# EUROPEAN PATENT APPLICATION

(11) **EP 1 964 850 A2**
(43) Date of publication of application: **03.09.2008**
(21) Application number: 08075288.4
(22) Date of filing: 09.04.1998
(51) Int. Cl.: C07K 14/47, C07K 14/82, C07K 14/15, C12Q 1/68, G01N 33/574, A61K 38/17, A61K 39/00

(54) **Compositions and methods for the treatment and diagnosis of breast cancer**

(30) Priority: 09.04.1997 US 838762; 11.12.1997 US 991789
(62) Divisional of application: 98915351.5
(71) Applicant: CORIXA CORPORATION, Wilmington, DE 19808 (US)
(72) Inventor: Frudakis, Tony N., Seattle, WA 99232-0232 (US); Smith, John M., Everett, WA 98208 (US); Reed, Steven G,, Bellevue, WA 98005 (US)
(74) Representative: Thornton, Neil

(57) **Abstract**

Compositions and methods for the detection and therapy of breast cancer are disclosed. The compounds provided include nucleotide sequences that are preferentially expressed in breast tumor tissue, as well as polypeptides encoded by such nucleotide sequences. Vaccines and pharmaceutical compositions comprising such compounds are also provided and may be used, for example, for the prevention and treatment of breast cancer. The polypeptides may also be used for the production of antibodies, which are useful for diagnosing and monitoring the progression of breast cancer in a patient.

SEQUENCE LISTING

(1) GENERAL INFORMATION:

(i) APPLICANT: Corixa Corporation

(ii) TITLE OF INVENTION: COMPOSITIONS AND METHODS FOR THE
TREATMENT AND DIAGNOSIS OF BREAST CANCER

(iii) NUMBER OF SEQUENCES: 297

(iv) CORRESPONDENCE ADDRESS:
(A) ADDRESSEE: Reddie & Grose
(B) STREET: 16 Theobalds Road
(C) CITY: London
(D) COUNTRY: United Kingdom
(E) POSTAL CODE: WC1X 8PL

(v) COMPUTER READABLE FORM:
(A) MEDIUM TYPE: Disk
(B) COMPUTER: IBM PC compatible
(C) OPERATING SYSTEM: PC-DOS/MS-DOS
(D) SOFTWARE: PatentIn Release #1.0, Version #1.30

(vi) CURRENT APPLICATION DATA:
(A) APPLICATION NUMBER:
(B) FILING DATE: 09 April 1998
(C) CLASSIFICATION:

(viii) ATTORNEY/AGENT INFORMATION:
(A) NAME: Thornton, Neil
(B) REGISTRATION NUMBER:
(C) REFERENCE/DOCKET NUMBER: P/50169.EP02/NT

(2) INFORMATION FOR SEQ ID NO:1:

(i) SEQUENCE CHARACTERISTICS:
(A) LENGTH: 363 base pairs
(B) TYPE: nucleic acid
(C) STRANDEDNESS: single
(D) TOPOLOGY: linear

(ix) FEATURE:
(A) NAME/KEY: CDS
(B) LOCATION: 1..363

(xi) SEQUENCE DESCRIPTION: SEQ ID NO:1:
TTA GAG ACC CAA TTG GGA CCT AAT TGG GAC CCA AAT TTC TCA AGT GGA 48
GGG AGA ACT TTT GAC GAT TTC CAC CGG TAT CTC CTC GTG GGT ATT CAG 96
GGA GCT GCC CAG AAA CCT ATA AAC TTG TCT AAG GCG ATT GAA GTC GTC 144
CAG GGG CAT GAT GAG TCA CCA GGA GTG TTT TTA GAG CAC CTC CAG GAG 192
GCT TAT CGG ATT TAC ACC CCT TTT GAC CTG GCA GCC CCC GAA AAT AGC 240
CAT GCT CTT AAT TTG GCA TTT GTG GCT CAG GCA GCC CCA GAT AGT AAA 288
AGG AAA CTC CAA AAA CTA GAG GGA TTT TGC TGG AAT GAA TAC CAG TCA 336
GCT TTT AGA GAT AGC CTA AAA GGT TTT 363

(2) INFORMATION FOR SEQ ID NO:2:

(i) SEQUENCE CHARACTERISTICS:
(A) LENGTH: 121 amino acids
(B) TYPE: amino acid
(D) TOPOLOGY: linear

(ii) MOLECULE TYPE: protein

(xi) SEQUENCE DESCRIPTION: SEQ ID NO:2:

(2) INFORMATION FOR SEQ ID NO:3:

     (i) SEQUENCE CHARACTERISTICS:
          (A) LENGTH: 1101 base pairs
          (B) TYPE: nucleic acid
          (C) STRANDEDNESS: single
          (D) TOPOLOGY: linear


    (xi) SEQUENCE DESCRIPTION: SEQ ID NO:3:

TCTTAGAATC TTCATACCCC GAACTCTTGG GAAAACTTTA ATCAGTCACC TACAGTCTAC      60
CACCCATTTA GGAGGAGCAA AGCTACCTCA GCTCCTCCGG AGCCGTTTTA AGATCCCCCA     120
TCTTCAAAGC CTAACAGATC AAGCAGCTCT CCGGTGCACA ACCTGCGCCC AGGTAAATGC     180
CAAAAAAGGT CCTAAACCCA GCCCAGGCCA CCGTCTCCAA GAAAACTCAC CAGGAGAAAA     240
GTGGGAAATT GACTTTACAG AAGTAAAACC ACACCGGGCT GGGTACAAAT ACCTTCTAGT     300
ACTGGTAGAC ACCTTCTCTG GATGGACTGA AGCATTTGCT ACCAAAAACG AAACTGTCAA     360
TATGGTAGTT AAGTTTTTAC TCAATGAAAT CATCCCTCGA CGTGGGCTGC CTGTTGCCAT     420
AGGGTCTGAT AATGGAACGG CCTTCGCCTT GTCTATAGTT TAATCAGTCA GTAAGGCGTT     480
AAACATTCAA TGGAAGCTCC ATTGTGCCTA TCGACCCAGA GCTCTGGGCA AGTAGAACGC     540
ATGAACTGCA CCCTAAAAAA ACACTCTTAC AAAATTAATC TTAAAAACCG GTGTTAATTG     600
TGTTAGTCTC CTTCCCTTAG CCCTACTTAG AGTTAAGGTG CACCCCTTAC TGGGCTGGGT     660
TCTTTACCTT TTGAAATCAT NTTTNGGAAG GGGCTGCCTA TCTTTNCTTA ACTAAAAAAN     720
GCCCATTTGG CAAAAATTTC NCAACTAATT TNTACGTNCC TACGTCTCCC CAACAGGTAN     780
AAAAATCTNC TGCCCTTTTC AAGGAACCAT CCCATCCATT CCTNAACAAA AGGCCTGCCN     840
TTCTTCCCCC AGTTAACTNT TTTTTNTTAA AATTCCCAAA AAANGAACCN CCTGCTGGAA     900
AAACNCCCCC CTCCAANCCC CGGCCNAAGN GGAAGGTTCC CTTGAATCCC NCCCCCNCNA     960
ANGGCCCGGA ACCNTTAAAN TNGTTCCNGG GGGTNNGGCC TAAAAGNCCN ATTTGGTAAA    1020
CCTANAAATT TTTTCTTTTN TAAAAACCAC NNTTTNNTTT TTCTTAAACA AAACCCTNTT    1080
TNTAGNANCN TATTTCCCNC C                                              1101

(2) INFORMATION FOR SEQ ID NO:4:

     (i) SEQUENCE CHARACTERISTICS:
          (A) LENGTH: 1087 base pairs
          (B) TYPE: nucleic acid
          (C) STRANDEDNESS: single
          (D) TOPOLOGY: linear


    (xi) SEQUENCE DESCRIPTION: SEQ ID NO:4:

TCTAGAGCTG CGCCTGGATC CCGCCACAGT GAGGAGACCT GAAGACCAGA GAAAACACAG      60
CAAGTAGGCC CTTTAAACTA CTCACCTGTG TTGTCTTCTA ATTTATTCTG TTTTATTTTG     120
TTTCCATCAT TTTAAGGGGT TAAAATCATC TTGTTCAGAC CTCAGCATAT AAAATGACCC     180
ATCTGTAGAC CTCAGGCTCC AACCATACCC CAAGAGTTGT CTGGTTTTGT TTAAATTACT     240
GCCAGGTTTC AGCTGCAGAT ATCCCTGGAA GGAATATTCC AGATTCCCTG AGTAGTTTCC     300

AGGTTAAAAT CCTATAGGCT TCTTCTGTTT TGAGGAAGAG TTCCTGTCAG AGAAAAACAT 360
GATTTTGGAT TTTTAACTTT AATGCTTGTG AAACGCTATA AAAAAAATTT TCTACCCCTA 420
GCTTTAAAGT ACTGTTAGTG AGAAATTAAA ATTCCTTCAG GAGGATTAAA CTGCCATTTC 480
AGTTACCCTA ATTCCAAATG TTTTGGTGGT TAGAATCTTC TTTAATGTTC TTGAAGAAGT 540
GTTTTATATT TTCCCATCNA GATAAATTCT CTCNCNCCTT NNTTTTNTNT CTNNTTTTTT 600
AAAACGGANT CTTGCTCCGT TGTCCANGCT GGGAATTTTN TTTTGGCCAA TCTCCGCTNC 660
CTTGCAANAA TNCTGCNTCC CAAAATTACC NCCTTTTTCC CACCTCCACC CCNNGGAATT 720
ACCTGGAATT ANAGGCCCCC NCCCCCCCCC CGGCTAATTT GTTTTTGTTT TTAGTAAAAA 780
ACGGGTTTCC TGTTTTAGTT AGGATGGCCC ANNTCTGACC CCNTNATCNT CCCCCTCNGC 840
CCTCNAATNT TNGGNNTANG GCTTACCCCC CCCNGNNGTT TTTCCTCCAT TNAAATTTTC 900
TNTGGANTCT TGAATNNCGG GTTTTCCCTT TTAAACCNAT TTTTTTTTTN NNNCCCCCAN 960
TTTTNCCTCC CCCNTNTNTA ANGGGGGTTT CCCAANCCGG GTCCNCCCCC ANGTCCCCAA 1020
TTTTTCTCCC CCCCCCTCTT TTTTCTTTNC CCCAAAANTC CTATCTTTTC CTNNAAATAT 1080
CNANTNT 1087

(2) INFORMATION FOR SEQ ID NO:5:

(i) SEQUENCE CHARACTERISTICS:
(A) LENGTH: 1010 base pairs
(B) TYPE: nucleic acid
(C) STRANDEDNESS: single
(D) TOPOLOGY: linear

(xi) SEQUENCE DESCRIPTION: SEQ ID NO:5:

TCTAGACCAA GAAATGGGAG GATTTTAGAG TGACTGATGA TTTCTCTATC ATCTGCAGTT 60
AGTAAACATT CTCCACAGTT TATGCAAAAA GTAACAAAAC CACTGCAGAT GACAAACACT 120
AGGTAACACA CATACTATCT CCCAAATACC TACCCACAAG CTCAACAATT TTAAACTGTT 180
AGGATCACTG GCTCTAATCA CCATGACATG AGGTCACCAC CAAACCATCA AGCGCTAAAC 240
AGACAGAATG TTTCCACTCC TGATCCACTG TGTGGGAAGA AGCACCGAAC TTACCCACTG 300
GGGGGCCTGC NTCANAANAA AAGCCCATGC CCCCGGGTNT NCCTTTNAAC CGGAACGAAT 360
NAACCCACCA TCCCCACANC TCCTCTGTTC NTGGGCCCTG CATCTTGTGG CCTCNTNTNC 420
TTTNGGGGAN ACNTGGGGAA GGTACCCCAT TTCNTTGACC CCNCNANAAA ACCCCNGTGG 480
CCCTTTGCCC TGATTCNCNT GGGCCTTTTC TCTTTTCCCT TTTGGGTTGT TTAAATTCCC 540
AATGTCCCCN GAACCCTCTC CNTNCTGCCC AAAACCTACC TAAATTNCTC NCTANGNNTT 600
TTCTTGGTGT TNCTTTTCAA AGGTNACCTT NCCTGTTCAN NCCCNACNAA AATTTNTTCC 660
NTATNNTGGN CCCNNAAAAA NNNATCNNCC CNAATTGCCC GAATTGGTTN GGTTTTTCCT 720
NCTGGGGGAA ACCCTTTAAA TTTCCCCCTT GGCCGGCCCC CCTTTTTTCC CCCCTTTNGA 780
AGGCAGGNGG TTCTTCCCGA ACTTCCAATT NCAACAGCCN TGCCCATTGN TGAAACCCTT 840
TTCCTAAAAT TAAAAAATAN CCGGTTNNGG NNGGCCTCTT TCCCCTCCNG GNGGGNNGNG 900
AAANTCCTTA CCCCNAAAAA GGTTGCTTAG CCCCCNGTCC CCACTCCCCC NGGAAAAATN 960
AACCTTTTCN AAAAAAGGAA TATAANTTTN CCACTCCTTN GTTCTCTTCC 1010

(2) INFORMATION FOR SEQ ID NO:6:

(i) SEQUENCE CHARACTERISTICS:
(A) LENGTH: 950 base pairs
(B) TYPE: nucleic acid
(C) STRANDEDNESS: single
(D) TOPOLOGY: linear

(xi) SEQUENCE DESCRIPTION: SEQ ID NO:6:

TCTAGAGCTC GCGGCCGCGA GCTCTAATAC GACTCACTAT AGGGCGTCGA CTCGATCTCA 60
GCTCACTGCA ATCTCTGCCC CCGGGGTCAT GCGATTCTCC TGCCTCAGCC TTCCAAGTAG 120
CTGGGATTAC AGGCGTGCAA CACCACACCC GGCTAATTTT GTATTTTTAA TAGAGATGGG 180
GTTTTCCCTT GTTGGCCANN ATGGTCTCNA ACCCCTGACC TCNNGTGATC CCCCCNCCCN 240
NGANCTCNNA CTGCTGGGGA TNNCCGNNNN NNNCCTCCCN NCNCNNNNNN NCNCNNTCCN 300
TNNTCCTTNC TCNNNNNNNN CNNTCNNTCC NNCTTCTCNC CNNNTNTTNT CNNCNNCCNN 360
CNNNCCNCNT NCCCNCNNNT TCNCNTNCNN TNTCCNNCNN NNTCNNCNNN CNNNNCNTNN 420
CCNNTACNTC NTNNNCNNNT CCNTCTNTNN CCTCNNCNNT CNCTNCNCNT TNTCTCCTCN 480
NTNNNNNNCT CCNNNNNTCT CNTCNCNNCN TNCCTCNNTN NCCNCNCCCC NCCTCNCNNC 540
CTNNTTTNNN CNNCNNNTCC NTNCCNTTCN NNTCCNNTNN CNNCNTCNCN NNCNTTNTTC 600
CCNCCNNTTC CTTNCNCNTN NNNTNTCNNN CNCNTCNNTC NTTTNCTCCT NNNTCCCNNC 660
TCNNTTCNCC CNNNTCCNCC CCCCNCCTNT CTCTCNCCCN NNTNNNTNTN NNNCNTCCNC 720
TNTCNCNTTC NTCNNTNCNT TNCTNTCNNC NNCNNTNCNC TNCCNTNTNT CTNNNTCNCN 780
TCNCNTNTCN CCNTCCNTTN CTNTCTCCTN TNTCCTTCCC CTCNCCTNCT CNTTCNCCNC 840
CCNNTNTNTN TNNCNCCNNT NCTNNNCNNC CNTCNTTTCN TCTCTNCTNN NNNTNNCCTC 900
NNCCCNTNCC CTNNTNCNCT NCTNNTACCN TNCTNCTCCN TCTTCCTTCC 950

(2) INFORMATION FOR SEQ ID NO:7:

(i) SEQUENCE CHARACTERISTICS:
(A) LENGTH: 1086 base pairs
(B) TYPE: nucleic acid
(C) STRANDEDNESS: single
(D) TOPOLOGY: linear

(xi) SEQUENCE DESCRIPTION: SEQ ID NO:7:

TCTAGAGCTC GCGGCCGCGA GCTCAATTAA CCCTCACTAA AGGGAGTCGA CTCGATCAGA 60
CTGTTACTGT GTCTATGTAG AAAGAAGTAG ACATAAGAGA TTCCATTTTG TTCTGTACTA 120
AGAAAAATTC TTCTGCCTTG AGATGCTGTT AATCTGTAAC CCTAGCCCCA ACCCTGTGCT 180
CACAGAGACA TGTGCTGTGT TGACTCAAGG TTCAATGGAT TTAGGGCTAT GCTTTGTTAA 240
AAAAGTGCTT GAAGATAATA TGCTTGTTAA AAGTCATCAC CATTCTCTAA TCTCAAGTAC 300
CCAGGGACAC AATACACTGC GGAAGGCCGC AGGGACCTCT GTCTAGGAAA GCCAGGTATT 360
GTCCAAGATT TCTCCCCATG TGATAGCCTG AGATATGGCC TCATGGGAAG GGTAAGACCT 420
GACTGTCCCC CAGCCCGACA TCCCCCAGCC CGACATCCCC CAGCCCGACA CCCGAAAAGG 480
GTCTGTGCTG AGGAAGATTA NTAAAAGAGG AAGGCTCTTT GCATTGAAGT AAGAAGAAGG 540
CTCTGTCTCC TGCTCGTCCC TGGGCAATAA AATGTCTTGG TGTTAAACCC GAATGTATGT 600
TCTACTTACT GAGAATAGGA GAAAACATCC TTAGGGCTGG AGGTGAGACA CCCTGGCGGC 660
ATACTGCTCT TTAATGCACG AGATGTTTGT NTAATTGCCA TCCAGGGCCA NCCCCTTTCC 720
TTAACTTTTT ATGANACAAA AACTTTGTTC NCTTTTCCTG CGAACCTCTC CCCCTATTAN 780
CCTATTGGCC TGCCCATCCC CTCCCCAAAN GGTGAAAANA TGTTCNTAAA TNCGAGGGAA 840
TCCAAAACNT TTTCCCGTTG GTCCCCTTTC CAACCCCGTC CCTGGGCCNN TTTCCTCCCC 900
AACNTGTCCC GGNTCCTTCN TTCCCNCCCC CTTCCCNGAN AAAAAACCCC GTNTGANGGN 960
GCCCCCTCAA ATTATAACCT TTCCNAAACA AANNGGTTCN AAGGTGGTTT GNTTCCGGTG 1020
CGGCTGGCCT TGAGGTCCCC CCTNCACCCC AATTTGGAAN CCNGTTTTTT TTATTGCCCN 1080
NTCCCC 1086

(2) INFORMATION FOR SEQ ID NO:8:

(i) SEQUENCE CHARACTERISTICS:
(A) LENGTH: 1177 base pairs
(B) TYPE: nucleic acid
(C) STRANDEDNESS: single
(D) TOPOLOGY: linear

(xi) SEQUENCE DESCRIPTION: SEQ ID NO:8:

NCCNTTTAGA TGTTGACAAN NTAAACAAGC NGCTCAGGCA GCTGAAAAAA GCCACTGATA 60
AAGCATCCTG GAGTATCAGA GTTTACTGTT AGATCAGCCT CATTTGACTT CCCCTCCCAC 120
ATGGTGTTTA AATCCAGCTA CACTACTTCC TGACTCAAAC TCCACTATTC CTGTTCATGA 180
CTGTCAGGAA CTGTTGGAAA CTACTGAAAC TGGCCGACCT GATCTTCAAA ATGTGCCCCT 240
AGGAAAGGTG GATGCCACCG TGTTCACAGA CAGTACCNCC TTCCTCGAGA AGGGACTACG 300
AGGGGCCGGT GCANCTGTTA CCAAGGAGAC TNATGTGTTG TGGGCTCAGG CTTTACCANC 360
AAACACCTCA NCNCNNAAGG CTGAATTGAT CGCCCTCACT CAGGCTCTCG GATGGGGTAA 420
GGGATATTAA CGTTAACACT GACAGCAGGT ACGCCTTTGC TACTGTGCAT GTACGTGGAG 480
CCATCTACCA GGAGCGTGGG CTACTCACTC GGCAGGTGGC TGTNATCCAC TGTAAANGGA 540
CATCAAAAGG AAAACNNGGC TGTTGCCCGT GGTAACCANA AANCTGATCN NCAGCTCNAA 600
GATGCTGTGT TGACTTTCAC TCNCNCCTCT TAAACTTGCT GCCCACANTC TCCTTTCCCA 660
ACCAGATCTG CCTGACAATC CCCATACTCA AAAAAAAAAN AANACTGGCC CCGAACCCNA 720
ACCAATAAAA ACGGGGANGG TNGGTNGANC NNCCTGACCC AAAAATAATG GATCCCCCGG 780
GCTGCAGGAA TTCAATTCAN CCTTATCNAT ACCCCCAACN NGGNGGGGGG GGCCNGTNCC 840
CATTNCCCCT NTATTNATTC TTTNNCCCCC CCCCCGGCNT CCTTTTTNAA CTCGTGAAAG 900
GGAAAACCTG NCTTACCAAN TTATCNCCTG GACCNTCCCC TTCCNCGGTN GNTTANAAAA 960
AAAAGCCCNC ANTCCCNTCC NAAATTTGCA CNGAAAGGNA AGGAATTTAA CCTTTATTTT 1020
TTNNTCCTTT ANTTTGTNNN CCCCCTTTTA CCCAGGCGAA CNGCCATCNT TTAANAAAAA 1080
AAANAGAANG TTTATTTTTC CTTNGAACCA TCCCAATANA AANCACCCGC NGGGGAACGG 1140
GGNGGNAGGC CNCTCACCCC CTTTNTGTNG GNGGGNC 1177

(2) INFORMATION FOR SEQ ID NO:9:

(i) SEQUENCE CHARACTERISTICS:
(A) LENGTH: 1146 base pairs
(B) TYPE: nucleic acid
(C) STRANDEDNESS: single
(D) TOPOLOGY: linear

(xi) SEQUENCE DESCRIPTION: SEQ ID NO:9:

NCCNNTTNNT GATGTTGTCT TTTTGGCCTC TCTTTGGATA CTTTCCCTCT CTTCAGAGGT 60
GAAAAGGGTC AAAAGGAGCT GTTGACAGTC ATCCCAGGTG GGCCAATGTG TCCAGAGTAC 120
AGACTCCATC AGTGAGGTCA AAGCCTGGGG CTTTTCAGAG AAGGGAGGAT TATGGGTTTT 180
CCAATTATAC AAGTCAGAAG TAGAAAGAAG GGACATAAAC CAGGAAGGGG GTGGAGCACT 240
CATCACCCAG AGGGACTTGT GCCTCTCTCA GTGGTAGTAG AGGGGCTACT TCCTCCCACC 300
ACGGTTGCAA CCAAGAGGCA ATGGGTGATG AGCCTACAGG GGACATANCC GAGGAGACAT 360
GGGATGACCC TAAGGGAGTA GGCTGGTTTT AAGGCGGTGG GACTGGGTGA GGGAAACTCT 420
CCTCTTCTTC AGAGAGAAGC AGTACAGGGC GAGCTGAACC GGCTGAAGGT CGAGGCGAAA 480
ACACGGTCTG GCTCAGGAAG ACCTTGGAAG TAAAATTATG AATGGTGCAT GAATGGAGCC 540
ATGGAAGGGG TGCTCCTGAC CAAACTCAGC CATTGATCAA TGTTAGGGAA ACTGATCAGG 600
GAAGCCGGGA ATTTCATTAA CAACCCGCCA CACAGCTTGA ACATTGTGAG GTTCAGTGAC 660
CCTTCAAGGG GCCACTCCAC TCCAACTTTG GCCATTCTAC TTTGCNAAAT TTCCAAAACT 720
TCCTTTTTTA AGGCCGAATC CNTANTCCCT NAAAAACNAA AAAAAATCTG CNCCTATTCT 780
GGAAAAGGCC CANCCCTTAC CAGGCTGGAA GAAATTTTNC CTTTTTTTTT TTTTTGAAGG 840
CNTTTNTTAA ATTGAACCTN AATTCNCCCC CCCAAAAAAA AACCCNCCNG GGGGGCGGAT 900
TTCCAAAAAC NAATTCCCTT ACCAAAAAAC AAAAACCCNC CCTTNTTCCC TTCCNCCCTN 960
TTCTTTTAAT TAGGGAGAGA TNAAGCCCCC CAATTTCCNG GNCTNGATNN GTTTCCCCCC 1020
CCCCCATTTT CCNAAACTTT TTCCCANCNA GGAANCCNCC CTTTTTTTNG GTCNGATTNA 1080
NCAACCTTCC AAACCATTTT TCCNNAAAAA NTTTGNTNGG NGGGAAAAAN ACCTNNTTTT 1140

ATAGAN                                                              1146

(2) INFORMATION FOR SEQ ID NO:10:

     (i) SEQUENCE CHARACTERISTICS:
          (A) LENGTH: 545 base pairs
          (B) TYPE: nucleic acid
          (C) STRANDEDNESS: single
          (D) TOPOLOGY: linear

    (xi) SEQUENCE DESCRIPTION: SEQ ID NO:10:

CTTCATTGGG TACGGGCCCC CTCGAGGTCG ACGGTATCGA TAAGCTTGAT ATCGAATTCC      60
TGCAGCCCGG GGGATCCACT AGTTCTAGAG TCAGGAAGAA CCACCAACCT TCCTGATTTT     120
TATTGGCTCT GAGTTCTGAG GCCAGTTTTC TTCTTCTGTT GAGTATGCGG GATTGTCAGG     180
CAGATCTGGC TGTGGAAAGG AGACTGTGGG CAGCAAGTTT AGAGGCGTGA CTGAAAGTCA     240
CACTGCATCT TGAGCTGCTG AATCAGCTTT CTGGTTACCA CGGGCAACAG CCGTGTTTTC     300
CTTTTGATGT CCTTTACAGT GGATTACAGC CACCTGCTGA GGTGAGTAGC CCACGCTCCT     360
GGTAGATGGC TCCACGTACA TGCACAGTAG CAAAGGCGTA CCTGCTGTCA GTGTTAACGT     420
TAATATCCTT ACCCCATCGG AGAGCCTGAG TGAGGGCGAT CAATTCAGCC CTTTTGTGCT     480
GAGGTGTTTG CTGGTTAAGC CCTGAACCCA CAACACATCT GTCTCCATGG TAACAGCTGC     540
ACCGG                                                               545

(2) INFORMATION FOR SEQ ID NO:11:

     (i) SEQUENCE CHARACTERISTICS:
          (A) LENGTH: 196 base pairs
          (B) TYPE: nucleic acid
          (C) STRANDEDNESS: single
          (D) TOPOLOGY: linear

    (xi) SEQUENCE DESCRIPTION: SEQ ID NO:11:

TCTCCTAGGC TGGGCACAGT GGCTCATACC TGTAATCCTG ACCGTTTCAG AGGCTCAGGT      60
GGGGGGATCG CTTGAGCCCA AGATTTCAAG ACTAGTCTGG GTAACATAGT GAGACCCTAT     120
CTCTACGAAA AAATAAAAAA ATGAGCCTGG TGTAGTGGCA CACACCAGCT GAGGAGGGAG     180
AATCGAGCCT AGGAGA                                                   196

(2) INFORMATION FOR SEQ ID NO:12:

     (i) SEQUENCE CHARACTERISTICS:
          (A) LENGTH: 388 base pairs
          (B) TYPE: nucleic acid
          (C) STRANDEDNESS: single
          (D) TOPOLOGY: linear

    (xi) SEQUENCE DESCRIPTION: SEQ ID NO:12:

TCTCCTAGGC TTGGGGGCTC TGACTAGAAA TTCAAGGAAC CTGGGATTCA AGTCCAACTG      60
TGACACCAAC TTACACTGTG GNCTCCAATA AACTGCTTCT TTCCTATTCC CTCTCTATTA     120
AATAAAATAA GGAAAACGAT GTCTGTGTAT AGCCAAGTCA GNTATCCTAA AAGGAGATAC     180
TAAGTGACAT TAAATATCAG AATGTAAAAC CTGGGAACCA GGTTCCCAGC CTGGGATTAA     240

ACTGACAGCA AGAAGACTGA ACAGTACTAC TGTGAAAAGC CCGAAGNGGC AATATGTTCA 300
CTCTACCGTT GAAGGATGGC TGGGAGAATG AATGCTCTGT CCCCCAGTCC CAAGCTCACT 360
TACTATACCT CCTTTATAGC CTAGGAGA 388

(2) INFORMATION FOR SEQ ID NO:13:

(i) SEQUENCE CHARACTERISTICS:
(A) LENGTH: 337 base pairs
(B) TYPE: nucleic acid
(C) STRANDEDNESS: single
(D) TOPOLOGY: linear

(xi) SEQUENCE DESCRIPTION: SEQ ID NO:13:

TAGTAGTTGC CTATAATCAT GTTTCTCATT ATTTTCACAT TTTATTAACC AATTTCTGTT 60
TACCCTGAAA AATATGAGGG AAATATATGA AACAGGGAGG CAATGTTCAG ATAATTGATC 120
ACAAGATATG ATTTCTACAT CAGATGCTCT TTCCTTTCCT GTTTATTTCC TTTTTATTTC 180
GGTTGTGGGG TCGAATGTAA TAGCTTTGTT TCAAGAGAGA GTTTTGGCAG TTTCTGTAGC 240
TTCTGACACT GCTCATGTCT CCAGGCATCT ATTTGCACTT TAGGAGGTGT CGTGGGAGAC 300
TGAGAGGTCT ATTTTTTCCA TATTTGGGCA ACTACTA 337

(2) INFORMATION FOR SEQ ID NO:14:

(i) SEQUENCE CHARACTERISTICS:
(A) LENGTH: 571 base pairs
(B) TYPE: nucleic acid
(C) STRANDEDNESS: single
(D) TOPOLOGY: linear

(xi) SEQUENCE DESCRIPTION: SEQ ID NO:14:

TAGTAGTTGC CATACAGTGC CTTTCCATTT ATTTAACCCC CACCTGAACG GCATAAACTG 60
AGTGTTCAGC TGGTGTTTTT TACTGTAAAC AATAAGGAGA CTTTGCTCTT CATTTAAACC 120
AAAATCATAT TTCATATTTT ACGCTCGAGG GTTTTTACCG GTTCCTTTTT ACACTCCTTA 180
AAACAGTTTT TAAGTCGTTT GGAACAAGAT ATTTTTTCTT TCCTGGCAGC TTTTAACATT 240
ATAGCAAATT TGTGTCTGGG GGACTGCTGG TCACTGTTTC TCACAGTTGC AAATCAAGGC 300
ATTTGCAACC AAGAAAAAAA AATTTTTTTG TTTTATTTGA AACTGGACCG GATAAACGGT 360
GTTTGGAGCG GCTGCTGTAT ATAGTTTTAA ATGGTTTATT GCACCTCCTT AAGTTGCACT 420
TATGTGGGGG GGGGNTTTTG NATAGAAAGT NTTTANTCAC ANAGTCACAG GGACTTTTNT 480
CTTTTGGNNA CTGAGCTAAA AAGGGCTGNT TTTCGGGTGG GGGCAGATGA AGGCTCACAG 540
GAGGCCTTTC TCTTAGAGGG GGGAACTNCT A 571

(2) INFORMATION FOR SEQ ID NO:15:

(i) SEQUENCE CHARACTERISTICS:
(A) LENGTH: 548 base pairs
(B) TYPE: nucleic acid
(C) STRANDEDNESS: single
(D) TOPOLOGY: linear

(xi) SEQUENCE DESCRIPTION: SEQ ID NO:15:

TATATATTTA ATAACTTAAA TATATTTTGA TCACCCACTG GGGTGATAAG ACAATAGATA 60
TAAAAGTATT TCCAAAAAGC ATAAAACCAA AGTATCATAC CAAACCAAAT TCATACTGCT 120
TCCCCCACCC GCACTGAAAC TTCACCTTCT AACTGTCTAC CTAACCAAAT TCTACCCTTC 180
AAGTCTTTGG TGCGTGCTCA CTACTCTTTT TTTTTTTTTT TTTNTTTTGG AGATGGAGTC 240
TGGCTGTGCA GCCCAGGGGT GGAGTACAAT GGCACAACCT CAGCTCACTG NAACCTCCGC 300
CTCCCAGGTT CATGAGATTC TCCTGNTTCA GCCTTCCCAG TAGCTGGGAC TACAGGTGTG 360
CATCACCATG CCTGGNTAAT CTTTTTTNGT TTTNGGGTAG AGATGGGGGT TTTACATGTT 420
GGCCAGGNTG GTNTCGAACT CCTGACCTCA AGTGATCCAC CCACCTCAGG CTCCCAAAGT 480
GCTAGGATTA CAGACATGAG CCACTGNGCC CAGNCCTGGT GCATGCTCAC TTCTCTAGGC 540
AACTACTA 548

(2) INFORMATION FOR SEQ ID NO:16:

(i) SEQUENCE CHARACTERISTICS:
(A) LENGTH: 638 base pairs
(B) TYPE: nucleic acid
(C) STRANDEDNESS: single
(D) TOPOLOGY: linear

(xi) SEQUENCE DESCRIPTION: SEQ ID NO:16:

TTCCGTTATG CACATGCAGA ATATTCTATC GGTACTTCAG CTATTACTCA TTTTGATGGC 60
GCAATCCGAG CCTATCCTCA AGATGAGTAT TTAGAAAGAA TTGATTTAGC GATAGACCAA 120
GCTGGTAAGC ACTCTGACTA CACGAAATTG TTCAGATGTG ATGGATTTAT GACAGTTGAT 180
CTTTGGAAGA GATTATTAAG TGATTATTTT AAAGGGAATC CATTAATTCC AGAATATCTT 240
GGTTTAGCTC AAGATGATAT AGAAATAGAA CAGAAAGAGA CTACAAATGA AGATGTATCA 300
CCAACTGATA TTGAAGAGCC TATAGTAGAA AATGAATTAG CTGCATTTAT TAGCCTTACA 360
CATAGCGATT TTCCTGATGA ATCTTATATT CAGCCATCGA CATAGCATTA CCTGATGGGC 420
AACCTTACGA ATAATAGAAA CTGGGTGCGG GGCTATTGAT GAATTCATCC NCAGTAAATT 480
TGGATATNAC AAAATATAAC TCGATTGCAT TTGGATGATG GAATACTAAA TCTGGCAAAA 540
GTAACTTTGG AGCTACTAGT AACCTCTCTT TTTGAGATGC AAAATTTTCT TTTAGGGTTT 600
CTTATTCTCT ACTTTACGGA TATTGGAGCA TAACGGGA 638

(2) INFORMATION FOR SEQ ID NO:17:

(i) SEQUENCE CHARACTERISTICS:
(A) LENGTH: 286 base pairs
(B) TYPE: nucleic acid
(C) STRANDEDNESS: single
(D) TOPOLOGY: linear

(xi) SEQUENCE DESCRIPTION: SEQ ID NO:17:

ACTGATGGAT GTCGCCGGAG GCGAGGGGCC TTATCTGATG CTCGGCTGCC TGTTCGTGAT 60
GTGCGCGGCG ATTGGGCTGT TTATCTCAAA CACCGCCACG GCGGTGCTGA TGGCGCCTAT 120
TGCCTTAGCG GCGGCGAAGT CAATGGGCGT CTCACCCTAT CCTTTTGCCA TGGTGGTGGC 180
GATGGCGGCT TCGGCGGCGT TTATGACCCC GGTCTCCTCG CCGGTTAACA CCCTGGTGCT 240
TGGCCCTGGC AAGTACTCAT TTAGCGATTT TGTCAAAATA GGCGTG 286

(2) INFORMATION FOR SEQ ID NO:18:

(i) SEQUENCE CHARACTERISTICS:
(A) LENGTH: 262 base pairs

(B) TYPE: nucleic acid
(C) STRANDEDNESS: single
(D) TOPOLOGY: linear

(xi) SEQUENCE DESCRIPTION: SEQ ID NO:18:

TCGGTCATAG CAGCCCCTTC TTCTCAATTT CATCTGTCAC TACCCTGGTG TAGTATCTCA 60
TAGCCTTACA TTTTTATAGC CTCCTCCCTG GTCTGTCTTT TGATTTTCCT GCCTGTAATC 120
CATATCACAC ATAACTGCAA GTAAACATTT CTAAAGTGTG GTTATGCTCA TGTCACTCCT 180
GTGNCAAGAA ATAGTTTCCA TTACCGTCTT AATAAAATTC GGATTTGTTC TTTNCTATTN 240
TCACTCTTCA CCTATGACCG AA 262

(2) INFORMATION FOR SEQ ID NO:19:

(i) SEQUENCE CHARACTERISTICS:
(A) LENGTH: 261 base pairs
(B) TYPE: nucleic acid
(C) STRANDEDNESS: single
(D) TOPOLOGY: linear

(xi) SEQUENCE DESCRIPTION: SEQ ID NO:19:

TCGGTCATAG CAAAGCCAGT GGTTTGAGCT CTCTACTGTG TAAACTCCTA AACCAAGGCC 60
ATTTATGATA AATGGTGGCA GGATTTTTAT TATAAACATG TACCCATGCA AATTTCCTAT 120
AACTCTGAGA TATATTCTTC TACATTTAAA CAATAAAAAT AATCTATTTT TAAAAGCCTA 180
ATTTGCGTAG TTAGGTAAGA GTGTTTAATG AGAGGGTATA AGGTATAAAT CACCAGTCAA 240
CGTTTCTCTG CCTATGACCG A 261

(2) INFORMATION FOR SEQ ID NO:20:

(i) SEQUENCE CHARACTERISTICS:
(A) LENGTH: 294 base pairs
(B) TYPE: nucleic acid
(C) STRANDEDNESS: single
(D) TOPOLOGY: linear

(xi) SEQUENCE DESCRIPTION: SEQ ID NO:20:

TACAACGAGG CGACGTCGGT AAAATCGGAC ATGAAGCCAC CGCTGGTCTT TTCGTCCGAG 60
CGATAGGCGC CGGCCAGCCA GCGGAACGGT TGCCCGGATG GCGAAGCGAG CCGGAGTTCT 120
TCGGACTGAG TATGAATCTT GTTGTGAAAA TACTCGCCGC CTTCGTTCGA CGACGTCGCG 180
TCGAAATCTT CGANCTCCTT ACGATCGAAG TCTTCGTGGG CGACGATCGC GGTCAGTTCC 240
GCCCCACCGA AATCATGGTT GAGCCGGATG CTGNCCCCGA AGNCCTCGTT TGTN 294

(2) INFORMATION FOR SEQ ID NO:21:

(i) SEQUENCE CHARACTERISTICS:
(A) LENGTH: 208 base pairs
(B) TYPE: nucleic acid
(C) STRANDEDNESS: single
(D) TOPOLOGY: linear

(xi) SEQUENCE DESCRIPTION: SEQ ID NO:21:

TTGGTAAAGG GCATGGACGC AGACGCCTGA CGTTTGGCTG AAAATCTTTC ATTGATTCGT 60
ATCAATGAAT AGGAAAATTC CCAAAGAGGG AATGTCCTGT TGCTCGCCAG TTTTTNTGTT 120
GTTCTCATGG ANAAGGCAAN GAGCTCTTCA GACTATTGGN ATTNTCGTTC GGTCTTCTGC 180
CAACTAGTCG NCTTGCNANG ATCTTCAT 208

(2) INFORMATION FOR SEQ ID NO:22:

(i) SEQUENCE CHARACTERISTICS:
(A) LENGTH: 287 base pairs
(B) TYPE: nucleic acid
(C) STRANDEDNESS: single
(D) TOPOLOGY: linear

(xi) SEQUENCE DESCRIPTION: SEQ ID NO:22:

NCCNTTGAGC TGAGTGATTG AGATNTGTAA TGGTTGTAAG GGTGATTCAG GCGGATTAGG 60
GTGGCGGGTC ACCCGGCAGT GGGTCTCCCG ACAGGCCAGC AGGATTTGGG GCAGGTACGG 120
NGTGCGCATC GCTCGACTAT ATGCTATGGC AGGCGAGCCG TGGAAGGNGG ATCAGGTCAC 180
GGCGCTGGAG CTTTCCACGG TCCATGNATT GNGATGGCTG TTCTAGGCGG CTGTTGCCAA 240
GCGTGATGGT ACGCTGGCTG GAGCATTGAT TTCTGGTGCC AAGGTGG 287

(2) INFORMATION FOR SEQ ID NO:23:

(i) SEQUENCE CHARACTERISTICS:
(A) LENGTH: 204 base pairs
(B) TYPE: nucleic acid
(C) STRANDEDNESS: single
(D) TOPOLOGY: linear

(xi) SEQUENCE DESCRIPTION: SEQ ID NO:23:

TTGGGTAAAG GGAGCAAGGA GAAGGCATGG AGAGGCTCAN GCTGGTCCTG GCCTACGACT 60
GGGCCAAGCT GTCGCCGGGG ATGGTGGAGA ACTGAAGCGG GACCTCCTCG AGGTCCTCCG 120
NCGTTACTTC NCCGTCCAGG AGGAGGGTCT TTCCGTGGTC TNGGAGGAGC GGGGGGAGAA 180
GATNCTCCTC ATGGTCNACA TCCC 204

(2) INFORMATION FOR SEQ ID NO:24:

(i) SEQUENCE CHARACTERISTICS:
(A) LENGTH: 264 base pairs
(B) TYPE: nucleic acid
(C) STRANDEDNESS: single
(D) TOPOLOGY: linear

(xi) SEQUENCE DESCRIPTION: SEQ ID NO:24:

TGGATTGGTC AGGAGCGGGT AGAGTGGCAC CATTGAGGGG ATATTCAAAA ATATTATTTT 60
GTCCTAAATG ATAGTTGCTG AGTTTTTCTT TGACCCATGA GTTATATTGG AGTTTATTTT 120
TTAACTTTCC AATCGCATGG ACATGTTAGA CTTATTTTCT GTTAATGATT NCTATTTTTA 180

TTAAATTGGA TTTGAGAAAT TGGTTNTTAT TATATCAATT TTTGGTATTT GTTGAGTTTG 240
ACATTATAGC TTAGTATGTG ACCA 264

(2) INFORMATION FOR SEQ ID NO:25:

(i) SEQUENCE CHARACTERISTICS:
(A) LENGTH: 376 base pairs
(B) TYPE: nucleic acid
(C) STRANDEDNESS: single
(D) TOPOLOGY: linear

(xi) SEQUENCE DESCRIPTION: SEQ ID NO:25:

TTACAACGAG GGGAAACTCC GTCTCTACAA AAATTAAAAA ATTAGCCAGG TGTGGTGGTG 60
TGCACCCGCA ATCCCAGCTA CTTGGGAGGT TGAGACACAA GANTCACCTA NATGTGGGAG 120
GTCAAGGTTG CATGAGTCAT GATTGTGCCA CTGCACTCCA GCCTGGGTGA CAGACCGAGA 180
CCCTGCCTCA ANAGANAANG AATAGGAAGT TCAGAAATCN TGGNTGTGGN GCCCAGCAAT 240
CTGCATCTAT NCAACCCCTG CAGGCAANGC TGATGCAGCC TANGTTCAAG AGCTGCTGTT 300
TCTGGAGGCA GCAGTTNGGG CTTCCATCCA GTATCACGGC CACACTCGCA CNAGCCATCT 360
GTCCTCCGTN TGTNAC 376

(2) INFORMATION FOR SEQ ID NO:26:

(i) SEQUENCE CHARACTERISTICS:
(A) LENGTH: 372 base pairs
(B) TYPE: nucleic acid
(C) STRANDEDNESS: single
(D) TOPOLOGY: linear

(xi) SEQUENCE DESCRIPTION: SEQ ID NO:26:

TTACAACGAG GGGAAACTCC GTCTCTACAA AAATTAAAAA ATTAGCCAGG TGTGGTGGTG 60
TGCACCTGTA ATCCCAGCTA CTTGGGCGGC TGAGACACAA GAACCACCTA AATGTGGGAG 120
GGTCAAGGTT GCATGAGTCA TGATCGCGCC ACTGCACTCC AGCCTGGGTG ACAGACTGAG 180
ACCCTGCCTC AAAAGAAAAA GAATAGGAAG TTCAGAAACC CTGGGTGTGG NGCCCAGCAA 240
TCTGCATTTA AACAATCCCT GCAGGCAATG CTGATGCAGC CTAAGTTCAA GAGCTGCTGT 300
TCTGGAGGCA GNAGTAAGGG CTTCCATCCA GCATCACGGN CAACACTGCA AAAGCACCTG 360
TCCTCGTTGG TA 372

(2) INFORMATION FOR SEQ ID NO:27:

(i) SEQUENCE CHARACTERISTICS:
(A) LENGTH: 477 base pairs
(B) TYPE: nucleic acid
(C) STRANDEDNESS: single
(D) TOPOLOGY: linear

(xi) SEQUENCE DESCRIPTION: SEQ ID NO:27:

TTCTGTCCAC ATCTACAAGT TTTATTTATT TTGTGGGTTT TCAGGGTGAC TAAGTTTTTC 60
CCTACATTGA AAAGAGAAGT TGCTAAAAGG TGCACAGGAA ATCATTTTTT TAAGTGAATA 120
TGATAATATG GGTCCGTGCT TAATACAACT GAGACATATT TGTTCTCTGT TTTTTTAGAG 180

TCACCTCTTA AAGTCCAATC CCACAATGGT GAAAAAAAAA TAGAAAGTAT TTGTTCTACC 240
TTTAAGGAGA CTGCAGGGAT TCTCCTTGAA AACGGAGTAT GGAATCAATC TTAAATAAAT 300
ATGAAATTGG TTGGTCTTCT GGGATAAGAA ATTCCCAACT CAGTGTGCTG AAATTCACCT 360
GACTTTTTTT GGGAAAAAAT AGTCGAAAAT GTCAATTTGG TCCATAAAAT ACATGTTACT 420
ATTAAAAGAT ATTTAAAGAC AAATTCTTTC AGAGCTCTAA GATTGGTGTG GACAGAA 477

(2) INFORMATION FOR SEQ ID NO:28:

(i) SEQUENCE CHARACTERISTICS:
(A) LENGTH: 438 base pairs
(B) TYPE: nucleic acid
(C) STRANDEDNESS: single
(D) TOPOLOGY: linear

(xi) SEQUENCE DESCRIPTION: SEQ ID NO:28:

TCTNCAACCT CTTGANTGTC AAAAACCTTN TAGGCTATCT CTAAAAGCTG ACTGGTATTC 60
ATTCCAGCAA AATCCCTCTA GTTTTTGGAG TTTCCTTTTA CTATCTGGGG CTGCCTGAGC 120
CACAAATGCC AAATTAAGAG CATGGCTATT TTCGGGGGCT GACAGGTCAA AAGGGGTGTA 180
AATCCGATAA GCCTCCTGGA GGTGCTCTAA AAACACTCCT GGTGACTCAT CATGCCCCTG 240
GACGACTTCA ATCGNCTTAG ACAAGTTTAT AGGTTTCTGG GCAGCTCCCT GAATACCCAC 300
GAGGAGATAC CGGTGGAAAT CGTCAAAAGT TCTCCCTCCA CTTGAGAAAT TTGGGTCCCA 360
ATTAGGTCCC AATTGGGTCT CTAATCACTA TTCCTCTAGC TTCCTCCTCC GGNCTATTGG 420
TTGATGTGAG GTTGAAGA 438

(2) INFORMATION FOR SEQ ID NO:29:

(i) SEQUENCE CHARACTERISTICS:
(A) LENGTH: 620 base pairs
(B) TYPE: nucleic acid
(C) STRANDEDNESS: single
(D) TOPOLOGY: linear

(xi) SEQUENCE DESCRIPTION: SEQ ID NO:29:

AAGAGGGTAC CAGCCCCAAG CCTTGACAAC TTCCATAGGG TGTCAAGCCT GTGGGTGCAC 60
AGAAGTCAAA AATTGAGTTT TGGGATCCTC AGCCTAGATT TCAGAGGATA TAAAGAAACA 120
CCTAACACCT AGATATTCAG ACAAAAGTTT ACTACAGGGA TGAAGCTTTC ACGGAAAACC 180
TCTACTAGGA AAGTACAGAA GAGAAATGTG GGTTTGGAGC CCCCAAACAG AATCCCCTCT 240
AGAACACTGC CTAATGAAAC TGTGAGAAGA TGGCCACTGT CATCCAGACA CCAGAATGAT 300
AGACCCACCA AAAACTTATG CCATATTGCC TATAAAACCT ACAGACACTC AATGCCAGCC 360
CCATGAAAAA AAAACTGAGA AGAAGACTGT NCCCTACAAT GCCACCGGAG CAGAACTGCC 420
CCAGGCCATG GAAGCACAGC TCTTATATCA ATGTGACCTG GATGTTGAGA CATGGAATCC 480
NANGAAATCN TTTTAANACT TCCACGGTTN AATGACTGCC CTATTANATT CNGAACTTAN 540
ATCCNGGCCT GTGACCTCTT TGCTTTGGCC ATTCCCCCTT TTTGGAATGG CTNTTTTTTT 600
CCCATGCCTG TNCCCTCTTA 620

(2) INFORMATION FOR SEQ ID NO:30:

(i) SEQUENCE CHARACTERISTICS:
(A) LENGTH: 100 base pairs
(B) TYPE: nucleic acid
(C) STRANDEDNESS: single

(D) TOPOLOGY: linear

(xi) SEQUENCE DESCRIPTION: SEQ ID NO:30:

TTACAACGAG GGGGTCAATG TCATAAATGT CACAATAAAA CAATCTCTTC TTTTTTTTTT 60
TTTTTTTTTT TTTTTTTTTT TTTTTTTTTT TTTTTTTTTT 100

(2) INFORMATION FOR SEQ ID NO:31:

(i) SEQUENCE CHARACTERISTICS:
(A) LENGTH: 762 base pairs
(B) TYPE: nucleic acid
(C) STRANDEDNESS: single
(D) TOPOLOGY: linear

(xi) SEQUENCE DESCRIPTION: SEQ ID NO:31:

TAGTCTATGC GCCGGACAGA GCAGAATTAA ATTGGAAGTT GCCCTCCGGA CTTTCTACCC 60
ACACTCTTCC TGAAAAGAGA AAGAAAAGAG GCAGGAAAGA GGTTAGGATT TCATTTTCAA 120
GAGTCAGCTA ATTAGGAGAG CAGAGTTTAG ACAGCAGTAG GCACCCCATG ATACAAACCA 180
TGGACAAAGT CCCTGTTTAG TAACTGCCAG ACATGATCCT GCTCAGGTTT TGAAATCTCT 240
CTGCCCATAA AAGATGGAGA GCAGGAGTGC CATCCACATC AACACGTGTC CAAGAAAGAG 300
TCTCAGGGAG ACAAGGGTAT CAAAAAACAA GATTCTTAAT GGGAAGGAAA TCAAACCAAA 360
AAATTAGATT TTTCTCTACA TATATATAAT ATACAGATAT TTAACACATT ATTCCAGAGG 420
TGGCTCCAGT CCTTGGGGCT TGAGAGATGG TGAAAACTTT TGTTCCACAT TAACTTCTGC 480
TCTCAAATTC TGAAGTATAT CAGAATGGGA CAGGCAATGT TTTGCTCCAC ACTGGGGCAC 540
AGACCCAAAT GGTTCTGTGC CCGAAGAAGA GAAGCCCGAA AGACATGAAG GATGCTTAAG 600
GGGGGTTGGG AAAGCCAAAT TGGTANTATC TTTTCCTCCT GCCTGTGTTC CNGAAGTCTC 660
CNCTGAAGGA ATTCTTAAAA CCCTTTGTGA GGAAATGCCC CCTTACCATG ACAANTGGTC 720
CCATTGCTTT TAGGGNGATG GAAACACCAA GGGTTTTGAT CC 762

(2) INFORMATION FOR SEQ ID NO:32:

(i) SEQUENCE CHARACTERISTICS:
(A) LENGTH: 276 base pairs
(B) TYPE: nucleic acid
(C) STRANDEDNESS: single
(D) TOPOLOGY: linear

(xi) SEQUENCE DESCRIPTION: SEQ ID NO:32:

TAGTCTATGC GTGTATTAAC CTCCCCTCCC TCAGTAACAA CCAAAGAGGC AGGAGCTGTT 60
ATTACCAACC CCATTTTACA GATGCATCAA TAATGACAGA GAAGTGAAGT GACTTGCGCA 120
CACAACCAGT AAATTGGCAG AGTCAGATTT GAATCCATGG AGTCTGGTCT GCACTTTCAA 180
TCACCGAATA CCCTTTCTAA GAAACGTGTG CTGAATGAGT GCATGGATAA ATCAGTGTCT 240
ACTCAACATC TTTGCCTAGA TATCCCGCAT AGACTA 276

(2) INFORMATION FOR SEQ ID NO:33:

(i) SEQUENCE CHARACTERISTICS:
(A) LENGTH: 477 base pairs
(B) TYPE: nucleic acid

(C) STRANDEDNESS: single
(D) TOPOLOGY: linear

(xi) SEQUENCE DESCRIPTION: SEQ ID NO:33:

TAGTAGTTGC CAAATATTTG AAAATTTACC CAGAAGTGAT TGAAAACTTT TTGGAAACAA 60
AAACAAATAA AGCCAAAAGG TAAAATAAAA ATATCTTTGC ACTCTCGTTA TTACCTATCC 120
ATAACTTTTT CACCGTAAGC TCTCCTGCTT GTTAGTGTAG TGTGGTTATA TTAAACTTTT 180
TAGTTATTAT TTTTTATTCA CTTTTCCACT AGAAAGTCAT TATTGATTTA GCACACATGT 240
TGATCTCATT TCATTTTTTC TTTTTATAGG CAAAATTTGA TGCTATGCAA CAAAAATACT 300
CAAGCCCATT ATCTTTTTTC CCCCCGAAAT CTGAAAATTG CAGGGGACAG AGGGAAGTTA 360
TCCCATTAAA AAATTGTAAA TATGTTCAGT TTATGTTTAA AAATGCACAA AACATAAGAA 420
AATTGTGTTT ACTTGAGCTG CTGATTGTAA GCAGTTTTAT CTCAGGGGCA ACTACTA 477

(2) INFORMATION FOR SEQ ID NO:34:

(i) SEQUENCE CHARACTERISTICS:
(A) LENGTH: 631 base pairs
(B) TYPE: nucleic acid
(C) STRANDEDNESS: single
(D) TOPOLOGY: linear

(xi) SEQUENCE DESCRIPTION: SEQ ID NO:34:

TAGTAGTTGC CAATTCAGAT GATCAGAAAT GCTGCTTTCC TCAGCATTGT CTTGTTAAAC 60
CGCATGCCAT TTGGAACTTT GGCAGTGAGA AGCCAAAAGG AAGAGGTGAA TGACATATAT 120
ATATATATAT ATTCAATGAA AGTAAAATGT ATATGCTCAT ATACTTTCTA GTTATCAGAA 180
TGAGTTAAGC TTTATGCCAT TGGGCTGCTG CATATTTTAA TCAGAAGATA AAAGAAAATC 240
TGGGCATTTT TAGAATGTGA TACATGTTTT TTTAAAACTG TTAAATATTA TTTCGATATT 300
TGTCTAAGAA CCGGAATGTT CTTAAAATTT ACTAAAACAG TATTGTTTGA GGAAGAGAAA 360
ACTGTACTGT TTGCCATTAT TACAGTCGTA CAAGTGCATG TCAAGTCACC CACTCTCTCA 420
GGCATCAGTA TCCACCTCAT AGCTTTACAC ATTTTGACGG GGAATATTGC AGCATCCTCA 480
GGCCTGACAT CTGGGAAAGG CTCAGATCCA CCTACTGCTC CTTGCTCGTT GATTTGTTTT 540
AAAATATTGT GCCTGGTGTC ACTTTTAAGC CACAGCCCTG CCTAAAAGCC AGCAGAGAAC 600
AGAACCCGCA CCATTCTATA GGCAACTACT A 631

(2) INFORMATION FOR SEQ ID NO:35:

(i) SEQUENCE CHARACTERISTICS:
(A) LENGTH: 578 base pairs
(B) TYPE: nucleic acid
(C) STRANDEDNESS: single
(D) TOPOLOGY: linear

(xi) SEQUENCE DESCRIPTION: SEQ ID NO:35:

TAGTAGTTGC CATCCCATAT TACAGAAGGC TCTGTATACA TGACTTATTT GGAAGTGATC 60
TGTTTTCTCT CCAAACCCAT TTATCGTAAT TTCACCAGTC TTGGATCAAT CTTGGTTTCC 120
ACTGATACCA TGAAACCTAC TTGGAGCAGA CATTGCACAG TTTTCTGTGG TAAAAACTAA 180
AGGTTTATTT GCTAAGCTGT CATCTTATGC TTAGTATTTT TTTTTTACAG TGGGGAATTG 240
CTGAGATTAC ATTTTGTTAT TCATTAGATA CTTTGGGATA ACTTGACACT GTCTTCTTTT 300
TTTCGCTTTT AATTGCTATC ATCATGCTTT TGAAACAAGA ACACATTAGT CCTCAAGTAT 360

TACATAAGCT TGCTTGTTAC GCCTGGTGGT TTAAAGGACT ATCTTTGGCC TCAGGTTCAC 420
AAGAATGGGC AAAGTGTTTC CTTATGTTCT GTAGTTCTCA ATAAAAGATT GCCAGGGGCC 480
GGGTACTGTG GCTCGCACTG TAATCCCAGC ACTTTGGGAA GCTGAGGCTG GCGGATCATG 540
TTAGGGCAGG TGTTCGAAAC CAGCCTGGGC AACTACTA 578

(2) INFORMATION FOR SEQ ID NO:36:

(i) SEQUENCE CHARACTERISTICS:
(A) LENGTH: 583 base pairs
(B) TYPE: nucleic acid
(C) STRANDEDNESS: single
(D) TOPOLOGY: linear

(xi) SEQUENCE DESCRIPTION: SEQ ID NO:36:

TAGTAGTTGC CTGTAATCCC AGCAACTCAG GAGGCTGGGG CAGGAGAATC AGTTGAACCT 60
GGGAGGCAGA AGTTGTAATT AGCAAAGATC GCACCATTGC ACTTCAGCCT GGGCAACAAG 120
AGTGAGATTC CATCTCAAAA ACAAAAAAAA GAAAAAGAAA AGAAAAGGAA AAAACGTATA 180
AACCCAGCCA AAACAAAATG ATCATTCTTT TAATAAGCAA GACTAATTTA ATGTGTTTAT 240
TTAATCAAAG CAGTTGAATC TTCTGAGTTA TTGGTGAAAA TACCCATGTA GTTAATTTAG 300
GGTTCTTACT TGGGTGAACG TTTGATGTTC ACAGGTTATA AAATGGTTAA CAAGGAAAAT 360
GATGCATAAA GAATCTTATA AACTACTAAA AATAAATAAA ATATAAATGG ATAGGTGCTA 420
TGGATGGAGT TTTTGTGTAA TTTAAAATCT TGAAGTCATT TTGGATGCTC ATTGGTTGTC 480
TGGTAATTTC CATTAGGAAA AGGTTATGAT ATGGGGAAAC TGTTTCTGGA AATTGCGGAA 540
TGTTTCTCAT CTGTAAAATG CTAGTATCTC AGGGCAACTA CTA 583

(2) INFORMATION FOR SEQ ID NO:37:

(i) SEQUENCE CHARACTERISTICS:
(A) LENGTH: 716 base pairs
(B) TYPE: nucleic acid
(C) STRANDEDNESS: single
(D) TOPOLOGY: linear

(xi) SEQUENCE DESCRIPTION: SEQ ID NO:37:

GATCTACTAG TCATNTGGAT TCTATCCATG GCAGCTAAGC CTTTCTGAAT GGATTCTACT 60
GCTTTCTTGT TCTTTAATCC AGACCCTTAT ATATGTTTAT GTTCACAGGC AGGGCAATGT 120
TTAGTGAAAA CAATTCTAAA TTTTTTATTT TGCATTTTCA TGCTAATTTC CGTCACACTC 180
CAGCAGGCTT CCTGGGAGAA TAAGGAGAAA TACAGCTAAA GACATTGTCC CTGCTTACTT 240
ACAGCCTAAT GGTATGCAAA ACCACTTCAA TAAAGTAACA GGAAAAGTAC TAACCAGGTA 300
GAATGGACCA AAACTGATAT AGAAAAATCA GAGGAAGAGA GGAACAAATA TTTACTGAGT 360
CCTAGAATGT ACAAGGCTTT TTAATTACAT ATTTTATGTA AGGCCTGCAA AAAACAGGTG 420
AGTAATCAAC ATTTGTCCCA TTTTACATAT AAGGAAACTG AAGCTTAAAT TGAATAATTT 480
AATGCATAGA TTTTATAGTT AGACCATGTT CAGGTCCCTA TGTTATACTT ACTAGCTGTA 540
TGAATATGAG AAAATAATTT TGTTATTTTC TTGGCATCAG TATTTTCATC TGCAAAATAA 600
AGCTAAAGTT ATTTAGCAAA CAGTCAGCAT AGTGCCTGAT ACATAGTAGG TGCTCCAAAC 660
ATGATTACNC TANTATTNGG TATTANAAAA ATCCAATATA GGCNTGGATA AAACCG 716

(2) INFORMATION FOR SEQ ID NO:38:

(i) SEQUENCE CHARACTERISTICS:
(A) LENGTH: 688 base pairs

(B) TYPE: nucleic acid
(C) STRANDEDNESS: single
(D) TOPOLOGY: linear

(xi) SEQUENCE DESCRIPTION: SEQ ID NO:38:

TTCTGTCCAC ATATCATCCC ACTTTAATTG TTAATCAGCA AAACTTTCAA TGAAAAATCA 60
TCCATTTTAA CCAGGATCAC ACCAGGAAAC TGAAGGTGTA TTTTTTTTTA CCTTAAAAAA 120
AAAAAAAAAA ACCAAACAAA CCAAAACAGA TTAACAGCAA AGAGTTCTAA AAAATTTACA 180
TTTCTCTTAC AACTGTCATT CAGAGAACAA TAGTTCTTAA GTCTGTTAAA TCTTGGCATT 240
AACAGAGAAA CTTGATGAAN AGTTGTACTT GGAATATTGT GGATTTTTTT TTTTGTCTAA 300
TCTCCCCCTA TTGTTTTGCC AACAGTAATT TAAGTTTGTG TGGAACATCC CCGTAGTTGA 360
AGTGTAAACA ATGTATAGGA AGGAATATAT GATAAGATGA TGCATCACAT ATGCATTACA 420
TGTAGGGACC TTCACAACTT CATGCACTCA GAAAACATGC TTGAAGAGGA GGAGAGGACG 480
GCCCAGGGTC ACCATCCAGG TGCCTTGAGG ACAGAGAATG CAGAAGTGGC ACTGTTGAAA 540
TTTAGAAGAC CATGTGTGAA TGGTTTCAGG CCTGGGATGT TTGCCACCAA GAAGTGCCTC 600
CGAGAAATTT CTTTCCCATT TGGAATACAG GGTGGCTTGA TGGGTACGGT GGGTGACCCA 660
ACGAAGAAAA TGAAATTCTG CCCTTTCC 688

(2) INFORMATION FOR SEQ ID NO:39:

(i) SEQUENCE CHARACTERISTICS:
(A) LENGTH: 585 base pairs
(B) TYPE: nucleic acid
(C) STRANDEDNESS: single
(D) TOPOLOGY: linear

(xi) SEQUENCE DESCRIPTION: SEQ ID NO:39:

TAGTAGTTGC CGCNNACCTA AAANTTGGAA AGCATGATGT CTAGGAAACA TANTAAAATA 60
GGGTATGCCT ATGTGCTACA GAGAGATGTT AGCATTTAAA GTGCATANTT TTATGTATTT 120
TGACAAATGC ATATNCCTCT ATAATCCACA ACTGATTACG AAGCTATTAC AATTAAAAAG 180
TTTGGCCGGG CGTGGTGGGC GGTGGCTGAC GCCTGTAATC CCAGCACTTT GGGAGGCCGA 240
GGCACGCGGA TCACGAGGTC GGGAGTTCAA GACCATCCTG GCTAACACGG TGAAAGTCCA 300
TCTCTACTAA AAATACGAAA AAATTACCCC GGCGTGGTGG CGGGCGCCTG TAGTCCCAGC 360
TACTCCGGAG GCTGAGGCAG GAGAATGGCG TGAACCCAGG ACACGGAGCT TGCAGTGTGC 420
CAACATCACG TCACTGCCCT CCAGCCTGGG GGACAGGAAC AAGANTCCCG TCCTCANAAA 480
AGAAAAATAC TACTNATANT TTCNACTTTA TTTTAANTTA CACAGAACTN CCTCTTGGTA 540
CCCCCTTACC ATTCATCTCA CCCACCTCCT ATAGGGCACN NCTAA 585

(2) INFORMATION FOR SEQ ID NO:40:

(i) SEQUENCE CHARACTERISTICS:
(A) LENGTH: 475 base pairs
(B) TYPE: nucleic acid
(C) STRANDEDNESS: single
(D) TOPOLOGY: linear

(xi) SEQUENCE DESCRIPTION: SEQ ID NO:40:

TCTGTCCACA CCAATCTTAG AAGCTCTGAA AAGAATTTGT CTTTAAATAT CTTTTAATAG 60
TAACATGTAT TTTATGGACC AAATTGACAT TTTCGACTGT TTTTTCCAAA AAAGTCAGGT 120

GAATTTCAGC ACACTGAGTT GGGAATTTCT TATCCCAGAA GACCAACCAA TTTCATATTT 180
ATTTAAGATT GATTCCATAC TCCGTTTTCA AGGAGAATCC CTGCAGTCTC CTTAAAGGTA 240
GAACAAATAC TTCCTATTTT TTTTTCACCA TTGTGGGATT GGACTTTAAG AGGTGACTCT 300
AAAAAAACAG AGAACAAATA TGTCTCAGTT GTATTAAGCA CGGACCCATA TTATCATATT 360
CACTTAAAAA AATGATTTCC TGTGCACCTT TTGGCAACTT CTCTTTTCAA TGTAGGGAAA 420
AACTTAGTCA CCCTGAAAAC CCACAAAATA AATAAAACTT GTAGATGTGG ACAGA 475

(2) INFORMATION FOR SEQ ID NO:41:

(i) SEQUENCE CHARACTERISTICS:
(A) LENGTH: 423 base pairs
(B) TYPE: nucleic acid
(C) STRANDEDNESS: single
(D) TOPOLOGY: linear

(xi) SEQUENCE DESCRIPTION: SEQ ID NO:41:

TAAGAGGGTA CATCGGGTAA GAACGTAGGC ACATCTAGAG CTTAGAGAAG TCTGGGGTAG 60
GAAAAAAATC TAAGTATTTA TAAGGGTATA GGTAACATTT AAAAGTAGGG CTAGCTGACA 120
TTATTTAGAA AGAACACATA CGGAGAGATA AGGGCAAAGG ACTAAGACCA GAGGAACACT 180
AATATTTAGT GATCACTTCC ATTCTTGGTA AAAATAGTAA CTTTTAAGTT AGCTTCAAGG 240
AAGATTTTTG GCCATGATTA GTTGTCAAAA GTTAGTTCTC TTGGGTTTAT ATTACTAATT 300
TTGTTTTAAG ATCCTTGTTA GTGCTTTAAT AAAGTCATGT TATATCAAAC GCTCTAAAAC 360
ATTGTAGCAT GTTAAATGTC ACAATATACT TACCATTTGT TGTATATGGC TGTACCCTCT 420
CTA 423

(2) INFORMATION FOR SEQ ID NO:42:

(i) SEQUENCE CHARACTERISTICS:
(A) LENGTH: 527 base pairs
(B) TYPE: nucleic acid
(C) STRANDEDNESS: single
(D) TOPOLOGY: linear

(xi) SEQUENCE DESCRIPTION: SEQ ID NO:42:

TCTCCTAGGC TAATGTGTGT GTTTCTGTAA AAGTAAAAAG TTAAAAATTT TAAAAATAGA 60
AAAAAGCTTA TAGAATAAGA ATATGAAGAA AGAAAATATT TTTGTACATT TGCACAATGA 120
GTTTATGTTT TAAGCTAAGT GTTATTACAA AAGAGCCAAA AAGGTTTTAA AAATTAAAAC 180
GTTTGTAAAG TTACAGTACC CTTATGTTAA TTTATAATTG AAGAAAGAAA AACTTTTTTT 240
TATAAATGTA GTGTAGCCTA AGCATACAGT ATTTATAAAG TCTGGCAGTG TTCAATAATG 300
TCCTAGGCCT TCACATTCAC TCACTGACTC ACCCAGAGCA ACTTCCAGTC CTGTAAGCTC 360
CATTCGTGGT AAGTGCCCTA TACAGGTGCA CCATTTATTT TACAGTATTT TTACTGTACC 420
TTCTCTATGT TTCCATATGT TTCGATATAC AAATACCACT GGTTACTATN GCCCNACAGG 480
TAATTCCAGT AACACGGCCT GTATACGTCT GGTANCCCTA GNGAAGA 527

(2) INFORMATION FOR SEQ ID NO:43:

(i) SEQUENCE CHARACTERISTICS:
(A) LENGTH: 331 base pairs
(B) TYPE: nucleic acid
(C) STRANDEDNESS: single
(D) TOPOLOGY: linear

(xi) SEQUENCE DESCRIPTION: SEQ ID NO:43:

TCTTCAACCT CGTAGGACAA CTCTCATATG CCTGGGCACT ATTTTTAGGT TACTACCTTG 60
GCTGCCCTTC TTTAAGAAAA AAAAAAGAAG AAAAAAGAAC TTTTCCACAA GTTTCTCTTC 120
CTCTAGTTGG AAAATTAGAG AAATCATGTT TTTAATTTTG TGTTATTTCA GATCACAAAT 180
TCAAACACTT GTAAACATTA AGCTTCTGTT CAATCCCCTG GGAAGAGGAT TCATTCTGAT 240
ATTTACGGTT CAAAAGAAGT TGTAATATTG TGCTTGGAAC ACAGAGAACC AGTTATTAAC 300
TTCCTACTAC TATTATATAA TAAATAATAA C 331

(2) INFORMATION FOR SEQ ID NO:44:

(i) SEQUENCE CHARACTERISTICS:
(A) LENGTH: 592 base pairs
(B) TYPE: nucleic acid
(C) STRANDEDNESS: single
(D) TOPOLOGY: linear

(xi) SEQUENCE DESCRIPTION: SEQ ID NO:44:

GGCTTAGTAG TTGCCAGGCA AAATARCGTT GATTCTCCTC AGGAGCCACC CCCAACACCC 60
CTGTTTGCTT CTAGACCTAT ACCTAGACTA AAGTCCCAGC AGACCCCTAG AGGTGAGGTT 120
CAGAGTGACC CTTGAGGAGA TGTGCTACAC TAGAAAAGAA CTGCTTGAGT TTTCTAATTT 180
ATATAAGCAG AAATCTGGAG AAGAGTCATA GGAATGGATA TTAAGGGTGT GAGATAATGG 240
CGGAAGGAAT ATAGAGTTGG ATCAGGCTGG ACTTATTGAT TTGAACCCAC TAAGTAGAGA 300
TTCTGCTTTT GATGTTGCAG CTCAGGGAGT TAAAAAAGGT TTTAATGGTT CTAATAGTTT 360
ATTTGCTTGG TTAGCTGAAA TATGGATAAA AGATGGCCCA CTGTGAGCAA GCTGGAAATG 420
CCTGATCTCT CTCAGTTTAA TGTAGAGGAA GGGATCCAAA AGTTTAGGGA GANTTGGATG 480
CTGGRAKTGG ATTGGTCACT TTGRGACCTA CCCWTCCCAG CTGGGAGGGT CCAGAAGATA 540
CACCCTTGAC CAACGCTTTG CGAAATGGAT TTGTGATGGC GGCAACTACT AA 592

(2) INFORMATION FOR SEQ ID NO:45:

(i) SEQUENCE CHARACTERISTICS:
(A) LENGTH: 567 base pairs
(B) TYPE: nucleic acid
(C) STRANDEDNESS: single
(D) TOPOLOGY: linear

(xi) SEQUENCE DESCRIPTION: SEQ ID NO:45:

GGCTTAGTAG TTGCCATTGC GAGTGCTTGC TCAACGAGCG TTGAACATGG CGGATTGTCT 60
AGATTCAACG GATTTGAGTT TTACCAGCAA AGCGAACCAA GCGCGGCCCA GAGAATTATG 120
GGTTGGTTGG CTTTGAAAAG ATGGAAATCC TGTAGGCCTA GTCAGAAAAG CCTTCTTGCA 180
GAACAGTTGG TTCTCGGGCG AACGCTCATC AAGATGCCCA TTGGAAAGGC TAGCGTGTAT 240
TTGGGAGAGC CTGATAGCGT GTCTTCTGAT GATGTTTGTG CTTGGACAGT GACAAAAGAT 300
ATGCAAAGCA AGTCCGAACT AGACGTCAAG CTTCGTGAGC AAATTATTGT AGACTCCTAC 360
TTATACTGTG AGGAATGATA GCCAAGGGTG GGGACTTTAA GACTAAGGTG GTTTGTACTT 420
GCGCCGATGA TCCCAGGCAG AAAGAMCTGA TCGCTAGTTT TATACGGGCA ACTACTAAGC 480
CGAATTCCAG CACACTGGCG GCCGTTACTA ATTGGATCCG ANCTCGGTAC CAGCTTGATG 540
CATASCTTGA GTTWTCTATA NTGTCNC 567

(2) INFORMATION FOR SEQ ID NO:46:

(i) SEQUENCE CHARACTERISTICS:
(A) LENGTH: 908 base pairs
(B) TYPE: nucleic acid
(C) STRANDEDNESS: single
(D) TOPOLOGY: linear

(xi) SEQUENCE DESCRIPTION: SEQ ID NO:46:

GAGCGAAAGA CCGAGGGCAG NGNNTANGNG CGANGAAGCG GAGAGGGCCA AAAAGCAACC 60
GCTTTCCCCG GGGGGTGCCG ATTCATTAAG GCAGGTGGAG GACAGGTTTC CCGATGGAAG 120
GCGGCAGGGG CGCAAGCAAT TAATGTGAGT AGGCCATTCA TTAGCACCCG GGCTTAACAT 180
TTAAGCTTCG GGTTGGTATG TGGTGGGAAT TGTGAGCGGA TAACAATTTC ACACAGGAAA 240
CAGCTATGAC CATGATTACG CCAAGCTATT TAGGTGACAT TATAGAATAA CTCAAGTTAT 300
GCATCAAGCT TGGTACCGAG TTCGGATCCA CTAGTAACGG CCGCCAGTGT GTGGAATTCG 360
GCTTAGTAGT TGCCGACCAT GGAGTGCTAC CTAGGCTAGA ATACCTGAGY TCCTCCCTAG 420
CCTCACTCAC ATTAAATTGT ATCTTTTCTA CATTAGATGT CCTCAGCGCC TTATTTCTGC 480
TGGACWATCG ATAAATTAAT CCTGATAGGA TGATAGCAGC AGATTAATTA CTGAGAGTAT 540
GTTAATGTGT CATCCCTCCT ATATAACGTA TTTGCATTTT AATGGAGCAA TTCTGGAGAT 600
AATCCCTGAA GGCAAAGGAA TGAATCTTGA GGGTGAGAAA GCCAGAATCA GTGTCCAGCT 660
GCAGTTGTGG GAGAAGGTGA TATTATGTAT GTCTCAGAAG TGACACCATA TGGGCAACTA 720
CTAAGCCCGA ATTCCAGCAC ACTGGCGGGC GTTACTAATG GATCCGAGCT CGGTACCAAG 780
CTTGATGCAT AGCTTGAGTA TCTATAGTGT CACTAAATAG CCTGGCGTTA TCATGGTCAT 840
AGCTGTTTCC TGTGTGAAAT TGTTATCCGC TCCCAATTCC CCCCACCATA CGAGCCGGAA 900
CATAAAGT 908

(2) INFORMATION FOR SEQ ID NO:47:

(i) SEQUENCE CHARACTERISTICS:
(A) LENGTH: 480 base pairs
(B) TYPE: nucleic acid
(C) STRANDEDNESS: single
(D) TOPOLOGY: linear

(xi) SEQUENCE DESCRIPTION: SEQ ID NO:47:

TGCCAACAAG GAAAGTTTTA AATTTCCCCT TGAGGATTCT TGGTGATCAT CAAATTCAGT 60
GGTTTTTAAG GTTGTTTTCT GTCAAATAAC TCTAACTTTA AGCCAAACAG TATATGGAAG 120
CACAGATAKA ATATTACACA GATAAAAGAG GAGTTGATCT AAAGTARAGA TAGTTGGGGG 180
CTTTAATTTC TGGAACCTAG GTCTCCCCAT CTTCTTCTGT GCTGAGGAAC TTCTTGGAAG 240
CGGGGATTCT AAAGTTCTTT GGAAGACAGT TTGAAAACCA CCATGTTGTT CTCAGTACCT 300
TTATTTTTAA AAAGTAGGTG AACATTTTGA GAGAGAAAAG GGCTTGGTTG AGATGAAGTC 360
CCCCCCCCCC CTTTTTTTTT TTTTAGCTGA AATAGATACC CTATGTTNAA RGAARGGATT 420
ATTATTTACC ATGCCAYTAR SCACATGCTC TTTGATGGGC NYCTCCSTAC CCTCCTTAAG 480

(2) INFORMATION FOR SEQ ID NO:48:

(i) SEQUENCE CHARACTERISTICS:
(A) LENGTH: 591 base pairs
(B) TYPE: nucleic acid
(C) STRANDEDNESS: single
(D) TOPOLOGY: linear

(xi) SEQUENCE DESCRIPTION: SEQ ID NO:48:

AAGAGGGTAC CGAGTGGAAT TTCCGCTTCA CTAGTCTGGT GTGGCTAGTC GGTTTCGTGG 60
TGGCCAACAT TACGAACTTC CAACTCAACC GTTCTTGGAC GTTCAAGCGG GAGTACCGGC 120
GAGGATGGTG GCGTGAATTC TGGCCTTTCT TTGCCGTGGG ATCGGTAGCC GCCATCATCG 180
GTATGTTTAT CAAGATCTTC TTTACTAACC CGACCTCTCC GATTTACCTG CCCGAGCCGT 240
GGTTTAACGA GGGGAGGGGG ATCCAGTCAC GCGAGTACTG GTCCCAGATC TTCGCCATCG 300
TCGTGACAAT GCCTATCAAC TTCGTCGTCA ATAAGTTGTG GACCTTCCGA ACGGTGAAGC 360
ACTCCGAAAA CGTCCGGTGG CTGCTGTGCG GTGACTCCCA AAATCTTGAT AACAACAAGG 420
TAACCGAATC GCGCTAAGGA ACCCCGGCAT CTCGGGTACT CTGCATATGC GTACCCCTTA 480
AGCCGAATTC CAGCACACTG GCGGCCGTTA CTAATTGGAT CCGAACTCCG TAACCAAGCC 540
TGATGCGTAA CTTGAGTTAT TCTATAGTGT CCCTAAAATA ACCTGGCGTT A 591

(2) INFORMATION FOR SEQ ID NO:49:

(i) SEQUENCE CHARACTERISTICS:
(A) LENGTH: 454 base pairs
(B) TYPE: nucleic acid
(C) STRANDEDNESS: single
(D) TOPOLOGY: linear

(xi) SEQUENCE DESCRIPTION: SEQ ID NO:49:

AAGAGGGTAC CTGCCTTGAA ATTTAAATGT CTAAGGAAAR TGGGAGATGA TTAAGAGTTG 60
GTGTGGCYTA GTCACACCAA AATGTATTTA TTACATCCTG CTCCTTTCTA GTTGACAGGA 120
AAGAAAGCTG CTGTGGGGAA AGGAGGGATA AATACTGAAG GGATTTACTA AACAAATGTC 180
CATCACAGAG TTTTCCTTTT TTTTTTTTTG AGACAGAGTC TTGCTCTGTC ACCCAGGCTG 240
GAATGAAGWG GTATGATCTC AGTTGAATGC AACCTCTACC TCCTAGGTTC AAGCGATTCT 300
CATGCCTCAG CCTCCTGAGC AGCTGGGACT ATAGGCGCAT GCTACCATGC CAGGCTAATT 360
TTTATATTTT TATTAGAGAC GGGGTGTTGC CATGTTGGCC AGGCAGGTCT CGAACTCCTG 420
GGCCTCAGAT GATCTGCCCC ACCGTACCCT CTTA 454

(2) INFORMATION FOR SEQ ID NO:50:

(i) SEQUENCE CHARACTERISTICS:
(A) LENGTH: 463 base pairs
(B) TYPE: nucleic acid
(C) STRANDEDNESS: single
(D) TOPOLOGY: linear

(xi) SEQUENCE DESCRIPTION: SEQ ID NO:50:

AAGAGGGTAC CAAAAAAAAG AAAAAGGAAA AAAAGAAAAA CAACTTGTAT AAGGCTTTCT 60
GCTGCATACA GCTTTTTTTT TTTAAATAAA TGGTGCCAAC AAATGTTTTT GCATTCACAC 120
CAATTGCTGG TTTTGAAATC GTACTCTTCA AAGGTATTTG TGCAGATCAA TCCAATAGTG 180
ATGCCCCGTA GGTTTTGTGG ACTGCCCACG TTGTCTACCT TCTCATGTAG GAGCCATTGA 240
GAGACTGTTT GGACATGCCT GTGTTCATGT AGCCGTGATG TCCGGGGGCC GTGTACATCA 300
TGTTACCGTG GGGTGGGGTC TGCATTGGCT GCTGGGCATA TGGCTGGGTG CCCATCATGC 360
CCATCTGCAT CTGCATAGGG TATTGGGGCG TTTGATCCAT ATAGCCATGA TTGCTGTGGT 420
AGCCACTGTT CATCATTGGC TGGGACATGC TGTTACCCTC TTA 463

(2) INFORMATION FOR SEQ ID NO:51:

(i) SEQUENCE CHARACTERISTICS:
(A) LENGTH: 399 base pairs
(B) TYPE: nucleic acid
(C) STRANDEDNESS: single
(D) TOPOLOGY: linear

(xi) SEQUENCE DESCRIPTION: SEQ ID NO:51:

CTTCAACCTC CCAAAGTGCT GGGATTACAG GACTGAGCCA CCACGCTCAG CCTAAGCCTC 60
TTTTTCACTA CCCTCTAAGC GATCTACCAC AGTGATGAGG GGCTAAAGAG CAGTGCAATT 120
TGATTACAAT AATGGAACTT AGATTTATTA ATTAACAATT TTTCCTTAGC ATGTTGGTTC 180
CATAATTATT AAGAGTATGG ACTTACTTAG AAATGAGCTT TCATTTTAAG AATTTCATCT 240
TTGACCTTCT CTATTAGTCT GAGCAGTATG ACACTATACG TATTTTATTT AACTAACCTA 300
CCTTGAGCTA TTACTTTTTA AAAGGCTATA TACATGAATG TGTATTGTCA ACTGTAAAGC 360
CCCACAGTAT TTAATTATAT CATGATGTCT TTGAGGTTG 399

(2) INFORMATION FOR SEQ ID NO:52:

(i) SEQUENCE CHARACTERISTICS:
(A) LENGTH: 392 base pairs
(B) TYPE: nucleic acid
(C) STRANDEDNESS: single
(D) TOPOLOGY: linear

(xi) SEQUENCE DESCRIPTION: SEQ ID NO:52:

CTTCAACCTC AATCAACCTT GGTAATTGAT AAAATCATCA CTTAACTTTC TGATATAATG 60
GCAATAATTA TCTGAGAAAA AAAAGTGGTG AAAGATTAAA CTTGCATTTC TCTCAGAATC 120
TTGAAGGATA TTTGAATAAT TCAAAAGCGG AATCAGTAGT ATCAGCCGAA GAAACTCACT 180
TAGCTAGAAC GTTGGACCCA TGGATCTAAG TCCCTGCCCT TCCACTAACC AGCTGATTGG 240
TTTTGTGTAA ACCTCCTACA CGCTTGGGCT TGGTCGCCTC ATTTGTCAAA GTAAAGGCTG 300
AAATAGGAAG ATAATGAACC GTGTCTTTTT GGTCTCTTTT CCATCCATTA CTCTGATTTT 360
ACAAAGAGGC CTGTATTCCC CTGGTGAGGT TG 392

(2) INFORMATION FOR SEQ ID NO:53:

(i) SEQUENCE CHARACTERISTICS:
(A) LENGTH: 179 base pairs
(B) TYPE: nucleic acid
(C) STRANDEDNESS: single
(D) TOPOLOGY: linear

(xi) SEQUENCE DESCRIPTION: SEQ ID NO:53:

TTCGGGTGAT GCCTCCTCAG GCTACAGTGA AGACTGGATT ACAGAAAGGT GCCAGCGAGA 60
TTTCAGATTC CTGTAAACCT CTAAAGAAAA GGAGTCGCGC CTCAACTGAT GTAGAAATGA 120
CTAGTTCAGC ATACNGAGAC ACNTCTGACT CCGATTCTAG AGGACTGAGT GACCTGCAN 179

(2) INFORMATION FOR SEQ ID NO:54:

(i) SEQUENCE CHARACTERISTICS:
(A) LENGTH: 112 base pairs
(B) TYPE: nucleic acid
(C) STRANDEDNESS: single
(D) TOPOLOGY: linear

(xi) SEQUENCE DESCRIPTION: SEQ ID NO:54:

TTCGGGTGAT GCCTCCTCAG GCTACATCAT NATAGAAGCA AAGTAGAANA ATCNNGTTTG 60
TGCATTTTCC CACANACAAA ATTCAAATGA NTGGAAGAAA TTGGGANAGT AT 112

(2) INFORMATION FOR SEQ ID NO:55:

(i) SEQUENCE CHARACTERISTICS:
(A) LENGTH: 225 base pairs
(B) TYPE: nucleic acid
(C) STRANDEDNESS: single
(D) TOPOLOGY: linear

(xi) SEQUENCE DESCRIPTION: SEQ ID NO:55:

TGAGCTTCCG CTTCTGACAA CTCAATAGAT AATCAAAGGA CAACTTTAAC AGGGATTCAC 60
AAAGGAGTAT ATCCAAATGC CAATAAACAT ATAAAAAGGA ATTCAGCTTC ATCATCATCA 120
GAAGWATGCA AATTAAAACC ATAATGAGAA ACCACTATGT CCCACTAGAA TAGATAAAAT 180
CTTAAAAGAC TGGTAAAACC AAGTGTTGGT AAGGCAAGAG GAGCA 225

(2) INFORMATION FOR SEQ ID NO:56:

(i) SEQUENCE CHARACTERISTICS:
(A) LENGTH: 175 base pairs
(B) TYPE: nucleic acid
(C) STRANDEDNESS: single
(D) TOPOLOGY: linear

(xi) SEQUENCE DESCRIPTION: SEQ ID NO:56:

GCTCCTCTTG CCTTACCAAC ACATTCTCAA AAACCTGTTA GAGTCCTAAG CATTCTCCTG 60
TTAGTATTGG GATTTTACCC CTGTCCTATA AAGATGTTAT GTACCAAAAA TGAAGTGGAG 120
GGCCATACCC TGAGGGAGGG GAGGGATCTC TAGTGTTGTC AGAAGCGGAA GCTCA 175

(2) INFORMATION FOR SEQ ID NO:57:

(i) SEQUENCE CHARACTERISTICS:
(A) LENGTH: 223 base pairs
(B) TYPE: nucleic acid
(C) STRANDEDNESS: single
(D) TOPOLOGY: linear

(xi) SEQUENCE DESCRIPTION: SEQ ID NO:57:

AGCCATTTAC CACCCATGGA TGAATGGATT TTGTAATTCT AGCTGTTGTA TTTTGTGAAT 60

TTGTTAATTT TGTTGTTTTT CTGTGAAACA CATACATTGG ATATGGGAGG TAAAGGAGTG 120
TCCCAGTTGC TCCTGGTCAC TCCCTTTATA GCCATTACTG TCTTGTTTCT TGTAACTCAG 180
GTTAGGTTTT GGTCTCTCTT GCTCCACTGC AAAAAAAAAA AAA 223

(2) INFORMATION FOR SEQ ID NO:58:

(i) SEQUENCE CHARACTERISTICS:
(A) LENGTH: 211 base pairs
(B) TYPE: nucleic acid
(C) STRANDEDNESS: single
(D) TOPOLOGY: linear

(xi) SEQUENCE DESCRIPTION: SEQ ID NO:58:

GTTCGAAGGT GAACGTGTAG GTAGCGGATC TCACAACTGG GGAACTGTCA AAGACGAATT 60
AACTGACTTG GATCAATCAA ATGTGACTGA GGAAACACCT GAAGGTGAAG AACATCATCC 120
AGTGGCAGAC ACTGAAAATA AGGAGAATGA AGTTGAAGAG GTAAAAGAGG AGGGTCCAAA 180
AGAGATGACT TTGGATGGGT GGTAAATGGC T 211

(2) INFORMATION FOR SEQ ID NO:59:

(i) SEQUENCE CHARACTERISTICS:
(A) LENGTH: 208 base pairs
(B) TYPE: nucleic acid
(C) STRANDEDNESS: single
(D) TOPOLOGY: linear

(xi) SEQUENCE DESCRIPTION: SEQ ID NO:59:

GCTCCTCTTG CCTTACCAAC TTTGCACCCA TCATCAACCA TGTGGCCAGG TTTGCAGCCC 60
AGGCTGCACA TCAGGGGACT GCCTCGCAAT ACTTCATGCT GTTGCTGCTG ACTGATGGTG 120
CTGTGACGGA TGTGGAAGCC ACACGTGAGG CTGTGGTGCG TGCCTCGAAC CTGCCCATGT 180
CAGTGATCAT TATGGGTGGT AAATGGCT 208

(2) INFORMATION FOR SEQ ID NO:60:

(i) SEQUENCE CHARACTERISTICS:
(A) LENGTH: 171 base pairs
(B) TYPE: nucleic acid
(C) STRANDEDNESS: single
(D) TOPOLOGY: linear

(xi) SEQUENCE DESCRIPTION: SEQ ID NO:60:

AGCCATTTAC CACCCATACT AAATTCTAGT TCAAACTCCA ACTTCTTCCA TAAAACATCT 60
AACCACTGAC ACCAGTTGGC AATAGCTTCT TCCTTCTTTA ACCTCTTAGA GTATTTATGG 120
TCAATGCCAC ACATTTCTGC AACTGAATAA AGTTGGTAAG GCAAGAGGAG C 171

(2) INFORMATION FOR SEQ ID NO:61:

(i) SEQUENCE CHARACTERISTICS:
(A) LENGTH: 134 base pairs

(B) TYPE: nucleic acid
(C) STRANDEDNESS: single
(D) TOPOLOGY: linear

(xi) SEQUENCE DESCRIPTION: SEQ ID NO:61:

CGGGTGATGC CTCCTCAGGC TTTGGTGTGT CCACTCNACT CACTGGCCTC TTCTCCAGCA 60
ACTGGTGAAN ATGTCCTCAN GAAAANCNCC ACACGCNGCT CAGGGTGGGG TGGGAANCAT 120
CANAATCATC NGGC 134

(2) INFORMATION FOR SEQ ID NO:62:

(i) SEQUENCE CHARACTERISTICS:
(A) LENGTH: 145 base pairs
(B) TYPE: nucleic acid
(C) STRANDEDNESS: single
(D) TOPOLOGY: linear

(xi) SEQUENCE DESCRIPTION: SEQ ID NO:62:

AGAGGGTACA TATGCAACAG TATATAAAGG AAGAAGTGCA CTGAGAGGAA CTTCATCAAG 60
GCCATTTAAT CAATAAGTGA TAGAGTCAAG GCTCAACCCA GGTGTGACGG ATTCCAGGTC 120
CCAAGCTCCT TACTGGTACC CTCTT 145

(2) INFORMATION FOR SEQ ID NO:63:

(i) SEQUENCE CHARACTERISTICS:
(A) LENGTH: 297 base pairs
(B) TYPE: nucleic acid
(C) STRANDEDNESS: single
(D) TOPOLOGY: linear

(xi) SEQUENCE DESCRIPTION: SEQ ID NO:63:

TGCACTGAGA GGAATTCAAA GGGTTTATGC CAAAGAACAA ACCAGTCCTC TGCAGCCTAA 60
CTCATTTGTT TTTGGGCTGC GAAGCCATGT AGAGGGCGAT CAGGCAGTAG ATGGTCCCTC 120
CCACAGTCAG CGCCATGGTG GTCCGGTAAA GCATTTGGTC AGGCAGGCCT CGTTTCAGGT 180
AGACGGGCAC ACATCAGCTT TCTGGAAAAA CTTTTGTAGC TCTGGAGCTT TGTTTTTCCC 240
AGCATAATCA TACACTGTGG AATCGGAGGT CAGTTTAGTT GGTAAGGCAA GAGGAGC 297

(2) INFORMATION FOR SEQ ID NO:64:

(i) SEQUENCE CHARACTERISTICS:
(A) LENGTH: 300 base pairs
(B) TYPE: nucleic acid
(C) STRANDEDNESS: single
(D) TOPOLOGY: linear

(xi) SEQUENCE DESCRIPTION: SEQ ID NO:64:

GCACTGAGAG GAACTTCCAA TACTATGTTG AATAGGAGTG GTGAGAGAGG GCATCCTTGT 60

CTTGTGCCGG TTTTCAAAGG GAATGCTTCC AGCTTTTGCC CATTCAGTAT AATATTAAAG 120
AATGTTTTAC CATTTTCTGT CTTGCCTGTT TTTCTGTGTT TTTGTTGGTC TCTTCATTCT 180
CCATTTTTAG GCCTTTACAT GTTAGGAATA TATTTCTTTT AATGATACTT CACCTTTGGT 240
ATCTTTTGTG AGACTCTACT CATAGTGTGA TAAGCACTGG GTTGGTAAGG CAAGAGGAGC 300

(2) INFORMATION FOR SEQ ID NO:65:

(i) SEQUENCE CHARACTERISTICS:
(A) LENGTH: 203 base pairs
(B) TYPE: nucleic acid
(C) STRANDEDNESS: single
(D) TOPOLOGY: linear

(xi) SEQUENCE DESCRIPTION: SEQ ID NO:65:

GCTCCTCTTG CCTTACCAAC TCACCCAGTA TGTCAGCAAT TTTATCRGCT TTACCTACGA 60
AACAGCCTGT ATCCAAACAC TTAACACACT CACCTGAAAA GTTCAGGCAA CAATCGCCTT 120
CTCATGGGTC TCTCTGCTCC AGTTCTGAAC CTTTCTCTTT TCCTAGAACA TGCATTTARG 180
TCGATAGAAG TTCCTCTCAG TGC 203

(2) INFORMATION FOR SEQ ID NO:66:

(i) SEQUENCE CHARACTERISTICS:
(A) LENGTH: 344 base pairs
(B) TYPE: nucleic acid
(C) STRANDEDNESS: single
(D) TOPOLOGY: linear

(xi) SEQUENCE DESCRIPTION: SEQ ID NO:66:

TACGGGGACC CCTGCATTGA GAAAGCGAGA CTCACTCTGA AGCTGAAATG CTGTTGCCCT 60
TGCAGTGCTG GTAGCAGGAG TTCTGTGCTT TGTGGGCTAA GGCTCCTGGA TGACCCCTGA 120
CATGGAGAAG GCAGAGTTGT GTGCCCCTTC TCATGGCCTC GTCAAGGCAT CATGGACTGC 180
CACACACAAA ATGCCGTTTT TATTAACGAC ATGAAATTGA AGGAGAGAAC ACAATTCACT 240
GATGTGGCTC GTAACCATGG ATATGGTCAC ATACAGAGGT GTGATTATGT AAAGGTTAAT 300
TCCACCCACC TCATGTGGAA ACTAGCCTCA ATGCAGGGGT CCCA 344

(2) INFORMATION FOR SEQ ID NO:67:

(i) SEQUENCE CHARACTERISTICS:
(A) LENGTH: 157 base pairs
(B) TYPE: nucleic acid
(C) STRANDEDNESS: single
(D) TOPOLOGY: linear

(xi) SEQUENCE DESCRIPTION: SEQ ID NO:67:

GCACTGAGAG GAACTTCGTA GGGAGGTTGA ACTGGCTGCT GAGGAGGGGG AACAACAGGG 60
TAACCAGACT GATAGCCATT GGATGGATAA TATGGTGGTT GAGGAGGGAC ACTACTTATA 120
GCAGAGGGTT GTGTATAGCC TGAGGAGGCA TCACCCG 157

(2) INFORMATION FOR SEQ ID NO:68:

(i) SEQUENCE CHARACTERISTICS:
(A) LENGTH: 137 base pairs
(B) TYPE: nucleic acid
(C) STRANDEDNESS: single
(D) TOPOLOGY: linear

(xi) SEQUENCE DESCRIPTION: SEQ ID NO:68:

GCACTGAGAG GAACTTCTAG AAAGTGAAAG TCTAGACATA AAATAAAATA AAAATTTAAA 60
ACTCAGGAGA GACAGCCCAG CACGGTGGCT CACGCCTGTA ATCCCAGAAC TTTGGGAGCC 120
TGAGGAGGCA TCACCCG 137

(2) INFORMATION FOR SEQ ID NO:69:

(i) SEQUENCE CHARACTERISTICS:
(A) LENGTH: 137 base pairs
(B) TYPE: nucleic acid
(C) STRANDEDNESS: single
(D) TOPOLOGY: linear

(xi) SEQUENCE DESCRIPTION: SEQ ID NO:69:

CGGGTGATGC CTCCTCAGGC TGTATTTTGA AGACTATCGA CTGGACTTCT TATCAACTGA 60
AGAATCCGTT AAAAATACCA GTTGTATTAT TTCTACCTGT CAAAATCCAT TTCAAATGTT 120
GAAGTTCCTC TCAGTGC 137

(2) INFORMATION FOR SEQ ID NO:70:

(i) SEQUENCE CHARACTERISTICS:
(A) LENGTH: 220 base pairs
(B) TYPE: nucleic acid
(C) STRANDEDNESS: single
(D) TOPOLOGY: linear

(xi) SEQUENCE DESCRIPTION: SEQ ID NO:70:

AGCATGTTGA GCCCAGACAC GCAATCTGAA TGAGTGTGCA CCTCAAGTAA ATGTCTACAC 60
GCTGCCTGGT CTGACATGGC ACACCATCNC GTGGAGGGCA CASCTCTGCT CNGCCTACWA 120
CGAGGGCANT CTCATWGACA GGTTCCACCC ACCAAACTGC AAGAGGCTCA NNAAGTACTR 180
CCAGGGTMYA SGGACMASGG TGGGAYTYCA YCACWCATCT 220

(2) INFORMATION FOR SEQ ID NO:71:

(i) SEQUENCE CHARACTERISTICS:
(A) LENGTH: 353 base pairs
(B) TYPE: nucleic acid
(C) STRANDEDNESS: single
(D) TOPOLOGY: linear

(xi) SEQUENCE DESCRIPTION: SEQ ID NO:71:

CGTTAGGGTC TCTATCCACT GCTAAACCAT ACACCTGGGT AAACAGGGAC CATTTAACAT 60
TCCCANCTAA ATATGCCAAG TGACTTCACA TGTTTATCTT AAAGATGTCC AAAACGCAAC 120
TGATTTTCTC CCCTAAACCT GTGATGGTGG GATGATTAAN CCTGAGTGGT CTACAGCAAG 180
TTAAGTGCAA GGTGCTAAAT GAANGTGACC TGAGATACAG CATCTACAAG GCAGTACCTC 240
TCAACNCAGG GCAACTTTGC TTCTCANAGG GCATTTAGCA GTGTCTGAAG TAATTTCTGT 300
ATTACAACTC ACGGGGCGGG GGGTGAATAT CTANTGGANA GNAGACCCTA ACG 353

(2) INFORMATION FOR SEQ ID NO:72:

(i) SEQUENCE CHARACTERISTICS:
(A) LENGTH: 343 base pairs
(B) TYPE: nucleic acid
(C) STRANDEDNESS: single
(D) TOPOLOGY: linear

(xi) SEQUENCE DESCRIPTION: SEQ ID NO:72:

GCACTGAGAG GAACTTCCAA TACYATKATC AGAGTGAACA RGCARCCYAC AGAACAGGAG 60
AAAATGTTYG CAATCTCTCC ATCTGACAAA AGGCTAATAT CCAGAWTCTA AWAGGAACTT 120
AAACAAATTT ATGAGAAAAG AACARACAAC CTCAWCAAAA AGTGGGTGAA GGAWATGCTS 180
AAARGAAGAC ATYTATTCAG CCAGTAAACA YATGAAAAAA AGGCTCATSA TCACTGAWCA 240
TTAGAGAAAT GCAAATCAAA ACCACAATGA GATACCATCT YAYRCCAGTT AGAAYGGTGA 300
TCATTAAAAR STCAGGAAAC AACAGATGCT GGACAAGGTG TCA 343

(2) INFORMATION FOR SEQ ID NO:73:

(i) SEQUENCE CHARACTERISTICS:
(A) LENGTH: 321 base pairs
(B) TYPE: nucleic acid
(C) STRANDEDNESS: single
(D) TOPOLOGY: linear

(xi) SEQUENCE DESCRIPTION: SEQ ID NO:73:

GCACTGAGAG GAACTTCAGA GAGAGAGAGA GAGTTCCACC CTGTACTTGG GGAGAGAAAC 60
AGAAGGTGAG AAAGTCTTTG GTTCTGAAGC AGCTTCTAAG ATCTTTTCAT TTGCTTCATT 120
TCAAAGTTCC CATGCTGCCA AAGTGCCATC CTTTGGGGTA CTGTTTTCTG AGCTCCAGTG 180
ATAACTCATT TATACAAGGG AGATACCCAG AAAAAAAGTG AGCAAATCTT AAAAAGGTGG 240
CTTGAGTTCA GCCTTAAATA CCATCTTGAA ATGACACAGA GAAAGAANGA TGTTGGGTGG 300
GAGTGGATAG AGACCCTAAC G 321

(2) INFORMATION FOR SEQ ID NO:74:

(i) SEQUENCE CHARACTERISTICS:
(A) LENGTH: 321 base pairs
(B) TYPE: nucleic acid
(C) STRANDEDNESS: single
(D) TOPOLOGY: linear

(xi) SEQUENCE DESCRIPTION: SEQ ID NO:74:

GCACTGAGAG GAACTTCAGA GAGAGAGAGA GAGTTCCACC CTGTACTTGG GGAGAGAAAC 60
AGAAGGTGAG AAAGTCTTTG GTTCTGAAGC AGCTTCTAAG ATCTTTTCAT TTGCTTCATT 120
TCAAAGTTCC CATGCTGCCA AAGTGCCATC CTTTGGGGTA CTGTTTTCTG AGCTCCAGTG 180
ATAACTCATT TATACAAGGG AGATACCCAG AAAAAAAGTG AGCAAATCTT AAAAAGGTGG 240
CTTGAGTTCA GYCTTAAATA CCATCTTGAA ATGAMACAGA GAAAGAAGGA TGTTGGGTGG 300
GAGTGGATAG AGACCCTAAC G 321

(2) INFORMATION FOR SEQ ID NO:75:

(i) SEQUENCE CHARACTERISTICS:
(A) LENGTH: 317 base pairs
(B) TYPE: nucleic acid
(C) STRANDEDNESS: single
(D) TOPOLOGY: linear

(xi) SEQUENCE DESCRIPTION: SEQ ID NO:75:

GCACTGAGAG GAACTTCCAC ATGCACTGAG AAATGCATGT TCACAAGGAC TGAAGTCTGG 60
AACTCAGTTT CTCAGTTCCA ATCCTGATTC AGGTGTTTAC CAGCTACACA ACCTTAAGCA 120
AGTCAGATAA CCTTAGCTTC CTCATATGCA AAATGAGAAT GAAAAGTACT CATCGCTGAA 180
TTGTTTTGAG GATTAGAAAA ACATCTGGCA TGCAGTAGAA ATTCAATTAG TATTCATTTT 240
CATTCTTCTA AATTAAACAA ATAGGATTTT TAGTGGTGGA ACTTCAGACA CCAGAAATGG 300
GAGTGGATAG AGACCCT 317

(2) INFORMATION FOR SEQ ID NO:76:

(i) SEQUENCE CHARACTERISTICS:
(A) LENGTH: 244 base pairs
(B) TYPE: nucleic acid
(C) STRANDEDNESS: single
(D) TOPOLOGY: linear

(xi) SEQUENCE DESCRIPTION: SEQ ID NO:76:

CGTTAGGGTC TCTATCCACT CCCACTACTG ATCAAACTCT ATTTATTTAA TTATTTTTAT 60
CATACTTTAA GTTCTGGGAT ACACGTGCAG CATGCGCAGG TTTGTTGCAT AGGTATACAC 120
TTGCCATGGT GGTTTGCTGC ACCCATCAGT CCATCATCTA CATTAGGTAT TTCTCCTAAT 180
GCTATCCCTC CCCTAGCCCC TTACACCCCC AACAGGCTCT AGTGTGTGAA GTTCCTCTCA 240
GTGC 244

(2) INFORMATION FOR SEQ ID NO:77:

(i) SEQUENCE CHARACTERISTICS:
(A) LENGTH: 254 base pairs
(B) TYPE: nucleic acid
(C) STRANDEDNESS: single
(D) TOPOLOGY: linear

(xi) SEQUENCE DESCRIPTION: SEQ ID NO:77:

CGTTAGGGTC TCTATCCACT GAAATCTGAA GCACAGGAGG AAGAGAAGCA GTYCTAGTGA 60

GATGGCAAGT TCWTTTACCA CACTCTTTAA CATTTYGTTT AGTTTTAACC TTTATTTATG 120
GATAATAAAG GTTAATATTA ATAATGATTT ATTTTAAGGC ATTCCCRAAT TTGCATAATT 180
CTCCTTTTGG AGATACCCTT TTATCTCCAG TGCAAGTCTG GATCAAAGTG ATASAMAGAA 240
GTTCCTCTCA GTGC 254

(2) INFORMATION FOR SEQ ID NO:78:

(i) SEQUENCE CHARACTERISTICS:
(A) LENGTH: 355 base pairs
(B) TYPE: nucleic acid
(C) STRANDEDNESS: single
(D) TOPOLOGY: linear

(xi) SEQUENCE DESCRIPTION: SEQ ID NO:78:

TTCGATACAG GCAAACATGA ACTGCAGGAG GGTGGTGACG ATCATGATGT TGCCGATGGT 60
CCGGATGGNC ACGAAGACGC ACTGGANCAC GTGCTTACGT CCTTTTGCTC TGTTGATGGC 120
CCTGAGGGGA CGCAGGACCC TTATGACCCT CAGAATCTTC ACAACGGGAG ATGGCACTGG 180
ATTGANTCCC ANTGACACCA GAGACACCCC AACCACCAGN ATATCANTAT ATTGATGTAG 240
TTCCTGTAGA NGGCCCCCTT GTGGAGGAAA GCTCCATNAG TTGGTCATCT TCAACAGGAT 300
CTCAACAGTT TCCGATGGCT GTGATGGGCA TAGTCATANT TAACCNTGTN TCGAA 355

(2) INFORMATION FOR SEQ ID NO:79:

(i) SEQUENCE CHARACTERISTICS:
(A) LENGTH: 406 base pairs
(B) TYPE: nucleic acid
(C) STRANDEDNESS: single
(D) TOPOLOGY: linear

(xi) SEQUENCE DESCRIPTION: SEQ ID NO:79:

TAAGAGGGTA CCAGCAGAAA GGTTAGTATC ATCAGATAGC ATCTTATACG AGTAATATGC 60
CTGCTATTTG AAGTGTAATT GAGAAGGAAA ATTTTAGCGT GCTCACTGAC CTGCCTGTAG 120
CCCCAGTGAC AGCTAGGATG TGCATTCTCC AGCCATCAAG AGACTGAGTC AAGTTGTTCC 180
TTAAGTCAGA ACAGCAGACT CAGCTCTGAC ATTCTGATTC GAATGACACT GTTCAGGAAT 240
CGGAATCCTG TCGATTAGAC TGGACAGCTT GTGGCAAGTG AATTTGCCTG TAACAAGCCA 300
GATTTTTTAA AATTTATATT GTAAATAATG TGTGTGTGTG TGTGTGTATA TATATATATA 360
TGTACAGTTA TCTAAGTTAA TTTAAAAGTT GTTTGGTACC CTCTTA 406

(2) INFORMATION FOR SEQ ID NO:80:

(i) SEQUENCE CHARACTERISTICS:
(A) LENGTH: 327 base pairs
(B) TYPE: nucleic acid
(C) STRANDEDNESS: single
(D) TOPOLOGY: linear

(xi) SEQUENCE DESCRIPTION: SEQ ID NO:80:

TTTTTTTTTT TTTACTCGGC TCAGTCTAAT CCTTTTTGTA GTCACTCATA GGCCAGACTT 60
AGGGCTAGGA TGATGATTAA TAAGAGGGAT GACATAACTA TTAGTGGCAG GTTAGTTGTT 120

TGTAGGGCTC ATGGTAGGGG TAAAAGGAGG GCAATTTCTA GATCAAATAA TAAGAAGGTA 180
ATAGCTACTA AGAAGAATTT TATGGAGAAA GGGACGCGGG CGGGGGATAT AGGGTCGAAG 240
CCGCACTCGT AAGGGGTGGA TTTTTCTATG TAGCCGTTGA GTTGTGGTAG TCAAAATGTA 300
ATAATTATTA GTAGTAAGCC TAGGAGA 327

(2) INFORMATION FOR SEQ ID NO:81:

(i) SEQUENCE CHARACTERISTICS:
(A) LENGTH: 318 base pairs
(B) TYPE: nucleic acid
(C) STRANDEDNESS: single
(D) TOPOLOGY: linear

(xi) SEQUENCE DESCRIPTION: SEQ ID NO:81:

TAGTCTATGC GGTTGATTCG GCAATCCATT ATTTGCTGGA TTTTGTCATG TGTTTTGCCA 60
ATTGCATTCA TAATTTATTA TGCATTTATG CTTGTATCTC CTAAGTCATG GTATATAATC 120
CATGCTTTTT ATGTTTTGTC TGACATAAAC TCTTATCAGA GCCCTTTGCA CACAGGGATT 180
CAATAAATAT TAACACAGTC TACATTTATT TGGTGAATAT TGCATATCTG CTGTACTGAA 240
AGCACATTAA GTAACAAAGG CAAGTGAGAA GAATGAAAAG CACTACTCAC AACAGTTATC 300
ATGATTGCGC ATAGACTA 318

(2) INFORMATION FOR SEQ ID NO:82:

(i) SEQUENCE CHARACTERISTICS:
(A) LENGTH: 338 base pairs
(B) TYPE: nucleic acid
(C) STRANDEDNESS: single
(D) TOPOLOGY: linear

(xi) SEQUENCE DESCRIPTION: SEQ ID NO:82:

TCTTCAACCT CTACTCCCAC TAATAGCTTT TTGATGACTT CTAGCAAGCC TCGCTAACCT 60
CGCCTTACCC CCCACTATTA ACCTACTGGG AGAACTCTCT GTGCTAGTAA CCACGTTCTC 120
CTGATCAAAT ATCACTCTCC TACTTACAGG ACTCAACATA CTAGTCACAG CCCTATACTC 180
CCTCTACATA TTTACCACAA CACAATGGGG CTCACTCACC CACCACATTA ACAACATAAA 240
ACCCTCATTC ACACGAGAAA ACACCCTCAT GTTCATACAC CTATCCCCCA TTCTCCTCCT 300
ATCCCTCAAC CCCGACATCA TTACCGGGTT TTCCTCTT 338

(2) INFORMATION FOR SEQ ID NO:83:

(i) SEQUENCE CHARACTERISTICS:
(A) LENGTH: 111 base pairs
(B) TYPE: nucleic acid
(C) STRANDEDNESS: single
(D) TOPOLOGY: linear

(xi) SEQUENCE DESCRIPTION: SEQ ID NO:83:

AGCCATTTAC CACCCATCCA CAAAAAAAAA AAAAAAAAAG AAAAATATCA AGGAATAAAA 60
ATAGACTTTG AACAAAAAGG AACATTTGCT GGCCTGAGGA GGCATCACCC G 111

(2) INFORMATION FOR SEQ ID NO:84:

(i) SEQUENCE CHARACTERISTICS:
(A) LENGTH: 224 base pairs
(B) TYPE: nucleic acid
(C) STRANDEDNESS: single
(D) TOPOLOGY: linear

(xi) SEQUENCE DESCRIPTION: SEQ ID NO:84:

TCGGGTGATG CCTCCTCAGG CCAAGAAGAT AAAGCTTCAG ACCCCTAACA CATTTCCAAA 60
AAGGAAGAAA GGAGAAAAAA GGGCATCATC CCCGTTCCGA AGGGTCAGGG AGGAGGAAAT 120
TGAGGTGGAT TCACGAGTTG CGGACAACTC CTTTGATGCC AAGCGAGGTG CAGCCGGAGA 180
CTGGGGAGAG CGAGCCAATC AGGTTTTGAA GTTCCTCTCA GTGC 224

(2) INFORMATION FOR SEQ ID NO:85:

(i) SEQUENCE CHARACTERISTICS:
(A) LENGTH: 348 base pairs
(B) TYPE: nucleic acid
(C) STRANDEDNESS: single
(D) TOPOLOGY: linear

(xi) SEQUENCE DESCRIPTION: SEQ ID NO:85:

GCACTGAGAG GAACTTCGTT GGAAACGGGT TTTTTTCATG TAAGGCTAGA CAGAAGAATT 60
CTCAGTAACT TCCTTGTGTT GTGTGTATTC AACTCACASA GTTGAACGAT CCTTTACACA 120
GAGCAGACTT GTAACACTCT TWTTGTGGAA TTTGCAAGTG GAGATTTCAG SCGCTTTGAA 180
GTSAAAGGTA GAAAAGGAAA TATCTTCCTA TAAAAACTAG ACAGAATGAT TCTCAGAAAC 240
TCCTTTGTGA TGTGTGCGTT CAACTCACAG AGTTTAACCT TTCWTTTCAT AGAAGCAGTT 300
AGGAAACACT CTGTTTGTAA AGTCTGCAAG TGGATAGAGA CCCTAACG 348

(2) INFORMATION FOR SEQ ID NO:86:

(i) SEQUENCE CHARACTERISTICS:
(A) LENGTH: 293 base pairs
(B) TYPE: nucleic acid
(C) STRANDEDNESS: single
(D) TOPOLOGY: linear

(xi) SEQUENCE DESCRIPTION: SEQ ID NO:86:

GCACTGAGAG GAACTTCYTT GTGWTGTKTG YATTCAACTC ACAGAGTTGA ASSWTSMTTT 60
ACABAGWKCA GGCTTKCAAA CACTCTTTTT GTMGAATYTG CAAGWGGAKA TTTSRRCCRC 120
TTTGWGGYCW WYSKTMGAAW MGGRWATATC TTCWYATMRA AMCTAGACAG AAKSATTCTC 180
AKAAWSTYYY YTGTGAWGWS TGCRTTCAAC TCACAGAGKT KAACMWTYCT KYTSATRGAG 240
CAGTTWKGAA ACTCTMTTTC TTTGGATTCT GCAAGTGGAT AGAGACCCTA ACG 293

(2) INFORMATION FOR SEQ ID NO:87:

(i) SEQUENCE CHARACTERISTICS:
(A) LENGTH: 10 base pairs

(B) TYPE: nucleic acid
(C) STRANDEDNESS: single
(D) TOPOLOGY: linear

(xi) SEQUENCE DESCRIPTION: SEQ ID NO:87:

CTCCTAGGCT 10

(2) INFORMATION FOR SEQ ID NO:88:

(i) SEQUENCE CHARACTERISTICS:
(A) LENGTH: 10 base pairs
(B) TYPE: nucleic acid
(C) STRANDEDNESS: single
(D) TOPOLOGY: linear

(xi) SEQUENCE DESCRIPTION: SEQ ID NO:88:

AGTAGTTGCC 10

(2) INFORMATION FOR SEQ ID NO:89:

(i) SEQUENCE CHARACTERISTICS:
(A) LENGTH: 11 base pairs
(B) TYPE: nucleic acid
(C) STRANDEDNESS: single
(D) TOPOLOGY: linear

(xi) SEQUENCE DESCRIPTION: SEQ ID NO:89:

TTCCGTTATG C 11

(2) INFORMATION FOR SEQ ID NO:90:

(i) SEQUENCE CHARACTERISTICS:
(A) LENGTH: 10 base pairs
(B) TYPE: nucleic acid
(C) STRANDEDNESS: single
(D) TOPOLOGY: linear

(xi) SEQUENCE DESCRIPTION: SEQ ID NO:90:

TGGTAAAGGG 10

(2) INFORMATION FOR SEQ ID NO:91:

(i) SEQUENCE CHARACTERISTICS:
(A) LENGTH: 10 base pairs
(B) TYPE: nucleic acid
(C) STRANDEDNESS: single
(D) TOPOLOGY: linear

(xi) SEQUENCE DESCRIPTION: SEQ ID NO:91:

TCGGTCATAG 10

(2) INFORMATION FOR SEQ ID NO:92:

(i) SEQUENCE CHARACTERISTICS:
(A) LENGTH: 10 base pairs
(B) TYPE: nucleic acid
(C) STRANDEDNESS: single
(D) TOPOLOGY: linear

(xi) SEQUENCE DESCRIPTION: SEQ ID NO:92:

TACAACGAGG 10

(2) INFORMATION FOR SEQ ID NO:93:

(i) SEQUENCE CHARACTERISTICS:
(A) LENGTH: 10 base pairs
(B) TYPE: nucleic acid
(C) STRANDEDNESS: single
(D) TOPOLOGY: linear

(xi) SEQUENCE DESCRIPTION: SEQ ID NO:93:

TGGATTGGTC 10

(2) INFORMATION FOR SEQ ID NO:94:

(i) SEQUENCE CHARACTERISTICS:
(A) LENGTH: 10 base pairs
(B) TYPE: nucleic acid
(C) STRANDEDNESS: single
(D) TOPOLOGY: linear

(xi) SEQUENCE DESCRIPTION: SEQ ID NO:94:

CTTTCTACCC 10

(2) INFORMATION FOR SEQ ID NO:95:

(i) SEQUENCE CHARACTERISTICS:
(A) LENGTH: 10 base pairs
(B) TYPE: nucleic acid
(C) STRANDEDNESS: single
(D) TOPOLOGY: linear

(xi) SEQUENCE DESCRIPTION: SEQ ID NO:95:

TTTTGGCTCC                                                          10

(2) INFORMATION FOR SEQ ID NO:96:

     (i) SEQUENCE CHARACTERISTICS:
          (A) LENGTH: 10 base pairs
          (B) TYPE: nucleic acid
          (C) STRANDEDNESS: single
          (D) TOPOLOGY: linear

    (xi) SEQUENCE DESCRIPTION: SEQ ID NO:96:

GGAACCAATC                                                          10

(2) INFORMATION FOR SEQ ID NO:97:

     (i) SEQUENCE CHARACTERISTICS:
          (A) LENGTH: 10 base pairs
          (B) TYPE: nucleic acid
          (C) STRANDEDNESS: single
          (D) TOPOLOGY: linear

    (xi) SEQUENCE DESCRIPTION: SEQ ID NO:97:

TCGATACAGG                                                          10

(2) INFORMATION FOR SEQ ID NO:98:

     (i) SEQUENCE CHARACTERISTICS:
          (A) LENGTH: 10 base pairs
          (B) TYPE: nucleic acid
          (C) STRANDEDNESS: single
          (D) TOPOLOGY: linear

    (xi) SEQUENCE DESCRIPTION: SEQ ID NO:98:

GGTACTAAGG                                                          10

(2) INFORMATION FOR SEQ ID NO:99:

     (i) SEQUENCE CHARACTERISTICS:
          (A) LENGTH: 10 base pairs
          (B) TYPE: nucleic acid
          (C) STRANDEDNESS: single
          (D) TOPOLOGY: linear

    (xi) SEQUENCE DESCRIPTION: SEQ ID NO:99:

AGTCTATGCG                                                          10

(2) INFORMATION FOR SEQ ID NO:100:

(i) SEQUENCE CHARACTERISTICS:
(A) LENGTH: 10 base pairs
(B) TYPE: nucleic acid
(C) STRANDEDNESS: single
(D) TOPOLOGY: linear

(xi) SEQUENCE DESCRIPTION: SEQ ID NO:100:

CTATCCATGG 10

(2) INFORMATION FOR SEQ ID NO:101:

(i) SEQUENCE CHARACTERISTICS:
(A) LENGTH: 10 base pairs
(B) TYPE: nucleic acid
(C) STRANDEDNESS: single
(D) TOPOLOGY: linear

(xi) SEQUENCE DESCRIPTION: SEQ ID NO:101:

TCTGTCCACA 10

(2) INFORMATION FOR SEQ ID NO:102:

(i) SEQUENCE CHARACTERISTICS:
(A) LENGTH: 10 base pairs
(B) TYPE: nucleic acid
(C) STRANDEDNESS: single
(D) TOPOLOGY: linear

(xi) SEQUENCE DESCRIPTION: SEQ ID NO:102:

AAGAGGGTAC 10

(2) INFORMATION FOR SEQ ID NO:103:

(i) SEQUENCE CHARACTERISTICS:
(A) LENGTH: 10 base pairs
(B) TYPE: nucleic acid
(C) STRANDEDNESS: single
(D) TOPOLOGY: linear

(xi) SEQUENCE DESCRIPTION: SEQ ID NO:103:

CTTCAACCTC 10

(2) INFORMATION FOR SEQ ID NO:104:

(i) SEQUENCE CHARACTERISTICS:

(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:104:

GCTCCTCTTG CCTTACCAAC 20

(2) INFORMATION FOR SEQ ID NO:105:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:105:

GTAAGTCGAG CAGTGTGATG 20

(2) INFORMATION FOR SEQ ID NO:106:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:106:

GTAAGTCGAG CAGTCTGATG 20

(2) INFORMATION FOR SEQ ID NO:107:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:107:

GACTTAGTGG AAAGAATGTA 20

(2) INFORMATION FOR SEQ ID NO:108:

(i) SEQUENCE CHARACTERISTICS:
(A) LENGTH: 20 base pairs
(B) TYPE: nucleic acid
(C) STRANDEDNESS: single

(D) TOPOLOGY: linear

(xi) SEQUENCE DESCRIPTION: SEQ ID NO:108:

GTAATTCCGC CAACCGTAGT 20

(2) INFORMATION FOR SEQ ID NO:109:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:109:

ATGGTTGATC GATAGTGGAA 20

(2) INFORMATION FOR SEQ ID NO:110:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:110:

ACGGGGACCC CTGCATTGAG 20

(2) INFORMATION FOR SEQ ID NO:111:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:111:

TATTCTAGAC CATTCGCTAC 20

(2) INFORMATION FOR SEQ ID NO:112:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:112:

ACATAACCAC TTTAGCGTTC 20

(2) INFORMATION FOR SEQ ID NO:113:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:113:

CGGGTGATGC CTCCTCAGGC 20

(2) INFORMATION FOR SEQ ID NO:114:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:114:

AGCATGTTGA GCCCAGACAC 20

(2) INFORMATION FOR SEQ ID NO:115:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:115:

GACACCTTGT CCAGCATCTG 20

(2) INFORMATION FOR SEQ ID NO:116:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:116:

TACGCTGCAA CACTGTGGAG 20

(2) INFORMATION FOR SEQ ID NO:117:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:117:

CGTTAGGGTC TCTATCCACT 20

(2) INFORMATION FOR SEQ ID NO:118:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:118:

AGACTGACTC ATGTCCCCTA 20

(2) INFORMATION FOR SEQ ID NO:119:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:119:

TCATCGCTCG GTGACTCAAG 20

(2) INFORMATION FOR SEQ ID NO:120:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:120:

CAAGATTCCA TAGGCTGACC 20

(2) INFORMATION FOR SEQ ID NO:121:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:121:

ACGTACTGGT CTTGAAGGTC 20

(2) INFORMATION FOR SEQ ID NO:122:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:122:

GACGCTTGGC CACTTGACAC 20

(2) INFORMATION FOR SEQ ID NO:123:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:123:

GTATCGACGT AGTGGTCTCC 20

(2) INFORMATION FOR SEQ ID NO:124:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:124:

TAGTGACATT ACGACGCTGG 20

(2) INFORMATION FOR SEQ ID NO:125:

(i) SEQUENCE CHARACTERISTICS:
(A) LENGTH: 20 base pairs
(B) TYPE: nucleic acid

(C) STRANDEDNESS: single
(D) TOPOLOGY: linear

(xi) SEQUENCE DESCRIPTION: SEQ ID NO:125:

CGGGTGATGC CTCCTCAGGC 20

(2) INFORMATION FOR SEQ ID NO:126:

(i) SEQUENCE CHARACTERISTICS:
(A) LENGTH: 23 base pairs
(B) TYPE: nucleic acid
(C) STRANDEDNESS: single
(D) TOPOLOGY: linear

(xi) SEQUENCE DESCRIPTION: SEQ ID NO:126:

ATGGCTATTT TCGGGGGCTG ACA 23

(2) INFORMATION FOR SEQ ID NO:127:

(i) SEQUENCE CHARACTERISTICS:
(A) LENGTH: 22 base pairs
(B) TYPE: nucleic acid
(C) STRANDEDNESS: single
(D) TOPOLOGY: linear

(xi) SEQUENCE DESCRIPTION: SEQ ID NO:127:

CCGGTATCTC CTCGTGGGTA TT 22

(2) INFORMATION FOR SEQ ID NO:128:

(i) SEQUENCE CHARACTERISTICS:
(A) LENGTH: 18 base pairs
(B) TYPE: nucleic acid
(C) STRANDEDNESS: single
(D) TOPOLOGY: linear

(xi) SEQUENCE DESCRIPTION: SEQ ID NO:128:

CTGCCTGAGC CACAAATG 18

(2) INFORMATION FOR SEQ ID NO:129:

(i) SEQUENCE CHARACTERISTICS:
(A) LENGTH: 24 base pairs
(B) TYPE: nucleic acid
(C) STRANDEDNESS: single
(D) TOPOLOGY: linear

(xi) SEQUENCE DESCRIPTION: SEQ ID NO:129:

CCGGAGGAGG AAGCTAGAGG AATA 24

(2) INFORMATION FOR SEQ ID NO:130:

(i) SEQUENCE CHARACTERISTICS:
(A) LENGTH: 14 base pairs
(B) TYPE: nucleic acid
(C) STRANDEDNESS: single
(D) TOPOLOGY: linear

(xi) SEQUENCE DESCRIPTION: SEQ ID NO:130:

TTTTTTTTTT TTAG 14

(2) INFORMATION FOR SEQ ID NO:131:

(i) SEQUENCE CHARACTERISTICS:
(A) LENGTH: 18 amino acids
(B) TYPE: amino acid
(C) STRANDEDNESS: single
(D) TOPOLOGY: linear

(xi) SEQUENCE DESCRIPTION: SEQ ID NO:131:

Ser Ser Gly Gly Arg Thr Phe Asp Asp Phe His Arg Tyr Leu Leu Val
1               5                   10                  15
Gly Ile

(2) INFORMATION FOR SEQ ID NO:132:

(i) SEQUENCE CHARACTERISTICS:
(A) LENGTH: 22 amino acids
(B) TYPE: amino acid
(C) STRANDEDNESS: single
(D) TOPOLOGY: linear

(xi) SEQUENCE DESCRIPTION: SEQ ID NO:132:

Gln Gly Ala Ala Gln Lys Pro Ile Asn Leu Ser Lys Xaa Ile Glu Val
1               5                   10                  15
Val Gln Gly His Asp Glu
            20

(2) INFORMATION FOR SEQ ID NO:133:

(i) SEQUENCE CHARACTERISTICS:
(A) LENGTH: 23 amino acids
(B) TYPE: amino acid

(C) STRANDEDNESS: single
(D) TOPOLOGY: linear

(xi) SEQUENCE DESCRIPTION: SEQ ID NO:133:

Ser Pro Gly Val Phe Leu Glu His Leu Gln Glu Ala Tyr Arg Ile Tyr
1               5                   10                  15
Thr Pro Phe Asp Leu Ser Ala
            20

(2) INFORMATION FOR SEQ ID NO:134:

(i) SEQUENCE CHARACTERISTICS:
(A) LENGTH: 9 amino acids
(B) TYPE: amino acid
(C) STRANDEDNESS: single
(D) TOPOLOGY: linear

(xi) SEQUENCE DESCRIPTION: SEQ ID NO:134:

Tyr Leu Leu Val Gly Ile Gln Gly Ala
1               5

(2) INFORMATION FOR SEQ ID NO:135:

(i) SEQUENCE CHARACTERISTICS:
(A) LENGTH: 9 amino acids
(B) TYPE: amino acid
(C) STRANDEDNESS: single
(D) TOPOLOGY: linear

(xi) SEQUENCE DESCRIPTION: SEQ ID NO:135:

Gly Ala Ala Gln Lys Pro Ile Asn Leu
1               5

(2) INFORMATION FOR SEQ ID NO:136:

(i) SEQUENCE CHARACTERISTICS:
(A) LENGTH: 9 amino acids
(B) TYPE: amino acid
(C) STRANDEDNESS: single
(D) TOPOLOGY: linear

(xi) SEQUENCE DESCRIPTION: SEQ ID NO:136:

Asn Leu Ser Lys Xaa Ile Glu Val Val
1               5

(2) INFORMATION FOR SEQ ID NO:137:

(i) SEQUENCE CHARACTERISTICS:
(A) LENGTH: 9 amino acids
(B) TYPE: amino acid
(C) STRANDEDNESS: single
(D) TOPOLOGY: linear

(xi) SEQUENCE DESCRIPTION: SEQ ID NO:137:

Glu Val Val Gln Gly His Asp Glu Ser
1               5

(2) INFORMATION FOR SEQ ID NO:138:

(i) SEQUENCE CHARACTERISTICS:
(A) LENGTH: 9 amino acids
(B) TYPE: amino acid
(C) STRANDEDNESS: single
(D) TOPOLOGY: linear

(xi) SEQUENCE DESCRIPTION: SEQ ID NO:138:

His Leu Gln Glu Ala Tyr Arg Ile Tyr
1               5

(2) INFORMATION FOR SEQ ID NO:139:

(i) SEQUENCE CHARACTERISTICS:
(A) LENGTH: 9 amino acids
(B) TYPE: amino acid
(C) STRANDEDNESS: single
(D) TOPOLOGY: linear

(xi) SEQUENCE DESCRIPTION: SEQ ID NO:139:

Asn Leu Ala Phe Val Ala Gln Ala Ala
1               5

(2) INFORMATION FOR SEQ ID NO:140:

(i) SEQUENCE CHARACTERISTICS:
(A) LENGTH: 9 amino acids
(B) TYPE: amino acid
(C) STRANDEDNESS: single
(D) TOPOLOGY: linear

(xi) SEQUENCE DESCRIPTION: SEQ ID NO:140:

Phe Val Ala Gln Ala Ala Pro Asp Ser
1               5

(2) INFORMATION FOR SEQ ID NO:141:

(i) SEQUENCE CHARACTERISTICS:
(A) LENGTH: 9388 base pairs
(B) TYPE: nucleic acid
(C) STRANDEDNESS: single
(D) TOPOLOGY: linear

(xi) SEQUENCE DESCRIPTION: SEQ ID NO:141:

GCTCGCGGCC GCGAGCTCAA TTAACCCTCA CTAAAGGGAG TCGACTCGAT CAGACTGTTA 60
CTGTGTCTAT GTAGAAAGAA GTAGACATAA GAGATTCCAT TTTGTTCTGT ACTAAGAAAA 120
ATTCTTCTGC CTTGAGATGC TGTTAATCTG TAACCCTAGC CCCAACCCTG TGCTCACAGA 180
GACATGTGCT GTGTTGACTC AAGGTTCAAT GGATTTAGGG CTATGCTTTG TTAAAAAAGT 240
GCTTGAAGAT AATATGCTTG TTAAAAGTCA TCACCATTCT CTAATCTCAA GTACCCAGGG 300
ACACAATACA CTGCGGAAGG CCGCAGGGAC CTCTGTCTAG GAAAGCCAGG TATTGTCCAA 360
GATTTCTCCC CATGTGATAG CCTGAGATAT GGCCTCATGG GAAGGGTAAG ACCTGACTGT 420
CCCCCAGCCC GACATCCCCC AGCCCGACAT CCCCCAGCCC GACACCCGAA AAGGGTCTGT 480
GCTGAGGAGG ATTAGTAAAA GAGGAAGGCC TCTTTGCAGT TGAGGTAAGA GGAAGGCATC 540
TGTCTCCTGC TCGTCCCTGG GCAATAGAAT GTCTTGGTGT AAAACCCGAT TGTATGTTCT 600
ACTTACTGAG ATAGGAGAAA ACATCCTTAG GGCTGGAGGT GAGACACGCT GGCGGCAATA 660
CTGCTCTTTA ATGCACCGAG ATGTTTGTAT AAGTGCACAT CAAGGCACAG CACCTTTCCT 720
TAAACTTATT TATGACACAG AGACCTTTGT TCACGTTTTC CTGCTGACCC TCTCCCCACT 780
ATTACCCTAT TGGCCTGCCA CATCCCCCTC TCCGAGATGG TAGAGATAAT GATCAATAAA 840
TACTGAGGGA ACTCAGAGAC CAGTGTCCCT GTAGGTCCTC CGTGTGCTGA GCGCCGGTCC 900
CTTGGGCTCA CTTTTCTTTC TCTATACTTT GTCTCTGTGT CTCTTTCTTT TCTCAGTCTC 960
TCGTTCCACC TGACGAGAAA TACCCACAGG TGTGGAGGGG CAGGCCACCC CTTCAATAAT 1020
TTACTAGCCT GTTCGCTGAC AACAAGACTG GTGGTGCAGA AGGTTGGGTC TTGGTGTTCA 1080
CCGGGTGGCA GGCATGGGCC AGGTGGGAGG GTCTCCAGCG CCTGGTGCAA ATCTCCAAGA 1140
AAGTGCAGGA AACAGCACCA AGGGTGATTG TAAATTTTGA TTTGGCGCGG CAGGTAGCCA 1200
TTCCAGCGCA AAAATGCGCA GGAAAGCTTT TGCTGTGCTT GTAGGCAGGT AGGCCCCAAG 1260
CACTTCTTAT TGGCTAATGT GGAGGGAACC TGCACATCCA TTGGCTGAAA TCTCCGTCTA 1320
TTTGAGGCTG ACTGAGCGCG TTCCTTTCTT CTGTGTTGCC TGGAAACGGA CTGTCTGCCT 1380
AGTAACATCT GATCACGTTT CCCATTGGCC GCCGTTTCCG GAAGCCCGCC CTCCCATTTC 1440
CGGAAGCCTG GCGCAAGGTT GGTCTGCAGG TGGCCTCCAG GTGCAAAGTG GGAAGTGTGA 1500
GTCCTCAGTC TTGGGCTATT CGGCCACGTG CCTGCCGGAC ATGGGACGCT GGAGGGTCAG 1560
CAGCGTGGAG TCCTGGCCTT TTGCGTCCAC GGGTGGGAAA TTGGCCATTG CCACGGCGGG 1620
AACTGGGACT CAGGCTGCCC CCCGGCCGTT TCTCATCCGT CCACCGGACT CGTGGGCGCT 1680
CGCACTGGCG CTGATGTAGT TTCCTGACCT CTGACCCGTA TTGTCTCCAG ATTAAAGGTA 1740
AAAACGGGGC TTTTTCAGCC CACTCGGGTA AAACGCCTTT TGATTTCTAG GCAGGTGTTT 1800
TGTTGCACGC CTGGGAGGGA GTGACCCGCA GGTTGAGGTT TATTAAAATA CATTCCTGGT 1860
TTATGTTATG TTTATAATAA AGCACCCCAA CCTTTACAAA ATCTCACTTT TTGCCAGTTG 1920
TATTATTTAG TGGACTGTCT CTGATAAGGA CAGCCAGTTA AAATGGAATT TTGTTGTTGC 1980
TAATTAAACC AATTTTTAGT TTTGGTGTTT GTCCTAATAG CAACAACTTC TCAGGCTTTA 2040
TAAAACCATA TTTCTTGGGG GAAATTTCTG TGTAAGGCAC AGCGAGTTAG TTTGGAATTG 2100
TTTTAAAGGA AGTAAGTTCC TGGTTTTGAT ATCTTAGTAG TGTAATGCCC AACCTGGTTT 2160
TTACTAACCC TGTTTTTAGA CTCTCCCTTT CCTTAAATCA CCTAGCCTTG TTTCCACCTG 2220
AATTGACTCT CCCTTAGCTA AGAGCGCCAG ATGGACTCCA TCTTGGCTCT TTCACTGGCA 2280
GCCCCTTCCT CAAGGACTTA ACTTGTGCAA GCTGACTCCC AGCACATCCA AGAATGCAAT 2340
TAACTGTTAA GATACTGTGG CAAGCTATAT CCGCAGTTCC GAGGAATTCA TCCGATTGAT 2400
TATGCCCAAA AGCCCCGCGT CTATCACCTT GTAATAATCT TAAAGCCCCT GCACCTGGAA 2460
CTATTAACTT TCCTGTAACC ATTTATCCTT TTAACTTTTT TGCTTACTTT ATTTCTGTAA 2520
AATTGTTTTA ACTAGACCTC CCCTCCCCTT TCTAAACCAA AGTATAAAAG AAGATCTAGC 2580
CCCTTCTTCA GAGCGGAGAG AATTTTGAGC ATTAGCCATC TCTTGGCGGC CAGCTAAATA 2640
AATGGACTTT TAATTTGTCT CAAAGTGTGG CGTTTTCTCT AACTCGCTCA GGTACGACAT 2700

TTGGAGGCCC CAGCGAGAAA CGTCACCGGG AGAAACGTCA CCGGGCGAGA GCCGGGCCCG 2760
CTGTGTGCTC CCCCGGAAGG ACAGCCAGCT TGTAGGGGGG AGTGCCACCT GAAAAAAAAA 2820
TTTCCAGGTC CCCAAAGGGT GACCGTCTTC CGGAGGACAG CGGATCGACT ACCATGCGGG 2880
TGCCCACCAA AATTCCACCT CTGAGTCCTC AACTGCTGAC CCCGGGGTCA GGTAGGTCAG 2940
ATTTGACTTT GGTTCTGGCA GAGGGAAGCG ACCCTGATGA GGGTGTCCCT CTTTTGACTC 3000
TGCCCATTTC TCTAGGATGC TAGAGGGTAG AGCCCTGGTT TTCTGTTAGA CGCCTCTGTG 3060
TCTCTGTCTG GGAGGGAAGT GGCCCTGACA GGGGCCATCC CTTGAGTCAG TCCACATCCC 3120
AGGATGCTGG GGGACTGAGT CCTGGTTTCT GGCAGACTGG TCTCTCTCTC TCTCTTTTTC 3180
TATCTCTAAT CTTTCCTTGT TCAGGTTTCT TGGAGAATCT CTGGGAAAGA AAAAAGAAAA 3240
ACTGTTATAA ACTCTGTGTG AATGGTGAAT GAATGGGGGA GGACAAGGGC TTGCGCTTGT 3300
CCTCCAGTTT GTAGCTCCAC GGCGAAAGCT ACGGAGTTCA AGTGGGCCCT CACCTGCGGT 3360
TCCGTGGCGA CCTCATAAGG CTTAAGGCAG CATCCGGCAT AGCTCGATCC GAGCCGGGGG 3420
TTTATACCGG CCTGTCAATG CTAAGAGGAG CCCAAGTCCC CTAAGGGGGA GCGGCCAGGC 3480
GGGCATCTGA CTGATCCCAT CACGGGACCC CCTCCCCTTG TTTGTCTAAA AAAAAAAAAA 3540
GAAGAAACTG TCATAACTGT TTACATGCCC TAGGGTCAAC TGTTTGTTTT ATGTTTATTG 3600
TTCTGTTCGG TGTCTATTGT CTTGTTTAGT GGTTGTCAAG GTTTTGCATG TCAGGACGTC 3660
GATATTGCCC AAGACGTCTG GGTAAGAACT TCTGCAAGGT CCTTAGTGCT GATTTTTTGT 3720
CACAGGAGGT TAAATTTCTC ATCAATCATT TAGGCTGGCC ACCACAGTCC TGTCTTTTCT 3780
GCCAGAAGCA AGTCAGGTGT TGTTACGGGA ATGAGTGTAA AAAAACATTC GCCTGATTGG 3840
GATTTCTGGC ACCATGATGG TTGTATTTAG ATTGTCATAC CCCACATCCA GGTTGATTGG 3900
ACCTCCTCTA AACTAAACTG GTGGTGGGTT CAAAACAGCC ACCCTGCAGA TTTCCTTGCT 3960
CACCTCTTTG GTCATTCTGT AACTTTTCCT GTGCCCTTAA ATAGCACACT GTGTAGGGAA 4020
ACCTACCCTC GTACTGCTTT ACTTCGTTTA GATTCTTACT CTGTTCCTCT GTGGCTACTC 4080
TCCCATCTTA AAAACGATCC AAGTGGTCCT TTTCCTCCTC CCTGCCCCCT ACCCCACACA 4140
TCTCGTTTTC CAGTGCGACA GCAAGTTCAG CGTCTCCAGG ACTTGGCTCT GCTCTCACTC 4200
CTTGAACCCT TAAAAGAAAA AGCTGGGTTT GAGCTATTTG CCTTTGAGTC ATGGAGACAC 4260
AAAAGGTATT TAGGGTACAG ATCTAGAAGA AGAGAGAGAA CACCTAGATC CAACTGACCC 4320
AGGAGATCTC GGGCTGGCCT CTAGTCCTCC TCCCTCAATC TTAAAGCTAC AGTGATGTGG 4380
CAAGTGGTAT TTAGCTGTTG TGGTTTTTCT GCTCTTTCTG GTCATGTTGA TTCTGTTCTT 4440
TCGATACTCC AGCCCCCCAG GGAGTGAGTT TCTCTGTCTG TGCTGGGTTT GATATCTATG 4500
TTCAAATCTT ATTAAATTGC CTTCAAAAAA AAAAAAAAAA GGGAAACACT TCCTCCCAGC 4560
CTTGTAAGGG TTGGAGCCCT CTCCAGTATA TGCTGCAGAA TTTTTCTCTC GGTTTCTCAG 4620
AGGATTATGG AGTCCGCCTT AAAAAAGGCA AGCTCTGGAC ACTCTGCAAA GTAGAATGGC 4680
CAAAGTTTGG AGTTGAGTGG CCCCTTGAAG GGTCACTGAA CCTCACAATT GTTCAAGCTG 4740
TGTGGCGGGT TGTTACTGAA ACTCCCGGCC TCCCTGATCA GTTTCCCTAC ATTGATCAAT 4800
GGCTGAGTTT GGTCAGGAGC ACCCCTTCCA TGGCTCCACT CATGCACCAT TCATAATTTT 4860
ACCTCCAAGG TCCTCCTGAG CCAGACCGTG TTTTCGCCTC GACCCTCAGC CGGTTCAGCT 4920
CGCCCTGTAC TGCCTCTCTC TGAAGAAGAG GAGAGTCTCC CTCACCCAGT CCCACCGCCT 4980
TAAAACCAGC CTACTCCCTT AGGGTCATCC CATGTCTCCT CGGCTATGTC CCCTGTAGGC 5040
TCATCACCCA TTGCCTCTTG GTTGCAACCG TGGTGGGAGG AAGTAGCCCC TCTACTACCA 5100
CTGAGAGAGG CACAAGTCCC TCTGGGTGAT GAGTGCTCCA CCCCCTTCCT GGTTTATGTC 5160
CCTTCTTTCT ACTTCTGACT TGTATAATTG GAAAACCCAT AATCCTCCCT TCTCTGAAAA 5220
GCCCCAGGCT TTGACCTCAC TGATGGAGTC TGTACTCTGG ACACATTGGC CCACCTGGGA 5280
TGACTGTCAA CAGCTCCTTT TGACCCTTTT CACCTCTGAA GAGAGGGAAA GTATCCAAAG 5340
AGAGGCCAAA AAGTACAACC TCACATCAAC CAATAGGCCG GAGGAGGAAG CTAGAGGAAT 5400
AGTGATTAGA GACCCAATTG GGACCTAATT GGGACCCAAA TTTCTCAAGT GGAGGGAGAA 5460
CTTTTGACGA TTTCCACCGG TATCTCCTCG TGGGTATTCA GGGAGCTGCT CAGAAACCTA 5520
TAAACTTGTC TAAGGCGACT GAAGTCGTCC AGGGGCATGA TGAGTCACCA GGAGTGTTTT 5580
TAGAGCACCT CCAGGAGGCT TATCGGATTT ACACCCCTTT TGACCTGGCA GCCCCCGAAA 5640
ATAGCCATGC TCTTAATTTG GCATTTGTGG CTCAGGCAGC CCCAGATAGT AAAAGGAAAC 5700
TCCAAAAACT AGAGGGATTT TGCTGGAATG AATACCAGTC AGCTTTTAGA GATAGCCTAA 5760
AAGGTTTTTG ACAGTCAAGA GGTTGAAAAA CAAAAACAAG CAGCTCAGGC AGCTGAAAAA 5820
AGCCACTGAT AAAGCATCCT GGAGTATCAG AGTTTACTGT TAGATCAGCC TCATTTGACT 5880
TCCCCTCCCA CATGGTGTTT AAATCCAGCT ACACTACTTC CTGACTCAAA CTCCACTATT 5940
CCTGTTCATG ACTGTCAGGA ACTGTTGGAA ACTACTGAAA CTGGCCGACC TGATCTTCAA 6000

AATGTGCCCC TAGGAAAGGT GGATGCCACC GTGTTCACAG ACAGTAGCAG CTTCCTCGAG 6060
AAGGGACTAC GAAAGGCCGG TGCAGCTGTT ACCATGGAGA CAGATGTGTT GTGGGCTCAG 6120
GCTTTACCAG CAAACACCTC AGCACAAAAG GCTGAATTGA TCGCCCTCAC TCAGGCTCTC 6180
CGATGGGGTA AGGATATTAA CGTTAACACT GACAGCAGGT ACGCCTTTGC TACTGTGCAT 6240
GTACGTGGAG CCATCTACCA GGAGCGTGGG CTACTCACCT CAGCAGGTGG CTGTAATCCA 6300
CTGTAAAGGA CATCAAAAGG AAAACACGGC TGTTGCCCGT GGTAACCAGA AAGCTGATTC 6360
AGCAGCTCAA GATGCAGTGT GACTTTCAGT CACGCCTCTA AACTTGCTGC CCACAGTCTC 6420
CTTTCCACAG CCAGATCTGC CTGACAATCC CGCATACTCA ACAGAAGAAG AAAACTGGCC 6480
TCAGAACTCA GAGCCAATAA AAATCAGGAA GGTTGGTGGA TTCTTCCTGA CTCTAGAATC 6540
TTCATACCCC GAACTCTTGG GAAAACTTTA ATCAGTCACC TACAGTCTAC CACCCATTTA 6600
GGAGGAGCAA AGCTACCTCA GCTCCTCCGG AGCCGTTTTA AGATCCCCCA TCTTCAAAGC 6660
CTAACAGATC AAGCAGCTCT CCGGTGCACA ACCTGCGCCC AGGTAAATGC CAAAAAAGGT 6720
CCTAAACCCA GCCCAGGCCA CCGTCTCCAA GAAAACTCAC CAGGAGAAAA GTGGGAAATT 6780
GACTTTACAG AAGTAAAACC ACACCGGGCT GGGTACAAAT ACCTTCTAGT ACTGGTAGAC 6840
ACCTTCTCTG GATGGACTGA AGCATTTGCT ACCAAAAACG AAACTGTCAA TATGGTAGTT 6900
AAGTTTTTAC TCAATGAAAT CATCCCTCGA CGTGGGCTGC CTGTTGCCAT AGGGTCTGAT 6960
AATGGACCGG CCTTCGCCTT GTCTATAGTT TAGTCAGTCA GTAAGGCGTT AAACATTCAA 7020
TGGAAGCTCC ATTGTGCCTA TCGACCCCAG AGCTCTGGGC AAGTAGAACG CATGAACTGC 7080
ACCCTAAAAA ACACTCTTAC AAAATTAATC TTAGAAACCG GTGTAAATTG TGTAAGTCTC 7140
CTTCCTTTAG CCCTACTTAG AGTAAGGTGC ACCCCTTACT GGGCTGGGTT CTTACCTTTT 7200
GAAATCATGT ATGGGAGGGC GCTGCCTATC TTGCCTAAGC TAAGAGATGC CCAATTGGCA 7260
AAAATATCAC AAACTAATTT ATTACAGTAC CTACAGTCTC CCCAACAGGT ACAAGATATC 7320
ATCCTGCCAC TTGTTCGAGG AACCCATCCC AATCCAATTC CTGAACAGAC AGGGCCCTGC 7380
CATTCATTCC CGCCAGGTGA CCTGTTGTTT GTTAAAAAGT TCCAGAGAGA AGGACTCCCT 7440
CCTGCTTGGA AGAGACCTCA CACCGTCATC ACGATGCCAA CGGCTCTGAA GGTGGATGGC 7500
ATTCCTGCGT GGATTCATCA CTCCCGCATC AAAAAGGCCA ACGGAGCCCA ACTAGAAACA 7560
TGGGTCCCCA GGGCTGGGTC AGGCCCCTTA AAACTGCACC TAAGTTGGGT GAAGCCATTA 7620
GATTAATTCT TTTTCTTAAT TTTGTAAAAC AATGCATAGC TTCTGTCAAA CTTATGTATC 7680
TTAAGACTCA ATATAACCCC CTTGTTATAA CTGAGGAATC AATGATTTGA TTCCCCAAAA 7740
ACACAAGTGG GGAATGTAGT GTCCAACCTG GTTTTTACTA ACCCTGTTTT TAGACTCTCC 7800
CTTTCCTTTA ATCACTCAGC CTTGTTTCCA CCTGAATTGA CTCTCCCTTA GCTAAGAGCG 7860
CCAGATGGAC TCCATCTTGG CTCTTTCACT GGCAGCCGCT TCCTCAAGGA CTTAACTTGT 7920
GCAAGCTGAC TCCCAGCACA TCCAAGAATG CAATTAACTG ATAAGATACT GTGGCAAGCT 7980
ATATCCGCAG TTCCCAGGAA TTCGTCCAAT TGATTACACC CAAAAGCCCC GCGTCTATCA 8040
CCTTGTAATA ATCTTAAAGC CCCTGCACCT GGAACTATTA ACGTTCCTGT AACCATTTAT 8100
CCTTTTAACT TTTTTGCCTA CTTTATTTCT GTAAAATTGT TTTAACTAGA CCCCCCCTCT 8160
CCTTTCTAAA CCAAAGTATA AAAGCAAATC TAGCCCCTTC TTCAGGCCGA GAGAATTTCG 8220
AGCGTTAGCC GTCTCTTGGC CACCAGCTAA ATAAACGGAT TCTTCATGTG TCTCAAAGTG 8280
TGGCGTTTTC TCTAACTCGC TCAGGTACGA CCGTGGTAGT ATTTTCCCCA ACGTCTTATT 8340
TTTAGGGCAC GTATGTAGAG TAACTTTTAT GAAAGAAACC AGTTAAGGAG GTTTTGGGAT 8400
TTCCTTTATC AACTGTAATA CTGGTTTTGA TTATTTATTT ATTTATTTAT TTTTTTTGAG 8460
AAGGAGTTTC ACTCTTGTTG CCCAGGCTGG AGTGCAATGG TGCGATCTTG GCTCACTGCA 8520
ACTTCCGCCT CCCAGGTTCA AGCGATTCTC CTGCCTCAGC CTCGAGAGTA GCTGGGATTA 8580
TAGGCATGCG CCACCACACC CAGCTAATTT TGTATTTTTA GTAAAGATGG GGTTTCTTCA 8640
TGTTGGTCAA GCTGGTCTGG AACTCCCCGC CTCGGGTGAT CTGCCCGCCT CGGCCTCCGA 8700
AAGTGCTGGG ATTACAGGTG TGATCCACCA CACCCAGCCG ATTTATATGT ATATAAATCA 8760
CATTCCTCTA ACCAAAATGT AGTGTTTCCT TCCATCTTGA ATATAGGCTG TAGACCCCGT 8820
GGGTATGGGA CATTGTTAAC AGTGAGACCA CAGCAGTTTT TATGTCATCT GACAGCATCT 8880
CCAAATAGCC TTCATGGTTG TCACTGCTTC CCAAGACAAT TCCAAATAAC ACTTCCCAGT 8940
GATGACTTGC TACTTGCTAT TGTTACTTAA TGTGTTAAGG TGGCTGTTAC AGACACTATT 9000
AGTATGTCAG GAATTACACC AAAATTTAGT GGCTCAAACA ATCATTTTAT TATGTATGTG 9060
GATTCTCATG GTCAGGTCAG GATTTCAGAC AGGGCACAAG GGTAGCCCAC TTGTCTCTGT 9120
CTATGATGTC TGGCCTCAGC ACAGGAGACT CAACAGCTGG GGTCTGGGAC CATTTGGAGG 9180
CTTGTTCCCT CACATCTGAT ACCTGGCTTG GGATGTTGGA AGAGGGGGTG AGCTGAGACT 9240
GAGTGCCTAT ATGTAGTGTT TCCATATGGC CTTGACTTCC TTACAGCCTG GCAGCCTCAG 9300

GGTAGTCAGA ATTCTTAGGA GGCACAGGGC TCCAGGGCAG ATGCTGAGGG GTCTTTTATG 9360
AGGTAGCACA GCAAATCCAC CCAGGATC 9388

(2) INFORMATION FOR SEQ ID NO:142:

(i) SEQUENCE CHARACTERISTICS:
(A) LENGTH: 419 base pairs
(B) TYPE: nucleic acid
(C) STRANDEDNESS: single
(D) TOPOLOGY: linear

(xi) SEQUENCE DESCRIPTION: SEQ ID NO:142:

TGTAAGTCGA GCAGTGTGAT GGAAGGAATG GTCTTTGGAG AGAGCATATC CATCTCCTCC 60
TCACTGCCTC CTAATGTCAT GAGGTACACT GAGCAGAATT AAACAGGGTA GTCTTAACCA 120
CACTATTTTT AGCTACCTTG TCAAGCTAAT GGTTAAAGAA CACTTTTGGT TTACACTTGT 180
TGGGTCATAG AAGTTGCTTT CCGCCATCAC GCAATAAGTT TGTGTGTAAT CAGAAGGAGT 240
TACCTTATGG TTTCAGTGTC ATTCTTTAGT TAACTTGGGA GCTGTGTAAT TTAGGCTTTG 300
CGTATTATTT CACTTCTGTT CTCCACTTAT GAAGTGATTG TGTGTTCGCG TGTGTGTGCG 360
TGCGCATGTG CTTCCGGCAG TTAACATAAG CAAATACCCA ACATCACACT GCTCGACTT 419

(2) INFORMATION FOR SEQ ID NO:143:

(i) SEQUENCE CHARACTERISTICS:
(A) LENGTH: 402 base pairs
(B) TYPE: nucleic acid
(C) STRANDEDNESS: single
(D) TOPOLOGY: linear

(xi) SEQUENCE DESCRIPTION: SEQ ID NO:143:

TGTAAGTCGA GCAGTGTGAT GTCCACTGCA GTGTGTTGCT GGGAACAGTT AATGAGCAAA 60
TTGTATACAA TGGCTAGTAC ATTGACCGGG ATTTGTTGAA GCTGGTGAGT GTTATGACTT 120
AGCCTGTTAG ACTAGTCTAT GCACATGGCT CTGGTCAACT ACCGCTCTCT CATTTCTCCA 180
GATAAATCCC CCATGCTTTA TATTCTCTTC CAAACATACT ATCCTCATCA CCACATAGTT 240
CCTTTGTTAA TGCTTTGTTC TAGACTTTCC CTTTTCTGTT TTCTTATTCA AACCTATATC 300
TCTTTGCATA GATTGTAAAT TCAAATGCCC TCAGGGTGCA GGCAGTTCAT GTAAGGGAGG 360
GAGGCTAGCC AGTGAGATCT GCATCACACT GCTCGACTTA CA 402

(2) INFORMATION FOR SEQ ID NO:144:

(i) SEQUENCE CHARACTERISTICS:
(A) LENGTH: 224 base pairs
(B) TYPE: nucleic acid
(C) STRANDEDNESS: single
(D) TOPOLOGY: linear

(xi) SEQUENCE DESCRIPTION: SEQ ID NO:144:

TCGGGTGATG CCTCCTCAGG CCAAGAAGAT AAAGCTTCAG ACCCCTAACA CATTTCCAAA 60
AAGGAAGAAA GGAGAAAAAA GGGCATCATC CCCGTTCCGA AGGGTCAGGG AGGAGGAAAT 120
TGAGGTGGAT TCACGAGTTG CGGACAACTC CTTTGATGCC AAGCGAGGTG CAGCCGGAGA 180

CTGGGGAGAG CGAGCCAATC AGGTTTTGAA GTTCCTCTCA GTGC 224

(2) INFORMATION FOR SEQ ID NO:145:

(i) SEQUENCE CHARACTERISTICS:
(A) LENGTH: 111 base pairs
(B) TYPE: nucleic acid
(C) STRANDEDNESS: single
(D) TOPOLOGY: linear

(xi) SEQUENCE DESCRIPTION: SEQ ID NO:145:

AGCCATTTAC CACCCATCCA CAAAAAAAAA AAAAAAAAAG AAAAATATCA AGGAATAAAA 60
ATAGACTTTG AACAAAAAGG AACATTTGCT GGCCTGAGGA GGCATCACCC G 111

(2) INFORMATION FOR SEQ ID NO:146:

(i) SEQUENCE CHARACTERISTICS:
(A) LENGTH: 585 base pairs
(B) TYPE: nucleic acid
(C) STRANDEDNESS: single
(D) TOPOLOGY: linear

(xi) SEQUENCE DESCRIPTION: SEQ ID NO:146:

TAGCATGTTG AGCCCAGACA CTTGTAGAGA GAGGAGGACA GTTAGAAGAA GAAGAAAAGT 60
TTTTAAATGC TGAAAGTTAC TATAAGAAAG CTTTGGCTTT GGATGAGACT TTTAAAGATG 120
CAGAGGATGC TTTGCAGAAA CTTCATAAAT ATATGCAGGT GATTCCTTAT TTCCTCCTAG 180
AAATTTAGTG ATATTTGAAA TAATGCCCAA ACTTAATTTT CTCCTGAGGA AAACTATTCT 240
ACATTACTTA AGTAAGGCAT TATGAAAAGT TTCTTTTTAG GTATAGTTTT TCCTAATTGG 300
GTTTGACATT GCTTCATAGT GCCTCTGTTT TTGTCCATAA TCGAAAGTAA AGATAGCTGT 360
GAGAAAACTA TTACCTAAAT TTGGTATGTT GTTTTGAGAA ATGTCCTTAT AGGGAGCTCA 420
CCTGGTGGTT TTTAAATTAT TGTTGCTACT ATAATTGAGC TAATTATAAA AACCTTTTTG 480
AGACATATTT TAAATTGTCT TTTCCTGTAA TACTGATGAT GATGTTTTCT CATGCATTTT 540
CTTCTGAATT GGGACCATTG CTGCTGTGTC TGGGCTCACA TGCTA 585

(2) INFORMATION FOR SEQ ID NO:147:

(i) SEQUENCE CHARACTERISTICS:
(A) LENGTH: 579 base pairs
(B) TYPE: nucleic acid
(C) STRANDEDNESS: single
(D) TOPOLOGY: linear

(xi) SEQUENCE DESCRIPTION: SEQ ID NO:147:

TAGCATGTTG AGCCCAGACA CTGGGCAGCG GGGGTGGCCA CGGCAGCTCC TGCCGAGCCC 60
AAGCGTGTTT GTCTGTGAAG GACCCTGACG TCACCTGCCA GGCTAGGGAG GGGTCAATGT 120
GGAGTGAATG TTCACCGACT TTCGCAGGAG TGTGCAGAAG CCAGGTGCAA CTTGGTTTGC 180
TTGTGTTCAT CACCCCTCAA GATATGCACA CTGCTTTCCA AATAAAGCAT CAACTGTCAT 240
CTCCAGATGG GGAAGACTTT TTCTCCAACC AGCAGGCAGG TCCCCATCCA CTCAGACACC 300
AGCACGTCCA CCTTCTCGGG CAGCACCACG TCCTCCACCT TCTGCTGGTA CACGGTGATG 360

ATGTCAGCAA AGCCGTTCTG CANGACCAGC TGCCCCGTGT GCTGTGCCAT CTCACTGGCC 420
TCCACCGCGT ACACCGCTCT AGGCCGCGCA TANTGTGCAC AGAANAAATG ATGATCCAGT 480
CCCACAGCCC ACGTCCAAGA NGACTTTATC CGTCAGGGAT TCTTTATTCT GCAGGATGAC 540
CTGTGGTATT AATTGTTCGT GTCTGGGCTC AACATGCTA 579

(2) INFORMATION FOR SEQ ID NO:148:

(i) SEQUENCE CHARACTERISTICS:
(A) LENGTH: 249 base pairs
(B) TYPE: nucleic acid
(C) STRANDEDNESS: single
(D) TOPOLOGY: linear

(xi) SEQUENCE DESCRIPTION: SEQ ID NO:148:

TGACACCTTG TCCAGCATCT GCAAGCCAGG AAGAGAGTCC TCACCAAGAT CCCCACCCCG 60
TTGGCACCAG GATCTTGGAC TTCCAATCTC CAGAACTGTG AGAAATAAGT ATTTGTCGCT 120
AAATAAATCT TTGTGGTTTC AGATATTTAG CTATAGCAGA TCAGGCTGAC TAAGAGAAAC 180
CCCATAAGAG TTACATACTC ATTAATCTCC GTCTCTATCC CCAGGTCTCA GATGCTGGAC 240
AAGGTGTCA 249

(2) INFORMATION FOR SEQ ID NO:149:

(i) SEQUENCE CHARACTERISTICS:
(A) LENGTH: 255 base pairs
(B) TYPE: nucleic acid
(C) STRANDEDNESS: single
(D) TOPOLOGY: linear

(xi) SEQUENCE DESCRIPTION: SEQ ID NO:149:

TGACACCTTG TCCAGCATCT GCTATTTTGT GACTTTTTAA TAATAGCCAT TCTGACTGGT 60
GTGAGATGGT AACTCATTGT GGGTTTGGTC TGCATTTCTC TAATGATCAG TGATATTAAG 120
CTTTTTTTAA ATATGCTTGT TGACCACATG TATATCATCT TTTGAGAAGT GTCTGTTCAT 180
ATCCTTTGCC CACTTTTTAA TTTTTTTATC TTGTAAATTT GTTTAATTTC CTTACAGATG 240
CTGGACAAGG TGTCA 255

(2) INFORMATION FOR SEQ ID NO:150:

(i) SEQUENCE CHARACTERISTICS:
(A) LENGTH: 318 base pairs
(B) TYPE: nucleic acid
(C) STRANDEDNESS: single
(D) TOPOLOGY: linear

(xi) SEQUENCE DESCRIPTION: SEQ ID NO:150:

TTACGCTGCA ACACTGTGGA GGCCAAGCTG GGATCACTTC TTCATTCTAA CTGGAGAGGA 60
GGGAAGTTCA AGTCCAGCAG AGGGTGGGTG GGTAGACAGT GGCACTCAGA AATGTCAGCT 120
GGACCCCTGT CCCCGCATAG GCAGGACAGC AAGGCTGTGG CTCTCCAGGG CCAGCTGAAG 180
AACAGGACAC TGTCTCCGCT GCCACAAAGC GTCAGAGACT CCCATCTTTG AAGCACGGCC 240
TTCTTGGTCT TCCTGCACTT CCCTGTTCTG TTAGAGACCT GGTTATAGAC AAGGCTTCTC 300

CACAGTGTTG CAGCGTAA 318

(2) INFORMATION FOR SEQ ID NO:151:

(i) SEQUENCE CHARACTERISTICS:
(A) LENGTH: 323 base pairs
(B) TYPE: nucleic acid
(C) STRANDEDNESS: single
(D) TOPOLOGY: linear

(xi) SEQUENCE DESCRIPTION: SEQ ID NO:151:

TNACGCNGCN ACNNTGTAGA GANGGNAAGG CNTTCCCCAC ATTNCCCCTT CATNANAGAA 60
TTATTCNACC AAGNNTGACC NATGCCNTTT ATGACTTACA TGCNNACTNC NTAATCTGTN 120
TCNNGCCTTA AAAGCNNNTC CACTACATGC NTCANCACTG TNTGTGTNAC NTCATNAACT 180
GTCNGNAATA GGGGCNCATA ACTACAGAAA TGCANTTCAT ACTGCTTCCA NTGCCATCNG 240
CGTGTGGCCT TNCCTACTCT TCTTNTATTC CAAGTAGCAT CTCTGGANTG CTTCCCCACT 300
CTCCACATTG TTGCAGCNAT AAT 323

(2) INFORMATION FOR SEQ ID NO:152:

(i) SEQUENCE CHARACTERISTICS:
(A) LENGTH: 311 base pairs
(B) TYPE: nucleic acid
(C) STRANDEDNESS: single
(D) TOPOLOGY: linear

(xi) SEQUENCE DESCRIPTION: SEQ ID NO:152:

TCAAGATTCC ATAGGCTGAC CAGTCCAAGG AGAGTTGAAA TCATGAAGGA GAGTCTATCT 60
GGAGAGAGCT GTAGTTTTGA GGGTTGCAAA GACTTAGGAT GGAGTTGGTG GGTGTGGTTA 120
GTCTCTAAGG TTGATTTTGT TCATAAATTT CATGCCCTGA ATGCCTTGCT TGCCTCACCC 180
TGGTCCAAGC CTTAGTGAAC ACCTAAAAGT CTCTGTCTTC TTGCTCTCCA AACTTCTCCT 240
GAGGATTTCC TCAGATTGTC TACATTCAGA TCGAAGCCAG TTGGCAAACA AGATGCAGTC 300
CAGAGGGTCA G 311

(2) INFORMATION FOR SEQ ID NO:153:

(i) SEQUENCE CHARACTERISTICS:
(A) LENGTH: 332 base pairs
(B) TYPE: nucleic acid
(C) STRANDEDNESS: single
(D) TOPOLOGY: linear

(xi) SEQUENCE DESCRIPTION: SEQ ID NO:153:

CAAGATTCCA TAGGCTGACC AGGAGGCTAT TCAAGATCTC TGGCAGTTGA GGAAGTCTCT 60
TTAAGAAAAT AGTTTAAACA ATTTGTTAAA ATTTTTCTGT CTTACTTCAT TTCTGTAGCA 120
GTTGATATCT GGCTGTCCTT TTTATAATGC AGAGTGGGAA CTTTCCCTAC CATGTTTGAT 180
AAATGTTGTC CAGGCTCCAT TGCCAATAAT GTGTTGTCCA AAATGCCTGT TTAGTTTTTA 240
AAGACGGAAC TCCACCCTTT GCTTGGTCTT AAGTATGTAT GGAATGTTAT GATAGGACAT 300
AGTAGTAGCG GTGGTCAGCC TATGGAATCT TG 332

(2) INFORMATION FOR SEQ ID NO:154:

(i) SEQUENCE CHARACTERISTICS:
(A) LENGTH: 345 base pairs
(B) TYPE: nucleic acid
(C) STRANDEDNESS: single
(D) TOPOLOGY: linear

(xi) SEQUENCE DESCRIPTION: SEQ ID NO:154:

TCAAGATTCC ATAGGCTGAC CTGGACAGAG ATCTCCTGGG TCTGGCCCAG GACAGCAGGC 60
TCAAGCTCAG TGGAGAAGGT TTCCATGACC CTCAGATTCC CCCAAACCTT GGATTGGGTG 120
ACATTGCATC TCCTCAGAGA GGGAGGAGAT GTANGTCTGG GCTTCCACAG GGACCTGGTA 180
TTTTAGGATC AGGGTACCGC TGGCCTGAGG CTTGGATCAT TCANAGCCTG GGGGTGGAAT 240
GGCTGGCAGC CTGTGGCCCC ATTGAAATAG GCTCTGGGGC ACTCCCTCTG TTCCTANTTG 300
AACTTGGGTA AGGAACAGGA ATGTGGTCAN CCTATGGAAT CTTGA 345

(2) INFORMATION FOR SEQ ID NO:155:

(i) SEQUENCE CHARACTERISTICS:
(A) LENGTH: 295 base pairs
(B) TYPE: nucleic acid
(C) STRANDEDNESS: single
(D) TOPOLOGY: linear

(xi) SEQUENCE DESCRIPTION: SEQ ID NO:155:

GACGCTTGGC CACTTGACAC ATTAAACAGT TTTGCATAAT CACTANCATG TATTTCTAGT 60
TTGCTGTCTG CTGTGATGCC CTGCCCTGAT TCTCTGGCGT TAATGATGGC AAGCATAATC 120
AAACGCTGTT CTGTTAATTC CAAGTTATAA CTGGCATTGA TTAAAGCATT ATCTTTCACA 180
ACTAAACTGT TCTTCATANA ACAGCCCATA TTATTATCAA ATTAAGAGAC AATGTATTCC 240
AATATCCTTT ANGGCCAATA TATTTNATGT CCCTTAATTA AGAGCTACTG TCCGT 295

(2) INFORMATION FOR SEQ ID NO:156:

(i) SEQUENCE CHARACTERISTICS:
(A) LENGTH: 406 base pairs
(B) TYPE: nucleic acid
(C) STRANDEDNESS: single
(D) TOPOLOGY: linear

(xi) SEQUENCE DESCRIPTION: SEQ ID NO:156:

GACGCTTGGC CACTTGACAC TGCAGTGGGA AAACCAGCAT GAGCCGCTGC CCCCAAGGAA 60
CCTCGAAGCC CAGGCAGAGG ACCAGCCATC CCAGCCTGCA GGTAAAGTGT GTCACCTGTC 120
AGGTGGGCTT GGGGTGAGTG GGTGGGGGAA GTGTGTGTGC AAAGGGGGTG TNAATGTNTA 180
TGCGTGTGAG CATGAGTGAT GGCTAGTGTG ACTGCATGTC AGGGAGTGTG AACAAGCGTG 240
CGGGGGTGTG TGTGCAAGTG CGTATGCATA TGAGAATATG TGTCTGTGGA TGAGTGCATT 300
TGAAAGTCTG TGTGTGTGCG TGTGGTCATG ANGGTAANTT ANTGACTGCG CAGGATGTGT 360
GAGTGTGCAT GGAACACTCA NTGTGTGTGT CAAGTGGCCN ANCGTC 406

(2) INFORMATION FOR SEQ ID NO:157:

(i) SEQUENCE CHARACTERISTICS:
(A) LENGTH: 208 base pairs
(B) TYPE: nucleic acid
(C) STRANDEDNESS: single
(D) TOPOLOGY: linear

(xi) SEQUENCE DESCRIPTION: SEQ ID NO:157:

TGACGCTTGG CCACTTGACA CACTAAAGGG TGTTACTCAT CACTTTCTTC TCTCCTCGGT 60
GGCATGTGAG TGCATCTATT CACTTGGCAC TCATTTGTTT GGCAGTGACT GTAANCCANA 120
TCTGATGCAT ACACCAGCTT GTAAATTGAA TAAATGTCTC TAATACTATG TGCTCACAAT 180
ANGGTANGGG TGAGGAGAAG GGGAGAGA 208

(2) INFORMATION FOR SEQ ID NO:158:

(i) SEQUENCE CHARACTERISTICS:
(A) LENGTH: 547 base pairs
(B) TYPE: nucleic acid
(C) STRANDEDNESS: single
(D) TOPOLOGY: linear

(xi) SEQUENCE DESCRIPTION: SEQ ID NO:158:

CTTCAACCTC CTTCAACCTC CTTCAACCTC CTGGATTCAA ACAATCATCC CACCTCAGAC 60
TCCTTAGTAG CTGAGACTAC AGACTCACGC CACTACATCT GGCTAAATTT TTGTAGAGAT 120
AGGGTTTCAT CATGTTGCCC TGGCTGGTCT CAAACTCCTG ACCTCAAGCA ATGTGCCCAC 180
CTCAGCCTCC CAAAGTGCTG GGATTACAGG CATAAGCCAC CATGCCCAGT CCATNTTTAA 240
TCTTTCCTAC CACATTCTTA CCACACTTTC TTTTATGTTT AGATACATAA ATGCTTACCA 300
TTATGATACA ATTGCCCACA GTATTAAGAC AGTAACATGC TGCACAGGTT TGTAGCCTAG 360
GAACAGTAGG CAATACCACA TAGCTTAGGT GTGTGGTAGA CTATACCATC TAGGTTTGTG 420
TAAGTTACAC TTTATGCTGT TTACACAATG ACAAAACCAT CTAATGATGC ATTTCTCAGA 480
ATGTATCCTT GTCAGTAAGC TATGATGTAC AGGGAACACT GCCCAAGGAC ACAGATATTG 540
TACCTGT 547

(2) INFORMATION FOR SEQ ID NO:159:

(i) SEQUENCE CHARACTERISTICS:
(A) LENGTH: 203 base pairs
(B) TYPE: nucleic acid
(C) STRANDEDNESS: single
(D) TOPOLOGY: linear

(xi) SEQUENCE DESCRIPTION: SEQ ID NO:159:

GCTCCTCTTG CCTTACCAAC TCACCCAGTA TGTCAGCAAT TTTATCRGCT TTACCTACGA 60
AACAGCCTGT ATCCAAACAC TTAACACACT CACCTGAAAA GTTCAGGCAA CAATCGCCTT 120
CTCATGGGTC TCTCTGCTCC AGTTCTGAAC CTTTCTCTTT TCCTAGAACA TGCATTTARG 180
TCGATAGAAG TTCCTCTCAG TGC 203

(2) INFORMATION FOR SEQ ID NO:160:

(i) SEQUENCE CHARACTERISTICS:
(A) LENGTH: 402 base pairs
(B) TYPE: nucleic acid
(C) STRANDEDNESS: single
(D) TOPOLOGY: linear

(xi) SEQUENCE DESCRIPTION: SEQ ID NO:160:

TGTAAGTCGA GCAGTGTGAT GGGTGGAACA GGGTTGTAAG CAGTAATTGC AAACTGTATT 60
TAAACAATAA TAATAATATT TAGCATTTAT AGAGCACTTT ATATCTTCAA AGTACTTGCA 120
AACATTAYCT AATTAAATAC CCTCTCTGAT TATAATCTGG ATACAAATGC ACTTAAACTC 180
AGGACAGGGT CATGAGARAA GTATGCATTT GAAAGTTGGT GCTAGCTATG CTTTAAAAAC 240
CTATACAATG ATGGGRAAGT TAGAGTTCAG ATTCTGTTGG ACTGTTTTTG TGCATTTCAG 300
TTCAGCCTGA TGGCAGAATT AGATCATATC TGCACTCGAT GACTYTGCTT GATAACTTAT 360
CACTGAAATC TGAGTGTTGA TCATCACACT GCTCGACTTA CA 402

(2) INFORMATION FOR SEQ ID NO:161:

(i) SEQUENCE CHARACTERISTICS:
(A) LENGTH: 193 base pairs
(B) TYPE: nucleic acid
(C) STRANDEDNESS: single
(D) TOPOLOGY: linear

(xi) SEQUENCE DESCRIPTION: SEQ ID NO:161:

AGCATGTTGA GCCCAGACAC TGACCAGGAG AAAAACCAAC CAATAGAAAC ACGCCCAGAC 60
ACTGACCAGG AGAAAAACCA ACCAATAAAA ACAGGCCCGG ACATAAGACA AATAATAAAA 120
TTAGCGGACA AGGACATGAA AACAGCTATT GTAAGAGCGG ATATAGTGGT GTGTGTCTGG 180
GCTCAACATG CTA 193

(2) INFORMATION FOR SEQ ID NO:162:

(i) SEQUENCE CHARACTERISTICS:
(A) LENGTH: 147 base pairs
(B) TYPE: nucleic acid
(C) STRANDEDNESS: single
(D) TOPOLOGY: linear

(xi) SEQUENCE DESCRIPTION: SEQ ID NO:162:

TGTTGAGCCC AGACACTGAC CAGGAGAAAA ACCAACCAAT AAAAACAGGC CCGGACATAA 60
GACAAATAAT AAAATTAGCG GACAAGGACA TGAAAACAGC TATTGTAAGA GCGGATATAG 120
TGGTGTGTGT CTGGGCTCAA CATGCTA 147

(2) INFORMATION FOR SEQ ID NO:163:

(i) SEQUENCE CHARACTERISTICS:
(A) LENGTH: 294 base pairs
(B) TYPE: nucleic acid
(C) STRANDEDNESS: single

(D) TOPOLOGY: linear

(xi) SEQUENCE DESCRIPTION: SEQ ID NO:163:

TAGCATGTTG AGCCCAGACA CAAATCTTTC CTTAAGCAAT AAATCATTTC TGCATATGTT 60
TTTAAAACCA CAGCTAAGCC ATGATTATTC AAAAGGACTA TTGTATTGGG TATTTTGATT 120
TGGGTTCTTA TCTCCCTCAC ATTATCTTCA TTTCTATCAT TGACCTCTTA TCCCAGAGAC 180
TCTCAAACTT TTATGTTATA CAAATCACAT TCTGTCTCAA AAAATATCTC ACCCACTTCT 240
CTTCTGTTTC TGCGTGTGTA TGTGTGTGTG TGTGTGTCTG GGCTCAACAT GCTA 294

(2) INFORMATION FOR SEQ ID NO:164:

(i) SEQUENCE CHARACTERISTICS:
(A) LENGTH: 412 base pairs
(B) TYPE: nucleic acid
(C) STRANDEDNESS: single
(D) TOPOLOGY: linear

(xi) SEQUENCE DESCRIPTION: SEQ ID NO:164:

CGGGATTGGC TTTGAGCTGC AGATGCTGCC TGTGACCGCA CCCGGCGTGG AACAGAAAGC 60
CACCTGGCTG CAAGTGCGCC AGAGCCGCCC TGACTACGTG CTGCTGTGGG GCTGGGGCGT 120
GATGAACTCC ACCGCCCTGA AGGAAGCCCA GGCCACCGGA TACCCCCGCG ACAAGATGTA 180
CGGCGTGTGG TGGGCCGGTG CGGAGCCCGA TGTGCGTGAC GTGGGCGAAG GCGCCAAGGG 240
CTACAACGCG CTGGCTCTGA ACGGCTACGG CACGCAGTCC AAGGTGATCC ANGACATCCT 300
GAAACACGTG CACGACAAGG GCCAGGGCAC GGGGCCCAAA GACGAAGTGG GCTCGGTGCT 360
GTACACCCGC GGCGTGATCA TCCAGATGCT GGACAAGGTG TCAATCACTA AT 412

(2) INFORMATION FOR SEQ ID NO:165:

(i) SEQUENCE CHARACTERISTICS:
(A) LENGTH: 361 base pairs
(B) TYPE: nucleic acid
(C) STRANDEDNESS: single
(D) TOPOLOGY: linear

(xi) SEQUENCE DESCRIPTION: SEQ ID NO:165:

TTGACACCTT GTCCAGCATC TGCATCTGAT GAGAGCCTCA GATGGCTACC ACTAATGGCA 60
GAAGGCAAAG GAGAACAGGC ATTGTATGGC AAGAAAGGAA GAAAGAGAGA GGGGAGAAAG 120
GTGCTAGGTT CTTTTCAACA ACCAGTTCTT GATGGAACTG AGAGTAAGAG CTCAAGGCCA 180
GGTGTGGTGA CTCCAACCAG TAATCCCAAC ATTTTAGGAG GCTGAGGCAG GCAGATGTCT 240
TGACCCCATG AGTTTGTGAC CAGCCTGAAC AACATCATGA GACTCCATCT CTACAATAAT 300
TACAAAAATT AATCAGGCAT TGTGGTATGC CCTGTAGTCC CAGATGCTGG ACAAGGTGTC 360
A 361

(2) INFORMATION FOR SEQ ID NO:166:

(i) SEQUENCE CHARACTERISTICS:
(A) LENGTH: 427 base pairs
(B) TYPE: nucleic acid
(C) STRANDEDNESS: single

(D) TOPOLOGY: linear

(xi) SEQUENCE DESCRIPTION: SEQ ID NO:166:

TWGACTGACT CATGTCCCCT ACACCCAACT ATCTTCTCCA GGTGGCCAGG CATGATAGAA 60
TCTGATCCTG ACTTAGGGGA ATATTTTCTT TTTACTTCCC ATCTTGATTC CCTGCCGGTG 120
AGTTTCCTGG TTCAGGGTAA GAAAGGAGCT CAGGCCAAAG TAATGAACAA ATCCATCCTC 180
ACAGACGTAC AGAATAAGAG AACWTGGACW TAGCCAGCAG AACMCAAKTG AAAMCAGAAC 240
MCTTAMCTAG GATRACAAMC MCRRARATAR KTGCYCMCMC WTATAATAGA AACCAAACTT 300
GTATCTAATT AAATATTTAT CCACYGTCAG GGCATTAGTG GTTTTGATAA ATACGCTTTG 360
GCTAGGATTC CTGAGGTTAG AATGGAARAA CAATTGCAMC GAGGGTAGGG GACATGAGTC 420
AKTCTAA 427

(2) INFORMATION FOR SEQ ID NO:167:

(i) SEQUENCE CHARACTERISTICS:
(A) LENGTH: 500 base pairs
(B) TYPE: nucleic acid
(C) STRANDEDNESS: single
(D) TOPOLOGY: linear

(xi) SEQUENCE DESCRIPTION: SEQ ID NO:167:

AACGTCGCAT GCTCCCGGCC GCCATGGCCG CGGGATAGAC TGACTCATGT CCCCTAAGAT 60
AGAGGAGACA CCTGCTAGGT GTAAGGAGAA GATGGTTAGG TCTACGGAGG CTCCAGGGTG 120
GGAGTAGTTC CCTGCTAAGG GAGGGTAGAC TGTTCAACCT GTTCCTGCTC CGGCCTCCAC 180
TATAGCAGAT GCGAGCAGGA GTAGGAGAGA GGGAGGTAAG AGTCAGAAGC TTATGTTGTT 240
TATGCGGGGA AACGCCRTAT CGGGGGCAGC CRAGTTATTA GGGGACANTR TAGWYARTCW 300
AGNTAGCATC CAAAGCGNGG GAGTTNTCCC ATATGGTTGG ACCTGCAGGC GGCCGCATTA 360
GTGATTAGCA TGTGAGCCCC AGACACGCAT AGCAACAAGG ACCTAAACTC AGATCCTGTG 420
CTGATTACTT AACATGAATT ATTGTATTTA TTTAACAACT TTGAGTTATG AGGCATATTA 480
TTAGGTCCAT ATTACCTGGA 500

(2) INFORMATION FOR SEQ ID NO:168:

(i) SEQUENCE CHARACTERISTICS:
(A) LENGTH: 358 base pairs
(B) TYPE: nucleic acid
(C) STRANDEDNESS: single
(D) TOPOLOGY: linear

(xi) SEQUENCE DESCRIPTION: SEQ ID NO:168:

TTCATCGCTC GGTGACTCAA GCCTGTAATC CCAGAACTTT GGGAGGCCGA GGGGAGCAGA 60
TCACCTGAGG TTGGGAGTTT GAGACCAGCC TGGCCAACAT GGTGACAACC CGTCTCTGCT 120
AAAAATACAA AAATTAGCCA AGCATGGTGG CATGCACTTG TAATCCCAGC TACTCGGGAG 180
GCTGAGGCAG GAGAATCACT TGAGGCCAGG AGGCAGAGGT TGCAGTGAGG CAGAGGTTGA 240
GATCATGCCA CTGCACTCCA GCCTGGGCAA CAGAGTAAGA CTCCATCTCA AAAAAAAAAA 300
AAAAAAAGAA TGATCAGAGC CACAAATACA GAAAACCTTG AGTCACCGAG CGATGAAA 358

(2) INFORMATION FOR SEQ ID NO:169:

(i) SEQUENCE CHARACTERISTICS:
(A) LENGTH: 1265 base pairs
(B) TYPE: nucleic acid
(C) STRANDEDNESS: single
(D) TOPOLOGY: linear

(xi) SEQUENCE DESCRIPTION: SEQ ID NO:169:

TTCTGTCCAC ACCAATCTTA GAGCTCTGAA AGAATTTGTC TTTAAATATC TTTTAATAGT 60
AACATGTATT TTATGGACCA AATTGACATT TTCGACTATT TTTTCCCAAA AAAAGTCAGG 120
TGAATTTCAG CACACTGAGT TGGGAATTTC TTATCCCAGA AGWCGGCACG AGCAATTTCA 180
TATTTATTTA AGATTGATTC CATACTCCGT TTTCAAGGAG AATCCCTGCA GTCTCCTTAA 240
AGGTAGAACA AATACTTTCT ATTTTTTTTT CACCATTGTG GGATTGGACT TTAAGAGGTG 300
ACTCTAAAAA AACAGAGAAC AAATATGTCT CAGTTGTATT AAGCACGGAC CCATATTATC 360
ATATTCACTT AAAAAAATGA TTTCCTGTGC ACCTTTTGGC AACTTCTCTT TTCAATGTAG 420
GGAAAAACTT AGTCACCCTG AAAACCCACA AAATAAATAA AACTTGTAGA TGTGGGCAGA 480
ARGTTTGGGG GTGGACATTG TATGTGTTTA AATTAAACCC TGTATCACTG AGAAGCTGTT 540
GTATGGGTCA GAGAAAATGA ATGCTTAGAA GCTGTTCACA TCTTCAAGAG CAGAAGCAAA 600
CCACATGTCT CAGCTATATT ATTATTTATT TTTTATGCAT AAAGTGAATC ATTTCTTCTG 660
TATTAATTTC CAAAGGGTTT TACCCTCTAT TTAAATGCTT TGAAAAACAG TGCATTGACA 720
ATGGGTTGAT ATTTTTCTTT AAAAGAAAAA TATAATTATG AAAGCCAAGA TAATCTGAAG 780
CCTGTTTTAT TTTAAAACTT TTTATGTTCT GTGGTTGATG TTGTTTGTTT GTTTGTTTCT 840
ATTTTGTTGG TTTTTTACTT TGTTTTTTGT TTTGTTTTGT TTTGGTTTDG CATACTACAT 900
GCAGTTTCTT TAACCAATGT CTGTTTGGCT AATGTAATTA AAGTTGTTAA TTTATATGAG 960
TGCATTTCAA CTATGTCAAT GGTTTCTTAA TATTTATTGT GTAGAAGTAC TGGTAATTTT 1020
TTTATTTACA ATATGTTTAA AGAGATAACA GTTTGATATG TTTTCATGTG TTTATAGCAG 1080
AAGTTATTTA TTTCTATGGC ATTCCAGCGG ATATTTTGGT GTTTGCGAGG CATGCAGTCA 1140
ATATTTTGTA CAGTTAGTGG ACAGTATTCA GCAACGCCTG ATAGCTTCTT TGGCCTTATG 1200
TTAAATAAAA AGACCTGTTT GGGATGTAAA AAAAAAAAAA AAAAAAAAAA AAAAAAAAAA 1260
AAAAA 1265

(2) INFORMATION FOR SEQ ID NO:170:

(i) SEQUENCE CHARACTERISTICS:
(A) LENGTH: 383 base pairs
(B) TYPE: nucleic acid
(C) STRANDEDNESS: single
(D) TOPOLOGY: linear

(xi) SEQUENCE DESCRIPTION: SEQ ID NO:170:

TGTAAGTCGA GCAGTGTGAT GACGATATTC TTCTTATTAA TGTGGTAATT GAACAAATGA 60
TCTGTGATAC TGATCCTGAG CTAGGAGGCG CTGTTCAGTT AATGGGACTT CTTCGTACTC 120
TAATTGATCC AGAGAACATG CTGGCTACAA CTAATAAAAC CGAAAAAAGT GAATTTCTAA 180
ATTTTTTCTA CAACCATTGT ATGCATGTTC TCACAGCACC ACTTTTGACC AATACTTCAG 240
AAGACAAATG TGAAAAGGAT AATATAGTTG GATCAAACAA AAACAACACA ATTTGTCCCG 300
ATAATTATCA AACAGCACAG CTACTTGCCT TAATTTTAGA GTTACTCACA TTTTGTGTGG 360
AACATCACAC TGCTCGACTT ACA 383

(2) INFORMATION FOR SEQ ID NO:171:

(i) SEQUENCE CHARACTERISTICS:
(A) LENGTH: 383 base pairs
(B) TYPE: nucleic acid
(C) STRANDEDNESS: single
(D) TOPOLOGY: linear

(xi) SEQUENCE DESCRIPTION: SEQ ID NO:171:

TGGGCACCTT CAATATCGCA AGTTAAAAAT AATGTTGAGT TTATTATACT TTTGACCTGT 60
TTAGCTCAAC AGGGTGAAGG CATGTAAAGA ATGTGGACTT CTGAGGAATT TTCTTTTAAA 120
AAGAACATAA TGAAGTAACA TTTTAATTAC TCAAGGACTA CTTTTGGTTG AAGTTTATAA 180
TCTAGATACC TCTACTTTTT GTTTTTGCTG TTCGACAGTT CACAAAGACC TTCAGCAATT 240
TACAGGGTAA AATCGTTGAA GTAGTGGAGG TGAAACTGAA ATTTAAAATT ATTCTGTAAA 300
TACTATAGGG AAAGAGGCTG AGCTTAGAAT CTTTTGGTTG TTCATGTGTT CTGTGCTCTT 360
ATCATCACAC TGCTCGACTT ACA 383

(2) INFORMATION FOR SEQ ID NO:172:

(i) SEQUENCE CHARACTERISTICS:
(A) LENGTH: 699 base pairs
(B) TYPE: nucleic acid
(C) STRANDEDNESS: single
(D) TOPOLOGY: linear

(xi) SEQUENCE DESCRIPTION: SEQ ID NO:172:

TCGGGTGATG CCTCCTCAGG CTTGTCGTTA GTGTACACAG AGCTGCTCAT GAAGCGACAG 60
CGGCTGCCCC TGGCACTTCA GAACCTCTTC CTCTACACTT TTGGTGCGCT TCTGAATCTA 120
GGTCTGCATG CTGGCGGCGG CTCTGGCCCA GGCCTCCTGG AAAGTTTCTC AGGATGGGCA 180
GCACTCGTGG TGCTGAGCCA GGCACTAAAT GGACTGCTCA TGTCTGCTGT CATGGAGCAT 240
GGCAGCAGCA TCACACGCCT CTTTGTGGTG TCCTGCTCGC TGGTGGTCAA CGCCGTGCTC 300
TCAGCAGTCC TGCTACGGCT GCAGCTCACA GCCGCCTTCT TCCTGGCCAC ATTGCTCATT 360
GGCCTGGCCA TGCGCCTGTA CTATGGCAGC CGCTAGTCCC TGACAACTTC CACCCTGATT 420
CCGGACCCTG TAGATTGGGC GCCACCACCA GATCCCCCTC CCAGGCCTTC CTCCCTCTCC 480
CATCAGCGGC CCTGTAACAA GTGCCTTGTG AGAAAAGCTG GAGAAGTGAG GGCAGCCAGG 540
TTATTCTCTG GAGGTTGGTG GATGAAGGGG TACCCCTAGG AGATGTGAAG TGTGGGTTTG 600
GTTAAGGAAA TGCTTACCAT CCCCCACCCC CAACCAAGTT NTTCCAGACT AAAGAATTAA 660
GGTAACATCA ATACCTAGGC CTGAGGAGGC ATCACCCGA 699

(2) INFORMATION FOR SEQ ID NO:173:

(i) SEQUENCE CHARACTERISTICS:
(A) LENGTH: 701 base pairs
(B) TYPE: nucleic acid
(C) STRANDEDNESS: single
(D) TOPOLOGY: linear

(xi) SEQUENCE DESCRIPTION: SEQ ID NO:173:

TCGGGTGATG CCTCCTCAGG CCAGATCAAA CTTGGGGTTG AAAACTGTGC AAAGAAATCA 60
ATGTCGGAGA AAGAATTTTG CAAAAGAAAA ATGCCTAATC AGTACTAATT TAATAGGTCA 120

CATTAGCAGT GGAAGAAGAA ATGTTGATAT TTTATGTCAG CTATTTTATA ATCACCAGAG 180
TGCTTAGCTT CATGTAAGCC ATCTCGTATT CATTAGAAAT AAGAACAATT TTATTCGTCG 240
GAAAGAACTT TTCAATTTAT AGCATCTTAA TTGCTCAGGA TTTTAAATTT TGATAAAGAA 300
AGCTCCACTT TTGGCAGGAG TAGGGGGCAG GGAGAGAGGA GGCTCCATCC ACAAGGACAG 360
AGACACCAGG GCCAGTAGGG TAGCTGGTGG CTGGATCAGT CACAACGGAC TGACTTATGC 420
CATGAGAAGA AACAACCTCC AAATCTCAGT TGCTTAATAC AACACAAGCT CATTTCTTGC 480
TCACGTTACA TGTCCTATGT AGATCAACAG CAGGTGACTC AGGGACCCAG GCTCCATCTC 540
CATATGAGCT TCCATAGTCA CCAGGACACG GGCTCTGAAA GTGTCCTCCA TGCAGGGACA 600
CATGCCTCTT CCTTTCATTG GGCAGAGCAA GTCACTTATG GCCAGAAGTC ACACTGCAGG 660
GCAGTGCCAT CCTGCTGTAT GCCTGAGGAG GCATCACCCG A 701

(2) INFORMATION FOR SEQ ID NO:174:

(i) SEQUENCE CHARACTERISTICS:
(A) LENGTH: 700 base pairs
(B) TYPE: nucleic acid
(C) STRANDEDNESS: single
(D) TOPOLOGY: linear

(xi) SEQUENCE DESCRIPTION: SEQ ID NO:174:

TCGGGTGATG CCTCCTCANG CCCCTAAATC AGAGTCCAGG GTCAGAGCCA CAGGAGACAG 60
GGAAAGACAT AGATTTTAAC CGGCCCCCTT CAGGAGATTC TGAGGCTCAG TTCACTTTGT 120
TGCAGTTTGA ACAGAGGCAG CAAGGCTAGT GGTTAGGGGC ACGGTCTCTA AAGCTGCACT 180
GCCTGGATCT GCCTCCCAGC TCTGCCAGGA ACCAGCTGCG TGGCCTTGAG CTGCTGACAC 240
GCAGAAAGCC CCCTGTGGAC CCAGTCTCCT CGTCTGTAAG ATGAGGACAG GACTCTAGGA 300
ACCCTTTCCC TTGGTTTGGC CTCACTTTCA CAGGCTCCCA TCTTGAACTC TATCTACTCT 360
TTTCCTGAAA CCTTGTAAAA GAAAAAAGTG CTAGCCTGGG CAACATGGCA AAACCCTGTC 420
TCTACAAAAA ATACAAAAAT TAGTTGGGTG TGGTGGCATG TGCCTGTAGT CCCAGCCACT 480
TGGGAGGTGC TGAGGTGGGA GGATCACTTG AGCCCGGGAG GTGGAGGTTG CAGTGAGCCA 540
AGATCATGCC ACTGCACTCC AGCCTGAGTA ATAGAGTAAG ACTCTGTCTC AAAAACAACA 600
ACAACAACAG TGAGTGTGCC TCTGTTTCCG GGTTGGATGG GGCACCACAT TTATGCATCT 660
CTCAGATTTG GACGCTGCAG CCTGAGGAGG CATCACCCGA 700

(2) INFORMATION FOR SEQ ID NO:175:

(i) SEQUENCE CHARACTERISTICS:
(A) LENGTH: 484 base pairs
(B) TYPE: nucleic acid
(C) STRANDEDNESS: single
(D) TOPOLOGY: linear

(xi) SEQUENCE DESCRIPTION: SEQ ID NO:175:

TATAGGGCGA ATTGGGCCCG AGTTGCATGN TCCCGGCCGC CATGGCCGCG GGATTCGGGT 60
GATGCCTCCT CAGGCTTGTC TGCCACAAGC TACTTCTCTG AGCTCAGAAA GTGCCCCTTG 120
ATGAGGGAAA ATGTCCTACT GCACTGCGAA TTTCTCAGTT CCATTTTACC TCCCAGTCCT 180
CCTTCTAAAC CAGTTAATAA ATTCATTCCA CAAGTATTTA CTGATTACCT GCTTGTGCCA 240
GGGACTATTC TCAGGCTGAA GAAGGTGGGA GGGGAGGGCG GAACCTGAGG AGCCACCTGA 300
GCCAGCTTTA TATTTCAACC ATGGCTGGCC CATCTGAGAG CATCTCCCCA CTCTCGCCAA 360
CCTATCGGGG CATAGCCCAG GGATGCCCCC AGGCGGCCCA GGTTAGATGC GTCCCTTTGG 420
CTTGTCAGTG ATGACATACA CCTTAGCTGC TTAGCTGGTG CTGGCCTGAG GAGGCATCAC 480
CCGA 484

(2) INFORMATION FOR SEQ ID NO:176:

(i) SEQUENCE CHARACTERISTICS:
(A) LENGTH: 432 base pairs
(B) TYPE: nucleic acid
(C) STRANDEDNESS: single
(D) TOPOLOGY: linear

(xi) SEQUENCE DESCRIPTION: SEQ ID NO:176:

TCGGGTGATG CCTCCTCAGG GCTCAAGGGA TGAGAAGTGA CTTCTTTCTG GAGGGACCGT 60
TCATGCCACC CAGGATGAAA ATGGATAGGG ACCCACTTGG AGGACTTGCT GATATGTTTG 120
GACAAATGCC AGGTAGCGGA ATTGGTACTG GTCCAGGAGT TATCCAGGAT AGATTTTCAC 180
CCACCATGGG ACGTCATCGT TCAAATCAAC TCTTCAATGG CCATGGGGGA CACATCATGC 240
CTCCCACACA ATCGCAGTTT GGAGAGATGG GAGGCAAGTT TATGAAAAGC CAGGGGCTAA 300
GCCAGCTCTA CCATAACCAG AGTCAGGGAC TCTTATCCCA GCTGCAAGGA CAGTCGAAGG 360
ATATGCCACC TCGGTTTTCT AAGAAAGGAC AGCTTAATGC AGATGAGATT AGCCTGAGGA 420
GGCATCACCC GA 432

(2) INFORMATION FOR SEQ ID NO:177:

(i) SEQUENCE CHARACTERISTICS:
(A) LENGTH: 788 base pairs
(B) TYPE: nucleic acid
(C) STRANDEDNESS: single
(D) TOPOLOGY: linear

(xi) SEQUENCE DESCRIPTION: SEQ ID NO:177:

TAGCATGTTG AGCCCAGACA CAGTAGCATT TGTGCCAATT TCTGGTTGGA ATGGTGACAA 60
CATGCTGGAG CCAAGTGCTA ACATGCCTTG GTTCAAGGGA TGGAAAGTCA CCCGTAAGGA 120
TGGCAATGCC AGTGGAACCA CGCTGCTTGA GGCTCTGGAC TGCATCCTAC CACCAACTCG 180
CCCAACTGAC AAGCCCTTGC GCCTGCCTCT CCAGGATGTC TACAAAATTG GTGGTATTGG 240
TACTGTTCCT GTTGGCCGAG TGGAGACTGG TGTTCTCAAA CCCGGTATGG TGGTCACCTT 300
TGCTCCAGTC AACGTTACAA CGGAAGTAAA ATCTGTCGAA ATGCACCATG AAGCTTTGAG 360
TGAAGCTCTT CCTGGGGACA ATGTGGGCTT CAATGTCAAG AATGTGTCTG TCAAGGATGT 420
TCGTCGTGGC AACGTTGCTG GTGACAGCAA AAATGACCCA CCAATGGAAG CAGCTGGCTT 480
CACTGCTCAG GTGATTATCC TGAACCATCC AGGCCAAATA AGTGCCGGCT ATGCCCCTGT 540
ATTGGATTGC CACACGGCTC ACATTGCATG CAAGTTTGCT GAGCTGAAGG AAAAGATTGA 600
TCGCCGTTCT GGTAAAAAGC TGGAAGATGG CCCTAAATTC TTGAAGTCTG GTGATGCTGC 660
CATTGTTGAT ATGGTTCCTG GCAAGCCCAT GTGTGTTGAG AGCTTCTCAG ACTATCCACC 720
TTTGGGTCGC TTTGCTGTTC GTGATATGAG ACAGACAGTT GCGGTGGGTG TCTGGGCTCA 780
ACATGCTA 788

(2) INFORMATION FOR SEQ ID NO:178:

(i) SEQUENCE CHARACTERISTICS:
(A) LENGTH: 786 base pairs
(B) TYPE: nucleic acid
(C) STRANDEDNESS: single
(D) TOPOLOGY: linear

(xi) SEQUENCE DESCRIPTION: SEQ ID NO:178:

TAGCATGTTG AGCCCAGACA CCTGTGTTTC TGGGAGCTCT GGCAGTGGCG GATTCATAGG 60
CACTTGGGCT GCACTTTGAA TGACACACTT GGCTTTATTA GATTCACTAG TTTTTAAAAA 120
ATTGTTGTTC GTTTCTTTTC ATTAAAGGTT TAATCAGACA GATCAGACAG CATAATTTTG 180
TATTTAATGA CAGAAACGTT GGTACATTTC TTCATGAATG AGCTTGCATT CTGAAGCAAG 240
AGCCTACAAA AGGCACTTGT TATAAATGAA AGTTCTGGCT CTAGAGGCCA GTACTCTGGA 300
GTTTCAGAGC AGCCAGTGAT TGTTCCAGTC AGTGATGCCT AGTTATATAG AGGAGGAGTA 360
CACTGTGCAC TCTTCTAGGT GTAAGGGTAT GCAACTTTGG ATCTTAAAAT TCTGTACACA 420
TACACACTTT ATATATATGT ATGTATGTAT GAAAACATGA AATTAGTTTG TCAAATATGT 480
GTGTGTTTAG TATTTTAGCT TAGTGCAACT ATTTCCACAT TATTTATTAA ATTGATCTAA 540
GACACTTTCT TGTTGACACC TTGAATATTA ATGTTCAAGG GTGCAATGTG TATTCCTTTA 600
GATTGTTAAA GCTTAATTAC TATGATTTGT AGTAAATTAA CTTTTAAAAT GTATTTGAGC 660
CCTTCTGTAG TGTCGTAGGG CTCTTACAGG GTGGGAAAGA TTTTAATTTT CCAGTTGCTA 720
ATTGAACAGT ATGGCCTCAT TATATATTTT GATTTATAGG AGTTTGTGTC TGGGCTCAAC 780
ATGCTA 786

(2) INFORMATION FOR SEQ ID NO:179:

(i) SEQUENCE CHARACTERISTICS:
(A) LENGTH: 796 base pairs
(B) TYPE: nucleic acid
(C) STRANDEDNESS: single
(D) TOPOLOGY: linear

(xi) SEQUENCE DESCRIPTION: SEQ ID NO:179:

TAGCATGTTG AGCCCAGACA CTGGTTACAA GACCAGACCT GCTTCCTCCA TATGTAAACA 60
GCTTTTAAAA AGCCAGTGAA CCTTTTTAAT ACTTTGGCAA CCTTCTTTCA CAGGCAAAGA 120
ACACCCCCAT CCGCCCCTTG TTTGGAGTGC AGAGTTTGGC TTTGGTTCTT TGCCTTGCCT 180
GGAGTATACT TCTAATTCCT GTTGTCCTGC ACAAGCTGAA TACCGAGCTA CCCACCGCCA 240
CCCAGGCCAG GTTTCCACTC ATTTATTACT TTATGTTTCT GTTCCATTGC TGGTCCACAG 300
AAATAAGTTT TCCTTTGGAG GAATGTGATT ATACCCCTTT AATTTCCTCC TTTTGCTTTT 360
TTTTAATATC ATTGGTATGT GTTTGGCCCA GAGGAAACTG AAATTCACCA TCATCTTGAC 420
TGGCAATCCC ATTACCATGC TTTTTTTAAA AAACGTAATT TTTCTTGCCT TACATTGGCA 480
GAGTAGCCCT TCCTGGCTAC TGGCTTAATG TAGTCACTCA GTTTCTAGGT GGCATTAGGC 540
ATGAGACCTG AAGCACAGAC TGTCTTACCA CAAAAGGTGA CAAGATCTCA AACCTTAGCC 600
AAAGGGCTAT GTCAGGTTTC AATGCTATCT GCTTCTGTTC CTGCTCACTG TTCTGGATTT 660
TGTCCTTCTT CATCCCTAGC ACCAGAATTT CCCAGTCTCC CTCCCTACCT TCCCTTGTTT 720
TAATTCTAAT CTATCAGCAA AATAACTTTT CAAATGTTTT AACCGGTATC TCCATGTGTC 780
TGGGCTCAAC ATGCTA 796

(2) INFORMATION FOR SEQ ID NO:180:

(i) SEQUENCE CHARACTERISTICS:
(A) LENGTH: 488 base pairs
(B) TYPE: nucleic acid
(C) STRANDEDNESS: single
(D) TOPOLOGY: linear

(xi) SEQUENCE DESCRIPTION: SEQ ID NO:180:

GGATGTGCTG CAAGGCGATT AAGTTGGGTA ACGCCAGGGT TTTCCCAGTC ACGACGTTGT 60
AAAACGACGG CCAGTGAATT GTAATACGAC TCACTATAGG GCGAATTGGG CCCGACGTCG 120
CATGCTCCCG GCCGCCATGG CCGCGGGATA GCATGTTGAG CCCAGACACC TGCAGGTCAT 180
TTGGAGAGAT TTTTCACGTT ACCAGCTTGA TGGTCTTTTT CAGGAGGAGA GACACTGAGC 240
ACTCCCAAGG TGAGGTTGAA GATTTCCTCT AGATAGCCGG ATAAGAAGAC TAGGAGGGAT 300
GCCTAGAAAA TGATTAGCAT GCAAATTTCT ACCTGCCATT TCAGAACTGT GTGTCAGCCC 360
ACATTCAGCT GCTTCTTGTG AACTGAAAAG AGAGAGGTAT TGAGACTTTT CTGATGGCCG 420
CTCTAACATT GTAACACAGT AATCTGTGTG TGTGTGGGTG TGTGTGTGTG TCTGGGCTCA 480
ACATGCTA 488

(2) INFORMATION FOR SEQ ID NO:181:

(i) SEQUENCE CHARACTERISTICS:
(A) LENGTH: 317 base pairs
(B) TYPE: nucleic acid
(C) STRANDEDNESS: single
(D) TOPOLOGY: linear

(xi) SEQUENCE DESCRIPTION: SEQ ID NO:181:

TAGCATGTTG AGCCCAGACA CGGCGACGGT ACCTGATGAG TGGGGTGATG GCACCTGTGA 60
AAAGGAGGAA CGTCATCCCC CATGATATTG GGGACCCAGA TGATGAACCA TGGCTCCGCG 120
TCAATGCATA TTTAATCCAT GATACTGCTG ATTGGAAGGA CCTGAACCTG AAGTTTGTGC 180
TGCAGGTTTA TCGGGACTAT TACCTCACGG GTGATCAAAA CTTCCTGAAG GACATGTGGC 240
CTGTGTGTCT AGTAAGGGAT GCACATGCAG TGGCCAGTGT GCCAGGGGTA TGGTTGGTGT 300
CTGGGCTCAA CATGCTA 317

(2) INFORMATION FOR SEQ ID NO:182:

(i) SEQUENCE CHARACTERISTICS:
(A) LENGTH: 507 base pairs
(B) TYPE: nucleic acid
(C) STRANDEDNESS: single
(D) TOPOLOGY: linear

(xi) SEQUENCE DESCRIPTION: SEQ ID NO:182:

TAGCATGTTG AGCCCAGACA CTGGCTGTTA GCCAAATCCT CTCTCAGCTG CTCCCTGTGG 60
TTTGGTGACT CAGGATTACA GAGGCATCCT GTTTCAGGGA ACAAAAAGAT TTTAGCTGCC 120
AGCAGAGAGC ACCACATACA TTAGAATGGT AAGGACTGCC ACCTCCTTCA AGAACAGGAG 180
TGAGGGTGGT GGTGAATGGG AATGGAAGCC TGCATTCCCT GATGCATTTG TGCTCTCTCA 240
AATCCTGTCT TAGTCTTAGG AAAGGAAGTA AAGTTTCAAG GACGGTTCCG AACTGCTTTT 300
TGTGTCTGGG CTCAACATGC TATCCCGCGG CCATGGCGGC CGGGAGCATG CGACGTCGGG 360
CCCAATTCGC CCTATAGTGA GTCGTATTAC AATTCACTGG CCGTCGTTTT ACAACGTCGT 420
GACTGGGAAA ACCCTGGCGT TACCCAACTT AATCGCCTTG CAGCACATCC CCCTTTCCCA 480
GCTGGCGTAA TANCGAAAAG GCCCGCA 507

(2) INFORMATION FOR SEQ ID NO:183:

(i) SEQUENCE CHARACTERISTICS:
(A) LENGTH: 227 base pairs
(B) TYPE: nucleic acid
(C) STRANDEDNESS: single

(D) TOPOLOGY: linear

(xi) SEQUENCE DESCRIPTION: SEQ ID NO:183:

GATTTACGCT GCAACACTGT GGAGGTAGCC CTGGAGCAAG GCAGGCATGG ATGCTTCTGC 60
AATCCCCAAA TGGAGCCTGG TATTTCAGCC AGGAATCTGA GCAGAGCCCC CTCTAATTGT 120
AGCAATGATA AGTTATTCTC TTTGTTCTTC AACCTTCCAA TAGCCTTGAG CTTCCAGGGG 180
AGTGTCGTTA ATCATTACAG CCTGGTCTCC ACAGTGTTGC AGCGTAA 227

(2) INFORMATION FOR SEQ ID NO:184:

(i) SEQUENCE CHARACTERISTICS:
(A) LENGTH: 225 base pairs
(B) TYPE: nucleic acid
(C) STRANDEDNESS: single
(D) TOPOLOGY: linear

(xi) SEQUENCE DESCRIPTION: SEQ ID NO:184:

TTACGCTGCA ACACTGTGGA GCAGATTAAC ATCAGACTTT TCTATCAACA TGACTGGGGT 60
TACTAAAAAG ACAACAAATC AATGGCTTCA AAAGTCTAAG GAATAATTTC GATACTTCAA 120
CTTTATAAAA CCTGACAAAA CTATCAATCA AGCATAAAGA CAGATGAAGA ACATTTCCAG 180
ATTTTGGCCA ATCAGATATT TTACCTCCAC AGTGTTGCAG CGTAA 225

(2) INFORMATION FOR SEQ ID NO:185:

(i) SEQUENCE CHARACTERISTICS:
(A) LENGTH: 597 base pairs
(B) TYPE: nucleic acid
(C) STRANDEDNESS: single
(D) TOPOLOGY: linear

(xi) SEQUENCE DESCRIPTION: SEQ ID NO:185:

GGCCCGACGT CGCATGCTCC CGGCCGCCAT GGCCGCGGGA TTCGTTAGGG TCTCTATCCA 60
CTGGGACCCA TAGGCTAGTC AGAGTATTTA GAGTTGAGTT CCTTTCTGCT TCCCAGAATT 120
TGAAAGAAAA GGAGTGAGGT GATAGAGCTG AGAGATCAGA TTTGCCTCTG AAGCCTGTTC 180
AAGATGTATG TGCTCAGACC CCACCACTGG GGCCTGTGGG TGAGGTCCTG GGCATCTATT 240
TGAATGAATT GCTGAAGGGG AGCACTATGC CAAGGAAGGG GAACCCATCC TGGCACTGGC 300
ACAGGGGTCA CCTTATCCAG TGCTCAGTGC TTCTTTGCTG CTACCTGGTT TTCTCTCATA 360
TGTGAGGGGC AGGTAAGAAG AAGTGCCCRG TGTTGTGCGA GTTTTAGAAC ATCTACCAGT 420
AAGTGGGGAA GTTTCACAAA GCAGCAGCTT TGTTTTGTGT ATTTTCACCT TCAGTTAGAA 480
GAGGAAGGCT GTGAGATGAA TGTTAGTTGA GTGGAAAAGA CGGGTAAGCT TAGTGGATAG 540
AGACCCTAAC GAATCACTAG TGCGGCCGCC TTGCAGGTCG ACCATATGGG AGAGCTC 597

(2) INFORMATION FOR SEQ ID NO:186:

(i) SEQUENCE CHARACTERISTICS:
(A) LENGTH: 597 base pairs
(B) TYPE: nucleic acid
(C) STRANDEDNESS: single
(D) TOPOLOGY: linear

(xi) SEQUENCE DESCRIPTION: SEQ ID NO:186:

GGCCCGAAGT TGCATGTTCC CGGCCGCCAT GGCCGCGGGA TTCGTTAGGG TCTCTATCCA 60
CTACCTAAAA AATCCCAAAC ATATAACTGA ACTCCTCACA CCCAATTGGA CCAATCCATC 120
ACCCCAGAGG CCTACAGATC CTCCTTTGAT ACATAAGAAA ATTTCCCCAA ACTACCTAAC 180
TATATCATTT TGCAAGATTT GTTTTACCAA ATTTTGATGG CCTTTCTGAG CTTGTCAGTG 240
TGAACCACTA TTACGAACGA TCGGATATTA ACTGCCCCTC ACCGTCCAGG TGTAGCTGGC 300
AACATCAAGT GCAGTAAATA TTCATTAAGT TTTCACCTAC TAAGGTGCTT AAACACCCTA 360
GGGTGCCATG TCGGTAGCAG ATCTTTTGAT TTGTTTTTAT TTCCCATAAG GGTCCTGTTC 420
AAGGTCAATC ATACATGTAG TGTGAGCAGC TAGTCACTAT CGCATGACTT GGAGGGTGAT 480
AATAGAGGCC TCCTTTGCTG TTAAAGAACT CTTGTCCCAG CCTGTCAAAG TGGATAGAGA 540
CCCTAACGAA TCACTAGTGC GGCCGCCTGC AGGTCGACCA TATGGGAGAG CTCCCAA 597

(2) INFORMATION FOR SEQ ID NO:187:

(i) SEQUENCE CHARACTERISTICS:
(A) LENGTH: 324 base pairs
(B) TYPE: nucleic acid
(C) STRANDEDNESS: single
(D) TOPOLOGY: linear

(xi) SEQUENCE DESCRIPTION: SEQ ID NO:187:

TCGTTAGGGT CTCTATCCAC TTGCAGGTAA AATCCAATCC TGTGTATATC TTATAGTCTT 60
CCATATGTAG TGGTTCAAGA GACTGCAGTT CCAGAAAGAC TAGCCGAGCC CATCCATGTC 120
TTCCACTTAA CCCTGCTTTG GGTTACACAT CTTAACTTTT CTGTTCAAGT TTCTCTGTGT 180
AGTTTATAGC ATGAGTATTG GGAWAATGCC CTGAAACCTG ACATGAGATC TGGGAAACAC 240
AAACTTACTC AATAAGAATT TCTCCCATAT TTTTATGATG GAAAAATTTC ACATGCACAG 300
AGGAGTGGAT AGAGACCCTA ACGA 324

(2) INFORMATION FOR SEQ ID NO:188:

(i) SEQUENCE CHARACTERISTICS:
(A) LENGTH: 178 base pairs
(B) TYPE: nucleic acid
(C) STRANDEDNESS: single
(D) TOPOLOGY: linear

(xi) SEQUENCE DESCRIPTION: SEQ ID NO:188:

GCGCGGGGAT TCGGGGTGAT ACCTCCTCAT GCCAAAATAC AACGTNTAAT TTCACAACTT 60
GCCTTCCAAT TTACGCATTT TCAATTTGCT CTCCCCATTT GTTGAGTCAC AACAAACACC 120
ATTGCCCAGA AACATGTATT ACCTAACATG CACATACTCT TAAAACTACT CATCCCTT 178

(2) INFORMATION FOR SEQ ID NO:189:

(i) SEQUENCE CHARACTERISTICS:
(A) LENGTH: 367 base pairs
(B) TYPE: nucleic acid
(C) STRANDEDNESS: single
(D) TOPOLOGY: linear

(xi) SEQUENCE DESCRIPTION: SEQ ID NO:189:

TGACACCTTG TCCAGCATCT GACACAGTCT TGGCTCTTGG AAAATATTGG ATAAATGAAA 60
ATGAATTTCT TTAGCAAGTG GTATAAGCTG AGAATATACG TATCACATAT CCTCATTCTA 120
AGACACATTC AGTGTCCCTG AAATTAGAAT AGGACTTACA ATAAGTGTGT TCACTTTCTC 180
AATAGCTGTT ATTCAATTGA TGGTAGGCCT TAAAAGTCAA AGAAATGAGA GGGCATGTGA 240
AAAAAAGCTC AACATCACTG ATCATTAGAA AACTTCCATT CAAACCCCCA ATGAGATACC 300
ATCTCATACC AGTCAGAATG GCTATTATTA AAAAGTCAAA AAATAACAGA TGCTGGACAA 360
GGTGTCA 367

(2) INFORMATION FOR SEQ ID NO:190:

(i) SEQUENCE CHARACTERISTICS:
(A) LENGTH: 369 base pairs
(B) TYPE: nucleic acid
(C) STRANDEDNESS: single
(D) TOPOLOGY: linear

(xi) SEQUENCE DESCRIPTION: SEQ ID NO:190:

GACACCTTGT CCAGCATCTG ACAACGCTAA CAGCCTGAGG AGATCTTTAT TTATTTATTT 60
AGTTTTTACT CTGGCTAGGC AGATGGTGGC TAAAACATTC ATTTACCCAT TTATTCATTT 120
AATTGTTCCT GCAAGGCCTA TGGATAGAGT ATTGTCCAGC ACTGCTCTGG AAGCTAGGAG 180
CATGGGGATG AACAAGATAG GCTACATCCT GTTCCCACAG AACTTCCACT TTAGTCTGGG 240
AAACAGATGA TATATACAAA TATATAAATG AATTCAGGTA GTTTTAAGTA CGAAAAGAAT 300
AAGAAAGCAG AGTCATGATT TANAATGCTG GAAACAGGGG CTATTGCTTG AGATATTGAA 360
GGTGCCCAA 369

(2) INFORMATION FOR SEQ ID NO:191:

(i) SEQUENCE CHARACTERISTICS:
(A) LENGTH: 369 base pairs
(B) TYPE: nucleic acid
(C) STRANDEDNESS: single
(D) TOPOLOGY: linear

(xi) SEQUENCE DESCRIPTION: SEQ ID NO:191:

TGACACCTTG TCCAGCATCT GCACAGGGAA AAGAAACTAT TATCAGAGTG AACAGGCAAC 60
CTACAGAATG GGAGAAAATT TTTGCAATCT ATCCATCTGA CAAAGGGCTA ATATCCAGAA 120
TCTACAAAGA ACTTATACAA ATTTACAAGA AACAAACAAA CAAACAACTC CTCAAAAAGT 180
GGGTGAAGGA TGTGAACAGA CACTTCTCAA AAGAAGACAT TTATGGGGCC AACAAACATA 240
TGAAAAAAAG CTCATCATCA CTGGTCACTA GATAAATGCA AATCAAAACC ACAATGAGAT 300
ACCATCTCAT TCCAGTTAGA ATGGCAATCA TTAAAAAGTC AGGAAACAAC AGATGCTGGA 360
CAAGGTGTC 369

(2) INFORMATION FOR SEQ ID NO:192:

(i) SEQUENCE CHARACTERISTICS:
(A) LENGTH: 449 base pairs
(B) TYPE: nucleic acid

(C) STRANDEDNESS: single
(D) TOPOLOGY: linear

(xi) SEQUENCE DESCRIPTION: SEQ ID NO:192:

TGACGCTTGG CCACTTGACA CTTCATCTTT GCACAGAAAA ACTTCTTTAC AGATTTAATT 60
CAAGACTGGT CTAGTGACAG TCCTCCAGAC ATTTTTTCAT TTGTTCCATA TACGTGGAAT 120
TTTAAAATCA TGTTTCATCA GTTTGAAATG ATTTGGGCTG CTAATCAACA CAATTGGATC 180
GACTGTTCTA CTAAACAACA GGAAAATGTG TATCTGGCAG CCTGTGGAGA AACACTAAAC 240
ATTGATTTTT CTTTGCCTTT TACGGACTTT GTTCCAGCTA CATGTAATAC CAAGTTCTCT 300
TTAAGAGGAG AAGATGTTGA TCTTCATTTG TTTCTACCAG ACTGCCACCC TAGTAAATAT 360
TCTTTATTTA TGCTGGTAAA AAATTGCCAT CCAAATAAGA TGATTCATGA TACTGGTATT 420
CCTGCTGAGT GTCAAGTGGC CAAGCGTCA 449

(2) INFORMATION FOR SEQ ID NO:193:

(i) SEQUENCE CHARACTERISTICS:
(A) LENGTH: 372 base pairs
(B) TYPE: nucleic acid
(C) STRANDEDNESS: single
(D) TOPOLOGY: linear

(xi) SEQUENCE DESCRIPTION: SEQ ID NO:193:

TGACGCTTGG CCACTTGACA CCAGGGATGT AKCAGTTGAA TATAATCCTG CAATTGTACA 60
TATTGGCAAT TTCCCATCAA ACATTCTAGA AAGAGACAAC CAGGATTGCT AGGCCATAAA 120
AGCTGCAATA AATAACTGGT AATTGCAGTA ATCATTTCAG GCCAATTCAA TCCAGTTTGG 180
CTCAGAGGTG CCTTTGGCTG AGAGAAGAGG TGAGATATAA TGTGTTTTCT TGCAACTTCT 240
TGGAAGAATA ACTCCACAAT AGTCTGAGGA CTAGATACAA ACCTATTTGC CATTAAAGCA 300
CCAGAGTCTG TTAATTCCAG TACTGATAAG TGTTGGAGAT TAGACTCCAG TGTGTCAAGT 360
GGCCAAGCGT CA 372

(2) INFORMATION FOR SEQ ID NO:194:

(i) SEQUENCE CHARACTERISTICS:
(A) LENGTH: 309 base pairs
(B) TYPE: nucleic acid
(C) STRANDEDNESS: single
(D) TOPOLOGY: linear

(xi) SEQUENCE DESCRIPTION: SEQ ID NO:194:

TGACGCTTGG CCACTTGACA CTTATGTAGA ATCCATCGTG GGCTGATGCA AGCCCTTTAT 60
TTAGGCTTAG TGTTGTGGGC ACCTTCAATA TCACACTAGA GACAAACGCC ACAAGATCTG 120
CAGAAACATT CAGTTCTGAN CACTCGAATG GCAGGATAAC TTTTTGTGTT GTAATCCTTC 180
ACATATACAA AAACAAACTC TGCANTCTCA CGTTACAAAA AAACGTACTG CTGTAAAATA 240
TTAAGAAGGG GTAAAGGATA CCATCTATAA CAAAGTAACT TACAACTAGT GTCAAGTGGC 300
CAAGCGTCA 309

(2) INFORMATION FOR SEQ ID NO:195:

(i) SEQUENCE CHARACTERISTICS:

(A) LENGTH: 312 base pairs
(B) TYPE: nucleic acid
(C) STRANDEDNESS: single
(D) TOPOLOGY: linear

(xi) SEQUENCE DESCRIPTION: SEQ ID NO:195:

TGACGCTTGG CCACTTGACA CCCAATCTCG CACTTCATCC TCCCAGCACC TGATGAAGTA 60
GGACTGCAAC TATCCCCACT TCCCAGATGA GGGGACCAAN GTACACATTA GGACCCGGAT 120
GGGAGCACAG ATTTGTCCGA TCCCAGACTC CAAGCACTCA GCGTCACTCC AGGACAGCGG 180
CTTTCAGATA AGGTCACAAA CATGAATGGC TCCGACAACC GGAGTCAGTC CGTGCTGAGT 240
TAAGGCAATG GTGACACGGA TGCACGTGTN ACCTGTAATG GTTCATCGTA AGTGTCAAGT 300
GGCCAAGCGT CA 312

(2) INFORMATION FOR SEQ ID NO:196:

(i) SEQUENCE CHARACTERISTICS:
(A) LENGTH: 288 base pairs
(B) TYPE: nucleic acid
(C) STRANDEDNESS: single
(D) TOPOLOGY: linear

(xi) SEQUENCE DESCRIPTION: SEQ ID NO:196:

TGTATCGACG TAGTGGTCTC CTCAGCCATG CAGAACTGTG ACTCAATTAA ACCTCTTTCC 60
TTTATGAATT ACCCAATCTC GGGTAGTGTC TTTATAGTAG TGTGAGAATG GACTAATACA 120
AGTACATTTT ACTTAGTAAT AATAATAAAC AAATATATTA CATTTTTGTG TATTTACTAC 180
ACCATATTTT TTATTGTTAT TGTAGTGTAC ACCTTCTACT TATTAAAAGA AATAGGCCCG 240
AGGCGGGCAG ATCACGAGGT CAGGAGATGG AGACCACTAC GTCGATAC 288

(2) INFORMATION FOR SEQ ID NO:197:

(i) SEQUENCE CHARACTERISTICS:
(A) LENGTH: 289 base pairs
(B) TYPE: nucleic acid
(C) STRANDEDNESS: single
(D) TOPOLOGY: linear

(xi) SEQUENCE DESCRIPTION: SEQ ID NO:197:

TTGGGCACCT TCAATATCAT GACAGGTGAT GTGATAACCA AGAAGGCTAC TAAGTGATTA 60
ATGGGTGGGT AATGTATACA GAGTAGGTAC ACTGGACAGA GGGGTAATTC ATAGCCAAGG 120
CAGGAGAAGC AGAATGGCAA AACATTTCAT CACACTACTC AGGATAGCAT GCAGTTTAAA 180
ACCTATAAGT AGTTTATTTT TGGAATTTTC CACTTAATAT TTTCAGACTG CAGGTAACTA 240
AACTGTGGAA CACAAGAACA TAGATAAGGG GAGACCACTA CGTCGATAC 289

(2) INFORMATION FOR SEQ ID NO:198:

(i) SEQUENCE CHARACTERISTICS:
(A) LENGTH: 288 base pairs
(B) TYPE: nucleic acid
(C) STRANDEDNESS: single

(D) TOPOLOGY: linear

(xi) SEQUENCE DESCRIPTION: SEQ ID NO:198:

GTATCGACGT AGTGGTCTCC CAAGCAGTGG GAAGAAAACG TGAACCAATT AAAATGTATC 60
AGATACCCCA AAGAAAGGCG CTTGAGTAAA GATTCCAAGT GGGTCACAAT CTCAGATCTT 120
AAAATTCAGG CTGTCAAAGA GATTTGCTAT GAGGTTGCTC TCAATGACTT CAGGCACAGT 180
CGGCAGGAGA TTGAAGCCCT GGCCATTGTC AAGATGAAGG AGCTTTGTGC CATGTATGGC 240
AAGAAAGACC CCAATGAGCG GGACTCCTGG AGACCACTAC GTCGATAC 288

(2) INFORMATION FOR SEQ ID NO:199:

(i) SEQUENCE CHARACTERISTICS:
(A) LENGTH: 1027 base pairs
(B) TYPE: nucleic acid
(C) STRANDEDNESS: single
(D) TOPOLOGY: linear

(xi) SEQUENCE DESCRIPTION: SEQ ID NO:199:

GCTTTTTGGG AAAAACNCAA NTGGGGGAAA GGGGGNTTNN TNGCAAGGGG ATAAAGGGGG 60
AANCCCAGGG TTTCCCCATT CAGGGAGGTG TAAAAAGNCG GCCAGGGGAT TGTAANAGGA 120
TTCAATAATA GGGGGAATGG GCCCNGAAGT TGCAAGGTTC CNGCCCGCCA TGNCCGCGGG 180
ATTTAGTGAC ATTACGACGS TGGTAATAAA GTGGGSCCAA WAAATATTTG TGATGTGATT 240
TTTSGACCAG TGAACCCATT GWACAGGACC TCATTTCCTY TGAGATGRTA GCCATAATCA 300
GATAAAAGRT TAGAAGTYTT TCTGCACGTT AACAGCATCA TTAAATGGAG TGGCATCACC 360
AATTTCACCC TTTGTTAGCC GATACCTTCC CCTTGAAGGC ATTCAATTAA GTGACCAATC 420
GTCATACGAG AGGGGATGGC ATGGGGATTG ATGATGATAT CAGGGGTGAT ACCTTCACAG 480
GTGAAAGGCA TATCCTCTTG TCTATACTGA ATACCACAAG TACCCTTTTG ACCATGTCGA 540
CTAGCAAATT TGTCTCCAAT CTGTGTWATC CCTAACAGAG CGTACCCTTA TTTTACAAAA 600
TTTATATCCT TCCTGATTGA GAGTTACCAT AACCTGATCC ACAATGCCCG TCTCGCTWGT 660
TCTGAGAAAA GTGCTACAGT CTCTCTTGGT ATAGCGTCTA TTGGTGCTCT CCAATTCATC 720
TTCATTTTTC AGGCAAGGTG AACTGTTTTG CCTATAATAA CMTCATCTCC TGATACMCGA 780
AACCCCKGGA RCTATCAAAC CATCATCATC CAGCGTTCKT WATGTYMCTA AATCCCTATT 840
GCGGCCGCCT GCAGGTCAAC ATATNGGAAA ACCCCCCACC CCTTNGGAGC NTACCTTGAA 900
TTTTCCATAT GTCCCNTAAA TTANCTNGNC TTANCCTGGC CNTAACCTNT TCCGGTTTAA 960
ATTGTTTCCG CCCCCNTTCC CCNCCTTNNA ACCGGAAACC TTAATTTTNA ACCNGGGGTT 1020
CCTATCC 1027

(2) INFORMATION FOR SEQ ID NO:200:

(i) SEQUENCE CHARACTERISTICS:
(A) LENGTH: 207 base pairs
(B) TYPE: nucleic acid
(C) STRANDEDNESS: single
(D) TOPOLOGY: linear

(xi) SEQUENCE DESCRIPTION: SEQ ID NO:200:

AGTGACATTA CGACGCTGGC CATCTTGAAT CCTAGGGCAT GAAGTTGCCC CAAAGTTCAG 60
CACTTGGTTA AGCCTGATCC CTCTGGTTTA TCACAAAGAA TAGGATGGGA TAAAGAAAGT 120
GGACACTTAA ATAAGCTATA AATTATATGG TCCTTGTCTA GCAGGAGACA ACTGCACAGG 180

TATACTACCA GCGTCGTAAT GTCACTA 207

(2) INFORMATION FOR SEQ ID NO:201:

(i) SEQUENCE CHARACTERISTICS:
(A) LENGTH: 209 base pairs
(B) TYPE: nucleic acid
(C) STRANDEDNESS: single
(D) TOPOLOGY: linear

(xi) SEQUENCE DESCRIPTION: SEQ ID NO:201:

TGGGCACCTT CAATATCTAT TAAAAGCACA AATACTGAAG AACACACCAA GACTATCAAT 60
GAGGTTACAT CTGGAGTCCT CGATATATCA GGAAAAAATG AAGTGAACAT TCACAGAGTT 120
TTACTTCTTT GGGAACTCAA ATGCTAGAAA AGAAAAGGGT GCCCTCTTTC TCTGGCTTCC 180
TGGTCCTATC CAGCGTCGTA ATGTCACTA 209

(2) INFORMATION FOR SEQ ID NO:202:

(i) SEQUENCE CHARACTERISTICS:
(A) LENGTH: 349 base pairs
(B) TYPE: nucleic acid
(C) STRANDEDNESS: single
(D) TOPOLOGY: linear

(xi) SEQUENCE DESCRIPTION: SEQ ID NO:202:

NTACGCTGCA ACACTGTGGA GCCACTGGTT TTTATTCCCG GCAGGTTATC CAGCAAACAG 60
TCACTGAACA CACCGAAGAC CGTGGTATGG TAACCGTTCA CAGTAATCGT TCCAGTCGTC 120
TGCGGGACCC CGACGAGCGT CACTGGGTAC AGACCAGATT CAGCCGGAAG AGAAAGCGCC 180
GCAGGGAGAG ACTCGAACTC CACTCCGCTG GTGAGCAGCC CCATGTTTTC AACTCGAAGT 240
TCAAACGGCA TTGGGTTATA TACCATCAGC TGAACTTCAC ACACATCTCC TTGAACCCAC 300
TGGAAATCTA TTTTCTTGTT CCGCTCTTCT CCACAGTGTT GCAGCGTAA 349

(2) INFORMATION FOR SEQ ID NO:203:

(i) SEQUENCE CHARACTERISTICS:
(A) LENGTH: 241 base pairs
(B) TYPE: nucleic acid
(C) STRANDEDNESS: single
(D) TOPOLOGY: linear

(xi) SEQUENCE DESCRIPTION: SEQ ID NO:203:

TGCTCCTCTT GCCTTACCAA CCCAAAGCCC ACTGTGAAAT ATGAAGTGAA TGACAAAATT 60
CAGTTTTCAA CGCAATATAG TATAGTTTAT CTGATTCTTT TGATCTCCAG GACACTTTAA 120
ACAACTGCTA CCACCACCAC CAACCTAGGG ATTTAGGATT CTCCACAGAC CAGAAATTAT 180
TTCTCCTTTG AGTTTCAGGC TCCTCTGGGA CTCCTGTTCA TCAATGGGTG GTAAATGGCT 240
A 241

(2) INFORMATION FOR SEQ ID NO:204:

(i) SEQUENCE CHARACTERISTICS:
(A) LENGTH: 248 base pairs
(B) TYPE: nucleic acid
(C) STRANDEDNESS: single
(D) TOPOLOGY: linear

(xi) SEQUENCE DESCRIPTION: SEQ ID NO:204:

TAGCCATTTA CCACCCATCT GCAAACCSWG ACMWWCARGR CYWGWACKYA GGCGATTTGA 60
AGTACTGGTA ATGCTCTGAT CATGTTAGTT ACATAAGTGT GGTCAGTTTA CAAAAATTCA 120
CAGAACTAAA TACTCAATGC TATGTGTTCA TGTCTGTGTT TATGTGTGTG TAATGTTTCA 180
ATTAAGTTTT TTTAAAAAAA AGAGATGATT TCCAAATAAG AAAGCCGTGT TGGTAAGGCA 240
AGAGGAGC 248

(2) INFORMATION FOR SEQ ID NO:205:

(i) SEQUENCE CHARACTERISTICS:
(A) LENGTH: 505 base pairs
(B) TYPE: nucleic acid
(C) STRANDEDNESS: single
(D) TOPOLOGY: linear

(xi) SEQUENCE DESCRIPTION: SEQ ID NO:205:

TACGCTGCAA CACTGTGGAG CCATTCATAC AGGTCCCTAA TTAAGGAACA AGTGATTATG 60
CTACCTTTGC ACGGTTAGGG TACCGCGGCC GTTAAACATG TGTCACTGGG CAGGCGGTGC 120
CTCTAATACT GGTGATGCTA GAGGTGATGT TTTTGGTAAA CAGGCGGGGT AAGATTTGCC 180
GAGTTCCTTT TACTTTTTTT AACCTTTCCT TATGAGCATG CCTGTGTTGG GTTGACAGTG 240
GGGGTAATAA TGACTTGTTG GTTGATTGTA GATATTGGGC TGTTAATTGT CAGTTCAGTG 300
TTTTAATCTG ACGCAGGCTT ATGCGGAGGA GAATGTTTTC ATGTTACTTA TACTAACATT 360
AGTTCTTCTA TAGGGTGATA GATTGGTCCA ATTGGGTGTG AGGAGTTCAG TTATATGTTT 420
GGGATTTTTT AGGTAGTGGG TGTTGANCTT GAACGCTTTC TTAATTGGTG GCTGCTTTTA 480
RGCCTACTAT GGGTGGTAAA TGGCT 505

(2) INFORMATION FOR SEQ ID NO:206:

(i) SEQUENCE CHARACTERISTICS:
(A) LENGTH: 179 base pairs
(B) TYPE: nucleic acid
(C) STRANDEDNESS: single
(D) TOPOLOGY: linear

(xi) SEQUENCE DESCRIPTION: SEQ ID NO:206:

TAGACTGACT CATGTCCCCT ACCAAAGCCC ATGTAAGGAG CTGAGTTCTT AAAGACTGAA 60
GACAGACTAT TCTCTGGAGA AAAATAAAAT GGAAATTGTA CTTTAAAAAA AAAAAAAATC 120
GGCCGGGCAT GGTAGCACAC ACCTGTAATC CCAGCTACTA GGGGACATGA GTCAGTCTA 179

(2) INFORMATION FOR SEQ ID NO:207:

(i) SEQUENCE CHARACTERISTICS:
(A) LENGTH: 176 base pairs

(B) TYPE: nucleic acid
(C) STRANDEDNESS: single
(D) TOPOLOGY: linear

(xi) SEQUENCE DESCRIPTION: SEQ ID NO:207:

AGACTGACTC ATGTCCCCTA CCCCACCTTC TGCTGTGCTG CCGTGTTCCT AACAGGTCAC 60
AGACTGGTAC TGGTCAGTGG CCTGGGGGTT GGGGACCTCT ATTATATGGG ATACAAATTT 120
AGGAGTTGGA ATTGACACGA TTTAGTGACT GATGGGATAT GGGTGGTAAA TGGCTA 176

(2) INFORMATION FOR SEQ ID NO:208:

(i) SEQUENCE CHARACTERISTICS:
(A) LENGTH: 196 base pairs
(B) TYPE: nucleic acid
(C) STRANDEDNESS: single
(D) TOPOLOGY: linear

(xi) SEQUENCE DESCRIPTION: SEQ ID NO:208:

AGACTGACTC ATGTCCCCTA TTTAACAGGG TCTCTAGTGC TGTGAAAAAA AAAAATGCTG 60
AACATTGCAT ATAACTTATA TTGTAAGAAA TACTGTACAA TGACTTTATT GCATCTGGGT 120
AGCTGTAAGG CATGAAGGAT GCCAAGAAGT TTAAGGAATA TGGGTGGTAA ATGGCTAGGG 180
GACATGAGTC AGTCTA 196

(2) INFORMATION FOR SEQ ID NO:209:

(i) SEQUENCE CHARACTERISTICS:
(A) LENGTH: 345 base pairs
(B) TYPE: nucleic acid
(C) STRANDEDNESS: single
(D) TOPOLOGY: linear

(xi) SEQUENCE DESCRIPTION: SEQ ID NO:209:

GACGCTTGGC CACTTGACAC CTTTTATTTT TTAAGGATTC TTAAGTCATT TANGTNACTT 60
TGTAAGTTTT TCCTGTGCCC CCATAAGAAT GATAGCTTTA AAAATTATGC TGGGGTAGCA 120
AAGAAGATAC TTCTAGCTTT AGAATGTGTA GGTATAGCCA GGATTCTTGT GAGGAGGGGT 180
GATTTAGAGC AAATTTCTTA TTCTCCTTGC CTCATCTGTA ACATGGGGAT AATAATAGAA 240
CTGGCTTGAC AAGGTTGGAA TTAGTATTAC ATGGTAAATA CATGTAAAAT GTTTAGAATG 300
GTGCCAAGTA TCTAGGAAGT ACTTGGGCAT GGGTGGTAAA TGGCT 345

(2) INFORMATION FOR SEQ ID NO:210:

(i) SEQUENCE CHARACTERISTICS:
(A) LENGTH: 178 base pairs
(B) TYPE: nucleic acid
(C) STRANDEDNESS: single
(D) TOPOLOGY: linear

(xi) SEQUENCE DESCRIPTION: SEQ ID NO:210:

GACGCTTGGC CACTTGACAC TAGAGTAGGG TTTGGCCAAC TTTTTCTATA AAGGACCAGA 60
GAGTAAATAT TTCAGGCTTT GTGGGTTGTG CAGTCTCTCT TGCAACTACT CAGCTCTGCC 120
ATTGTAGCAT AGAAATCAGC CATAGACAGG ACAGAAATGA ATGGGTGGTA AATGGCTA 178

(2) INFORMATION FOR SEQ ID NO:211:

(i) SEQUENCE CHARACTERISTICS:
(A) LENGTH: 454 base pairs
(B) TYPE: nucleic acid
(C) STRANDEDNESS: single
(D) TOPOLOGY: linear

(xi) SEQUENCE DESCRIPTION: SEQ ID NO:211:

TGGGCACCTT CAATATCTAT CCAGCGCATC TAAATTCGCT TTTTTCTTGA TTAAAAATTT 60
CACCACTTGC TGTTTTTGCT CATGTATACC AAGTAGCAGT GGTGTGAGGC CATGCTTGTT 120
TTTTGATTCG ATATCAGCAC CGTATAAGAG CAGTGCTTTG GCCATTAATT TATCTTCATT 180
GTAGACAGCA TAGTGTAGAG TGGTATCTCC ATACTCATCT GGAATATTTG GATCAGTGCC 240
ATGTTCCAGC AACATTAACG CACATTCATC TTCCTGGCAT TGTACGGCCT TTGTCAGAGC 300
TGTCCTCTTT TTGTTGTCAA GGACATTAAG TTGACATCGT CTGTCCAGCA CGAGTTTTAC 360
TACTTCTGAA TTCCCATTGG CAGAGGCCAG ATGTAGAGCA GTCCTCTTTT GCTTGTCCCT 420
CTTGTTCACA TCAGTGTCCC TGAGCATAAC GGAA 454

(2) INFORMATION FOR SEQ ID NO:212:

(i) SEQUENCE CHARACTERISTICS:
(A) LENGTH: 337 base pairs
(B) TYPE: nucleic acid
(C) STRANDEDNESS: single
(D) TOPOLOGY: linear

(xi) SEQUENCE DESCRIPTION: SEQ ID NO:212:

TCCGTTATGC CACCCAGAAA ACCTACTGGA GTTACTTATT AACATCAAGG CTGGAACCTA 60
TTTGCCTCAG TCCTATCTGA TTCATGAGCA CATGGTTATT ACTGATCGCA TTGAAAACAT 120
TGATCACCTG GGTTTCTTTA TTTATCGACT GTGTCATGAC AAGGAAACTT ACAAACTGCA 180
ACGCAGAGAA ACTATTAAAG GTATTCAGAA ACGTGAAGCC AGCAATTGTT TCGCAATTCG 240
GCATTTTGAA AACAAATTTG CCGTGGAAAC TTTAATTTGT TCTTGAACAG TCAAGAAAAA 300
CATTATTGAG GAAAATTAAT ATCACAGCAT AACGGAA 337

(2) INFORMATION FOR SEQ ID NO:213:

(i) SEQUENCE CHARACTERISTICS:
(A) LENGTH: 715 base pairs
(B) TYPE: nucleic acid
(C) STRANDEDNESS: single
(D) TOPOLOGY: linear

(xi) SEQUENCE DESCRIPTION: SEQ ID NO:213:

TCGGGTGATG CCTCCTCAGG CATCTTCCAT CCATCTCTTC AAGATTAGCT GTCCCAAATG 60

TTTTTCCTTC TCTTCTTTAC TGATAAATTT GGACTCCTTC TTGACACTGA TGACAGCTTT 120
AGTATCCTTC TTGTCACCTT GCAGACTTTA AACATAAAAA TACTCATTGG TTTTAAAAGG 180
AAAAAAGTAT ACATTAGCAC TATTAAGCTT GGCCTTGAAA CATTTTCTAT CTTTTATTAA 240
ATGTCGGTTA GCTGAACAGA ATTCATTTTA CAATGCAGAG TGAGAAAAGA AGGGAGCTAT 300
ATGCATTTGA GAATGCAAGC ATTGTCAAAT AAACATTTTA AATGCTTTCT TAAAGTGAGC 360
ACATACAGAA ATACATTAAG ATATTAGAAA GTGTTTTTGC TTGTGTACTA CTAATTAGGG 420
AAGCACCTTG TATAGTTCCT CTTCTAAAAT TGAAGTAGAT TTTAAAAACC CATGTAATTT 480
AATTGAGCTC TCAGTTCAGA TTTTAGGAGA ATTTTAACAG GGATTTGGTT TTGTCTAAAT 540
TTTGTCAATT TNTTTAGTTA ATCTGTATAA TTTTATAAAT GTCAAACTGT ATTTAGTCCG 600
TTTTCATGCT GCTATGAAAG AAATACCCAN GACAGGGTTA TTTATAAANG GAAAGANGTT 660
AATTTGACTC CCAGTTCACA GGCCTGAGGA NGNATCNCCC GAAATCCTTA TTGCG 715

(2) INFORMATION FOR SEQ ID NO:214:

(i) SEQUENCE CHARACTERISTICS:
(A) LENGTH: 345 base pairs
(B) TYPE: nucleic acid
(C) STRANDEDNESS: single
(D) TOPOLOGY: linear

(xi) SEQUENCE DESCRIPTION: SEQ ID NO:214:

GGTAANGNGC ATACNTCGGT GCTCCGGCCG CCGGAGTCGG GGGATTCGGG TGATGCCTCC 60
TCAGGCCCAC TTGGGCCTGC TTTTCCCAAA TGGCAGCTCC TCTGGACATG CCATTCCTTC 120
TCCCACCTGC CTGATTCTTC ATATGTTGGG TGTCCCTGTT TTTCTGGTGC TATTTCCTGA 180
CTGCTGTTCA GCTGCCACTG TCCTGCAAAG CCTGCCTTTT TAAATGCCTC ACCATTCCTT 240
CATTTGTTTC TTAAATATGG GAAGTGAAAG TGCCACCTGA GGCCGGGCAC AGTGGCTCAC 300
GCCTGTAATC CCAGCACTTT GGGAGCCTGA GGAGGCATCA CCCGA 345

(2) INFORMATION FOR SEQ ID NO:215:

(i) SEQUENCE CHARACTERISTICS:
(A) LENGTH: 429 base pairs
(B) TYPE: nucleic acid
(C) STRANDEDNESS: single
(D) TOPOLOGY: linear

(xi) SEQUENCE DESCRIPTION: SEQ ID NO:215:

GGTGATGCCT CCTCAGGCGA AGCTCAGGGA GGACAGAAAC CTCCCGTGGA GCAGAAGGGC 60
AAAAGCTCGC TTGATCTTGA TTTTCAGTAC GAATACAGAC CGTGAAAGCG GGGCCTCACG 120
ATCCTTCTGA CCTTTTGGGT TTTAAGCAGG AGGTGTCAGA AAAGTTACCA CAGGGATAAC 180
TGGCTTGTGG CGGCCAAGCG TTCATAGCGA CGTCGCTTTT TGATCCTTCG ATGTCGGCTC 240
TTCCTATCAT TGTGAAGCAG AATTCACCAA GCGTTGGATT GTTCACCCAC TAATAGGGAA 300
CGTGAGCTGG GTTTAGACCG TCGTGAGACA GGTTAGTTTT ACCCTACTGA TGATGTGTKG 360
TTGCCATGGT AATCCTGCTC AGTACGAGAG GAACCGCAGG TTCASACATT TGGTGTATGT 420
GCTTGCCTT 429

(2) INFORMATION FOR SEQ ID NO:216:

(i) SEQUENCE CHARACTERISTICS:
(A) LENGTH: 593 base pairs
(B) TYPE: nucleic acid

(C) STRANDEDNESS: single
(D) TOPOLOGY: linear

(xi) SEQUENCE DESCRIPTION: SEQ ID NO:216:

TGACACCTAT GTCCNGCATC TGTTCACAGT TTCCACAAAT AGCCAGCCTT TGGCCACCTC 60
TCTGTCCTGA GGTATACAAG TATATCAGGA GGTGTATACC TTCTCTTCTC TTCCCCACCA 120
AAGAGAACAT GCAGGCTCTG GAAGCTGTCT TAGGAGCCTT TGGGCTCAGA ATTTCAGAGT 180
CTTGGGTACC TTGGATGTGG TCTGGAAGGA GAAACATTGG CTCTGGATAA GGAGTACAGC 240
CGGAGGAGGG TCACAGAGCC CTCAGCTCAA GCCCCTGTGC CTTAGTCTAA AAGCAGCTTT 300
GGATGAGGAA GCAGGTTAAG TAACATACGT AAGCGTACAC AGGTAGAAAG TGCTGGGAGT 360
CAGAATTGCA CAGTGTGTAG GAGTAGTACC TCAATCAATG AGGGCAAATC AACTGAAAGA 420
AGAAGACCNA TTAATGAATT GCTTANGGGG AAGGATCAAG GCTATCATGG AGATCTTTCT 480
AGGAAGATTA TTGTTTANAA TTATGAAAGG ANTAGGGCAG GGACAGGGCC AGAAGTANAA 540
GANAACATTG CCTATANCCC TTGTCTTGCA CCCAGATGCT GGACAAGGTG TCA 593

(2) INFORMATION FOR SEQ ID NO:217:

(i) SEQUENCE CHARACTERISTICS:
(A) LENGTH: 335 base pairs
(B) TYPE: nucleic acid
(C) STRANDEDNESS: single
(D) TOPOLOGY: linear

(xi) SEQUENCE DESCRIPTION: SEQ ID NO:217:

TGACACCTTG TCCAGCATCT GACGTGAAGA TGAGCAGCTC AGAGGAGGTG TCCTGGATTT 60
CCTGGTTCTG TGGGCTCCGT GGCAATGAAT TCTTCTGTGA AGTGGATGAA GACTACATCC 120
AGGACAAATT TAATCTTACT GGACTCAATG AGCAGGTCCC TCACTATCGA CAAGCTCTAG 180
ACATGATCTT GGACCTGGAG CCTGATGAAG AACTGGAAGA CAACCCCAAC CAGAGTGACC 240
TGATTGAGCA GGCAGCCGAG ATGCTTTATG GATTGATCCA CGCCCGCTAC ATCCTTACCA 300
ACCGTGGCAT CGCCCAGATG CTGGACAAGG TGTCA 335

(2) INFORMATION FOR SEQ ID NO:218:

(i) SEQUENCE CHARACTERISTICS:
(A) LENGTH: 248 base pairs
(B) TYPE: nucleic acid
(C) STRANDEDNESS: single
(D) TOPOLOGY: linear

(xi) SEQUENCE DESCRIPTION: SEQ ID NO:218:

TACGTACTGG TCTTGAAGGT CTTAGGTAGA GAAAAAATGT GAATATTTAA TCAAAGACTA 60
TGTATGAAAT GGGACTGTAA GTACAGAGGG AAGGGTGGCC CTTATCGCCA GAAGTTGGTA 120
GATGCGTCCC CGTCATGAAA TGTTGTGTCA CTGCCCGACA TTTGCCGAAT TACTGAAATT 180
CCGTAGAATT AGTGCAAATT CTAACGTTGT TCATCTAAGA TTATGGTTCC ATGTTTCTAG 240
TACTTTTA 248

(2) INFORMATION FOR SEQ ID NO:219:

(i) SEQUENCE CHARACTERISTICS:

(A) LENGTH: 530 base pairs
(B) TYPE: nucleic acid
(C) STRANDEDNESS: single
(D) TOPOLOGY: linear

(xi) SEQUENCE DESCRIPTION: SEQ ID NO:219:

TGACGCTTGG CCACTTGACA CAAGTAGGGG ATAAGGACAA AGACCCATNA GGTGGCCTGT 60
CAGCCTTTTG TTACTGTTGC TTCCCTGTCA CCACGGCCCC CTCTGTAGGG GTGTGCTGTG 120
CTCTGTGGAC ATTGGTGCAT TTTCACACAT ACCATTCTCT TTCTGCTTCA CAGCAGTCCT 180
GAGGCGGGAG CACACAGGAC TACCTTGTCA GATGANGATA ATGATGTCTG GCCAACTCAC 240
CCCCCAACCT TCTCACTAGT TATANGAAGA GCCANGCCTA NAACCTTCTA TCCTGNCCCC 300
TTGCCCTATG ACCTCATCCC TGTTCCATGC CCTATTCTGA TTTCTGGTGA ACTTTGGAGC 360
AGCCTGGTTT NTCCTCCTCA CTCCAGCCTC TCTCCATACC ATGGTANGGG GGTGCTGTTC 420
CACNCAAANG GTCAGGTGTG TCTGGGGAAT CCTNANANCT GCCNGGAGTT TCCNANGCAT 480
TCTTAAAAAC CTTCTTGCCT AATCANATNG TGTCCAGTGG CCAACCNTCN 530

(2) INFORMATION FOR SEQ ID NO:220:

(i) SEQUENCE CHARACTERISTICS:
(A) LENGTH: 531 base pairs
(B) TYPE: nucleic acid
(C) STRANDEDNESS: single
(D) TOPOLOGY: linear

(xi) SEQUENCE DESCRIPTION: SEQ ID NO:220:

TGACGCTTGG CCACTTGACA CTAAATAGCA TCTTCTAAAG GCCTGATTCA GAGTTGTGGA 60
AAATTCTCCC AGTGTCAGGG ATTGTCAGGA ACAGGGCTGC TCCTGTGCTC ACTTTACCTG 120
CTGTGTTTCT GCTGGAAAAG GAGGGAAGAG GAATGGCTGA TTTTTACCTA ATGTCTCCCA 180
GTTTTTCATA TTCTTCTTGG ATCCTCTTCT CTGACAACTG TTCCCTTTTG GTCTTCTTCT 240
TCTTGCTCAG AGAGCAGGTC TCTTTAAAAC TGAGAAGGGA GAATGAGCAA ATGATTAAAG 300
AAAACACACT TCTGAGGCCC AGAGATCAAA TATTAGGTAA ATACTAAACC GCTTGCCTGC 360
TGTGGTCACT TTTCTCCTCT TTCACATGCT CTATCCCTCT ATCCCCCACC TATTCATATG 420
GCTTTTATCT GCCAAGTTAT CCGGCCTCTC ATCAACCTTC TCCCCTAGCC TACTGGGGGA 480
TATCCATCTG GGTCTGTCTC TGGTGTATTG GTGTCAAGTG GCCAAGCGTC A 531

(2) INFORMATION FOR SEQ ID NO:221:

(i) SEQUENCE CHARACTERISTICS:
(A) LENGTH: 530 base pairs
(B) TYPE: nucleic acid
(C) STRANDEDNESS: single
(D) TOPOLOGY: linear

(xi) SEQUENCE DESCRIPTION: SEQ ID NO:221:

ATTGACGCTT GGCCACTTGA CACCCGCCTG CCTGCAATAC TGGGGCAAGG GCCTTCACTG 60
CTTTCCTGCC ACCAGCTGCC ACTGCACACA GAGATCAGAA ATGCTACCAA CCAAGACTGT 120
TGGTCCTCAG CCTCTCTGAG GAGAAAGAGC AGAAGCCTGG AAGTCAGAAG AGAAGCTAGA 180
TCGGCTACGG CCTTGGCAGC CAGCTTCCCC ACCTGTGGCA ATAAAGTCGT GCATGGCTTA 240
ACAATGGGGG CACCTCCTGA GAAACACATT GTTAGGCAAT TCGGCGTGTG TTCATCAGAG 300

CATATTTACA CAAACCTCGA TAGTGCAGCC TACTATCCAC TATTGCTCCT ACGCTGCAAA 360
CCTGAACAGC ATGGGACTGT ACTGAATACT GGAAGCAGCT GGTGATGGTA CTTATTTGTG 420
TATCTAAACA CAGAGAAGGT ACAGTAAGAA TATGGTATCA TAAACTTACA GGGACCGCCA 480
TCCTATATGC AGTCTGTTGT GACCAAAATG TGTCAAGTGG CCAAGCGTCA 530

(2) INFORMATION FOR SEQ ID NO:222:

(i) SEQUENCE CHARACTERISTICS:
(A) LENGTH: 578 base pairs
(B) TYPE: nucleic acid
(C) STRANDEDNESS: single
(D) TOPOLOGY: linear

(xi) SEQUENCE DESCRIPTION: SEQ ID NO:222:

TGTATCGACG TAGTGGTCTC CGGGCTACTA GGCCGTTGTG TGCTGGTAGT ACCTGGTTCA 60
CTGAAAGGCG CATCTCCCTC CCCGCGTCGC CCTGAAGCAG GGGGAGGACT TCGCCCAGCC 120
AAGGCAGTTG TATGAGTTTT AGCTGCGGCA CTTCGAGACC TCTGAGCCCA CCTCCTTCAG 180
GAGCCTTCCC CGATTAAGGA AGCCAGGGTA AGGATTCCTT CCTCCCCCAG ACACCACGAA 240
CAAACCACCA CCCCCCCTAT TCTGGCAGCC CATATACATC AGAACGAAAC AAAAATAACA 300
AATAAACNAA AACCAAAAAA AAAAGAGAAG GGGAAATGTA TATGTCTGTC CATCCTGTTG 360
CTTTAGCCTG TCAGCTCCTA NAGGGCAGGG ACCGTGTCTT CCGAATGGTC TGTGCAGCGC 420
CGACTGCGGG AAGTATCGGA GGAGGAAGCA GAGTCAGCAG AAGTTGAACG GTGGGCCCGG 480
CGGCTCTTGG GGGCTGGTGT TGTACTTCGA GACCGCTTTC GCTTTTTGTC TTAGATTTAC 540
GTTTGCTCTT TGGAGTGGGA NACCACTACN TCNATACA 578

(2) INFORMATION FOR SEQ ID NO:223:

(i) SEQUENCE CHARACTERISTICS:
(A) LENGTH: 578 base pairs
(B) TYPE: nucleic acid
(C) STRANDEDNESS: single
(D) TOPOLOGY: linear

(xi) SEQUENCE DESCRIPTION: SEQ ID NO:223:

TGTATCGACG TAGTGGTCTC CTCTTGCAAA GGACTGGCTG GTGAATGGTT TCCCTGAATT 60
ATGGACTTAC CCTAAACATA TCTTATCATC ATTACCAGTT GCAAAATATT AGAATGTGTT 120
GTCACTGTTT CATTTGATTC CTAGAAGGTT AGTCTTAGAT ATGTTACTTT AACCTGTATG 180
CTGTAGTGCT TTGAATGCAT TTTTTGTTTG CATTTTTGTT TGCCCAACCT GTCAATTATA 240
GCTGCTTAGG TCTGGACTGT CCTGGATAAA GCTGTTAAAA TATTCACCAG TCCAGCCATC 300
TTACAAGCTA ATTAAGTCAA CTAAATGCTT CCTTGTTTTG CCAGACTTGT TATGTCAATC 360
CTCAATTTCT GGGTTCATTT TGGGTGCCCT AAATCTTAGG GTGTGACTTT CTTAGCATCC 420
TGTAACATCC ATTCCCAAGC AAGCACAACT TCACATAATA CTTTCCAGAA GTTCATTGCT 480
GAAGCCTTTC CTTCACCCAG CGGAGCAACT TGATTTTCTA CAACTTCCCT CATCAGAGCC 540
ACAAGAGTAT GGGATATGGA GACCACTACG TCGATACA 578

(2) INFORMATION FOR SEQ ID NO:224:

(i) SEQUENCE CHARACTERISTICS:
(A) LENGTH: 345 base pairs
(B) TYPE: nucleic acid
(C) STRANDEDNESS: single
(D) TOPOLOGY: linear

(xi) SEQUENCE DESCRIPTION: SEQ ID NO:224:

TGTATCGACG TANTGGTCTC CCAAGGTGCT GGGATTGCAG GCATGAGCCA CCACTCCCAG 60
GTGGATCTTT TTCTTTATAC TTACTTCATT AGGTTTCTGT TATTCAAGAA GTGTAGTGGT 120
AAAAGTCTTT TCAATCTACA TGGTTAAATA ATGATAGCCT GGGAAATAAA TAGAAATTTT 180
TTCTTTCATC TTTAGGTTGA ATAAAGAAAC AGAAAAAATA GAACATACTG AAAATAATCT 240
AAGTTCCAAC CATAGAAGAA CTGCAGAAGA AATGAAGAAA GTGATGATGA TTTAGATTTT 300
GATATTGATT TAGAAGACAC AGGAGGAGAC CACTACGTCG ATACA 345

(2) INFORMATION FOR SEQ ID NO:225:

(i) SEQUENCE CHARACTERISTICS:
(A) LENGTH: 347 base pairs
(B) TYPE: nucleic acid
(C) STRANDEDNESS: single
(D) TOPOLOGY: linear

(xi) SEQUENCE DESCRIPTION: SEQ ID NO:225:

TGTATCGACG TAGTGGTCTC CAAACTGAGG TATGTGTGCC ACTAGCACAC AAAGCCTTCC 60
AACAGGGACG CAGGCACAGG CAGTTTAAAG GGAATCTGTT TCTAAATTAA TTTCCACCTT 120
CTCTAAGTAT TCTTTCCTAA AACTGATCAA GGTGTGAAGC CTGTGCTCTT TCCCAACTCC 180
CCTTTGACAA CAGCCTTCAA CTAACACAAG AAAAGGCATG TCTGACACTC TTCCTGAGTC 240
TGACTCTGAT ACGTTGTTCT GATGTCTAAA GAGCTCCAGA ACACCAAAGG GACAATTCAG 300
AATGCTGGTG TATAACAGAC TCCAATGGAG ACCACTACGT CGATACA 347

(2) INFORMATION FOR SEQ ID NO:226:

(i) SEQUENCE CHARACTERISTICS:
(A) LENGTH: 281 base pairs
(B) TYPE: nucleic acid
(C) STRANDEDNESS: single
(D) TOPOLOGY: linear

(xi) SEQUENCE DESCRIPTION: SEQ ID NO:226:

AGGNGNGGGA NTGTATCGAC GTAGTGGTCT CCCAACAGTC TGTCATTCAG TCTGCAGGTG 60
TCAGTGTTTT GGACAATGAG GCACCATTGT CACTTATTGA CTCCTCAGCT CTAAATGCTG 120
AAATTAAATC TTGTCATGAC AAGTCTGGAA TTCCTGATGA GGTTTTACAA AGTATTTTGG 180
ATCAATACTC CAACAAATCA GAAAGCCAGA AAGAGGATCC TTTCAATATT GCAGAACCAC 240
GAGTGGATTT ACACACCTCA GGAGACCACT ACGTCGATAC A 281

(2) INFORMATION FOR SEQ ID NO:227:

(i) SEQUENCE CHARACTERISTICS:
(A) LENGTH: 3646 base pairs
(B) TYPE: nucleic acid
(C) STRANDEDNESS: single
(D) TOPOLOGY: linear

(xi) SEQUENCE DESCRIPTION: SEQ ID NO:227:

GGGAAACACT TCCTCCCAGC CTTGTAAGGG TTGGAGCCCT CTCCAGTATA TGCTGCAGAA 60
TTTTTCTCTC GGTTTCTCAG AGGATTATGG AGTCCGCCTT AAAAAAGGCA AGCTCTGGAC 120
ACTCTGCAAA GTAGAATGGC CAAAGTTTGG AGTTGAGTGG CCCCTTGAAG GGTCACTGAA 180

CCTCACAATT GTTCAAGCTG TGTGGCGGGT TGTTACTGAA ACTCCCGGCC TCCCTGATCA 240
GTTTCCCTAC ATTGATCAAT GGCTGAGTTT GGTCAGGAGC ACCCCTTCCG TGGCTCCACT 300
CATGCACCAT TCATAATTTT ACCTCCAAGG TCCTCCTGAG CCAGACCGTG TTTTCGCCTC 360
GACCCTCAGC CGGTTCGGCT CGCCCTGTAC TGCCTCTCTC TGAAGAAGAG GAGAGTCTCC 420
CTCACCCAGT CCCACCGCCT TAAAACCAGC CTACTCCCTT AGGGTCATCC CATGTCTCCT 480
CGGCTATGTC CCCTGTAGGC TCATCACCCA TTGCCTCTTG GTTGCAACCG TGGTGGGAGG 540
AAGTAGCCCC TCTACTACCA CTGAGAGAGG CACAAGTCCC TCTGGGTGAT GAGTGCTCCA 600
CCCCCTTCCT GGTTTATGTC CCTTCTTTCT ACTTCTGACT TGTATAATTG GAAAACCCAT 660
AATCCTCCCT TCTCTGAAAA GCCCCAGGCT TTGACCTCAC TGATGGAGTC TGTACTCTGG 720
ACACATTGGC CCACCTGGGA TGACTGTCAA CAGCTCCTTT TGACCCTTTT CACCTCTGAA 780
GAGAGGGAAA GTATCCAAAG AGAGGCCAAA AAGTACAACC TCACATCAAC CAATAGGCCG 840
GAGGAGGAAG CTAGAGGAAT AGTGATTAGA GACCCAATTG GGACCTAATT GGGACCCAAA 900
TTTCTCAAGT GGAGGGAGAA CTTTTGACGA TTTCCACCGG TATCTCCTCG TGGGTATTCA 960
GGGAGCTGCT CAGAAACCTA TAAACTTGTC TAAGGCGACT GAAGTCGTCC AGGGGCATGA 1020
TGAGTCACCA GGAGTGTTTT TAGAGCACCT CCAGGAGGCT TATCAGATTT ACACCCCTTT 1080
TGACCTGGCA GCCCCCGAAA ATAGCCATGC TCTTAATTTG GCATTTGTGG CTCAGGCAGC 1140
CCCAGATAGT AAAAGGAAAC TCCAAAAACT AGAGGGATTT TGCTGGAATG AATACCAGTC 1200
AGCTTTTAGA GATAGCCTAA AAGGTTTTTG ACAGTCAAGA GGTTGAAAAA CAAAAACAAG 1260
CAGCTCAGGC AGCTGAAAAA AGCCACTGAT AAAGCATCCT GGAGTATCAG AGTTTACTGT 1320
TAGATCAGCC TCATTTGACT TCCCCTCCCA CATGGTGTTT AAATCCAGCT ACACTACTTC 1380
CTGACTCAAA CTCCACTATT CCTGTTCATG ACTGTCAGGA ACTGTTGGAA ACTACTGAAA 1440
CTGGCCGACC TGATCTTCAA AATGTGCCCC TAGGAAAGGT GGATGCCACC ATGTTCACAG 1500
ACAGTAGCAG CTTCCTCGAG AAGGGACTAC GAAAGGCCGG TGCAGCTGTT ACCATGGAGA 1560
CAGATGTGTT GTGGGCTCAG GCTTTACCAG CAAACACCTC AGCACAAAAG GCTGAATTGA 1620
TCGCCCTCAC TCAGGCTCTC CGATGGGGTA AGGATATTAA CGTTAACACT GACAGCAGGT 1680
ACGCCTTTGC TACTGTGCAT GTACGTGGAG CCATCTACCA GGAGCGTGGG CTACTCACCT 1740
CAGCAGGTGG CTGTAATCCA CTGTAAAGGA CATCAAAAGG AAAACACGGC TGTTGCCCGT 1800
GGTAACCAGA AAGCTGATTC AGCAGCTCAA GATGCAGTGT GACTTTCAGT CACGCCTCTA 1860
AACTTGCTGC CCACAGTCTC CTTTCCACAG CCAGATCTGC CTGACAATCC CGCATACTCA 1920
ACAGAAGAAG AAAACTGGCC TCAGAACTCA GAGCCAATAA AAATCAGGAA GGTTGGTGGA 1980
TTCTTCCTGA CTCTAGAATC TTCATACCCC GAACTCTTGG GAAAACTTTA ATCAGTCACC 2040
TACAGTCTAC CACCCATTTA GGAGGAGCAA AGCTACCTCA GCTCCTCCGG AGCCGTTTTA 2100
AGATCCCCCA TCTTCAAAGC CTAACAGATC AAGCAGCTCT CCGGTGCACA ACCTGCGCCC 2160
AGGTAAATGC CAAAAAAGGT CCTAAACCCA GCCCAGGCCA CCGTCTCCAA GAAAACTCAC 2220
CAGGAGAAAA GTGGGAAATT GACTTTACAG AAGTAAAACC ACACCGGGCT GGGTACAAAT 2280
ACCTTCTAGT ACTGGTAGAC ACCTTCTCTG GATGGACTGA AGCATTTGCT ACCAAAAACG 2340
AAACTGTCAA TATGGTAGTT AAGTTTTTAC TCAATGAAAT CATCCCTCGA CATGGGCTGC 2400
CTGTTTGCCA TAGGGTCTGA TAATGGACCG GCCTTCGCCT TGTCTATAGT TTAGTCAGTC 2460
AGTAAGGCGT TAAACATTCA ATGGAAGCTC CATTGTGCCT ATCGACCCCA GAGCTCTGGG 2520
CAAGTAGAAC GCATGAACTG CACCCTAAAA AACACTCTTA CAAAATTAAT CTTAGAAACC 2580
GGTGTAAATT GTGTAAGTCT CCTTCCTTTA GCCCTACTTA GAGTAAGGTG CACCCCTTAC 2640
TGGGCTGGGT TCTTACCTTT TGAAATCATG TATGGAGGGG TGCTGCCTAT CTTGCCTAAG 2700
CTAAGAGATG CCCAATTGGC AAAAATATCA CAAACTAATT TATTACAGTA CCTACAGTCT 2760
CCCCAACAGG TACAAGATAT CATCCTGCCA CTTGTTCGAG GAACCCATCC CAATCCAATT 2820
CCTGAACAGA CAGGGCCCTG CCATTCATTC CCGCCAGGTG ACCTGTTGTT TGTTAAAAAG 2880
TTCCAGAGAG AAGGACTCCC TCCTGCTTGG AAGAGACCTC ACACCGTCAT CACGATGCCA 2940
ACGGCTCTGA AGGTGGATGG CATTCCTGCG TGGATTCATC ACTCCCGCAT CAAAAAGGCC 3000
AACAGAGCCC AACTAGAAAC ATGGGTCCCC AGGGCTGGGT CAGGCCCCTT AAAACTGCAC 3060
CTAAGTTGGG TGAAGCCATT AGATTAATTC TTTTTCTTAA TTTTGTAAAA CAATGCATAG 3120
CTTCTGTCAA ACTTATGTAT CTTAAGACTC AATATAACCC CCTTGTTATA ACTGAGGAAT 3180
CAATGATTTG ATTCCCCCAA AAACACAAGT GGGGAATGTA GTGTCCAACC TGGTTTTTAC 3240
TAACCCTGTT TTTAGACTCT CCCTTTCCTT TAATCACTCA GCTTGTTTCC ACCTGAATTG 3300
ACTCTCCCTT AGCTAAGAGC GCCAGATGGA CTCCATCTTG GCTCTTTCAC TGGCAGCCGC 3360
TTCCTCAAGG ACTTAACTTG TGCAAGCTGA CTCCCAGCAC ATCCAAGAAT GCAATTAACT 3420
GATAAGATAC TGTGGCAAGC TATATCCGCA GTTCCCAGGA ATTCGTCCAA TTGATCACAG 3480

CCCCTCTACC CTTCAGCAAC CACCACCCTG ATCAGTCAGC AGCCATCAGC ACCGAGGCAA 3540
GGCCCTCCAC CAGCAAAAAG ATTCTGACTC ACTGAAGACT TGGATGATCA TTAGTATTTT 3600
TAGCAGTAAA GTTTTTTTTT CTTTTTCTTT CTTTTTTTCT CGTGCC 3646

(2) INFORMATION FOR SEQ ID NO:228:

(i) SEQUENCE CHARACTERISTICS:
(A) LENGTH: 419 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO:228:

TAAGAGGGTA CAAGATCTAA GCACAGCCGT CAATGCAGAA CACAGAACGT AGCCTGGTAA 60
GTGTGTTAAG AGTGGGAATT TTTGGAGTAC AGAGTAAGGC ACCTAACCCT AGCTGGGGTT 120
TGGTGACGGT CCCAGATGGC TTACAGAAGA AAGTGTCCTG AGATGAGTTT TTAAGAATGA 180
ATAAGGATAG ACACAAGTGA GGACTGACTT GGCAGTGGTG AATGGTGGGT GGCAAAAAAC 240
TTCGCATGTA TGGAAACTGC ACGTACAGGA ATGAAGAATG AGACTGTGTG GTGTTTAATG 300
AGCTGCAAAT ACTAATTTTA TCCTGAAAGT TTTGAAGAGT TAACTAAAAA GTATTTTTTA 360
GTAAGGAAAT AACCCTACAT TTCAGGGTTA TTGTTTGTTT ANATATTGAA GGTGCCCAA 419

(2) INFORMATION FOR SEQ ID NO:229:

(i) SEQUENCE CHARACTERISTICS:
(A) LENGTH: 148 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO:229:

AAGAGGGTAC CTGTATGTAG CCATGGTGGC AATGAGAGAC TGATTACTAC CTGCTGGAGA 60
TTGTTTAAGT GAGTTAATAT ATTAAGGATA AAGGGAGCCA GGTTTTTTGA CTGTTGGAGA 120
AGGAAATTAC AGATATTGAA GGTCCCAA 148

(2) INFORMATION FOR SEQ ID NO:230:

(i) SEQUENCE CHARACTERISTICS:
(A) LENGTH: 257 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo Sapiens

(xi) SEQUENCE DESCRIPTION: SEQ ID NO:230:

TAAGAGGGTA CMAAAAAAAA AAAATAGAAC GAATGAGTAA GACCTACTAT TTGATAGTAC 60
AACAGGGTGA CTATAGTCAA TGATAACTTA ATTATACATT TAACATAGAG TGTAATTGGA 120
TTGTTTGTAA CTCGAAGGAT AAATGCTTGA GAGGATGGAT ACCCCATTCT CCATGATGTA 180
CTTATTTCAC ATTACATGCC TGTATCAAAG CATCTCATAT ACCCTATAAA TATGTACACC 240
TACTATGTAC CCTCTTA 257

(2) INFORMATION FOR SEQ ID NO:231:

(i) SEQUENCE CHARACTERISTICS:
(A) LENGTH: 260 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO:231:

TAAGAGGGTA CGGGTATTTG CTGATGGGAT TTTTTTTTCT TTCTTTTTCT TTGGAAAACA 60
AAATGAAAGC CAGAACAAAA TTATTGAACA AAAGACAGGG ACTAAATCTG GAGAAATGAA 120
GTCCCCTCAC CTGACTGCCA TTTCATTCTA TCTGACCTTC CAGTCTAGGT TAGGAGAATA 180
GGGGGTGGAG GGGATTAATC TGATACAGGT ATATTTAAAG CAACTCTGCA TGTGTGCCAG 240
AAGTCCATGG TACCCTCTTA 260

(2) INFORMATION FOR SEQ ID NO:232:

(i) SEQUENCE CHARACTERISTICS:
(A) LENGTH: 596 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO:232:

TGCTCCTCTT GCCTTACCAA CCACAAATTA GAACCATAAT GAGATGTCAC CTCATACCTG 60
GTGGGATTAA CATTATTTAA AAAATCAGAA GTATTGACAA GGATGTGAAG AAATTAGAAC 120
ATCTGTGCAC TGTTGGTGGG AATGTAAAAA AGGTGTGGCC ACTATGGGTA ACAGCATGAA 180
GGTTCCTCAA AAAAAATTTT TTTTAATCTA CTCTATGATC GATCTTGAGG TTGTTTATGC 240
AAAAGAACTG AAATCAGGAT TTTGAGGAAA TATTCACATT CCCACATCCA TTTCTGCTTT 300
ATTCATAATA CTCAAGAGAT GGAAACAACC TAAATGTCCA TCCCGGGATG AATGGATAAA 360
CACAGTGTGG TATATGCATA CAATGGAATA TTATTTAGTC TTTAAAAAGA AAAATTCTAT 420

CATATACTAC AACTTANATN AACCTTGAGG ACACAATGCT NAGTGAAATA AGCCACGGAA 480
GGACGAATAC TGCATTATTC CCTTATATGA AGTATCTAAA GTGGTCAAAC TCTTANAGCA 540
NAAAGTAAAA ATGGGTGGTT GCCANACAGT TGGTTAGGCN AGAAGANAAN CCTANT 596

(2) INFORMATION FOR SEQ ID NO:233:

(i) SEQUENCE CHARACTERISTICS:
(A) LENGTH: 96 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO:233:

TCTTCTGAAG ACCTTTCGCG ACTCTTAAGC TCGTGGTTGG TAAGGCAAGA GGAGCGTTGG 60
TAAGGCAAGA GGAGCGTTGG TAAGGCAAGA GGAGCA 96

(2) INFORMATION FOR SEQ ID NO:234:

(i) SEQUENCE CHARACTERISTICS:
(A) LENGTH: 313 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO:234:

TGTAAGTCGA GCAGTGTGAT GATAAAACTT GAATGGATCA ATAGTTGCTT CTTATGGATG 60
AGCAAAGAAA GTAGTTTCTT GTGATGGAAT CTGCTCCTGG CAAAAATGCT GTGAACGTTG 120
TTGAAAAGAC AACAAAGAGT TTAGAGTAGT ACATAAATTT AGAATAGTAC ATAAACTTAG 180
AATAGTACAT AAACTTAGTA CATAAATAAT GCACGAAGCA GGGGCAGGGC TTGAGAGAAT 240
TGACTTCAAT TTGGAAAGAG TATCTACTGT AGGTTAGATG CTCTCAAACA GCATCACACT 300
GCTCGACTTA CAA 313

(2) INFORMATION FOR SEQ ID NO:235:

(i) SEQUENCE CHARACTERISTICS:
(A) LENGTH: 550 base pairs
(B) TYPE: nucleic acid
(C) STRANDEDNESS: single
(D) TOPOLOGY: linear

(ii) MOLECULE TYPE: cDNA

(vi) ORIGINAL SOURCE:

(A) ORGANISM: Homo sapiens

(xi) SEQUENCE DESCRIPTION: SEQ ID NO:235:

AACGAGGACA GATCCTTAAA AAGAATGTTG AGTGAAAAAA GTAGAAAATA AGATAATCTC 60
CAAAGTCCAG TAGCATTATT TAAACATTTT TAAAAAATAC ACTGATAAAA ATTTTGTACA 120
TTTCCCAAAA ATACATATGG AAGCACAGCA GCATGAATGC CTATGGGRTT GAGGATAGGG 180
GTTGGGAGTA GGGATGGGGA TAAAGGGGGA AAATAAAACC AGAGAGGAGT CTTACACATT 240
TCATGAACCA AGGAGTATAA TTATTTCAAC TATTTGTACC WGAAGTCCAG AAAGAGTGGA 300
GGCAGAAGGG GGAGAAGAGG GCGAAGAAAC GTTTTTGGGA GAGGGGTCCC ASAAGAGAGA 360
TTTTCGCGAT GTGGCGCTAC ATACGTTTTT CCAGGATGCC TTAAGCTCTG CACCCTATTT 420
TTCTCATCAC TAATATTAGA TTAAACCCTT TGAAGACAGC GTCTGTGGTT TCTCTACTTC 480
AGCTTTCCCT CCGTGTCTTG CACACAGTAG CTGTTTTACA AGGGTTGAAC TGACTGAAGT 540
GAGATTATTC 550

(2) INFORMATION FOR SEQ ID NO:236:

(i) SEQUENCE CHARACTERISTICS:
(A) LENGTH: 325 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO:236:

TAGACTGACT CATGTCCCCT ACCAGAGTAG CTAGAATTAA TAGCACAAGC CTCTACACCC 60
AGGAACTCAC TATTGAATAC ATAAATGGAA TTTATTCAGC CTTAAAAAGT TTGGAAGGAA 120
ATTCTGACAT ATGCTAAAAC ATGGATGAAC CTTGAAGACT TTATGATAAG TAAAAGAAGC 180
CAGTCATAAA AGGAAAAATA TTGCATGATT CCACTTATAT GAGGTACCTA GAGTAGTCAA 240
TTTCATAGAA ACACAAAATA GAATGGTGTT TGCCAGGGCT TTTGAGGAAA AGGGAATGAC 300
AAGTTAGGGG ACATGAGTCA GTCTA 325

(2) INFORMATION FOR SEQ ID NO:237:

(i) SEQUENCE CHARACTERISTICS:
(A) LENGTH: 373 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO:237:

TAGACTGACT CATGTCCCCT ATCTACTCAA CATTTCCACT TGAAGTCTGA TAGGCATCTC 60
AGACTTATCT TGTCCCAAAG CAAACTCTTT ATTTCTTTTC ATCCTAGTCT TTATTTCTTG 120
TGCTGTCTTA CCCATCTCAA AAGAGTGCCA AAATCCACCA AGTTGCTGAA ACAGAAATCT 180

AAGAAATATC CTTGATTCTT CTTTTTCCCA TCTACTTCAC TTCTAATTCA TTAGTAAATA 240
ATCTGTTTCA GAAAACCAAA CACCTCATGT TCTCACTCAT AAGGGGGAGT TGAACAATGA 300
GAACACACAG ACACAGGGAG GGGAACATCA CACACCACGG CCCGTCAGGG AGTANGGGAC 360
ATGAGTCAGT CTA 373

(2) INFORMATION FOR SEQ ID NO:238:

(i) SEQUENCE CHARACTERISTICS:
(A) LENGTH: 492 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO:238:

TAGACTGACT CATGTCCCCT ATAATGCTCC CAGGCATCAG AAAGCATCTC AAACTGGAGC 60
TGACACCATG GCAGAGGTTT CAGGTAAGTC ACAAAAGGGG TCCTAAAGAA TTTGCCCTCA 120
ATATCAGAGT GATTAGAAGA AGTGGACAGA GCTACCCAAG TTAAACATAT GCGAGATAAA 180
AAAAATATGG CACTTGTGAA CACACACTAC AGGAGGAAAA TAAGGAACAT AATAGCATAT 240
TGTGCTATTA TGATGATGAA GAACCTCTCT ANAAGAAAAC ATAACCAAAG AAACAAAGAA 300
AATTCCTGCN AATGTTTAAT GCTATAGAAG AAATTAACAA AAACATATAT TCAATGAATT 360
CAGAAAAGTT AGCAGGTCAN AAGAAAACAA ATCAAAGACC AGAATAATCC CATTTTAGAT 420
TGTCGAGTAA ACTANAACAG AAAGAATACC ACTGGAAATT GAATTCCTAC GTANGGGACA 480
TGANTCANTC TA 492

(2) INFORMATION FOR SEQ ID NO:239:

(i) SEQUENCE CHARACTERISTICS:
(A) LENGTH: 482 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO:239:

TGGAAAGTAT TTAATGATGG GCAACTTGCT GTTTACTTCC TACATATCCC ATCATCTTCT 60
GTATTTTTTT AAATAACTTT TTTTTGGATT TTTAAAGTAA CCTTATTCTG AGAGGTAACA 120
TGGATTACAT ACTTCTAAGC CATTAGGAGA CTCTATGTTA AACCAAAAGG AAATGTTACT 180
AGATCTTCAT TTGATCAATA GGATGTGATA ATCATCATCT TTCTGCTCTA ATGGAAAAGT 240
ACTANAAACA TGGAACCATA ATCTTAGATG AACAACGTTA GAATTTGCAC TAATTCTACG 300
GAATTTCAGT AATTCGGCAA ATGTCGGGCA GTGACACAAC ATTTCATGAC GGGGACGCAT 360
CTACCAACTT CTGGCGATAA GGGCCACCCT TCCCTCTGTA CTTACAGTCC CATTTCATAC 420
ACAGTCTTTG ATTAAATATT CACATTTTTT CTCTACCTAA AGACCTTCAA GACCAGTACG 480
TA 482

(2) INFORMATION FOR SEQ ID NO:240:

(i) SEQUENCE CHARACTERISTICS:
(A) LENGTH: 519 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO:240:

TGTATCGACG TAGTGGTCTC CCCATGTGAT AGTCTGAAAT ATAGCCTCAT GGGATGAGAG 60
GCTGTGCCCC AGCCCGACAC CCGTAAAGGG TCTGTGCTGA GGTGGATTAG TAAAAGAGGA 120
AAGCCTTGCA GTTGAGATAG AGGAAGGGCA CTGTCTCCTG CCTGCCCCTG GGAACTGAAT 180
GTCTCGGTAT AAAACCCGAT TGTACATTTG TTCAATTCTG AGATAGGAGA AAAACCACCC 240
TATGGCGGGA GGCGAGACAT GTTGGCAGCA ATGCTGCCTT GTTATGCTTT ACTCCACAGA 300
TGTTTGGGCG GAGGGAAACA TAAATCTGGC CTACGTGCAC ATCCAGGCAT AGTACCTCCC 360
TTTGAACTTA ATTATGACAC AGATTCCTTT GCTCACATGT TTTTTTGCTG ACCTTCTCCT 420
TATTATCACC CTGCTCTCCT ACCGCATTCC TTGTGCTGAG ATAATGAAAA TAATATCAAT 480
AAAAACTTGA NGGAACTCGG AGACCACTAC GTCGATACA 519

(2) INFORMATION FOR SEQ ID NO:241:

(i) SEQUENCE CHARACTERISTICS:
(A) LENGTH: 771 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO:241:

TGTATCGACG TAGTGGTCTC CACTCCCGCC TTGACGGGGC TGCTATCTGC CTTCCAGGCC 60
ACTGTCACGG CTCCCGGGTA GAAGTCACTT ATGAGACACA CCAGTGTGGC CTTGTTGGCT 120
TGAAGCTCCT CAGAGGAGGG TGGGAACAGA GTGACCGAGG GGGCAGCCTT GGGCTGACCT 180
AGGACGGTCA GCTTGGTCCC TCCGCCAAAC ACGAGAGTGC TGCTGCTTGT ATATGAGCTG 240
CAGTAATAAT CAGCCTCGTC CTCAGCCTGG AGCCCAGAGA TGGTCAGGGA GGCCGTGTTG 300
CCANACTTGG AGCCAGAGAA GCGATTAGAA ACCCCTGAGG GCCGATTACC GACCTCATAA 360
ATCATGAATT TGGGGGCTTT GCCTGGGTGC TGTTGGTACC ANGAGACATT ATTATAACCA 420
CCAACGTCAC TGCTGGTTCC ANTGCAGGGA AAATGGTTGA TCNAACTGTC CAAGAAAACC 480
ACTACGTCCA TACCAATCCA CTAATTGCCN GCCGCCTGCA GGTTCAACCA TATTGGGGAA 540
NAACTCCCCN CCGCCGTTTG GGATTGNCAT NAACCTTTGA AATTTTTTCC TATTANTTGT 600
CCCCCTAAAA TAAACCNTTG GGCNTTAATC CATTGGGTCC ATANCTTNTT TNCCCGGTTT 660
TTAAAANTTG TTTATCCCGC CNCCCNATTT CCCCCCCAAC TTTCCAAAAC CCGAAACCNT 720
TNAAATTTNT TNAAACCCTG GGGGGTTCCC NNAATTNNAN TTNAANCTNC C 771

(2) INFORMATION FOR SEQ ID NO:242:

(i) SEQUENCE CHARACTERISTICS:
(A) LENGTH: 167 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO:242:

TGGGCACCTT CAATATCGGG CTCATCGATA ACATCACGCT GCTGATGCTG CTGTTGCTGG 60
TCCTCTCTAG GAACCTCTGG ATTTTCAAAT TCTTTGAGGA ATTCATCCAA ATTATCTGCC 120
TCTCCTCCTT TCCTCCTTTT TCTAAGGTCT TCTGGTACAA GCGGTCA 167

(2) INFORMATION FOR SEQ ID NO:243:

(i) SEQUENCE CHARACTERISTICS:
(A) LENGTH: 338 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO:243:

TTGGGCACCT TCAATATCTA CTGATCTAAA TAGTGTGGTT TGAGGCCTCT TGTTCCTGGC 60
TAAAAATCCT TGGCAAGAGT CAATCTCCAC TTTACAATAG AGGTAAAAAT CTTACAATGG 120
ATATTCTTGA CAAAGCTAGC ATAGAGACAG CAATTTTACA CAAGGTATTT TTCACCTGTT 180
TAATAACAGT GGTTTTCCTA CACCCATAGG GTGCCACCAA GGGAGGAGTG CACAGTTGCA 240
GAAACAAATT AAGATACTGA AGACAACACT ACTTACCATT TCCCGTATAG CTAACCACCA 300
GTTCAACTGT ACATGTATGT TCTTATGGGC AATCAAGA 338

(2) INFORMATION FOR SEQ ID NO:244:

(i) SEQUENCE CHARACTERISTICS:
(A) LENGTH: 346 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO:244:

TTTTTGGCTC CCATACAGCA CACTCTCATG GGAAATGTCT GTTCTAAGGT CAACCCATAA 60
TGCAAAAATC ATCAATATAC TTGAAGATCC CCGTGTAAGG TACAATGTAT TTAATATTAT 120
CACTGATACA ATTGATCCAA TACCAGTTTT AGTCTGGCAT TGAATCAAAT CACTGTTTTT 180
GTTGTATAAA AAGAGAAATA TTTAGCTTAT ATTTAAGTAC CATATTGTAA GAAAAAAGAT 240
GCTTATCTTT ACATGCTAAA ATCATGATCT GTACATTGGT GCAGTGAATA TTACTGTAAA 300
AGGGAAGAAG GAATGAAGAC GAGCTAAGGA TATTGAAGGT GCCCAA 346

(2) INFORMATION FOR SEQ ID NO:245:

(i) SEQUENCE CHARACTERISTICS:
(A) LENGTH: 521 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO:245:

ACCAATCCCA CACGGATACT GAGGGACAAG TATATCATCC CATTTCATCC CTACAGCAGC 60
AACTTCATGA GGCAGGAGTT ATTAGTCCCA TTTTACAGAA GAGGAAACTG AGACTTAGGG 120
AGATCAAGTA ATTTGCCCAG GTCGCACAAT TAGTGATAGA GCCAGGGCTT GAAGCGACGT 180
CTGTCTTAAG CCAATGACCC CTGCAGATTA TTAGAGCAAC TGTTCTCCAC AACAGTGTAA 240
GCCTCTTGCT ANAAGCTCAG GTCCACAAGG GCAGAGATTT TTGTCTGTTT TGCTCATTGC 300
TCCTTCCCCA TTGCTTAGAG CAGGGTCTGC CACGAANCAG GTTCTCAATG CATAGTTATT 360
AAATGTATAT AAGAGCAAAC ATATGTTACA GAGAACTTTC TGTATGCTTG TCACTTACAT 420
GAATCACCTG TGANATGGGT ATGCTTGTTC CCCANTGTTG CAGATNAAGA TATTGAANGT 480
GCCCAAATCA CTANTTGCGG GCGCCTGCAN GTCCANCATA T 521

(2) INFORMATION FOR SEQ ID NO:246:

(i) SEQUENCE CHARACTERISTICS:
(A) LENGTH: 482 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO:246:

TGGAACCAAT CCAAATACCC ATCAATGATA GACTGGATAA AGAAAATTTG GCACATGTTC 60
ACCATGAAAT ACTATGCAGC CATAAAAAAG GATGAGTTCA TATCCTTTGC AGGGACATGG 120
ATGAAGCTGG AGACCATCAT TCTCAGCAAA CTAACAAGGG AACAGAAAAC CAAACACTGC 180
ATGTTCTCAC TCTTAAGTGG GAGCTGAACA ATGAGAACAC ATGGACACAG GGAGGGGAAC 240
ATCACACAGT GGGGCCTGCT GGTGGGTAGG GGTCTAGGGG AGGGATAGCA TTAGGAGAAA 300
TACCTAATGT AGATGACGGG TTGATGGGTG CAGCAAACCA CCATGACACG TGTATACCTA 360
TGTAACAAAC CTGCATGTTC TGCACATGTA CCCCAGAACT TAAAGTGTTA ATAAAAAAAT 420
TAAGAAAAAA GTTAAGTATG TCATAGATAC ATAAAATATT GTANATATTG AAGGTGCCCA 480

AA 482

(2) INFORMATION FOR SEQ ID NO:247:

(i) SEQUENCE CHARACTERISTICS:
(A) LENGTH: 474 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO:247:

TTCGATACAG GCACAGAGTA AGCAGAAAAA TGGCTGTGGT TTAACCAAGT GAGTACAGTT 60
AAGTGAGAGA GGGGCAGAGA AGACAAGGGC ATATGCAGGG GGTGATTATA ACAGGTGGTT 120
GTGCTGGGAA GTGAGGGTAC TCGGGGATGA GGAACAGTGA AAAAGTGGCA AAAAGTGGTA 180
AGATCAGTGA ATTGTACTTC TCCAGAATTT GATTTCTGGN GGAGTCAAAT AACTATCCAG 240
TTTGGGGTAT CATANGGCAA CAGTTGAGGT ATAGGAGGTA GAAGTCNCAG TGGGATAATT 300
GAGGTTATGA ANGGTTTGGT ACTGACTGGT ACTGACAANG TCTGGGTTAT GACCATGGGA 360
ATGAATGACT GTANAAGCGT ANAGGATGAA ACTATTCCAC GANAAAGGGG TCCNAAAACT 420
AAAAANNNAA GNNNNNGGGG AATATTATTT ATGTGGATAT TGAANGTGCC CAAA 474

(2) INFORMATION FOR SEQ ID NO:248:

(i) SEQUENCE CHARACTERISTICS:
(A) LENGTH: 355 base pairs
(B) TYPE: nucleic acid
(C) STRANDEDNESS: single
(D) TOPOLOGY: linear

(xi) SEQUENCE DESCRIPTION: SEQ ID NO:248:

TTCGATACAG GCAAACATGA ACTGCAGGAG GGTGGTGACG ATCATGATGT TGCCGATGGT 60
CCGGATGGNC ACGAAGACGC ACTGGANCAC GTGCTTACGT CCTTTTGCTC TGTTGATGGC 120
CCTGAGGGGA CGCAGGACCC TTATGACCCT CAGAATCTTC ACAACGGGAG ATGGCACTGG 180
ATTGANTCCC ANTGACACCA GAGACACCCC AACCACCAGN ATATCANTAT ATTGATGTAG 240
TTCCTGTAGA NGGCCCCCTT GTGGAGGAAA GCTCCATNAG TTGGTCATCT TCAACAGGAT 300
CTCAACAGTT TCCGATGGCT GTGATGGGCA TAGTCATANT TAACCNTGTN TCGAA 355

(2) INFORMATION FOR SEQ ID NO:249:

(i) SEQUENCE CHARACTERISTICS:
(A) LENGTH: 434 base pairs
(B) TYPE: nucleic acid
(C) STRANDEDNESS: single
(D) TOPOLOGY: linear

(xi) SEQUENCE DESCRIPTION: SEQ ID NO:249:

TTGGATTGGT CCTCCAGGAG AACAAGGGGA AAAAGGTGAC CGAGGGCTCC CTGGAACTCA 60
AGGATCTCCA GGAGCAAAAG GGGATGGGGG AATTCCTGGT CCTGCTGGTC CCTTAGGTCC 120

ACCTGGTCCT CCAGGCTTAC CAGGTCCTCA AGGCCCAAAG GGTAACAAAG GCTCTACTGG 180
ACCCGCTGGC CAGAAAGGTG ACAGTGGTCT TCCAGGGCCT CCTGGGCCTC CAGGTCCACC 240
TGGTGAAGTC ATTCAGCCTT TACCAATCTT GTCCTCCAAA AAAACGAGAA GACATACTGA 300
AGGCATGCAA GCAGATGCAG ATGATAATAT TCTTGATTAC TCGGATGGAA TGGAAGAAAT 360
ATTTGGTTCC CTCAATTCCC TGAAACAAGA CATCGAGCAT ATGAAATTTC CAATGGGTAC 420
TCAGACCAAT CCAA 434

(2) INFORMATION FOR SEQ ID NO:250:

(i) SEQUENCE CHARACTERISTICS:
(A) LENGTH: 430 base pairs
(B) TYPE: nucleic acid
(C) STRANDEDNESS: single
(D) TOPOLOGY: linear

(xi) SEQUENCE DESCRIPTION: SEQ ID NO:250:

TGGATTGGTC ACATGGCAGA GACAGGATTC CAAGGCAGTG AGAGGAGGAT ACAATGCTTC 60
TCACTAGTTA TTATTATTTA TTTTATTTTT GAGATGAAGT CTCGCTTTGT CTCCCAGGCT 120
GGAGAGCGGT GGTGCGATCT TGGCTCTCTG CAACCCCCGC CTCAAGCAAT TCTCCTGTCT 180
TAGCCTCGCG GGTAGATGGA ATTACAGGCG CCCACCGCCA TGCCCAACTA ATTTTTTTGT 240
GTCTTCAGTA GAGACAGGGT TTCGCCATGT TGGGCAGGCT GGTCTTGAAC TCCTGACCTC 300
NAGTGATCTG CCCTCCTCGG CCTCACAAAG TGCTGGAATT ACAGGCATGG GCTGCTGCAC 360
CCAGTCAACT TCTCACTAGT TATGGCCTTA TCATTTTCAC CACATTCTAT TGGCCCAAAA 420
AAAAAAAAAN 430

(2) INFORMATION FOR SEQ ID NO:251:

(i) SEQUENCE CHARACTERISTICS:
(A) LENGTH: 329 base pairs
(B) TYPE: nucleic acid
(C) STRANDEDNESS: single
(D) TOPOLOGY: linear

(xi) SEQUENCE DESCRIPTION: SEQ ID NO:251:

TGGTACTCCA CCATYATGGG GTCAACCGCC ATCCTCGCCC TCCTCCTGGC TGTTCTCCAA 60
GGAGTCTGTG CCGAGGTGCA GCTGRTGCAG TCTGGAGCAG AGGTGAAAAA GTCCGGGGAG 120
TCTCTGAAGA TCTCCTGTAA GGGTTCTGGA TACACCTTTA AGATCTACTG GATCGCCTGG 180
GTGCGCCAGT TGCCCGGGAA AGGCCTGGAG TGGATGGGGC TCATCTTTCC TGATGACTCT 240
GATACCAGAT ACAGCCCGTC CTTCCAAGGC CAGGTCACCA TCTCAGTCGA TAAGTCCATC 300
AGCACCGCCT ATCTGCAGTG GAGTACCAA 329

(2) INFORMATION FOR SEQ ID NO:252:

(i) SEQUENCE CHARACTERISTICS:
(A) LENGTH: 536 base pairs
(B) TYPE: nucleic acid
(C) STRANDEDNESS: single
(D) TOPOLOGY: linear

(xi) SEQUENCE DESCRIPTION: SEQ ID NO:252:

TGGTACTCCA CTCAGCCCAA CCTTAATTAA GAATTAAGAG GGAACCTATT ACTATTCTCC 60
CAGGCTCCTC TGCTCTAACC AGGCTTCTGG GACAGTATTA GAAAAGGATG TCTCAACAAG 120

TATGTAGATC CTGTACTGGC CTAAGAAGTT AAACTGAGAA TAGCATAAAT CAGACCAAAC 180
TTAATGGTCG TTGAGACTTG TGTCCTGGAG CAGCTGGGAT AGGAAAACTT TTGGGCAGCA 240
AGAGGAAGAA CTGCCTGGAA GGGGGCATCA TGTTAAAAAT TACAAGGGGA ACCCACACCA 300
GGCCCCCTTC CCAGCTCTCA GCCTAGAGTA TTAGCATTTC TCAGCTAGAG ACTCACAACT 360
TCCTTGCTTA GAATGTGCCA CCGGGGGGAG TCCCTGTGGG TGATGAGGCT CTCAAGAGTG 420
AGAGTGGCAT CCTATCTTCT GTGTGCCCAC AGGAGCCTGG CCCGAGACTT AGCAGGTGAA 480
GTTTCTGGTC CAGGCTTTGC CCTTGACTCA CTATGTGACC TCTGGTGGAG TACCAA 536

(2) INFORMATION FOR SEQ ID NO:253:

(i) SEQUENCE CHARACTERISTICS:
(A) LENGTH: 507 base pairs
(B) TYPE: nucleic acid
(C) STRANDEDNESS: single
(D) TOPOLOGY: linear

(xi) SEQUENCE DESCRIPTION: SEQ ID NO:253:

NTGTTGCGAT CCCAGTAACT CGGGAAGCTG AGGCGGGAGG ATCACCTGAG CTCAGGAGGT 60
TGAGGCCGCA GTGAGCCGGG ACCACGCCAC TACACTCCAG CCTGGGGCAT AGAGTGAGAC 120
CCTCCAAGAC AGAAAAGAAA AGAAAGGAAG GGAAAGGGAA AGGGAAAAGG AAAAGGAAAA 180
GGAAAAGGAA AAGGAAAAGA CAAGACAAAA CAAGACTTGA ATTTGGATCT CCTGACTTCA 240
ATTTTATGTT CTTTCTACAC CACAATTCCT CTGCTTACTA AGATGATAAT TTAGAAACCC 300
CTCGTTCCAT TCTTTACAGC AAGCTGGAAG TTTGGTCAAG TAATTACAAT AATAGTAACA 360
AATTTGAATA TTATATGCCA GGTGTTTTTC ATTCCTGCTC TCACTTAATT CTCACCACTC 420
TGATATAAAT ACAATTGCTG CCGGGTGTGG TGGCTCATGC CTGTAATCCC GGCACTTTGG 480
GAGACCGAGG TGGGCGGATS GCAACAA 507

(2) INFORMATION FOR SEQ ID NO:254:

(i) SEQUENCE CHARACTERISTICS:
(A) LENGTH: 222 base pairs
(B) TYPE: nucleic acid
(C) STRANDEDNESS: single
(D) TOPOLOGY: linear

(xi) SEQUENCE DESCRIPTION: SEQ ID NO:254:

TTGGATTGGT CACTGTGAGG AAGCCAAATC GGATCCGAGA GTCTTTTTCT AAAGGCCAGT 60
ACTGGCCACA CTTTCTCCTG CCGCCTTCCT CAAAGCTGAA GACACACAGA GCAAGGCGCT 120
TCTGTTTTAC TCCCCAATGG TAACTCCAAA CCATAGATGG TTAGCTNCCC TGCTCATCTT 180
TCCACATCCC TGCTATTCAG TATAGTCCGT GGACCAATCC AA 222

(2) INFORMATION FOR SEQ ID NO:255:

(i) SEQUENCE CHARACTERISTICS:
(A) LENGTH: 463 base pairs
(B) TYPE: nucleic acid
(C) STRANDEDNESS: single
(D) TOPOLOGY: linear

(xi) SEQUENCE DESCRIPTION: SEQ ID NO:255:

TGTTGCGATC CATAAATGCT GAAATGGAAA TAAACAACAT GATGAGGGAG GATTAAGTTG 60
GGGAGGGAGC ACATTAAGGT GGCCATGAAG TTTGTTGGAA GAAGTGACTT TTGAACAAGG 120

CCTTGGTGTT AAGAGCTGAT GAGAGTGTCC CAGACAGAGG GGCCACTGGT ACAATAGACG 180
AGATGGGAGA GGGCTTGGAA GGTGTGCGAA ATAGGAAGGA GTTTGTTCTG GTATGAGTCT 240
AGTGAACACA GAGGCGAGAG GCCCTGGTGG GTGCAGCTGG AGAGTTATGC AGAATAACAT 300
TAGGCCCTGT GGGGGACTGT AGACTGTCAG CAATAATCCA CAGTTTGGAT TTTATTCTAA 360
GAGTGATGGG AAGCCGTGGA AAGGGGGTTA AGCAAGGAGT GAAATTATCA GATTTACAGT 420
GATAAAAATA AATTGGTCTG GCTACTGGGG AAAAAAAAAA AAA 463

(2) INFORMATION FOR SEQ ID NO:256:

(i) SEQUENCE CHARACTERISTICS:
(A) LENGTH: 262 base pairs
(B) TYPE: nucleic acid
(C) STRANDEDNESS: single
(D) TOPOLOGY: linear

(xi) SEQUENCE DESCRIPTION: SEQ ID NO:256:

TTGGATTGGT CAACCTGCTC AACTCTACYT TTCCTCCTTC TTCCTAAAAA ATTAATGAAT 60
CCAATACATT AATGCCAAAA CCCTTGGGTT TTATCAATAT TTCTGTTAAA AAGTATTATC 120
CAGAACTGGA CATAATACTA CATAATAATA CATAACAACC CCTTCATCTG GATGCAAACA 180
TCTATTAATA TAGCTTAAGA TCACTTTCAC TTTACAGAAG CAACATCCTG TTGATGTTAT 240
TTTGATGTTT GGACCAATCC AA 262

(2) INFORMATION FOR SEQ ID NO:257:

(i) SEQUENCE CHARACTERISTICS:
(A) LENGTH: 461 base pairs
(B) TYPE: nucleic acid
(C) STRANDEDNESS: single
(D) TOPOLOGY: linear

(xi) SEQUENCE DESCRIPTION: SEQ ID NO:257:

GNGGNNNNNN NNNCAATTCG ACTCNGTTCC CNTGGTANCC GGTCGACATG GCCGCGGGAT 60
TACCGCTTGT NNCTGGGGGT GTATGGGGGA CTATGACCGC TTGTAGCTGG GGGTGTATGG 120
GGGACTATGA CCGCTTGTAG MTGGKGGTGT ATGGGGGACT ATGACCGCTT GTCGGGTGGT 180
CGGATAAACC GACGCAAGGG ACGTGATCGA AGCTGCGTTC CCGCTCTTTC GCATCGGTAG 240
GGATCATGGA CAGCAATATC CGCATTCGYC TGAAGGCGTT CGACCATCGC GTGCTCGATC 300
AGGCGACCGG CGACATCGCC GACACCGCAC GCCGTACCGG CGCGCTCATC CGCGGTCCGA 360
TCCCGCTTCC CACGCGCATC GAGAAGTTCA CGGTCAACCG TGGCCCGCAC GTCGACAAGA 420
AGTCGCGCGA GCAGTTCGAG GTGCGTACCT ACAAGCGGTC A 461

(2) INFORMATION FOR SEQ ID NO:258:

(i) SEQUENCE CHARACTERISTICS:
(A) LENGTH: 332 base pairs
(B) TYPE: nucleic acid
(C) STRANDEDNESS: single
(D) TOPOLOGY: linear

(xi) SEQUENCE DESCRIPTION: SEQ ID NO:258:

TGACCGCTTG TAGCTGGGGG TGTATGGGGG ACTACGACCG CTTGTAGCTG GGGGTGTATG 60
GGGGACTATG ACCGCTTGTA GCTGGGGGTG TATGGGGGAC TATGACCGCT TGTAGCTGGG 120
GGTGTATGGG GGACTAGGAC CGCTTGTAGC TGGGGGTGTA TGGGGGACTA TGACCGCTTG 180

TAGCTGGGGG TGTATGGGGG ACTACGACCG CTTGTAGCTG GGGGTGTATG GGGGACTATG 240
ACCGCTTGTA NCTGGGGGTG TATGGGGGAC TATGACCGCT TGTGCTGCCT GGGGGATGGG 300
AGGAGAGTTG TGGTTGGGGA AAAAAAAAAA AA 332

(2) INFORMATION FOR SEQ ID NO:259:

(i) SEQUENCE CHARACTERISTICS:
(A) LENGTH: 291 base pairs
(B) TYPE: nucleic acid
(C) STRANDEDNESS: single
(D) TOPOLOGY: linear

(xi) SEQUENCE DESCRIPTION: SEQ ID NO:259:

TACCGCTTGT GACCGCTTGT GACCGCTTGT GACCGCTTGT GACCGCTTGT GACCGCTTGT 60
GACCGCTTGT GACCGCTTGT GACCGCTTGT GACCGCTTGT GACCGCTTGT GACCGCTTGT 120
GACCGCTTGT GACCGCTTGT NACNGGGGGT GTCTGGGGGA CTATGANNGA NTGTNACTGG 180
GGGTGTCTGG GGGNCTATGA NNGANTGTNA CNGGGGGTGT CTGGGGGACT ATGANNGACT 240
GTGCNNCCTG GGGGATCNGA GGAGANTNGN GGNTAGNGAT GGTTNGGGAN A 291

(2) INFORMATION FOR SEQ ID NO:260:

(i) SEQUENCE CHARACTERISTICS:
(A) LENGTH: 238 base pairs
(B) TYPE: nucleic acid
(C) STRANDEDNESS: single
(D) TOPOLOGY: linear

(xi) SEQUENCE DESCRIPTION: SEQ ID NO:260:

TAAGAGGGTA CTGGTTAAAA TACAGGAAAT CTGGGGTAAT GAGGCAGAGA ACCAGGATAC 60
TTTGAGGTCA GGGATGAAAA CTAGAATTTT TTTCTTTTTT TTTGCCTGAG AAACTTGCTG 120
CTCTGAAGAG GCCCATGTAT TAATTGCTTT GATCTTCCTT TTCTTACAGC CCTTTCAAGG 180
GCAGAGCCCT CCTTATCCTG AAGGAATCTT ATCCTTAGCT ATAGTATGTA CCCTCTTA 238

(2) INFORMATION FOR SEQ ID NO:261:

(i) SEQUENCE CHARACTERISTICS:
(A) LENGTH: 746 base pairs
(B) TYPE: nucleic acid
(C) STRANDEDNESS: single
(D) TOPOLOGY: linear

(xi) SEQUENCE DESCRIPTION: SEQ ID NO:261:

TTGGGCACCT TCAATATCAA TAGCTAACAT TTATTGAGTG TTTATCGTAT CATAAAACAC 60
TGTTCTAAGC CTTTAAACGT ACTAATTCAT TTAATGCTCA TAATCACTTT AGAAGGTGGG 120
TACTAGTATT AGTCTCATTT ACAGATGCAA CATGCAGGCA CAGAGAGGTT AATTAACTTG 180
CCCAAGGTAA CACAGCTAAG AAATAGAAAA AATATTGAAT CTGGAAAGTT GGGCTTCTGG 240
GTAACCCACA GAGTCTTCAA TGAGCCTGGG GCCTCACTCA GTTTGCTTTT ACAAAGCGAA 300
TGAGTAACAT CACTTAATTC AGTGAGTAGG CCAAATGGAG GTCAGCTACG AGTTTCTGCT 360
GTTCTTGCAG TGGACTGACA GATGTTTACA ACGTCTGGCC ATCAGTWAAT GGACTGATTA 420
TCATTGGGAW GTGGGTGGGC TGAATGTTGG CCAGTGAAGT TTATTCAWGC CATATTTTTA 480
TGTTTAGGAT GACTTTTGGC TGGTCCTAGG GCAAGCTCTG TCTGSCACGG AACACAGAAT 540
WACACAGGGA CCCCCTCAAT TTCTGGTGTG GCTAGAACCA TGAACCACTG GTTGGGGGAA 600

CAAGCGGTCA AAACCTAAGT GCGGCCGGCT GGCAGGGTCC ACCCATATGG GGAAAACTCC 660
CNACGCGTTT GGAATGCCTN AGCTNGAATT ATTCTAANAG TTGTCCNCNT AAAATTAGCC 720
TGGGCGTTAA TCANGGGTCN NAAGCC 746

(2) INFORMATION FOR SEQ ID NO:262:

(i) SEQUENCE CHARACTERISTICS:
(A) LENGTH: 588 base pairs
(B) TYPE: nucleic acid
(C) STRANDEDNESS: single
(D) TOPOLOGY: linear

(xi) SEQUENCE DESCRIPTION: SEQ ID NO:262:

TGACCGCTTG TCATCTCACA TGGGGTCCTG CACGCTTTTG CCTTTGTAGG AAACCTGACA 60
TTTGTCTGTT TCTTCTTTCT CTTTTCCTTC CCATATCCTC CTAATTTACG TTTGACTTGT 120
TTGCTGAGGA GGCAGGAGCT AGAGACTGCT GTGAGCTCAT AGGGGTGGGA AGTTTATCCT 180
TCAAGTCCCG CCCACTCATC ACTGCTTCTC ACCTTCCCCT GACCAGGCTT ACAAGTGGGT 240
TCTTGCCTGC TTTCCCTTTG GACCCAACAA GCCCCTGTAA TGAGTGTGCA TGACTCTGAC 300
AGCTGTGGAC TCAGGGTCCT TGGCTACAGC TGCCATGTAA AATATCTCAT CCAGTTCTCG 360
CAAATTGTTA AAATAACCAC ATTTCTTAGA TTCCAGTACC CAAATCATGT CTTTACGAAC 420
TGCTCCTCAC ACCCAGAAGT GGCACAATAA TTCTTGGGGA ATTATTACTT TTTTTTTTCT 480
CTCTNTTNNC GNNNGNNNNG GNNNGNCCAG GAATTACCAC NTTGGAAGAC CTGGCCNGAA 540
TTTATTATAN AGGGGAGCCG ATTNTTTTTC CTAACACAAA GCGGGTCA 588

(2) INFORMATION FOR SEQ ID NO:263:

(i) SEQUENCE CHARACTERISTICS:
(A) LENGTH: 730 base pairs
(B) TYPE: nucleic acid
(C) STRANDEDNESS: single
(D) TOPOLOGY: linear

(xi) SEQUENCE DESCRIPTION: SEQ ID NO:263:

TTTTTTTTTT TTTGGCCTGA GCAACTGAAA TTATGAAATT TCCATATACT CAAAAGAGTA 60
AGACTGCAAA AAGATTAAAT GTAAAAGTTG TCTTGTATAC AGTAATGTTT AAGATACCTA 120
TTANATTTAT AAATGGAAAA TTAGGGCATT TGGATATACA AGTTGAAAAT TCAGGAGTGA 180
GGTTGGGCTG GCTGGGTATA TACTGAAAAC TGTCAGTACA CAGATGACAT CTAAAACCAC 240
AAATCTGGTT TTATTTTAGC AGTGATATGT GTCACTCCCA CAAAAGCCTT CCCAATTGGC 300
CTCAGCATAC ACAACAAGTC ACCTCCCCAC AGCCCTCTAC ACATAAACAA ATTCCTTAGT 360
TTAGTTCAGG AGGAAATGCG CCCTTTTCCT TCCGCTCTAG GTGACCGCAA GGCCCAGTTC 420
TCGTCACCAA GATGTTAAGG GAAGTCTGCC AAAGAGGCAT CTGAAAGGAA ATAAGGGGAA 480
TGGGAGTGAC CACAAAGGAA AGCCAAGGAN AAACTTTGGA GACCGTTTCT AGANCCCTGG 540
CATTTCACAA CAAAACTCNG GAACAAACCT TGTCTCATCA ATCATTTAAG CCCTTCGTTT 600
GGANNAGACT TTCTGAACTG GGCGCTGAAC ATAANCCTCA TTGAATGTCT TCACAGTCTC 660
CCAGCTGAAG GCACACCTTG GGCCAGAAGG GGAATCTTCC AGGTCCTCAA NACAGGGCTC 720
GCCCTTTGNC 730

(2) INFORMATION FOR SEQ ID NO:264:

(i) SEQUENCE CHARACTERISTICS:
(A) LENGTH: 715 base pairs
(B) TYPE: nucleic acid
(C) STRANDEDNESS: single

(D) TOPOLOGY: linear

(xi) SEQUENCE DESCRIPTION: SEQ ID NO:264:

TTTTTTTTTT TTTGGCCAGT ATGATAGTCT CTACCACTAT ATTGAAGCTC TTAGGTCATT 60
TACACTTAAT GTGGTTATAG ATGCTGTTGA GCTTACTTCT ACCACCTTGC TATTTCTCCC 120
GTCTCTTTTT TGTTCCTTTT CTCTTCTTTT CCTCCCTTAT TTTATAATTG AATTTTTTAG 180
GATTCTATTT TATATAGATT TATCAGCTAT AACACTTTGT ATTCTTTTGT TTTGTGGTTC 240
TTCTGTCATT TCAATGTGCA TCTTAAACTC ATCACAATCT ATTTTCAAAT AATATCATAT 300
AACCTTACAT ATAATGTAAG AATCTACCAC CATATATTTC CATTTCTCCC TTCCATCCTA 360
TGTNTGTCAT ATTTTTTCCT TTATATATGT TTTAAAGACA TAATAGTATA TGGGAGGTTT 420
TTGCTTAAAA TGTGATCAAT ATTCCTTCAA NGAAACGTAA AAATTCAAAA TAAATNTCTG 480
TTTATTCTCA AATNNACCTA ATATTTCCTA CCATNTCTNA TACNTTTCAA GAATCTGAAG 540
GCATTGGTTT TTTCCGGCTT AAGAACCTCC TCTAAAGCAC TCTAAGCAGA ATTAAGTCTT 600
CTGGGAGAGG AATTCTCCCA AGCTTGGGCC TTNANNTGTA CTCCNTNANG GTTAAANTTT 660
GGCCGGGAAA TAGAAATTCC AAGTTAACAG GNTANTTTTT NTTTTTNTTN TCNCC 715

(2) INFORMATION FOR SEQ ID NO:265:

(i) SEQUENCE CHARACTERISTICS:
(A) LENGTH: 152 base pairs
(B) TYPE: nucleic acid
(C) STRANDEDNESS: single
(D) TOPOLOGY: linear

(xi) SEQUENCE DESCRIPTION: SEQ ID NO:265:

TTTTTTTTTT TTTCCCAACA CAAAGCACCA TTATCTTTCC TCACAATTTT CAACATAGTT 60
TGATTCCCAT GAAGAGGTTA TGATTTCTAA AGAAAACATG GCTACTATAC TATCAATCAG 120
GGTTAAATCT TTTTTTTTTG AGACGGAGTT TA 152

(2) INFORMATION FOR SEQ ID NO:266:

(i) SEQUENCE CHARACTERISTICS:
(A) LENGTH: 193 base pairs
(B) TYPE: nucleic acid
(C) STRANDEDNESS: single
(D) TOPOLOGY: linear

(xi) SEQUENCE DESCRIPTION: SEQ ID NO:266:

TAAACTCCGT CCCCTTCTTA ATCAATATGG AGGCTACCCA CTCCACATTA CCTTCTTTTC 60
AAGGGACTGT TTCCGTAACT GTTGTGGGTA TTCACGACCA GGCTTCTAAA CCTCTTAAAA 120
CTCCCCAATT CTGGTGCCAA CTTGGACAAC ATGCTTTTTT TTTTTTTTTT TTTTTTTTTN 180
GAGACGGAGT TTA 193

(2) INFORMATION FOR SEQ ID NO:267:

(i) SEQUENCE CHARACTERISTICS:
(A) LENGTH: 460 base pairs
(B) TYPE: nucleic acid
(C) STRANDEDNESS: single
(D) TOPOLOGY: linear

(xi) SEQUENCE DESCRIPTION: SEQ ID NO:267:

TGTTGCGATC CCTTAAGCAT GGGTGCTATT AAAAAAATGG TGGAGAAGAA AATACCTGGA 60
ATTTACGTCT TATCTTTAGA GATTGGGAAG ACCCTGATGG AGGACGTGGA GAACAGCTTC 120
TTCTTGAATG TCAATTCCCA AGTAACAACA GTGTGTCAGG CACTTGCTAA GGATCCTAAA 180
TTGCAGCAAG GCTACAATGC TATGGGATTC TCCCAGGGAG GCCAATTTCT GAGGGCAGTG 240
GCTCAGAGAT GCCCTTCACC TCCCATGATC AATCTGATCT CGGTTGGGGG ACAACATCAA 300
GGTGTTTTTG GACTCCCTCG ATGCCCAGGA GAGAGCTCTC ACATCTGTGA CTTCATCCGA 360
AAAACACTGA ATGCTGGGGC GTACTCCAAA GTTGTTCAGG AACGCCTCGT GCAAGCCGAA 420
TACTGGCATG ACCCATAAAA GGAGGATGTG GATCGCAACA 460

(2) INFORMATION FOR SEQ ID NO:268:

(i) SEQUENCE CHARACTERISTICS:
(A) LENGTH: 533 base pairs
(B) TYPE: nucleic acid
(C) STRANDEDNESS: single
(D) TOPOLOGY: linear

(xi) SEQUENCE DESCRIPTION: SEQ ID NO:268:

TGTTGCGATC CGTTGATAGA ATAGCGACGT GGTAATGAGT GCATGGCACG CCTCCGACTT 60
ACCTTCGCCC GTGGGGACCC CGAGTACGTC TACGGCGTCG TCACTTAGAG TACCCTCTGG 120
ACGCCCGGGC GCGTTCGATT TACCGGAAGC GCGAGCTGCA GTGGGCTTGC GCCCCCGGCC 180
AAATTCTTTG GGGGGTTTAA GGCCGCGGGG AATTTGAGGT ATCTCTATCA GTATGTAGCC 240
AAGTTGGAAC AGTCGCCATT CCCGAAATCG CTTTCTTTGA ATCCGCACCG CCTCCAGCAT 300
TGCCTCATTC ATCAACCTGA AGGCACGCAT AAGTGACGGT TGTGTCTTCA GCAGCTCCAC 360
TCCATAACTA GCGCGCTCGA CCTCGTCTTC GTACGCGCCA GGTCCGTGCG TGCGAATTCC 420
CAACTCCGGT GAGTTGCGCA TTTCAAGTTN CGAAACTGTT CGCCTCCACN ATTTGGCATG 480
TTCACGCATG ACACGGAATA AACTCGTCCA GTACCGGGAA TGGGATCGCA ACA 533

(2) INFORMATION FOR SEQ ID NO:269:

(i) SEQUENCE CHARACTERISTICS:
(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:269:

TTTTTTTTTT TTCGCCTGAA TTAGCTACAG ATCCTCCTCA CAAGCGGTCA 50

(2) INFORMATION FOR SEQ ID NO:270:

(i) SEQUENCE CHARACTERISTICS:
(A) LENGTH: 519 base pairs
(B) TYPE: nucleic acid
(C) STRANDEDNESS: single
(D) TOPOLOGY: linear

(xi) SEQUENCE DESCRIPTION: SEQ ID NO:270:

TGTTGCGATC CAAATAACCC ACCAGCTTCT TGCACACTTC GCAGAAGCCA CCGTCCTTTG 60
GCTGAGTCAC GTGAACGGTC AGTGCAAGCA GCCGCGTGCC AGAGCAGAGG TGCAGCATGC 120
TGCACACCAG CTCAGGGCTG ACCTCCTCCA GCAGGATGGA CAGGATGGAG CTGCCGTACG 180

TGTCCACCAC CTCCTGGCAC TCTTCCGACA GGGACTTCGG CAGCTTCGAG CACATTTTGT 240
CAAAAGCGTC GAGTATTTCT TTCTCAGTCT TGTTGTTGTC AATCAGCTTG GTCACCTCCT 300
TCACCAGGAA TTCACACACC TCACAGTAAA CATCAGACTT TGCTGGGACC TCGTGCTTCT 360
TAATGGGCTC CACCAGTTCC AGGGCAGGGA TGACATTCTT GGAGGCCACT TTGGCGGGGA 420
CCAGAGTCTG CATGGGCATC TCTTTCACCT CATCACAGAA CCCAACCAGC GCACAGATCT 480
CCTTGGGTTG CATGTGCATC ATCATCTGGG ATCGCAACA 519

(2) INFORMATION FOR SEQ ID NO:271:

(i) SEQUENCE CHARACTERISTICS:
(A) LENGTH: 457 base pairs
(B) TYPE: nucleic acid
(C) STRANDEDNESS: single
(D) TOPOLOGY: linear

(xi) SEQUENCE DESCRIPTION: SEQ ID NO:271:

TTTTTTTTTT TTCGGGCGGC GACCGGACGT GCACTCCTCC AGTAGCGGCT GCACGTCGTG 60
CCAATGGCCC GCTATGAGGA GGTGAGCGTG TCCGGCTTCG AGGAGTTCCA CCGGGCCGTG 120
GAACAGCACA ATGGCAAGAC CATTTTCGCC TACTTTACGG GTTCTAAGGA CGCCGGGGGG 180
AAAAGCTGGT GCCCCGACTG CGTGCAGGCT GAACCAGTCG TACGAGAGGG GCTGAAGCAC 240
ATTAGTGAAG GATGTGTGTT CATCTACTGC CAAGTAGGAG AAGAGCCTTA TTGGAAAGAT 300
CCAAATAATG ACTTCAGAAA AAACTTGAAA GTAACAGCAG TGCCTACACT ACTTAAGTAT 360
GGAACACCTC AAAAACTGGT AGAATCTGAG TGTCTTCAGG CCAACCTGGT GGAAATGTTG 420
TTCTCTGAAG ATTAAGATTT TAGGATGGCA ATCAAGA 457

(2) INFORMATION FOR SEQ ID NO:272:

(i) SEQUENCE CHARACTERISTICS:
(A) LENGTH: 102 base pairs
(B) TYPE: nucleic acid
(C) STRANDEDNESS: single
(D) TOPOLOGY: linear

(xi) SEQUENCE DESCRIPTION: SEQ ID NO:272:

TTTTTTTTTT TTGGGCAACA ACCTGAATAC CTTTTCAAGG CTCTGGCTTG GGCTCAAGCC 60
CGCAGGGGAA ATGCAACTGG CCAGGTCACA GGGCAATCAA GA 102

(2) INFORMATION FOR SEQ ID NO:273:

(i) SEQUENCE CHARACTERISTICS:
(A) LENGTH: 455 base pairs
(B) TYPE: nucleic acid
(C) STRANDEDNESS: single
(D) TOPOLOGY: linear

(xi) SEQUENCE DESCRIPTION: SEQ ID NO:273:

TTTTTTTTTT TTGGCAATCA ACAGGTTTAA GTCTTCGGCC GAAGTTAATC TCGTGTTTTT 60
GGCAATCAAC AGGTTTAAGT CTTCGGCCGA AGTTAATCTC GTGTTTTTGG CAATCAACAG 120
GTTTAAGTCT TCGGCCGAAG TTAATCTCGT GTTTTTGGCA ATCAACAGGT TTAAGTCTTC 180
GGCCGAAGTT AATCTCGTGT TTTTGGCAAT CAACAGGTTT AAGTCTTCGG CCGAAGTTAA 240
TCTCGTGTTT TTGGCAATCA ACAGGTTTAA GTCTTCGGCC GAAGTTAATC TCGTGTTTTT 300
GGCAATCAAG AGGTTTAAGT CTTCGGCCGA AGTTAATCTC GTGTTTTTGG CAATCAACAG 360

GTTTAAGTCT TCGGCCGAAN TTAATCTCGT GTTTTTGGCA ATCAACAGGT TTAANTCTTC 420
GGCCGAAGTT AATCTCGTGT TTTTGGCAAT CAANA 455

(2) INFORMATION FOR SEQ ID NO:274:

(i) SEQUENCE CHARACTERISTICS:
(A) LENGTH: 461 base pairs
(B) TYPE: nucleic acid
(C) STRANDEDNESS: single
(D) TOPOLOGY: linear

(xi) SEQUENCE DESCRIPTION: SEQ ID NO:274:

TTTTTTTTTT TTGGCCAATA CCCTTGATGA ACATCAATGT GAAAATCCTC GGTAAAATAC 60
TGGCAAACCA AATCCAGCAG CACATCAAAA AGCTTATCCA CCATGATCAA GTGGGCTTCA 120
TCCCTGGGAT GCAAGGCTGG TTCAACATAA GAAAATCAAT AAATGTAATC CATCACATAA 180
ACAGAACCAA AGACAAAAAC CACATGATTA TCTCAATAGA TGCAGAAAAG GCCTTGGACA 240
AATTCAACAG CCCTTCATGC TAAACACTCT TAATAAACTA GATATTGATG GAATGTATCT 300
CAAAATAATA AGAGCTATTT ATGACAAACC CACAGCCAAT ATCATACTGA ATGGGCAAAG 360
ACTGGAAGCA TTCCCTTTGA AAACTGGCAC AAGACAAGGA TGCCCTCTCT CACCGCTCCT 420
ATTCAACATA GTATTGGAAG TTCTGGCCAG GGCAATCAAG A 461

(2) INFORMATION FOR SEQ ID NO:275:

(i) SEQUENCE CHARACTERISTICS:
(A) LENGTH: 729 base pairs
(B) TYPE: nucleic acid
(C) STRANDEDNESS: single
(D) TOPOLOGY: linear

(xi) SEQUENCE DESCRIPTION: SEQ ID NO:275:

TTTTTTTTTT TTGGCCAACA CCAAGTCTTC CACGTGGGAG GTTTTATTAT GTTTTACAAC 60
CATGAAAACA TAGGAAGGTG GCTGTTACAG CAAACATTTC AGATAGACGA ATCGGCCAAG 120
CTCCCCAAAC CCCACCTTCA CAGCCTCTTC CACACGTCTC CCANAGATTG TTGTCCTTCA 180
CTTGCAAATT CANGGATGTT GGAAGTNGAC ATTTNNAGTN GCNGGAACCC CATCAGTGAA 240
NCANTAAGCA GAANTACGAT GACTTTGANA NACANCTGAT GAAGAACACN CTACNGANAA 300
CCCTTTCTNT CGTGTTANGA TCTCNNGTCC NTCACTAATG CGGCCCCCTG CNGGTCCACC 360
ATTTGGGAGA ACTCCCCCCN CGTTGGATCC CCCCTTGAGT NTCCCATTCT NGTCCCCCAN 420
ACCNGNCTTG NGNGNCANTN CNNCCTCNCA CCNTGTTTCC CTGNNGTNAA AATNNGTTTT 480
NCCGCCNCCC NAATTCCCAC CCNAATCACA GCGAANCCNG AAGGCCTTCN NAAGTGTTTA 540
ANGCCCNGNG GTTTCCTCNT NTANTTGCAG CCTACCCTCC CNCTTNNNNT TNCGNGTTGG 600
TCGCGCCCTG GNCNCGCCTN GTTCCTCTTT NNGGNNACAA CCTNGNTCNN NGGCNCNTCN 660
NNNCTNTTCC TNNNACTAGC TNGCCTNTCC NCNCCGNGGN NCANNGCACA TTNCNCNNAC 720
TNTGTNNCC 729

(2) INFORMATION FOR SEQ ID NO:276:

(i) SEQUENCE CHARACTERISTICS:
(A) LENGTH: 339 base pairs
(B) TYPE: nucleic acid
(C) STRANDEDNESS: single
(D) TOPOLOGY: linear

(xi) SEQUENCE DESCRIPTION: SEQ ID NO:276:

TGACCTGACA TGTAGTAGAT ACTTAATAAA TATTTGTGGA ATGAATGGAT GAAGTGGAGT 60
TACAGAGAAA AATAGAAAAG TACAAATTGT TGTCAGTGTT TTGAAGGAAA ATTATGATCT 120
TTCCCAAAGT TCTGACTTCA TTCTAAGACA GGGTTAGTAT CTCCATACAT AATTTTACTT 180
GCTTTTGAAA ATCAAATGAG ATAATCTATT TAGATTGATA ATTTATTTAG ACTGGCTATA 240
AACTATTAAG TGCTAGCAAA TATACATTTT AATCTCATTT TCCACCTCTT GTGATATAGC 300
TATGTAGGTG TTGACTTTAA TGGATGTCAG GTCAATCCC 339

(2) INFORMATION FOR SEQ ID NO:277:

(i) SEQUENCE CHARACTERISTICS:
(A) LENGTH: 664 base pairs
(B) TYPE: nucleic acid
(C) STRANDEDNESS: single
(D) TOPOLOGY: linear

(xi) SEQUENCE DESCRIPTION: SEQ ID NO:277:

TGACCTGACA TCCATAACAA AATCTTTCTC CATTATATTC TTCTAGGGGA ATTTCTTGAA 60
AAGCATCCAA AGGAAACAAA TGATGGTAAG ACCGTGCCAA GTGGGGAGCA GACACCAAAG 120
TAAGACCACA GATTTTACAT TCAACAGGTA GCTCACAGTA CTTTGCCCGA CACTGTGGGC 180
AGAAATAGCC TCCTAATGTA AGCCCTGGCT CAGTATTGCC ATCCAAATGC GCCATGCTGA 240
AAGAGGGTTT TGCATCCTGG TCAGATNAAG AAGCAATGGT GTGCTGAGGA AATCCCATAC 300
GAATAAGTGA GCATTCAGAA CTTGAGCTAG CAGGAGGAGG ACTAAGATGA TGTGTGAGCA 360
ACTCTTTGTA ATGGCTTTCA TCTAAAATAA CATGGTACGT GCCACCAGTT TCACGAGCAA 420
GTACAGTGCA AACGCGAACT TCTGCAGACA ATCCAATAAC AGATACTCTA ATTTTAGCTG 480
CCTTTAGGGT CTTGATTAAA TCATAAATAT TAGATGGATC GCAAGTTGTA AGGNTGCTAA 540
AAGATGATTA GTACTTCTCG ACTTGTATGT CCAGGCATGT TGTTTTAAAN TCTGCCTTAG 600
NCCCTGCTTA GGGGAATTTT TAAAGAAGAT GGCTCTCCAT GTTCANGGTC AATCACNAAT 660
TGCC 664

(2) INFORMATION FOR SEQ ID NO:278:

(i) SEQUENCE CHARACTERISTICS:
(A) LENGTH: 452 base pairs
(B) TYPE: nucleic acid
(C) STRANDEDNESS: single
(D) TOPOLOGY: linear

(xi) SEQUENCE DESCRIPTION: SEQ ID NO:278:

TGACCTGACA TTGAGGAAGA GCACACACCT CTGAAATTCC TTAGGTTCAG AAGGGCATTT 60
GACACAGAGT GGGCCTCTGA TAATTCATGA AATGCATTCT GAAGTCATCC AGAATGGAGG 120
CTGCAATCTG CTGTGCTTTG GGGGTTGCCT CACTGTGCTC CTGGATATCA CACAAAAGCT 180
GCAATCCTTC TTCTTCAACT AACATTTTGC AGTATTTGCT GGGATTTTTA CTGCAGACAT 240
GATACATAGC CCATAGTGCC CAGAGCTGAA CCTCTGGTTG AGAGAAGTTG CCAAGGAGCG 300
GGAAAAATGT CTTGAAAGAT CTATAGGTCA CCAATGCTGT CATCTTACAA CTTGAACTTG 360
GCCAATTCTG TATGGTTGCA TGCAGATCTT GGAGAAGAGT ACGCCTCTGG AAGTCACGGG 420
ATATCCAAAN CTGTCTGTCA GATGTCAGGT CA 452

(2) INFORMATION FOR SEQ ID NO:279:

(i) SEQUENCE CHARACTERISTICS:
(A) LENGTH: 274 base pairs
(B) TYPE: nucleic acid

(C) STRANDEDNESS: single
(D) TOPOLOGY: linear

(xi) SEQUENCE DESCRIPTION: SEQ ID NO:279:

TTTTTTTTTT TTCGGCAAGG CAAATTTACT TCTGCAAAAG GGTGCTGCTT GCACTTTTGG 60
CCACTGCGAG AGCACACCAA ACAAAGTAGG GAAGGGGTTT TTATCCCTAA CGCGGTTATT 120
CCCTGGTTCT GTGTCGTGTC CCCATTGGCT GGAGTCAGAC TGCACAATCT ACACTGACCC 180
AACTGGCTAC TGTTTAAAAT TGAATATGAA TAATTAGGTA GGAAGGGGGA GGCTGTTTGT 240
TACGGTACAA GACGTGTTTG GGCATGTCAG GTCA 274

(2) INFORMATION FOR SEQ ID NO:280:

(i) SEQUENCE CHARACTERISTICS:
(A) LENGTH: 272 base pairs
(B) TYPE: nucleic acid
(C) STRANDEDNESS: single
(D) TOPOLOGY: linear

(xi) SEQUENCE DESCRIPTION: SEQ ID NO:280:

TACCTGACAT GGAGAAATAA CTTGTAGTAT TTTGCGTGCA ATGGAATACT ATATGAGGGT 60
GAAAATGAAT GAACTAGCAA TGCGTGTATC AACATGAATA AATCCCCAAA ACATAATAAT 120
GTTGAATGGA AAAGGTGAGT TTCAGAAGGA TATATATGCC CTCTAAATCC ATTTATGTAA 180
ACCTTTAAAA AACTACATTA TTTATGGTCA TAAGTCCATC CAGAAAATAT TTAAAAACCT 240
ACATGGGATT GATAACTACT GATGTCAGGT CA 272

(2) INFORMATION FOR SEQ ID NO:281:

(i) SEQUENCE CHARACTERISTICS:
(A) LENGTH: 431 base pairs
(B) TYPE: nucleic acid
(C) STRANDEDNESS: single
(D) TOPOLOGY: linear

(xi) SEQUENCE DESCRIPTION: SEQ ID NO:281:

TTTTTTTTTT TTGGCCAATA GCATGATTTA AACATTGGAA AAAGTCAAAT GAGCAATGCG 60
AATTTTTATG TTCTCTTGAA TAATCAAAAG AGTAGGCAAC ATTGGTTCCT CATTCTTGAA 120
TAGCATTAAT CAGAAAATAT TGCATAGCCT CTAGCCTCCT TAGAGTAGGT GTGCTCTCTC 180
AAATATATCA TAGTCCCACA GTTTATTTCA TGTATATTTT CTGCCTGAAT CACATAGACA 240
TTTGAATTTG CAACGCCTGA TGTAAATATA TAAATTCTTA CCAATCAGAA ACATAGCAAG 300
AAATTCAGGG ACTTGGTCAT YATCAGGGTA TGACAGCANA TCCCTGTARA AACACTGATA 360
CACACTCACA CACGTATGCA ACGTGGAGAT GTCGCYTTWW KKKTWYWCWM RMRYCRWCGN 420
AATCACTTAN N 431

(2) INFORMATION FOR SEQ ID NO:282:

(i) SEQUENCE CHARACTERISTICS:
(A) LENGTH: 98 base pairs
(B) TYPE: nucleic acid
(C) STRANDEDNESS: single
(D) TOPOLOGY: linear

(xi) SEQUENCE DESCRIPTION: SEQ ID NO:282:

ATTCGATTCG ATGCTTGAGC CCAGGAGTTC AAGACTGCAG TGAGCCACTG CACTTCAGGC 60
TGGACAACAG AGCGAGTCCC TGTGCCAAAA AAAAAAAA 98

(2) INFORMATION FOR SEQ ID NO:283:

(i) SEQUENCE CHARACTERISTICS:
(A) LENGTH: 764 base pairs
(B) TYPE: nucleic acid
(C) STRANDEDNESS: single
(D) TOPOLOGY: linear

(xi) SEQUENCE DESCRIPTION: SEQ ID NO:283:

TTTTTTTTTT TTCGCAAGCA CGTGCACTTT ATTGAATGAC ACTGTAGACA GGTGTGTGGG 60
TATAAACTGC TGTATCTAGG GGCAGGACCA AGGGGGCAGG GGCAACAGCC CCAGCGTGCA 120
GGGCCASCAT TGCACAGTGG ASTGCAAAGG TTGCAGGCTA TGGGCGGCTA CTAVTAACCC 180
CGTTTTTCCT GTATTATCTG TAACATAATA TGGTAGACTG TCACAGAGCC GAATWCCART 240
HACASGATGA ATCCAAWGGT CAYGAGGATG CCCASAATCA GGGCCCASAT STTCAGGCAC 300
TTGGCGGTGG GGGCATASGC CTGKGCCCCG GTCACGTCSC CAACCWTCTY CCTGTCCCTA 360
CMCTTGAWTC CNCNCCTTNN NNTNCCNTNA TNTGCCCGCC CNCCTCCTNG NGTCAACCNG 420
NATCTGCACT ANCTCCCTCN CCCCTTNTGG ANTCTCNTCC TTCAANTAAN NTTATCCTTN 480
ACNCCCCCCT CNCCTTTCCC CTNCCNCCCN TNATCCCNGN NCCNCTATCA NTCNTNCCCT 540
CNCTNTNCTN CNNATCGTTC CNCCTNNTAA CTACNCTTTN NACNANNCCT CACTNATNCC 600
NGNNANTTCT TTCCTTCCCT CCCNACGCNN TGCGTGCGCC CGTCTNGCCT NNNCTNCGNA 660
CCCNNACTTT ATTTACCTTT NCACCCTAGC NCTCTACTTN ACCCANCCNC TCCTACCTCC 720
NGGNCCACCC NNCCCTNATC NCTNNCTCTN TCNNCTCNTT CCCC 764

(2) INFORMATION FOR SEQ ID NO:284:

(i) SEQUENCE CHARACTERISTICS:
(A) LENGTH: 157 base pairs
(B) TYPE: nucleic acid
(C) STRANDEDNESS: single
(D) TOPOLOGY: linear

(xi) SEQUENCE DESCRIPTION: SEQ ID NO:284:

CAAGTGTAGG CACAGTGATG AAAGCCTGGA GCAAACACAA TCTGTGGGTA ATTAACGTTT 60
ATTTCTCCCC TTCCAGGAAC GTCTTGCATG GATGATCAAA GATCAGCTCC TGGTCAACAT 120
AAATAAGCTA GTTTAAGATA CGTTCCCCTA CACTTGA 157

(2) INFORMATION FOR SEQ ID NO:285:

(i) SEQUENCE CHARACTERISTICS:
(A) LENGTH: 150 base pairs
(B) TYPE: nucleic acid
(C) STRANDEDNESS: single
(D) TOPOLOGY: linear

(xi) SEQUENCE DESCRIPTION: SEQ ID NO:285:

ATTCGATTGT ACTCAGACAA CAATATGCTA AGTGGAAGAA GTCAGTCACA AAAGACCACA 60
TACTGTATGA CTTCATTTAC ATTAAGTGTC CAGAATAGGC AAATCCGTAG AGACAGAAAG 120
TAGATGAGCA GCTGCCTAGG TCTGAGTACA 150

(2) INFORMATION FOR SEQ ID NO:286:

(i) SEQUENCE CHARACTERISTICS:
(A) LENGTH: 219 base pairs
(B) TYPE: nucleic acid
(C) STRANDEDNESS: single
(D) TOPOLOGY: linear

(xi) SEQUENCE DESCRIPTION: SEQ ID NO:286:

ATTCGATTTT TTTTTTTTTG GCCATGATGA AATTCTTACT CCCTCAGATT TTTTGTCTGG 60
ATAAATGCAA GTCTCACCAC CAGATGTGAA ATTACAGTAA ACTTTGAAGG AATCTCCTGA 120
GCAACCTTGG TTAGGATCAA TCCAATATTC ACCATCTGGG AAGTCAGGAT GGCTGAGTTG 180
CAGGTCTTTA CAAGTTCGGG CTGGATTGGT CTGAGTACA 219

(2) INFORMATION FOR SEQ ID NO:287:

(i) SEQUENCE CHARACTERISTICS:
(A) LENGTH: 196 base pairs
(B) TYPE: nucleic acid
(C) STRANDEDNESS: single
(D) TOPOLOGY: linear

(xi) SEQUENCE DESCRIPTION: SEQ ID NO:287:

ATTCGATTCT TGAGGCTACC AGGAGCTAGG AGAAGAGGCA TGGAACAAAT TTTCCCTCAT 60
ATCCATACTC AGAAGGAACC AACCCTGCTG ACACCTTAAT TTCAGCTTCT GGCCTCTAGA 120
ACTGTGAGAG AGTACATTTC TCTTGGTTTA AGCCAAGAGA ATCTGTCTTT TGGTACTTTA 180
TATCATAGCC TCAAGA 196

(2) INFORMATION FOR SEQ ID NO:288:

(i) SEQUENCE CHARACTERISTICS:
(A) LENGTH: 199 base pairs
(B) TYPE: nucleic acid
(C) STRANDEDNESS: single
(D) TOPOLOGY: linear

(xi) SEQUENCE DESCRIPTION: SEQ ID NO:288:

ATTCGATTTC AGTCCAGTCC CAGAACCCAC ATTGTCAATT ACTACTCTGT ARAAGATTCA 60
TTTGTTGAAA TTCATTGAGT AAAACATTTA TGATCCCTTA ATATATGCCA ATTACCATGC 120
TAGGTACTGA AGATTCAAGT GACCGAGATG CTAGCCCTTG GGTTCAAGTG ATCCCTCTCC 180
CAGAGTGCAC TGGACTGAA 199

(2) INFORMATION FOR SEQ ID NO:289:

(i) SEQUENCE CHARACTERISTICS:
(A) LENGTH: 182 base pairs
(B) TYPE: nucleic acid
(C) STRANDEDNESS: single
(D) TOPOLOGY: linear

(xi) SEQUENCE DESCRIPTION: SEQ ID NO:289:

ATTCGATTCT TGAGGCTACA AACCTGTACA GTATGTTACT CTACTGAATA CTGTAGGCAA 60
TAGTAATACA GAAGCAAGTA TCTGTATATG TAAACATTAA AAAGGTACAG TGAAACTTCA 120
GTATTATAAT CTTAGGGACC ACCATTATAT ATGTGGTCCA TCATTGGCCA AAAAAAAAAA 180
AA 182

(2) INFORMATION FOR SEQ ID NO:290:

(i) SEQUENCE CHARACTERISTICS:
(A) LENGTH: 1646 base pairs
(B) TYPE: nucleic acid
(C) STRANDEDNESS: single
(D) TOPOLOGY: linear

(xi) SEQUENCE DESCRIPTION: SEQ ID NO:290:

GGCACGAGGA GAAATGTAAT TCCATATTTT ATTTGAAACT TATTCCATAT TTTAATTGGA 60
TATTGAGTGA TTGGGTTATC AAACACCCAC AAACTTTAAT TTTGTTAAAT TTATATGGCT 120
TTGAAATAGA AGTATAAGTT GCTACCATTT TTTGATAACA TTGAAAGATA GTATTTTACC 180
ATCTTTAATC ATCTTGGAAA ATACAAGTCC TGTGAACAAC CACTCTTTCA CCTAGCAGCA 240
TGAGGCCAAA AGTAAAGGCT TTAAATTATA ACATATGGGA TTCTTAGTAG TATGTTTTTT 300
TCTTGAAACT CAGTGGCTCT ATCTAACCTT ACTATCTCCT CACTCTTTCT CTAAGACTAA 360
ACTCTAGGCT CTTAAAAATC TGCCCACACC AATCTTAGAA GCTCTGAAAA GAATTTGTCT 420
TTAAATATCT TTTAATAGTA ACATGTATTT TATGGACCAA ATTGACATTT TCGACTATTT 480
TTTCCAAAAA AGTCAGGTGA ATTTCAGCAC ACTGAGTTGG GAATTTCTTA TCCCAGAAGA 540
CCAACCAATT TCATATTTAT TTAAGATTGA TTCCATACTC CGTTTTCAAG GAGAATCCCT 600
GCAGTCTCCT TAAAGGTAGA ACAAATACTT TCTATTTTTT TTTCACCATT GTGGGATTGG 660
ACTTTAAGAG GTGACTCTAA AAAAACAGAG AACAAATATG TCTCAGTTGT ATTAAGCACG 720
GACCCATATT ATCATATTCA CTTAAAAAAA TGATTTCCTG TGCACCTTTT GGCAACTTCT 780
CTTTTCAATG TAGGGAAAAA CTTAGTCACC CTGAAAACCC ACAAAATAAA TAAAACTTGT 840
AGATGTGGGC AGAAGGTTTG GGGGTGGACA TTGTATGTGT TTAAATTAAA CCCTGTATCA 900
CTGAGAAGCT GTTGTATGGG TCAGAGAAAA TGAATGCTTA GAAGCTGTTC ACATCTTCAA 960
GAGCAGAAGC AAACCACATG TCTCAGCTAT ATTATTATTT ATTTTTTATG CATAAAGTGA 1020
ATCATTTCTT CTGTATTAAT TTCCAAAGGG TTTTACCCTC TATTTAAATG CTTTGAAAAA 1080
CAGTGCATTG ACAATGGGTT GATATTTTTC TTTAAAAGAA AAATATAATT ATGAAAGCCA 1140
AGATAATCTG AAGCCTGTTT TATTTTAAAA CTTTTTATGT TCTGTGGTTG ATGTTGTTTG 1200
TTTGTTTGTT TCTATTTTGT TGGTTTTTTA CTTTGTTTTT TGTTTTGTTT TGTTTTGTTT 1260
KGCATACTAC ATGCAGTTCT TTAACCAATG TCTGTTTGGC TAATGTAATT AAAGTTGTTA 1320
ATTTATATGA GTGCATTTCA ACTATGTCAA TGGTTTCTTA ATATTTATTG TGTAGAAGTA 1380
CTGGTAATTT TTTTATTTAC AATATGTTTA AAGAGATAAC AGTTTGATAT GTTTTCATGT 1440
GTTTATAGCA GAAGTTATTT ATTTCTATGG CATTCCAGCG GATATTTTGG TGTTTGCGAG 1500
GCATGCAGTC AATATTTTGT ACAGTTAGTG GACAGTATTC AGCAACGCCT GATAGCTTCT 1560
TTGGCCTTAT GTTAAATAAA AAGACCTGTT TGGGATGTAT TTTTTATTTT TAAAAAAAAA 1620
AAAAAAAAAA AAAAAAAAAA AAAAAA 1646

(2) INFORMATION FOR SEQ ID NO:291:

(i) SEQUENCE CHARACTERISTICS:
(A) LENGTH: 1851 base pairs
(B) TYPE: nucleic acid
(C) STRANDEDNESS: single
(D) TOPOLOGY: linear

(xi) SEQUENCE DESCRIPTION: SEQ ID NO:291:

TCATCACCAT TGCCAGCAGC GGCACCGTTA GTCAGGTTTT CTGGGAATCC CACATGAGTA 60
CTTCCGTGTT CTTCATTCTT CTTCAATAGC CATAAATCTT CTAGCTCTGG CTGGCTGTTT 120
TCACTTCCTT TAAGCCTTTG TGACTCTTCC TCTGATGTCA GCTTTAAGTC TTGTTCTGGA 180
TTGCTGTTTT CAGAAGAGAT TTTTAACATC TGTTTTTCTT TGTAGTCAGA AAGTAACTGG 240
CAAATTACAT GATGATGACT AGAAACAGCA TACTCTCTGG CCGTCTTTCC AGATCTTGAG 300
AAGATACATC AACATTTTGC TCAAGTAGAG GGCTGACTAT ACTTGCTGAT CCACAACATA 360
CAGCAAGTAT GAGAGCAGTT CTTCCATATC TATCCAGCGC ATTTAAATTC GCTTTTTTCT 420
TGATTAAAAA TTTCACCACT TGCTGTTTTT GCTCATGTAT ACCAAGTAGC AGTGGTGTGA 480
GGCCATGCTT GTTTTTTGAT TCGATATCAG CACCGTATAA GAGCAGTGCT TTGGCCATTA 540
ATTTATCTTC ATTGTAGACA GCATAGTGTA GAGTGGTATT TCCATACTCA TCTGGAATAT 600
TTGGATCAGT GCCATGTTCC AGCAACATTA ACGCACATTC ATCTTCCTGG CATTGTACGG 660
CCTTTGTCAG AGCTGTCCTC TTTTTGTTGT CAAGGACATT AAGTTGACAT CGTCTGTCCA 720
GCACGAGTTT TACTACTTCT GAATTCCCAT TGGCAGAGGC CAGATGTAGA GCAGTCCTCT 780
TTTGCTTGTC CCTCTTGTTC ACATCCGTGT CCCTGAGCAT GACGATGAGA TCCTTTCTGG 840
GGACTTTACC CCACCAGGCA GCTCTGTGGA GCTTGTCCAG ATCTTCTCCA TGGACGTGGT 900
ACCTGGGATC CATGAAGGCG CTGTCATCGT AGTCTCCCCA AGCGACCACG TTGCTCTTGC 960
CGCTCCCCTG CAGCAGGGGA AGCAGTGGCA GCACCACTTG CACCTCTTGC TCCCAAGCGT 1020
CTTCACAGAG GAGTCGTTGT GGTCTCCAGA AGTGCCCACG TTGCTCTTGC CGCTCCCCCT 1080
GTCCATCCAG GGAGGAAGAA ATGCAGGAAA TGAAAGATGC ATGCACGATG GTATACTCCT 1140
CAGCCATCAA ACTTCTGGAC AGCAGGTCAC TTCCAGCAAG GTGGAGAAAG CTGTCCACCC 1200
ACAGAGGATG AGATCCAGAA ACCACAATAT CCATTCACAA ACAAACACTT TTCAGCCAGA 1260
CACAGGTACT GAAATCATGT CATCTGCGGC AACATGGTGG AACCTACCCA ATCACACATC 1320
AAGAGATGAA GACACTGCAG TATATCTGCA CAACGTAATA CTCTTCATCC ATAACAAAAT 1380
AATATAATTT TCCTCTGGAG CCATATGGAT GAACTATGAA GGAAGAACTC CCCGAAGAAG 1440
CCAGTCGCAG AGAAGCCACA CTGAAGCTCT GTCCTCAGCC ATCAGCGCCA CGGACAGGAR 1500
TGTGTTTCTT CCCCAGTGAT GCAGCCTCAA GTTATCCCGA AGCTGCCGCA GCACACGGTG 1560
GCTCCTGAGA AACACCCCAG CTCTTCCGGT CTAACACAGG CAAGTCAATA AATGTGATAA 1620
TCACATAAAC AGAATTAAAA GCAAAGTCAC ATAAGCATCT CAACAGACAC AGAAAAGGCA 1680
TTTGACAAAA TCCAGCATCC TTGTATTTAT TGTTGCAGTT CTCAGAGGAA ATGCTTCTAA 1740
CTTTTCCCCA TTTAGTATTA TGTTGGCTGT GGGCTTGTCA TAGGTGGTTT TTATTACTTT 1800
AAGGTATGTC CCTTCTATGC CTGTTTTGCT GAGGGTTTTA ATTCTCGTGC C 1851

(2) INFORMATION FOR SEQ ID NO:292:

(i) SEQUENCE CHARACTERISTICS:
(A) LENGTH: 1851 base pairs
(B) TYPE: nucleic acid
(C) STRANDEDNESS: single
(D) TOPOLOGY: linear

(xi) SEQUENCE DESCRIPTION: SEQ ID NO:292:

TCATCACCAT TGCCAGCAGC GGCACCGTTA GTCAGGTTTT CTGGGAATCC CACATGAGTA 60
CTTCCGTGTT CTTCATTCTT CTTCAATAGC CATAAATCTT CTAGCTCTGG CTGGCTGTTT 120
TCACTTCCTT TAAGCCTTTG TGACTCTTCC TCTGATGTCA GCTTTAAGTC TTGTTCTGGA 180
TTGCTGTTTT CAGAAGAGAT TTTTAACATC TGTTTTTCTT TGTAGTCAGA AAGTAACTGG 240
CAAATTACAT GATGATGACT AGAAACAGCA TACTCTCTGG CCGTCTTTCC AGATCTTGAG 300
AAGATACATC AACATTTTGC TCAAGTAGAG GGCTGACTAT ACTTGCTGAT CCACAACATA 360
CAGCAAGTAT GAGAGCAGTT CTTCCATATC TATCCAGCGC ATTTAAATTC GCTTTTTTCT 420
TGATTAAAAA TTTCACCACT TGCTGTTTTT GCTCATGTAT ACCAAGTAGC AGTGGTGTGA 480
GGCCATGCTT GTTTTTTGAT TCGATATCAG CACCGTATAA GAGCAGTGCT TTGGCCATTA 540
ATTTATCTTC ATTGTAGACA GCATAGTGTA GAGTGGTATT TCCATACTCA TCTGGAATAT 600
TTGGATCAGT GCCATGTTCC AGCAACATTA ACGCACATTC ATCTTCCTGG CATTGTACGG 660
CCTTTGTCAG AGCTGTCCTC TTTTTGTTGT CAAGGACATT AAGTTGACAT CGTCTGTCCA 720
GCACGAGTTT TACTACTTCT GAATTCCCAT TGGCAGAGGC CAGATGTAGA GCAGTCCTCT 780

TTTGCTTGTC CCTCTTGTTC ACATCCGTGT CCCTGAGCAT GACGATGAGA TCCTTTCTGG 840
GGACTTTACC CCACCAGGCA GCTCTGTGGA GCTTGTCCAG ATCTTCTCCA TGGACGTGGT 900
ACCTGGGATC CATGAAGGCG CTGTCATCGT AGTCTCCCCA AGCGACCACG TTGCTCTTGC 960
CGCTCCCCTG CAGCAGGGGA AGCAGTGGCA GCACCACTTG CACCTCTTGC TCCCAAGCGT 1020
CTTCACAGAG GAGTCGTTGT GGTCTCCAGA AGTGCCCACG TTGCTCTTGC CGCTCCCCCT 1080
GTCCATCCAG GGAGGAAGAA ATGCAGGAAA TGAAAGATGC ATGCACGATG GTATACTCCT 1140
CAGCCATCAA ACTTCTGGAC AGCAGGTCAC TTCCAGCAAG GTGGAGAAAG CTGTCCACCC 1200
ACAGAGGATG AGATCCAGAA ACCACAATAT CCATTCACAA ACAAACACTT TTCAGCCAGA 1260
CACAGGTACT GAAATCATGT CATCTGCGGC AACATGGTGG AACCTACCCA ATCACACATC 1320
AAGAGATGAA GACACTGCAG TATATCTGCA CAACGTAATA CTCTTCATCC ATAACAAAAT 1380
AATATAATTT TCCTCTGGAG CCATATGGAT GAACTATGAA GGAAGAACTC CCCGAAGAAG 1440
CCAGTCGCAG AGAAGCCACA CTGAAGCTCT GTCCTCAGCC ATCAGCGCCA CGGACAGGAR 1500
TGTGTTTCTT CCCCAGTGAT GCAGCCTCAA GTTATCCCGA AGCTGCCGCA GCACACGGTG 1560
GCTCCTGAGA AACACCCCAG CTCTTCCGGT CTAACACAGG CAAGTCAATA AATGTGATAA 1620
TCACATAAAC AGAATTAAAA GCAAAGTCAC ATAAGCATCT CAACAGACAC AGAAAAGGCA 1680
TTTGACAAAA TCCAGCATCC TTGTATTTAT TGTTGCAGTT CTCAGAGGAA ATGCTTCTAA 1740
CTTTTCCCCA TTTAGTATTA TGTTGGCTGT GGGCTTGTCA TAGGTGGTTT TTATTACTTT 1800
AAGGTATGTC CCTTCTATGC CTGTTTTGCT GAGGGTTTTA ATTCTCGTGC C 1851

(2) INFORMATION FOR SEQ ID NO:293:

(i) SEQUENCE CHARACTERISTICS:
(A) LENGTH: 668 base pairs
(B) TYPE: nucleic acid
(C) STRANDEDNESS: single
(D) TOPOLOGY: linear

(xi) SEQUENCE DESCRIPTION: SEQ ID NO:293:

CTTGAGCTTC CAAATAYGGA AGACTGGCCC TTACACASGT CAATGTTAAA ATGAATGCAT 60
TTCAGTATTT TGAAGATAAA ATTRGTAGAT CTATACCTTG TTTTTTGATT CGATATCAGC 120
ACCRTATAAG AGCAGTGCTT TGGCCATTAA TTTATCTTTC ATTRTAGACA GCRTAGTGYA 180
GAGTGGTATT TCCATACTCA TCTGGAATAT TTGGATCAGT GCCATGTTCC AGCAACATTA 240
ACGCACATTC ATCTTCCTGG CATTGTACGG CCTGTCAGTA TTAGACCCAA AAACAAATTA 300
CATATCTTAG GAATTCAAAA TAACATTCCA CAGCTTTCAC CAACTAGTTA TATTTAAAGG 360
AGAAAACTCA TTTTTATGCC ATGTATTGAA ATCAAACCCA CCTCATGCTG ATATAGTTGG 420
CTACTGCATA CCTTTATCAG AGCTGTCCTC TTTTTGTTGT CAAGGACATT AAGTTGACAT 480
CGTCTGTCCA GCAGGAGTTT TACTACTTCT GAATTCCCAT TGGCAGAGGC CAGATGTAGA 540
GCAGTCCTAT GAGAGTGAGA AGACTTTTTA GGAAATTGTA GTGCACTAGC TACAGCCATA 600
GCAATGATTC ATGTAACTGC AAACACTGAA TAGCCTGCTA TTACTCTGCC TTCAAAAAAA 660
AAAAAAAA 668

(2) INFORMATION FOR SEQ ID NO:294:

(i) SEQUENCE CHARACTERISTICS:
(A) LENGTH: 1512 base pairs
(B) TYPE: nucleic acid
(C) STRANDEDNESS: single
(D) TOPOLOGY: linear

(xi) SEQUENCE DESCRIPTION: SEQ ID NO:294:

GGGTCGCCCA GGGGGSGCGT GGGCTTTCCT CGGGTGGGTG TGGGTTTTCC CTGGGTGGGG 60
TGGGCTGGGC TRGAATCCCC TGCTGGGGTT GGCAGGTTTT GGCTGGGATT GACTTTTYTC 120
TTCAAACAGA TTGGAAACCC GGAGTTACCT GCTAGTTGGT GAAACTGGTT GGTAGACGCG 180

ATCTGTTGGC TACTACTGGC TTCTCCTGGC TGTTAAAAGC AGATGGTGGT TGAGGTTGAT 240
TCCATGCCGG CTGCTTCTTC TGTGAAGAAG CCATTTGGTC TCAGGAGCAA GATGGGCAAG 300
TGGTGCTGCC GTTGCTTCCC CTGCTGCAGG GAGAGCGGCA AGAGCAACGT GGGCACTTCT 360
GGAGACCACG ACGACTCTGC TATGAAGACA CTCAGGAGCA AGATGGGCAA GTGGTGCCGC 420
CACTGCTTCC CCTGCTGCAG GGGGAGTGGC AAGAGCAACG TGGGCGCTTC TGGAGACCAC 480
GACGAYTCTG CTATGAAGAC ACTCAGGAAC AAGATGGGCA AGTGGTGCTG CCACTGCTTC 540
CCCTGCTGCA GGGGGAGCRG CAAGAGCAAG GTGGGCGCTT GGGGAGACTA CGATGACAGT 600
GCCTTCATGG AGCCCAGGTA CCACGTCCGT GGAGAAGATC TGGACAAGCT CCACAGAGCT 660
GCCTGGTGGG GTAAAGTCCC CAGAAAGGAT CTCATCGTCA TGCTCAGGGA CACTGACGTG 720
AACAAGAAGG ACAAGCAAAA GAGGACTGCT CTACATCTGG CCTCTGCCAA TGGGAATTCA 780
GAAGTAGTAA AACTCSTGCT GGACAGACGA TGTCAACTTA ATGTCCTTGA CAACAAAAAG 840
AGGACAGCTC TGAYAAAGGC CGTACAATGC CAGGAAGATG AATGTGCGTT AATGTTGCTG 900
GAACATGGCA CTGATCCAAA TATTCCAGAT GAGTATGGAA ATACCACTCT RCACTAYGCT 960
RTCTAYAATG AAGATAAATT AATGGCCAAA GCACTGCTCT TATAYGGTGC TGATATCGAA 1020
TCAAAAAACA AGGTATAGAT CTACTAATTT TATCTTCAAA ATACTGAAAT GCATTCATTT 1080
TAACATTGAC GTGTGTAAGG GCCAGTCTTC CGTATTTGGA AGCTCAAGCA TAACTTGAAT 1140
GAAAATATTT TGAAATGACC TAATTATCTM AGACTTTATT TTAAATATTG TTATTTTCAA 1200
AGAAGCATTA GAGGGTACAG TTTTTTTTTT TTAAATGCAC TTCTGGTAAA TACTTTTGTT 1260
GAAAACACTG AATTTGTAAA AGGTAATACT TACTATTTTT CAATTTTTCC CTCCTAGGAT 1320
TTTTTTCCCC TAATGAATGT AAGATGGCAA AATTTGCCCT GAAATAGGTT TTACATGAAA 1380
ACTCCAAGAA AAGTTAAACA TGTTTCAGTG AATAGAGATC CTGCTCCTTT GGCAAGTTCC 1440
TAAAAAACAG TAATAGATAC GAGGTGATGC GCCTGTCAGT GGCAAGGTTT AAGATATTTC 1500
TGATCTCGTG CC 1512

(2) INFORMATION FOR SEQ ID NO:295:

(i) SEQUENCE CHARACTERISTICS:
(A) LENGTH: 1853 base pairs
(B) TYPE: nucleic acid
(C) STRANDEDNESS: single
(D) TOPOLOGY: linear

(xi) SEQUENCE DESCRIPTION: SEQ ID NO:295:

GGGTCGCCCA GGGGGSGCGT GGGCTTTCCT CGGGTGGGTG TGGGTTTTCC CTGGGTGGGG 60
TGGGCTGGGC TRGAATCCCC TGCTGGGGTT GGCAGGTTTT GGCTGGGATT GACTTTTYTC 120
TTCAAACAGA TTGGAAACCC GGAGTTACCT GCTAGTTGGT GAAACTGGTT GGTAGACGCG 180
ATCTGTTGGC TACTACTGGC TTCTCCTGGC TGTTAAAAGC AGATGGTGGT TGAGGTTGAT 240
TCCATGCCGG CTGCTTCTTC TGTGAAGAAG CCATTTGGTC TCAGGAGCAA GATGGGCAAG 300
TGGTGCTGCC GTTGCTTCCC CTGCTGCAGG GAGAGCGGCA AGAGCAACGT GGGCACTTCT 360
GGAGACCACG ACGACTCTGC TATGAAGACA CTCAGGAGCA AGATGGGCAA GTGGTGCCGC 420
CACTGCTTCC CCTGCTGCAG GGGGAGTGGC AAGAGCAACG TGGGCGCTTC TGGAGACCAC 480
GACGAYTCTG CTATGAAGAC ACTCAGGAAC AAGATGGGCA AGTGGTGCTG CCACTGCTTC 540
CCCTGCTGCA GGGGGAGCRG CAAGAGCAAG GTGGGCGCTT GGGGAGACTA CGATGACAGY 600
GCCTTCATGG AKCCCAGGTA CCACGTCCRT GGAGAAGATC TGGACAAGCT CCACAGAGCT 660
GCCTGGTGGG GTAAAGTCCC CAGAAAGGAT CTCATCGTCA TGCTCAGGGA CACKGAYGTG 720
AACAAGARGG ACAAGCAAAA GAGGACTGCT CTACATCTGG CCTCTGCCAA TGGGAATTCA 780
GAAGTAGTAA AACTCSTGCT GGACAGACGA TGTCAACTTA ATGTCCTTGA CAACAAAAAG 840
AGGACAGCTC TGAYAAAGGC CGTACAATGC CAGGAAGATG AATGTGCGTT AATGTTGCTG 900
GAACATGGCA CTGATCCAAA TATTCCAGAT GAGTATGGAA ATACCACTCT RCACTAYGCT 960
RTCTAYAATG AAGATAAATT AATGGCCAAA GCACTGCTCT TATAYGGTGC TGATATCGAA 1020
TCAAAAAACA AGCATGGCCT CACACCACTG YTACTTGGTR TACATGAGCA AAAACAGCAA 1080
GTSGTGAAAT TTTTAATYAA GAAAAAAGCG AATTTAAAAT GCRCTGGATA GATATGGAAG 1140
RACTGCTCTC ATACTTGCTG TATGTTGTGG ATCAGCAAGT ATAGTCAGCC YTCTACTTGA 1200
GCAAAATRTT GATGTATCTT CTCAAGATCT GGAAAGACGG CCAGAGAGTA TGCTGTTTCT 1260

AGTCATCATC ATGTAATTTG CCAGTTACTT TCTGACTACA AAGAAAAACA GATGTTAAAA 1320
ATCTCTTCTG AAAACAGCAA TCCAGAACAA GACTTAAAGC TGACATCAGA GGAAGAGTCA 1380
CAAAGGCTTA AAGGAAGTGA AAACAGCCAG CCAGAGGCAT GGAAACTTTT AAATTTAAAC 1440
TTTTGGTTTA ATGTTTTTTT TTTTTGCCTT AATAATATTA GATAGTCCCA AATGAAATWA 1500
CCTATGAGAC TAGGCTTTGA GAATCAATAG ATTCTTTTTT TAAGAATCTT TTGGCTAGGA 1560
GCGGTGTCTC ACGCCTGTAA TTCCAGCACC TTGAGAGGCT GAGGTGGGCA GATCACGAGA 1620
TCAGGAGATC GAGACCATCC TGGCTAACAC GGTGAAACCC CATCTCTACT AAAAATACAA 1680
AAACTTAGCT GGGTGTGGTG GCGGGTGCCT GTAGTCCCAG CTACTCAGGA RGCTGAGGCA 1740
GGAGAATGGC ATGAACCCGG GAGGTGGAGG TTGCAGTGAG CCGAGATCCG CCACTACACT 1800
CCAGCCTGGG TGACAGAGCA AGACTCTGTC TCAAAAAAAA AAAAAAAAAA AAA 1853

(2) INFORMATION FOR SEQ ID NO:296:

(i) SEQUENCE CHARACTERISTICS:
(A) LENGTH: 2184 base pairs
(B) TYPE: nucleic acid
(C) STRANDEDNESS: single
(D) TOPOLOGY: linear

(xi) SEQUENCE DESCRIPTION: SEQ ID NO:296:

GGCACGAGAA TTAAAACCCT CAGCAAAACA GGCATAGAAG GGACATACCT TAAAGTAATA 60
AAAACCACCT ATGACAAGCC CACAGCCAAC ATAATACTAA ATGGGGAAAA GTTAGAAGCA 120
TTTCCTCTGA GAACTGCAAC AATAAATACA AGGATGCTGG ATTTTGTCAA ATGCCTTTTC 180
TGTGTCTGTT GAGATGCTTA TGTGACTTTG CTTTTAATTC TGTTTATGTG ATTATCACAT 240
TTATTGACTT GCCTGTGTTA GACCGGAAGA GCTGGGGTGT TTCTCAGGAG CCACCGTGTG 300
CTGCGGCAGC TTCGGGATAA CTTGAGGCTG CATCACTGGG GAAGAAACAC AYTCCTGTCC 360
GTGGCGCTGA TGGCTGAGGA CAGAGCTTCA GTGTGGCTTC TCTGCGACTG GCTTCTTCGG 420
GGAGTTCTTC CTTCATAGTT CATCCATATG GCTCCAGAGG AAAATTATAT TATTTTGTTA 480
TGGATGAAGA GTATTACGTT GTGCAGATAT ACTGCAGTGT CTTCATCTCT TGATGTGTGA 540
TTGGGTAGGT TCCACCATGT TGCCGCAGAT GACATGATTT CAGTACCTGT GTCTGGCTGA 600
AAAGTGTTTG TTTGTGAATG GATATTGTGG TTTCTGGATC TCATCCTCTG TGGGTGGACA 660
GCTTTCTCCA CCTTGCTGGA AGTGACCTGC TGTCCAGAAG TTTGATGGCT GAGGAGTATA 720
CCATCGTGCA TGCATCTTTC ATTTCCTGCA TTTCTTCCTC CCTGGATGGA CAGGGGGAGC 780
GGCAAGAGCA ACGTGGGCAC TTCTGGAGAC CACAACGACT CCTCTGTGAA GACGCTTGGG 840
AGCAAGAGGT GCAAGTGGTG CTGCCACTGC TTCCCCTGCT GCAGGGGAGC GGCAAGAGCA 900
ACGTGGTCGC TTGGGGAGAC TACGATGACA GCGCCTTCAT GGATCCCAGG TACCACGTCC 960
ATGGAGAAGA TCTGGACAAG CTCCACAGAG CTGCCTGGTG GGGTAAAGTC CCCAGAAAGG 1020
ATCTCATCGT CATGCTCAGG GACACGGATG TGAACAAGAG GGACAAGCAA AAGAGGACTG 1080
CTCTACATCT GGCCTCTGCC AATGGGAATT CAGAAGTAGT AAAACTCGTG CTGGACAGAC 1140
GATGTCAACT TAATGTCCTT GACAACAAAA AGAGGACAGC TCTGACAAAG GCCGTACAAT 1200
GCCAGGAAGA TGAATGTGCG TTAATGTTGC TGGAACATGG CACTGATCCA AATATTCCAG 1260
ATGAGTATGG AAATACCACT CTACACTATG CTGTCTACAA TGAAGATAAA TTAATGGCCA 1320
AAGCACTGCT CTTATACGGT GCTGATATCG AATCAAAAAA CAAGCATGGC CTCACACCAC 1380
TGCTACTTGG TATACATGAG CAAAAACAGC AAGTGGTGAA ATTTTTAATC AAGAAAAAAG 1440
CGAATTTAAA TGCGCTGGAT AGATATGGAA GAACTGCTCT CATACTTGCT GTATGTTGTG 1500
GATCAGCAAG TATAGTCAGC CCTCTACTTG AGCAAAATGT TGATGTATCT TCTCAAGATC 1560
TGGAAAGACG GCCAGAGAGT ATGCTGTTTC TAGTCATCAT CATGTAATTT GCCAGTTACT 1620
TTCTGACTAC AAAGAAAAAC AGATGTTAAA AATCTCTTCT GAAAACAGCA ATCCAGAACA 1680
AGACTTAAAG CTGACATCAG AGGAAGAGTC ACAAAGGCTT AAAGGAAGTG AAAACAGCCA 1740
GCCAGAGGCA TGGAAACTTT TAAATTTAAA CTTTTGGTTT AATGTTTTTT TTTTTTGCCT 1800
TAATAATATT AGATAGTCCC AAATGAAATW ACCTATGAGA CTAGGCTTTG AGAATCAATA 1860
GATTCTTTTT TTAAGAATCT TTTGGCTAGG AGCGGTGTCT CACGCCTGTA ATTCCAGCAC 1920
CTTGAGAGGC TGAGGTGGGC AGATCACGAG ATCAGGAGAT CGAGACCATC CTGGCTAACA 1980
CGGTGAAACC CCATCTCTAC TAAAAATACA AAAACTTAGC TGGGTGTGGT GGCGGGTGCC 2040

TGTAGTCCCA GCTACTCAGG ARGCTGAGGC AGGAGAATGG CATGAACCCG GGAGGTGGAG 2100
GTTGCAGTGA GCCGAGATCC GCCACTACAC TCCAGCCTGG GTGACAGAGC AAGACTCTGT 2160
CTCAAAAAAA AAAAAAAAAA AAAA 2184

(2) INFORMATION FOR SEQ ID NO:297:

(i) SEQUENCE CHARACTERISTICS:
(A) LENGTH: 1855 base pairs
(B) TYPE: nucleic acid
(C) STRANDEDNESS: single
(D) TOPOLOGY: linear

(xi) SEQUENCE DESCRIPTION: SEQ ID NO:297:

TGCACGCATC GGCCAGTGTC TGTGCCACGT ACACTGACGC CCCCTGAGAT GTGCACGCCG 60
CACGCGCACG TTGCACGCGC GGCAGCGGCT TGGCTGGCTT GTAACGGCTT GCACGCGCAC 120
GCCGCCCCCG CATAACCGTC AGACTGGCCT GTAACGGCTT GCAGGCGCAC GCCGCACGCG 180
CGTAACGGCT TGGCTGCCCT GTAACGGCTT GCACGTGCAT GCTGCACGCG CGTTAACGGC 240
TTGGCTGGCA TGTAGCCGCT TGGCTTGGCT TTGCATTYTT TGCTKGGCTK GGCGTTGKTY 300
TCTTGGATTG ACGCTTCCTC CTTGGATKGA CGTTTCCTCC TTGGATKGAC GTTTCYTYTY 360
TCGCGTTCCT TTGCTGGACT TGACCTTTTY TCTGCTGGGT TTGGCATTCC TTTGGGGTGG 420
GCTGGGTGTT TTCTCCGGGG GGGKTKGCCC TTCCTGGGGT GGGCGTGGGK CGCCCCCAGG 480
GGGCGTGGGC TTTCCCCGGG TGGGTGTGGG TTTTCCTGGG GTGGGGTGGG CTGTGCTGGG 540
ATCCCCCTGC TGGGGTTGGC AGGGATTGAC TTTTTTCTTC AAACAGATTG GAAACCCGGA 600
GTAACNTGCT AGTTGGTGAA ACTGGTTGGT AGACGCGATC TGCTGGTACT ACTGTTTCTC 660
CTGGCTGTTA AAAGCAGATG GTGGCTGAGG TTGATTCAAT GCCGGCTGCT TCTTCTGTGA 720
AGAAGCCATT TGGTCTCAGG AGCAAGATGG GCAAGTGGTG CGCCACTGCT TCCCCTGCTG 780
CAGGGGGAGC GGCAAGAGCA ACGTGGGCAC TTCTGGAGAC CACAACGACT CCTCTGTGAA 840
GACGCTTGGG AGCAAGAGGT GCAAGTGGTG CTGCCCACTG CTTCCCCTGC TGCAGGGGAG 900
CGGCAAGAGC AACGTGGKCG CTTGGGGAGA CTACGATGAC AGCGCCTTCA TGGAKCCCAG 960
GTACCACGTC CRTGGAGAAG ATCTGGACAA GCTCCACAGA GCTGCCTGGT GGGGTAAAGT 1020
CCCCAGAAAG GATCTCATCG TCATGCTCAG GGACACTGAY GTGAACAAGA RGGACAAGCA 1080
AAAGAGGACT GCTCTACATC TGGCCTCTGC CAATGGGAAT TCAGAAGTAG TAAAACTCGT 1140
GCTGGACAGA CGATGTCAAC TTAATGTCCT TGACAACAAA AAGAGGACAG CTCTGACAAA 1200
GGCCGTACAA TGCCAGGAAG ATGAATGTGC GTTAATGTTG CTGGAACATG GCACTGATCC 1260
AAATATTCCA GATGAGTATG GAAATACCAC TCTACACTAT GCTGTCTACA ATGAAGATAA 1320
ATTAATGGCC AAAGCACTGC TCTTATACGG TGCTGATATC GAATCAAAAA ACAAGGTATA 1380
GATCTACTAA TTTTATCTTC AAAATACTGA AATGCATTCA TTTTAACATT GACGTGTGTA 1440
AGGGCCAGTC TTCCGTATTT GGAAGCTCAA GCATAACTTG AATGAAAATA TTTTGAAATG 1500
ACCTAATTAT CTAAGACTTT ATTTTAAATA TTGTTATTTT CAAAGAAGCA TTAGAGGGTA 1560
CAGTTTTTTT TTTTTAAATG CACTTCTGGT AAATACTTTT GTTGAAAACA CTGAATTTGT 1620
AAAAGGTAAT ACTTACTATT TTTCAATTTT TCCCTCCTAG GATTTTTTTC CCCTAATGAA 1680
TGTAAGATGG CAAAATTTGC CCTGAAATAG GTTTTACATG AAAACTCCAA GAAAAGTTAA 1740
ACATGTTTCA GTGAATAGAG ATCCTGCTCC TTTGGCAAGT TCCTAAAAAA CAGTAATAGA 1800
TACGAGGTGA TGCGCCTGTC AGTGGCAAGG TTTAAGATAT TTCTGATCTC GTGCC 1855

## Description

### TECHNICAL FIELD

The present invention relates generally to the detection and therapy of breast cancer. The invention is more specifically related to nucleotide sequences that are preferentially expressed in breast tumor tissue and to polypeptides encoded by such nucleotide sequences. The nucleotide sequences and polypeptides may be used in vaccines and pharmaceutical compositions for the prevention and treatment of breast cancer. The polypeptides may also be used for the production of compounds, such as antibodies, useful for diagnosing and monitoring the progression of breast cancer in a patient.

### BACKGROUND OF THE INVENTION

Breast cancer is a significant health problem for women in the United States and throughout the world. Although advances have been made in detection and treatment of the disease, breast cancer remains the second leading cause of cancer-related deaths in women, affecting more than 180,000 women in the United States each year. For women in North America, the life-time odds of getting breast cancer are now one in eight.

No vaccine or other universally successful method for the prevention or treatment of breast cancer is currently available. Management of the disease currently relies on a combination of early diagnosis (through routine breast screening procedures) and aggressive treatment, which may include one or more of a variety of treatments such as surgery, radiotherapy, chemotherapy and hormone therapy. The course of treatment for a particular breast cancer is often selected based on a variety of prognostic parameters, including an analysis of specific tumor markers. *See*, *e*.*g*., Porter-Jordan and Lippman, Breast Cancer 8:73-100 (1994). However, the use of established markers often leads to a result that is difficult to interpret, and the high mortality observed in breast cancer patients indicates that improvements are needed in the treatment, diagnosis and prevention of the disease.

Accordingly, there is a need in the art for improved methods for therapy and diagnosis of breast cancer. The present invention fulfills these needs and further provides other related advantages.

### SUMMARY OF THE INVENTION

Briefly stated, the subject invention provides compositions and methods for the diagnosis and therapy of breast cancer. In one aspect, isolated DNA molecules are provided, comprising (a) a nucleotide sequence preferentially expressed in breast cancer tissue, relative to normal tissue; (b) a variant of such a sequence that contains one or more nucleotide substitutions, deletions, insertions and/or modifications at no more than 20% (preferably no more than 5%) of the nucleotide positions, such that the antigenic and/or immunogenic properties of the polypeptide encoded by the nucleotide sequence are retained; or (c) a nucleotide sequence encoding an epitope of a polypeptide encoded by at least one of the above sequences. In one embodiment, the isolated DNA molecule comprises a human endogenous retroviral sequence recited in SEQ ID NO:1. In other embodiments, the isolated DNA molecule comprises a nucleotide sequence recited in any one of SEQ ID NO: 3-26, 28-77, 142, 143, 146-152, 154-166, 168-176, 178-192, 194-198, 200-204, 206, 207, 209-214, 216, 218, 219, 221-240, 243-245, 247, 250, 251, 253, 255, 257-266, 268, 269, 271-273, 275, 276, 278, 280, 281, 284, 288 and 291-297.

In related embodiments, the isolated DNA molecule encodes an epitope of a polypeptide, wherein the polypeptide is encoded by a nucleotide sequence that: (a) hybridizes to a sequence recited in any one of SEQ ID NO: 1, 3-26, 28-77, 142, 143, 146-152, 154-166, 168-176, 178-192, 194-198, 200-204, 206, 207, 209-214, 216, 218, 219, 221-240, 243-245, 247, 250, 251, 253, 255, 257-266, 268, 269, 271-273, 275, 276, 278, 280, 281, 284, 288 and 291-297 under stringent conditions; and (b) is at least 80% identical to a sequence recited in any one of SEQ ID NO: 1, 3-26, 28-77, 142, 143, 146-152, 154-166, 168-176, 178-192, 194-198, 200-204, 206, 207, 209-214, 216, 218, 219, 221-240, 243-245, 247, 250, 251, 253, 255, 257-266, 268, 269, 271-273, 275, 276, 278, 280, 281, 284, 288 and 291-297; and wherein RNA corresponding to said nucleotide sequence is expressed at a greater level in human breast tumor tissue than in normal breast tissue.

In another embodiment, the present invention provides an isolated DNA molecule encoding an epitope of a polypeptide, the polypeptide being encoded by: (a) a nucleotide sequence transcribed from the sequence of SEQ ID NO: 141; or (b) a variant of said nucleotide sequence that contains one or more nucleotide substitutions, deletions, insertions and/or modifications at no more than 20% of the nucleotide positions, such that the antigenic and/or immunogenic properties of the polypeptide encoded by the nucleotide sequence are retained. Isolated DNA and RNA molecules comprising a nucleotide sequence complementary to a DNA molecule as described above are also provided.

In related aspects, the present invention provides recombinant expression vectors comprising a DNA molecule as described above and host cells transformed or transfected with such expression vectors.

In further aspects, polypeptides, comprising an amino acid sequence encoded by a DNA molecule as described above, and monoclonal antibodies that bind to such polypeptides are provided.

In yet another aspect, methods are provided for determining the presence of breast cancer in a patient. In one embodiment, the method comprises detecting, within a biological sample, a polypeptide as described above. In another embodiment, the method comprises detecting, within a biological sample, an RNA molecule encoding a polypeptide as described above. In yet another embodiment, the method comprises (a) intradermally injecting a patient with a polypeptide as described above; and (b) detecting an immune response on the patient's skin and therefrom detecting the presence of breast cancer in the patient. In further embodiments, the present invention provides methods for determining the presence of breast cancer in a patient as described above wherein the polypeptide is encoded by a nucleotide sequence selected from the group consisting of SEQ ID NO: 78-86, 144, 145, 153, 167, 177, 193, 199, 205, 208, 215, 217, 220, 241, 242, 246, 248, 249, 252, 256, 267, 270, 274, 277, 279, 282, 283, 285-287, 289, 290 and sequences that hybridize thereto under stringent conditions.

In a related aspect, diagnostic kits useful in the determination of breast cancer are provided. The diagnostic kits generally comprise either one or more monoclonal antibodies as described above, or one or more monoclonal antibodies that bind to a polypeptide encoded by a nucleotide sequence selected from the group consisting of sequences provided in SEQ ID NO: 78-86, 144, 145, 153, 167, 177, 193, 199, 205, 208, 215, 217, 220, 241, 242 and 246, 248, 249, 252, 256, 267, 270, 274, 277, 279, 282, 283, 285-287, 289, 290 and a detection reagent.

Within a related aspect, the diagnostic kit comprises a first polymerase chain reaction primer and a second polymerase chain reaction primer, at least one of the primers being specific for an RNA molecule described herein. In one embodiment, at least one of the primers comprises at least about 10 contiguous nucleotides of an RNA molecule as described above, or an RNA molecule encoding a polypeptide encoded by a nucleotide sequence selected from the group consisting of SEQ ID NO: 78-86, 144, 145, 153, 167, 177, 193, 199, 205, 208, 215, 217, 220, 241, 242 246, 248, 249, 252, 256, 267, 270, 274, 277, 279, 282, 283, 285-287, 289 and 290.

Within another related aspect, the diagnostic kit comprises at least one oligonucleotide probe, the probe being specific for a DNA molecule described herein. In one embodiment, the probe comprises at least about 15 contiguous nucleotides of a DNA molecule as described above, or a DNA molecule selected from the group consisting of SEQ ID NO: 78-86, 144, 145, 153, 167, 177, 193, 199, 205, 208, 215, 217, 220, 241, 242 246, 248, 249, 252, 256, 267, 270, 274, 277, 279, 282, 283, 285-287, 289 and 290.

In another related aspect, the present invention provides methods for monitoring the progression of breast cancer in a patient. In one embodiment, the method comprises: (a) detecting an amount, in a biological sample, of a polypeptide as described above at a first point in time; (b) repeating step (a) at a subsequent point in time; and (c) comparing the amounts of polypeptide detected in steps (a) and (b), and therefrom monitoring the progression of breast cancer in the patient. In another embodiment, the method comprises (a) detecting an amount, within a biological sample, of an RNA molecule encoding a polypeptide as described above at a first point in time; (b) repeating step (a) at a subsequent point in time; and (c) comparing the amounts of RNA molecules detected in steps (a) and (b), and therefrom monitoring the progression of breast cancer in the patient. In yet other embodiments, the present invention provides methods for monitoring the progression of breast cancer in a patient as described above wherein the polypeptide is encoded by a nucleotide sequence selected form the group consisting of SEQ ID NO: 78-86, 144, 145, 153, 167, 177, 193, 199, 205, 208, 215, 217, 220, 241, 242, 246, 248, 249, 252, 256, 267, 270, 274, 277, 279, 282, 283, 285-287, 289, 290 and sequences that hybridize thereto under stringent conditions.

In still other aspects, pharmaceutical compositions, which comprise a polypeptide as described above in combination with a physiologically acceptable carrier, and vaccines, which comprise a polypeptide as described above in combination with an immune response enhancer or adjuvant, are provided. In yet other aspects, the present invention provides pharmaceutical compositions and vaccines comprising a polypeptide encoded by a nucleotide sequence selected from the group consisting of SEQ ID NO: 78-86, 144, 145, 153, 167, 177, 193, 199, 205, 208, 215, 217, 220, 241, 242 and 246, 248, 249, 252, 256, 267, 270, 274, 277, 279, 282, 283, 285-287, 289, 290 and sequences that hybridize thereto under stringent conditions.

In related aspects, the present invention provides methods for inhibiting the development of breast cancer in a patient, comprising administering to a patient a pharmaceutical composition or vaccine as described above.

These and other aspects of the present invention will become apparent upon reference to the following detailed description and attached drawings. All references disclosed herein are hereby incorporated by reference in their entirety as if each was incorporated individually.

### BRIEF DESCRIPTION OF THE DRAWINGS

Figure 1 shows the differential display PCR products, separated by gel electrophoresis, obtained from cDNA prepared from normal breast tissue (lanes 1 and 2) and from cDNA prepared from breast tumor tissue from the same patient (lanes 3 and 4). The arrow indicates the band corresponding to B18Ag1.
Figure 2 is a northern blot comparing the level of B18Ag1 mRNA in breast tumor tissue (lane 1) with the level in normal breast tissue.
Figure 3 shows the level of B18Ag1 mRNA in breast tumor tissue compared to that in various normal and non-breast tumor tissues as determined by RNase protection assays.
Figure 4 is a genomic clone map showing the location of additional retroviral sequences obtained from ends of XbaI restriction digests (provided in SEQ ID NO:3 - SEQ ID NO: 10) relative to B18Ag1.
Figures 5A and 5B show the sequencing strategy, genomic organization and predicted open reading frame for the retroviral element containing B18Ag1.
Figure 6 shows the nucleotide sequence of the representative breast tumor-specific cDNA B18Ag1.
Figure 7 shows the nucleotide sequence of the representative breast tumor-specific cDNA B17Agl.
Figure 8 shows the nucleotide sequence of the representative breast tumor-specific cDNA B17Ag2.
Figure 9 shows the nucleotide sequence of the representative breast tumor-specific cDNA B 13Ag2a.
Figure 10 shows the nucleotide sequence of the representative breast tumor-specific cDNA B13Ag1b.
Figure 11 shows the nucleotide sequence of the representative breast tumor-specific cDNA B13Ag1a.
Figure 12 shows the nucleotide sequence of the representative breast tumor-specific cDNA B11Ag1.
Figure 13 shows the nucleotide sequence of the representative breast tumor-specific cDNA B3CA3c.
Figure 14 shows the nucleotide sequence of the representative breast tumor-specific cDNA B9CG1.
Figure 15 shows the nucleotide sequence of the representative breast tumor-specific cDNA B9CG3.
Figure 16 shows the nucleotide sequence of the representative breast tumor-specific cDNA B2CA2.
Figure 17 shows the nucleotide sequence of the representative breast tumor-specific cDNA B3CA1.
Figure 18 shows the nucleotide sequence of the representative breast tumor-specific cDNA B3CA2.
Figure 19 shows the nucleotide sequence of the representative breast tumor-specific cDNA B3CA3.
Figure 20 shows the nucleotide sequence of the representative breast tumor-specific cDNA B4CA1.
Figure 21A depicts RT-PCR analysis of breast tumor genes in breast tumor tissues (lanes 1-8) and normal breast tissues (lanes 9-13) and H₂O (lane 14).
Figure 21B depicts RT-PCR analysis of breast tumor genes in prostate tumors (lane 1, 2), colon tumors (lane 3), lung tumor (lane 4), normal prostate (lane 5), normal colon (lane 6), normal kidney (lane 7), normal liver (lane 8), normal lung (lane 9), normal ovary (lanes 10, 18), normal pancreases (lanes 11, 12), normal skeletal muscle (lane 13), normal skin (lane 14), normal stomach (lane 15), normal testes (lane 16), normal small intestine (lane 17), HBL-100 (lane 19), MCF-12A (lane 20), breast tumors (lanes 21-23), H₂O (lane 24), and colon tumor (lane 25).

### DETAILED DESCRIPTION OF THE INVENTION

As noted above, the present invention is generally directed to compositions and methods for the diagnosis, monitoring and therapy of breast cancer. The compositions described herein include polypeptides, nucleic acid sequences and antibodies. Polypeptides of the present invention generally comprise at least a portion of a protein that is expressed at a greater level in human breast tumor tissue than in normal breast tissue (i.e., the level of RNA encoding the polypeptide is at least 2-fold higher in tumor tissue). Such polypeptides are referred to herein as breast tumor-specific polypeptides, and cDNA molecules encoding such polypeptides are referred to as breast tumor-specific cDNAs. Nucleic acid sequences of the subject invention generally comprise a DNA or RNA sequence that encodes all or a portion of a polypeptide as described above, or that is complementary to such a sequence. Antibodies are generally immune system proteins, or fragments thereof, that are capable of binding to a portion of a polypeptide as described above. Antibodies can be produced by cell culture techniques, including the generation of monoclonal antibodies as described herein, or via transfection of antibody genes into suitable bacterial or mammalian cell hosts, in order to allow for the production of recombinant antibodies.

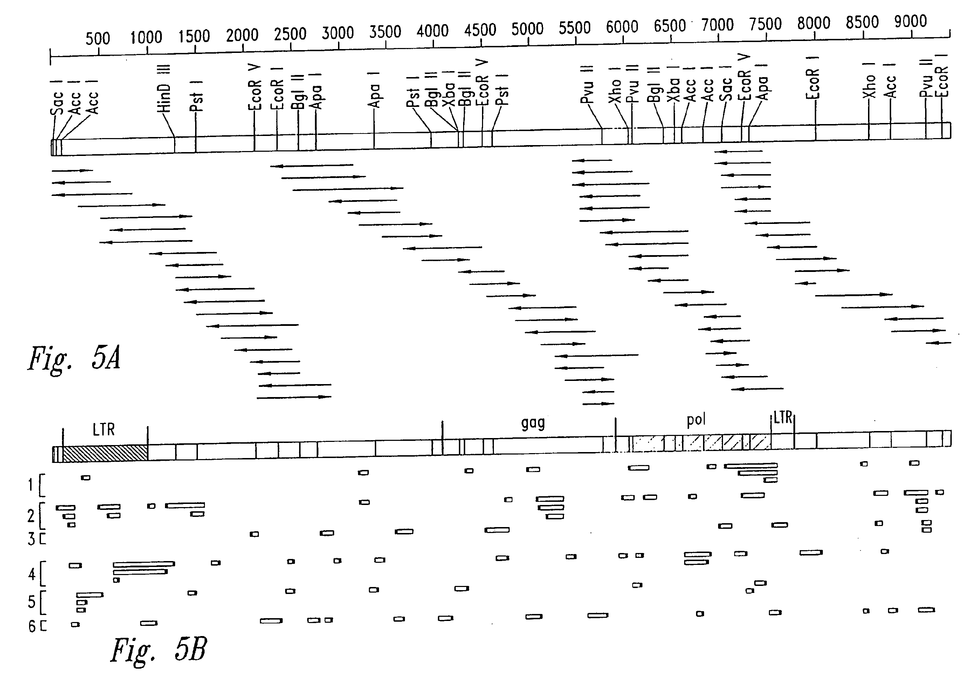

Polypeptides within the scope of this invention include, but are not limited to, polypeptides (and epitopes thereof) encoded by a human endogenous retroviral sequence, such as the sequence designated B18Ag1 (Figure 5 and SEQ ID NO:1). Also within the scope of the present invention are polypeptides encoded by other sequences within the retroviral genome containing B18Ag1 (SEQ ID NO: 141). Such sequences include, but are not limited to, the sequences recited in SEQ ID NO:3 - SEQ ID NO:10. B18Ag1 has homology to the *gag* p30 gene of the endogenous human retroviral element S71, as described in Werner et al., Virology 174:225-238 (1990) and also shows homology to about thirty other retroviral gag genes. As discussed in more detail below, the present invention also includes a number of additional breast tumor-specific polypeptides, such as those encoded by the nucleotide sequences recited in SEQ ID NO: 11-26, 28-77, 142, 143, 146-152, 154-166, 168-176, 178-192, 194-198, 200-204, 206, 207, 209-214, 216, 218, 219, 221-240, 243-245, 247, 250, 251, 253, 255, 257-266, 268, 269, 271-273, 275, 276, 278, 280, 281, 284, 288 and 291-297. As used herein, the term "polypeptide" encompasses amino acid chains of any length, including full length proteins containing the sequences recited herein. A polypeptide comprising an epitope of a protein containing a sequence as described herein may consist entirely of the epitope, or may contain additional sequences. The additional sequences may be derived from the native protein or may be heterologous, and such sequences may (but need not) possess immunogenic or antigenic properties.

An "epitope," as used herein is a portion of a polypeptide that is recognized (*i*.*e*., specifically bound) by a B-cell and/or T-cell surface antigen receptor. Epitopes may generally be identified using well known techniques, such as those summarized in Paul, Fundamental Immunology, 3rd ed., 243-247 (Raven Press, 1993) and references cited therein. Such techniques include screening polypeptides derived from the native polypeptide for the ability to react with antigen-specific antisera and/or T-cell lines or clones. An epitope of a polypeptide is a portion that reacts with such antisera and/or T-cells at a level that is similar to the reactivity of the full length polypeptide (e.g., in an ELISA and/or T-cell reactivity assay). Such screens may generally be performed using methods well known to those of ordinary skill in the art, such as those described in Harlow and Lane, Antibodies: A Laboratory Manual, Cold Spring Harbor Laboratory, 1988. B-cell and T-cell epitopes may also be predicted via computer analysis. Polypeptides comprising an epitope of a polypeptide that is preferentially expressed in a tumor tissue (with or without additional amino acid sequence) are within the scope of the present invention.

The compositions and methods of the present invention also encompass variants of the above polypeptides and nucleic acid sequences encoding such polypeptides. A polypeptide "variant," as used herein, is a polypeptide that differs from the native polypeptide in substitutions and/or modifications, such that the antigenic and/or immunogenic properties of the polypeptide are retained. Such variants may generally be identified by modifying one of the above polypeptide sequences and evaluating the reactivity of the modified polypeptide with antisera and/or T-cells as described above. Nucleic acid variants may contain one or more substitutions, deletions, insertions and/or modifications such that the antigenic and/or immunogenic properties of the encoded polypeptide are retained. One preferred variant of the polypeptides described herein is a variant that contains nucleotide substitutions, deletions, insertions and/or modifications at no more than 20% of the nucleotide positions.

Preferably, a variant contains conservative substitutions. A "conservative substitution" is one in which an amino acid is substituted for another amino acid that has similar properties, such that one skilled in the art of peptide chemistry would expect the secondary structure and hydropathic nature of the polypeptide to be substantially unchanged. In general, the following groups of amino acids represent conservative changes: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his.

Variants may also (or alternatively) be modified by, for example, the deletion or addition of amino acids that have minimal influence on the immunogenic or antigenic properties, secondary structure and hydropathic nature of the polypeptide. For example, a polypeptide may be conjugated to a signal (or leader) sequence at the N-terminal end of the protein which co-translationally or post-translationally directs transfer of the protein. The polypeptide may also be conjugated to a linker or other sequence for ease of synthesis, purification or identification of the polypeptide (e.g., poly-His), or to enhance binding of the polypeptide to a solid support. For example, a polypeptide may be conjugated to an immunoglobulin Fc region.

In general, nucleotide sequences encoding all or a portion of the polypeptides described herein may be prepared using any of several techniques. For example, cDNA molecules encoding such polypeptides may be cloned on the basis of the breast tumor-specific expression of the corresponding mRNAs, using differential display PCR. This technique compares the amplified products from RNA template prepared from normal and breast tumor tissue. cDNA may be prepared by reverse transcription of RNA using a (dT)₁₂AG primer. Following amplification of the cDNA using a random primer, a band corresponding to an amplified product specific to the tumor RNA may be cut out from a silver stained gel and subcloned into a suitable vector (e.g., the T-vector, Novagen, Madison, WI). Nucleotide sequences encoding all or a portion of the breast tumor-specific polypeptides disclosed herein may be amplified from cDNA prepared as described above using the random primers shown in SEQ ID NO.:87-125.

Alternatively, a gene encoding a polypeptide as described herein (or a portion thereof) may be amplified from human genomic DNA, or from breast tumor cDNA, via polymerase chain reaction. For this approach, B18Ag1 sequence-specific primers may be designed based on the sequence provided in SEQ ID NO:1, and may be purchased or synthesized. One suitable primer pair for amplification from breast tumor cDNA is (5'ATG GCT ATT TTC GGG GGC TGA CA) (SEQ ID NO.:126) and (5'CCG GTA TCT CCT CGT GGG TAT T) (SEQ ID NO.:127). An amplified portion of B18Ag1 may then be used to isolate the full length gene from a human genomic DNA library or from a breast tumor cDNA library, using well known techniques, such as those described in Sambrook et al., Molecular Cloning: A Laboratory Manual, Cold Spring Harbor Laboratories, Cold Spring Harbor, NY (1989). Other sequences within the retroviral genome of which B18Ag1 is a part may be similarly prepared by screening human genomic libraries using B18Ag1-specific sequences as probes. Nucleotides translated into protein from the retroviral genome shown in SEQ ID NO: 141 may then be determined by cloning the corresponding cDNAs, predicting the open reading frames and cloning the appropriate cDNAs into a vector containing a viral promoter, such as T7. The resulting constructs can be employed in a translation reaction, using techniques known to those of skill in the art, to identify nucleotide sequences which result in expressed protein. Similarly, primers specific for the remaining breast tumor-specific polypeptides described herein may be designed based on the nucleotide sequences provided in SEQ ID NO:11 - SEQ ID NO:86 and SEQ ID NO:142 - SEQ ID NO:297.

Recombinant polypeptides encoded by the DNA sequences described above may be readily prepared from the DNA sequences. For example, supernatants from suitable host/vector systems which secrete recombinant protein or polypeptide into culture media may be first concentrated using a commercially available filter. Following concentration, the concentrate may be applied to a suitable purification matrix such as an affinity matrix or an ion exchange resin. Finally, one or more reverse phase HPLC steps can be employed to further purify a recombinant polypeptide.

In general, any of a variety of expression vectors known to those of ordinary skill in the art may be employed to express recombinant polypeptides of this invention. Expression may be achieved in any appropriate host cell that has been transformed or transfected with an expression vector containing a DNA molecule that encodes a recombinant polypeptide. Suitable host cells include prokaryotes, yeast and higher eukaryotic cells. Preferably, the host cells employed are *E. coli,* yeast or a mammalian cell line such as COS or CHO.

Such techniques may also be used to prepare polypeptides comprising epitopes or variants of the native polypeptides. For example, variants of a native polypeptide may generally be prepared using standard mutagenesis techniques, such as oligonucleotide-directed site-specific mutagenesis, and sections of the DNA sequence may be removed to permit preparation of truncated polypeptides. Portions and other variants having fewer than about 100 amino acids, and generally fewer than about 50 amino acids, may also be generated by synthetic means, using techniques well known to those of ordinary skill in the art. For example, such polypeptides may be synthesized using any of the commercially available solid-phase techniques, such as the Merrifield solid-phase synthesis method, where amino acids are sequentially added to a growing amino acid *chain. See* Merrifield, J. Am. Chem. Soc. 85:2149-2146 (1963). Equipment for automated synthesis of polypeptides is commercially available from suppliers such as Perkin Elmer/Applied BioSystems Division,, Foster City, CA, and may be operated according to the manufacturer's instructions.

In specific embodiments, polypeptides of the present invention encompass amino acid sequences encoded by a DNA molecule having a sequence recited in any one of SEQ ID NO:1, 3-26, 28-77, 142, 143, 146-152, 154-166, 168-176, 178-192, 194-198, 200-204, 206, 207, 209-214, 216, 218, 219, 221-240, 243-245, 247, 250, 251, 253, 255, 257-266, 268, 269, 271-273, 275, 276, 278, 280, 281, 284, 288 and 291-297, variants of such polypeptides that are encoded by DNA molecules containing one or more nucleotide substitutions, deletions, insertions and/or modifications at no more than 20% of the nucleotide positions, and epitopes of the above polypeptides. Polypeptides within the scope of the present invention also include polypeptides (and epitopes thereof) encoded by DNA sequences that hybridize to a DNA molecule having a sequence recited in any one of SEQ ID NO:1, 3-26, 28-77, 142, 143, 146-152, 154-166, 168-176, 178-192, 194-198, 200-204, 206, 207, 209-214, 216, 218, 219, 221-240, 243-245, 247, 250, 251, 253, 255, 257-266, 268, 269, 271-273, 275, 276, 278, 280, 281, 284, 288 and 291-297 under stringent conditions, wherein the DNA sequences are at least 80% identical in overall sequence to a recited sequence and wherein RNA corresponding to the nucleotide sequence is expressed at a greater level in human breast tumor tissue than in normal breast tissue. As used herein, "stringent conditions" refers to prewashing in a solution of 6X SSC, 0.2% SDS; hybridizing at 65°C, 6X SSC, 0.2% SDS overnight; followed by two washes of 30 minutes each in 1X SSC, 0.1% SDS at 65°C and two washes of 30 minutes each in 0.2 X SSC, 0.1% SDS at 65°C. DNA molecules according to the present invention include molecules that encode any of the above polypeptides.

In another aspect of the present invention, antibodies are provided. Such antibodies may be prepared by any of a variety of techniques known to those of ordinary skill in the *art. See, e.g.,* Harlow and Lane, Antibodies: A Laboratory Manual, Cold Spring Harbor Laboratory, 1988. In one such technique, an immunogen comprising the polypeptide is initially injected into any of a wide variety of mammals (e.g., mice, rats, rabbits, sheep or goats). In this step, the polypeptides of this invention may serve as the immunogen without modification. Alternatively, particularly for relatively short polypeptides, a superior immune response may be elicited if the polypeptide is joined to a carrier protein, such as bovine serum albumin or keyhole limpet hemocyanin. The immunogen is injected into the animal host, preferably according to a predetermined schedule incorporating one or more booster immunizations, and the animals are bled periodically. Polyclonal antibodies specific for the polypeptide may then be purified from such antisera by, for example, affinity chromatography using the polypeptide coupled to a suitable solid support.

Monoclonal antibodies specific for the antigenic polypeptide of interest may be prepared, for example, using the technique of Kohler and Milstein, Eur. J. Immunol. 6:511-519 (1976), and improvements thereto. Briefly, these methods involve the preparation of immortal cell lines capable of producing antibodies having the desired specificity (i.e., reactivity with the polypeptide of interest). Such cell lines may be produced, for example, from spleen cells obtained from an animal immunized as described above. The spleen cells are then immortalized by, for example, fusion with a myeloma cell fusion partner, preferably one that is syngeneic with the immunized animal. A variety of fusion techniques may be employed. For example, the spleen cells and myeloma cells may be combined with a nonionic detergent for a few minutes and then plated at low density on a selective medium that supports the growth of hybrid cells, but not myeloma cells. A preferred selection technique uses HAT (hypoxanthine, aminopterin, thymidine) selection. After a sufficient time, usually about 1 to 2 weeks, colonies of hybrids are observed. Single colonies are selected and their culture supernatants tested for binding activity against the polypeptide. Hybridomas having high reactivity and specificity are preferred.

Monoclonal antibodies may be isolated from the supernatants of growing hybridoma colonies. In addition, various techniques may be employed to enhance the yield, such as injection of the hybridoma cell line into the peritoneal cavity of a suitable vertebrate host, such as a mouse. Monoclonal antibodies may then be harvested from the ascites fluid or the blood. Contaminants may be removed from the antibodies by conventional techniques, such as chromatography, gel filtration, precipitation, and extraction. The polypeptides of this invention may be used in the purification process in, for example, an affinity chromatography step.

Antibodies may be used, for example, in methods for detecting breast cancer in a patient. Such methods involve using an antibody to detect the presence or absence of a breast tumor-specific polypeptide as described herein in a suitable biological sample. As used herein, suitable biological samples include tumor or normal tissue biopsy, mastectomy, blood, lymph node, serum or urine samples, or other tissue, homogenate, or extract thereof obtained from a patient.

There are a variety of assay formats known to those of ordinary skill in the art for using an antibody to detect polypeptide markers in a sample. *See, e.g.,* Harlow and Lane, Antibodies: A Laboratory Manual, Cold Spring Harbor Laboratory, 1988. For example, the assay may be performed in a Western blot format, wherein a protein preparation from the biological sample is submitted to gel electrophoresis, transferred to a suitable membrane and allowed to react with the antibody. The presence of the antibody on the membrane may then be detected using a suitable detection reagent, as described below.

In another embodiment, the assay involves the use of antibody immobilized on a solid support to bind to the polypeptide and remove it from the remainder of the sample. The bound polypeptide may then be detected using a second antibody or reagent that contains a reporter group. Alternatively, a competitive assay may be utilized, in which a polypeptide is labeled with a reporter group and allowed to bind to the immobilized antibody after incubation of the antibody with the sample. The extent to which components of the sample inhibit the binding of the labeled polypeptide to the antibody is indicative of the reactivity of the sample with the immobilized antibody, and as a result, indicative of the concentration of polypeptide in the sample.

The solid support may be any material known to those of ordinary skill in the art to which the antibody may be attached. For example, the solid support may be a test well in a microtiter plate or a nitrocellulose filter or other suitable membrane. Alternatively, the support may be a bead or disc, such as glass, fiberglass, latex or a plastic material such as polystyrene or polyvinylchloride. The support may also be a magnetic particle or a fiber optic sensor, such as those disclosed, for example, in U.S. Patent No. 5,359,681.

The antibody may be immobilized on the solid support using a variety of techniques known to those in the art, which are amply described in the patent and scientific literature. In the context of the present invention, the term "immobilization" refers to both noncovalent association, such as adsorption, and covalent attachment (which may be a direct linkage between the antigen and functional groups on the support or may be a linkage by way of a cross-linking agent). Immobilization by adsorption to a well in a microtiter plate or to a membrane is preferred. In such cases, adsorption may be achieved by contacting the antibody, in a suitable buffer, with the solid support for a suitable amount of time. The contact time varies with temperature, but is typically between about 1 hour and 1 day. In general, contacting a well of a plastic microtiter plate (such as polystyrene or polyvinylchloride) with an amount of antibody ranging from about 10 ng to about 1 µg, and preferably about 100-200 ng, is sufficient to immobilize an adequate amount of polypeptide.

Covalent attachment of antibody to a solid support may also generally be achieved by first reacting the support with a bifunctional reagent that will react with both the support and a functional group, such as a hydroxyl or amino group, on the antibody. For example, the antibody may be covalently attached to supports having an appropriate polymer coating using benzoquinone or by condensation of an aldehyde group on the support with an amine and an active hydrogen on the binding partner *(see, e*.*g*., Pierce Immunotechnology Catalog and Handbook (1991) at A12-A13).

In certain embodiments, the assay for detection of polypeptide in a sample is a two-antibody sandwich assay. This assay may be performed by first contacting an antibody that has been immobilized on a solid support, commonly the well of a microtiter plate, with the biological sample, such that the polypeptide within the sample are allowed to bind to the immobilized antibody. Unbound sample is then removed from the immobilized polypeptide-antibody complexes and a second antibody (containing a reporter group) capable of binding to a different site on the polypeptide is added. The amount of second antibody that remains bound to the solid support is then determined using a method appropriate for the specific reporter group.

More specifically, once the antibody is immobilized on the support as described above, the remaining protein binding sites on the support are typically blocked. Any suitable blocking agent known to those of ordinary skill in the art, such as bovine serum albumin or Tween 20^{™} (Sigma Chemical Co., St. Louis, MO). The immobilized antibody is then incubated with the sample, and polypeptide is allowed to bind to the antibody. The sample may be diluted with a suitable diluent, such as phosphate-buffered saline (PBS) prior to incubation. In general, an appropriate contact time (i.e., incubation time) is that period of time that is sufficient to detect the presence of polypeptide within a sample obtained from an individual with breast cancer. Preferably, the contact time is sufficient to achieve a level of binding that is at least 95% of that achieved at equilibrium between bound and unbound polypeptide. Those of ordinary skill in the art will recognize that the time necessary to achieve equilibrium may be readily determined by assaying the level of binding that occurs over a period of time. At room temperature, an incubation time of about 30 minutes is generally sufficient.

Unbound sample may then be removed by washing the solid support with an appropriate buffer, such as PBS containing 0.1% Tween 20^{™}. The second antibody, which contains a reporter group, may then be added to the solid support. Preferred reporter groups include enzymes (such as horseradish peroxidase), substrates, cofactors, inhibitors, dyes, radionuclides, luminescent groups, fluorescent groups and biotin. The conjugation of antibody to reporter group may be achieved using standard methods known to those of ordinary skill in the art.

The second antibody is then incubated with the immobilized antibody-polypeptide complex for an amount of time sufficient to detect the bound polypeptide. An appropriate amount of time may generally be determined by assaying the level of binding that occurs over a period of time. Unbound second antibody is then removed and bound second antibody is detected using the reporter group. The method employed for detecting the reporter group depends upon the nature of the reporter group. For radioactive groups, scintillation counting or autoradiographic methods are generally appropriate. Spectroscopic methods may be used to detect dyes, luminescent groups and fluorescent groups. Biotin may be detected using avidin, coupled to a different reporter group (commonly a radioactive or fluorescent group or an enzyme). Enzyme reporter groups may generally be detected by the addition of substrate (generally for a specific period of time), followed by spectroscopic or other analysis of the reaction products.

To determine the presence or absence of breast cancer, the signal detected from the reporter group that remains bound to the solid support is generally compared to a signal that corresponds to a predetermined cut-off value established from non-tumor tissue. In one preferred embodiment, the cut-off value is the average mean signal obtained when the immobilized antibody is incubated with samples from patients without breast cancer. In general, a sample generating a signal that is three standard deviations above the predetermined cut-off value may be considered positive for breast cancer. In an alternate preferred embodiment, the cut-off value is determined using a Receiver Operator Curve, according to the method of Sackett et al., Clinical Epidemiology: A Basic Science for Clinical Medicine, p. 106-7 (Little Brown and Co., 1985). Briefly, in this embodiment, the cut-off value may be determined from a plot of pairs of true positive rates (*i*.*e*., sensitivity) and false positive rates (100%-specificity) that correspond to each possible cut-off value for the diagnostic test result. The cut-off value on the plot that is the closest to the upper left-hand corner (i.e., the value that encloses the largest area) is the most accurate cut-off value, and a sample generating a signal that is higher than the cut-off value determined by this method may be considered positive. Alternatively, the cut-off value may be shifted to the left along the plot, to minimize the false positive rate, or to the right, to minimize the false negative rate. In general, a sample generating a signal that is higher than the cut-off value determined by this method is considered positive for breast cancer.

In a related embodiment, the assay is performed in a flow-through or strip test format, wherein the antibody is immobilized on a membrane, such as nitrocellulose. In the flow-through test, the polypeptide within the sample bind to the immobilized antibody as the sample passes through the membrane. A second, labeled antibody then binds to the antibody-polypeptide complex as a solution containing the second antibody flows through the membrane. The detection of bound second antibody may then be performed as described above. In the strip test format, one end of the membrane to which antibody is bound is immersed in a solution containing the sample. The sample migrates along the membrane through a region containing second antibody and to the area of immobilized antibody. Concentration of second antibody at the area of immobilized antibody indicates the presence of breast cancer. Typically, the concentration of second antibody at that site generates a pattern, such as a line, that can be read visually. The absence of such a pattern indicates a negative result. In general, the amount of antibody immobilized on the membrane is selected to generate a visually discernible pattern when the biological sample contains a level of polypeptide that would be sufficient to generate a positive signal in the two-antibody sandwich assay, in the format discussed above. Preferably, the amount of antibody immobilized on the membrane ranges from about 25 ng to about 1 µg, and more preferably from about 50 ng to about 1 µg. Such tests can typically be performed with a very small amount of biological sample.

The presence or absence of breast cancer in a patient may also be determined by evaluating the level of mRNA encoding a breast tumor-specific polypeptide as described herein within the biological sample (e.g., a biopsy, mastectomy and/or blood sample from a patient) relative to a predetermined cut-off value. Such an evaluation may be achieved using any of a variety of methods known to those of ordinary skill in the art such as, for *example, in situ* hybridization and amplification by polymerase chain reaction.

For example, polymerase chain reaction may be used to amplify sequences from cDNA prepared from RNA that is isolated from one of the above biological samples. Sequence-specific primers for use in such amplification may be designed based on the sequences provided in any one of SEQ ID NO: 1, 11-86 and 142-297, and may be purchased or synthesized. In the case of B18Ag1, as noted herein, one suitable primer pair is B18Ag1-2 (5'ATG GCT ATT TTC GGG GGC TGA CA) (SEQ ID NO.:126) and B18Ag1-3 (5'CCG GTA TCT CCT CGT GGG TAT T) (SEQ ID NO.:127). The PCR reaction products may then be separated by gel electrophoresis and visualized according to methods well known to those of ordinary skill in the art. Amplification is typically performed on samples obtained from matched pairs of tissue (tumor and non-tumor tissue from the same individual) or from unmatched pairs of tissue (tumor and non-tumor tissue from different individuals). The amplification reaction is preferably performed on several dilutions of cDNA spanning two orders of magnitude. A two-fold or greater increase in expression in several dilutions of the tumor sample as compared to the same dilution of the non-tumor sample is considered positive.

As used herein, the term "primer/probe specific for a DNA/RNA molecule" means an oligonucleotide sequence that has at least about 80% identity, preferably at least about 90% and more preferably at least about 95%, identity to the DNA/RNA molecule in question. Primers and/or probes which may be usefully employed in the inventive diagnostic methods preferably have at least about 10-40 nucleotides. In a preferred embodiment, the polymerase chain reaction primers comprise at least about 10 contiguous nucleotides of a DNA/RNA molecule encoding one of the polypeptides disclosed herein. Preferably, oligonucleotide probes for use in the inventive diagnostic methods comprise at least about 15 contiguous oligonucleotides of a DNA/RNA molecule encoding one of the polypeptides disclosed herein. Techniques for both PCR based assays and *in situ* hybridization assays are well known in the art.

Conventional RT-PCR protocols using agarose and ethidium bromide staining while important in defining gene specificity do not lend themselves to diagnostic kit development because of the time and effort required in making them quantitative (i.e., construction of saturation and/or titration curves), and their sample throughput. This problem is overcome by the development of procedures such as real time RT-PCR which allows for assays to be performed in single tubes, and in turn can be modified for use in 96 well plate formats. Instrumentation to perform such methodologies are available from Perkin Elmer/Applied Biosystems Division. Alternatively, other high throughput assays using labeled probes (e.g., digoxygenin) in combination with labeled (e.g., enzyme fluorescent, radioactive) antibodies to such probes can also be used in the development of 96 well plate assays.

In yet another method for determining the presence or absence of breast cancer in a patient, one or more of the breast tumor-specific polypeptides described may be used in a skin test. As used herein, a "skin test" is any assay performed directly on a patient in which a delayed-type hypersensitivity (DTH) reaction (such as swelling, reddening or dermatitis) is measured following intradermal injection of one or more polypeptides as described above. Such injection may be achieved using any suitable device sufficient to contact the polypeptide or polypeptides with dermal cells of the patient, such as a tuberculin syringe or 1 mL syringe. Preferably, the reaction is measured at least 48 hours after injection, more preferably 48-72 hours.

The DTH reaction is a cell-mediated immune response, which is greater in patients that have been exposed previously to a test antigen (*i*.*e*., an immunogenic portion of a polypeptide employed, or a variant thereof). The response may measured visually, using a ruler. In general, a response that is greater than about 0.5 cm in diameter, preferably greater than about 5.0 cm in diameter, is a positive response, indicative of breast cancer.

The breast tumor-specific polypeptides described herein are preferably formulated, for use in a skin test, as pharmaceutical compositions containing at least one polypeptide and a physiologically acceptable carrier, such as water, saline, alcohol, or a buffer. Such compositions typically contain one or more of the above polypeptides in an amount ranging from about 1 µg to 100 µg, preferably from about 10 µg to 50 µg in a volume of 0.1 mL. Preferably, the carrier employed in such pharmaceutical compositions is a saline solution with appropriate preservatives, such as phenol and/or Tween 80^{™}.

In other aspects of the present invention, the progression and/or response to treatment of a breast cancer may be monitored by performing any of the above assays over a period of time, and evaluating the change in the level of the response (*i*.*e*., the amount of polypeptide or mRNA detected or, in the case of a skin test, the extent of the immune response detected). For example, the assays may be performed every month to every other month for a period of 1 to 2 years. In general, breast cancer is progressing in those patients in whom the level of the response increases over time. In contrast, breast cancer is not progressing when the signal detected either remains constant or decreases with time.

In further aspects of the present invention, the compounds described herein may be used for the immunotherapy of breast cancer. In these aspects, the compounds (which may be polypeptides, antibodies or nucleic acid molecules) are preferably incorporated into pharmaceutical compositions or vaccines. Pharmaceutical compositions comprise one or more such compounds and a physiologically acceptable carrier. Vaccines may comprise one or more polypeptides and an immune response enhancer, such as an adjuvant or a liposome (into which the compound is incorporated). Pharmaceutical compositions and vaccines may additionally contain a delivery system, such as biodegradable microspheres which are disclosed, for example, in U.S. Patent Nos. 4,897,268 and 5,075,109. Pharmaceutical compositions and vaccines within the scope of the present invention may also contain other compounds, including one or more separate polypeptides.

Alternatively, a vaccine may contain DNA encoding one or more of the polypeptides as described above, such that the polypeptide is generated *in situ.* In such vaccines, the DNA may be present within any of a variety of delivery systems known to those of ordinary skill in the art, including nucleic acid expression systems, bacteria and viral expression systems. Appropriate nucleic acid expression systems contain the necessary DNA sequences for expression in the patient (such as a suitable promoter and terminating signal). Bacterial delivery systems involve the administration of a bacterium (such as *Bacillus-Calmette-Guerrin)* that expresses an immunogenic portion of the polypeptide on its cell surface. In a preferred embodiment, the DNA may be introduced using a viral expression system (e.g., vaccinia or other pox virus, retrovirus, or adenovirus), which may involve the use of a non-pathogenic (defective), replication competent virus. Techniques for incorporating DNA into such expression systems are well known to those of ordinary skill in the art. The DNA may also be "naked," as described, for example, in Ulmer et al., Science 259:1745-1749 (1993), and reviewed by Cohen, Science 259:1691-1692 (1993). The uptake of naked DNA may be increased by coating the DNA onto biodegradable beads, which are efficiently transported into the cells.

While any suitable carrier known to those of ordinary skill in the art may be employed in the pharmaceutical compositions of this invention, the type of carrier will vary depending on the mode of administration. For parenteral administration, such as subcutaneous injection, the carrier preferably comprises water, saline, alcohol, a fat, a wax or a buffer. For oral administration, any of the above carriers or a solid carrier, such as mannitol, lactose, starch, magnesium stearate, sodium saccharine, talcum, cellulose, glucose, sucrose, and magnesium carbonate, may be employed. Biodegradable microspheres (e.g., polylactate polyglycolate) may also be employed as carriers for the pharmaceutical compositions of this invention.

Any of a variety of adjuvants may be employed in the vaccines of this invention to nonspecifically enhance the immune response. Most adjuvants contain a substance designed to protect the antigen from rapid catabolism, such as aluminum hydroxide or mineral oil, and a stimulator of immune responses, such as lipid A, *Bortadella pertussis* or *Mycobacterium tuberculosis* derived proteins. Suitable adjuvants are commercially available as, for example, Freund's Incomplete Adjuvant and Complete Adjuvant (Difco Laboratories, Detroit, MI), Merck Adjuvant 65 (Merck and Company, Inc., Rahway, NJ), alum, biodegradable microspheres, monophosphoryl lipid A and quil A. Cytokines, such as GM-CSF or interleukin-2, -7, or -12, may also be used as adjuvants.

The above pharmaceutical compositions and vaccines may be used, for example, for the therapy of breast cancer in a patient. As used herein, a "patient" refers to any warm-blooded animal, preferably a human. A patient may or may not be afflicted with breast cancer. Accordingly, the above pharmaceutical compositions and vaccines may be used to prevent the development of breast cancer or to treat a patient afflicted with breast cancer. To prevent the development of breast cancer, a pharmaceutical composition or vaccine comprising one or more polypeptides as described herein may be administered to a patient. Alternatively, naked DNA or plasmid or viral vector encoding the polypeptide may be administered. For treating a patient with breast cancer, the pharmaceutical composition or vaccine may comprise one or more polypeptides, antibodies or nucleotide sequences complementary to DNA encoding a polypeptide as described herein (e.g., antisense RNA or antisense deoxyribonucleotide oligonucleotides).

Routes and frequency of administration, as well as dosage, will vary from individual to individual. In general, the pharmaceutical compositions and vaccines may be administered by injection (e.g., intracutaneous, intramuscular, intravenous or subcutaneous), intranasally (e.g., by aspiration) or orally. Between 1 and 10 doses may be administered for a 52-week period. Preferably, 6 doses are administered, at intervals of 1 month, and booster vaccinations may be given periodically thereafter. Alternate protocols may be appropriate for individual patients. A suitable dose is an amount of a compound that, when administered as described above, is capable of promoting an anti-tumor immune response. Such response can be monitored by measuring the anti-tumor antibodies in a patient or by vaccine-dependent generation of cytolytic effector cells capable of killing the patient's tumor cells *in vitro.* Such vaccines should also be capable of causing an immune response that leads to an improved clinical outcome (*e*.*g*., more frequent remissions, complete or partial or longer disease-free survival) in vaccinated patients as compared to non-vaccinated patients. In general, for pharmaceutical compositions and vaccines comprising one or more polypeptides, the amount of each polypeptide present in a dose ranges from about 100 µg to 5 mg. Suitable dose sizes will vary with the size of the patient, but will typically range from about 0.1 mL to about 5 mL.

Aspects of the invention are defined in the numbered paragraphs below:
1. An isolated DNA molecule, comprising:
   (a) a nucleotide sequence selected from the group consisting of SEQ ID NO:1, 3-26, 28-77, 142, 143, 146-152, 154-166, 168-176, 178-192, 194-198, 200-204, 206, 207, 209-214, 216, 218, 219, 221-240, 243-245, 247, 250, 251, 253, 255, 257-266, 268, 269, 271-273, 275, 276, 278, 280, 281, 284, 288 and 291-297;
   (b) a variant of said nucleotide sequence that contains one or more nucleotide substitutions, deletions, insertions and/or modifications at no more than 20% of the nucleotide positions, such that the antigenic and/or immunogenic properties of the polypeptide encoded by the nucleotide sequence are retained; or
   (c) a nucleotide sequence encoding an epitope of a polypeptide encoded by at least one sequence selected from the group consisting of SEQ ID NO: 1, 3-26, 28-77, 142, 143, 146-152, 154-166, 168-176, 178-192, 194-198, 200-204, 206, 207, 209-214, 216, 218, 219, 221-240, 243-245, 247, 250, 251, 253, 255, 257-266, 268, 269, 271-273, 275, 276, 278, 280, 281, 284, 288 and 291-297.
2. An isolated DNA molecule encoding an epitope of a polypeptide, wherein said polypeptide is encoded by a nucleotide sequence that:
   (a) hybridizes to a sequence selected from the group consisting of SEQ ID NO:1, 3-26, 28-77, 142, 143, 146-152, 154-166, 168-176, 178-192, 194-198, 200-204, 206, 207, 209-214, 216, 218, 219, 221-240, 243-245, 247, 250, 251, 253, 255, 257-266, 268, 269, 271-273, 275, 276, 278, 280, 281, 284, 288 and 291-297 under stringent conditions; and
   (b) is at least 80% identical to a sequence selected from the group consisting of SEQ ID NO:1, 3-26, 28-77, 142, 143, 146-152, 154-166, 168-176, 178-192, 194-198, 200-204, 206, 207, 209-214, 216, 218, 219, 221-240, 243-245, 247, 250, 251, 253, 255, 257-266, 268, 269, 271-273, 275, 276, 278, 280, 281, 284, 288 and 291-297.
3. An isolated DNA molecule encoding an epitope of a polypeptide, wherein said polypeptide is encoded by:
   (a) a nucleotide sequence transcribed from the sequence of SEQ ID NO: 141; or
   (b) a variant of said nucleotide sequence that contains one or more nucleotide substitutions, deletions, insertions and/or modifications at no more than 20% of the nucleotide positions, such that the antigenic and/or immunogenic properties of the polypeptide encoded by the nucleotide sequence are retained.
4. An isolated DNA or RNA molecule comprising a nucleotide sequence complementary to a DNA molecule according to any one of paragraphs 1-3.
5. A recombinant expression vector comprising a DNA molecule according to any one of paragraphs 1-3.
6. A host cell transformed or transfected with an expression vector according to paragraph 5.
7. A polypeptide comprising an amino acid sequence encoded by a DNA molecule according to any one of paragraphs 1-3.
8. A polypeptide according to paragraph 7 wherein said polypeptide comprises an epitope of an amino acid sequence encoded by at least one nucleotide sequence selected from the group consisting of SEQ ID NO:1, 3-26, 28-77, 142, 143, 146-152, 154-166, 168-176, 178-192, 194-198, 200-204, 206, 207, 209-214, 216, 218, 219, 221-240, 243-245, 247, 250, 251, 253, 255, 257-266, 268, 269, 271-273, 275, 276, 278, 280, 281, 284, 288 and 291-297.
9. A monoclonal antibody that binds to a polypeptide according to paragraph 7.
10. A method for determining the presence of breast cancer in a patient comprising detecting, within a biological sample, at least one polypeptide according to paragraph 7, and therefrom determining the presence of breast cancer in the patient.
11. A method for determining the presence of breast cancer in a patient comprising detecting within a biological sample, at least one polypeptide encoded by a nucleotide sequence selected from the group consisting of SEQ ID NO: 78-86, 144, 145, 153, 167, 177, 193, 199, 205, 208, 215, 217, 220, 241, 242, 246, 248, 249, 252, 256, 267, 270, 274, 277, 279, 282, 283, 285-287, 289, 290 and sequences that hybridize thereto under stringent conditions.
12. The method of paragraphs 10 or 11 wherein the biological sample is a portion of a breast tumor.
13. The method of paragraph 10 wherein the step of detecting comprises contacting the biological sample with a monoclonal antibody according to claim 9.
14. The method of paragraph 11 wherein the step of detecting comprises contacting the biological sample with a monoclonal antibody that binds to a polypeptide encoded by a nucleotide sequence selected from the group consisting of SEQ ID NO: 78-86, 144, 145, 153, 167, 177, 193, 199, 205, 208, 215, 217, 220, 241, 242, 246, 248, 249, 252, 256, 267, 270, 274, 277, 279, 282, 283, 285-287, 289, 290 and sequences that hybridize thereto under stringent conditions.
15. A method for determining the presence of breast cancer in a patient comprising detecting, within a biological sample, an RNA molecule encoding at least one polypeptide according to paragraph 7, and therefrom determining the presence of breast cancer in the patient.
16. A method for determining the presence of breast cancer in a patient comprising detecting, within a biological sample, at least one RNA molecule encoding a polypeptide encoded by a nucleotide sequence selected from the group consisting of SEQ ID NO: 78-86, 144, 145, 153, 167, 177, 193, 199, 205, 208, 215, 217, 220, 241, 242, 246, 248, 249, 252, 256, 267, 270, 274, 277, 279, 282, 283, 285-287, 289, 290 and sequences that hybridize thereto under stringent conditions; and therefrom determining the presence of breast cancer in the patient.
17. The method of paragraphs 15 or 16 wherein the biological sample is a portion of a breast tumor.
18. The method of paragraph 15 wherein the step of detecting comprises:
   (a) preparing cDNA from RNA molecules within the biological sample; and
   (b) specifically amplifying cDNA molecules that are capable of encoding at least a portion of a polypeptide according to paragraph 7, and therefrom determining the presence of breast cancer in the patient.
19. The method of paragraph 16 wherein the step of detecting comprises:
   (a) preparing cDNA from RNA molecules within the biological sample; and
   (b) specifically amplifying cDNA molecules that are capable of encoding at least a portion of a polypeptide encoded by a nucleotide sequence selected from the group consisting of SEQ ID NO: 78-86, 144, 145, 153, 167, 177, 193, 199, 205, 208, 215, 217, 220, 241, 242, 246, 248, 249, 252, 256, 267, 270, 274, 277, 279, 282, 283, 285-287, 289, 290 and sequences that hybridize thereto under stringent conditions; and therefrom determining the presence of breast cancer in the patient.
20. A method for monitoring the progression of breast cancer in a patient, comprising:
   (a) detecting an amount, in a biological sample, of at least one polypeptide according to paragraph 7 at a first point in time;
   (b) repeating step (a) at a subsequent point in time; and
   (c) comparing the amounts of polypeptide detected in steps (a) and (b), and therefrom monitoring the progression of breast cancer in the patient.
21. A method for monitoring the progression of breast cancer in a patient, comprising:
   (a) detecting in a biological sample an amount of at least one polypeptide at a first point in time, the polypeptide being encoded by a nucleotide sequence selected from the group consisting of SEQ ID NO: 78-86, 144, 145, 153, 167, 177, 193, 199, 205, 208, 215, 217, 220, 241, 242, 246, 248, 249, 252, 256, 267, 270, 274, 277, 279, 282, 283, 285-287, 289, 290 and sequences that hybridize thereto under stringent conditions;
   (b) repeating step (a) at a subsequent point in time; and
   (c) comparing the amounts of polypeptide detected in steps (a) and (b), and therefrom monitoring the progression of breast cancer in the patient.
22. The method of paragraphs 20 or 21 wherein the biological sample is a portion of a breast tumor.
23. The method of paragraph 20 wherein the step of detecting comprises contacting a portion of the biological sample with a monoclonal antibody according to claim 9.
24. The method of paragraph 21 wherein the step of detecting comprises contacting the biological sample with a monoclonal antibody that binds to a polypeptide encoded by a nucleotide sequence selected from the group consisting of SEQ ID NO: 78-86, 144, 145, 153, 167, 177, 193, 199, 205, 208, 215, 217, 220, 241, 242, 246, 248, 249, 252, 256, 267, 270, 274, 277, 279, 282, 283, 285-287, 289, 290 and sequences that hybridize thereto under stringent conditions.
25. The method of paragraph 20 wherein said polypeptide comprises an epitope of an amino acid sequence encoded by at least one nucleotide sequence selected from the group consisting of SEQ ID NO:1, 3-26, 28-77, 142, 143, 146-152, 154-166, 168-176, 178-192, 194-198, 200-204, 206, 207, 209-214, 216, 218, 219, 221-240, 243-245, 247, 250, 251, 253, 255, 257-266, 268, 269, 271-273, 275, 276, 278, 280, 281, 284, 288 and 291-297.
26. A method for monitoring the progression of breast cancer in a patient, comprising:
   (a) detecting an amount, within a biological sample, of at least one RNA molecule encoding a polypeptide according to paragraph 7 at a first point in time;
   (b) repeating step (a) at a subsequent point in time; and
   (c) comparing the amounts of RNA molecules detected in steps (a) and (b), and therefrom monitoring the progression of breast cancer in the patient.
27. The method of paragraph 26 wherein the step of detecting comprises:
   (a) preparing cDNA from RNA molecules within the biological sample; and
   (b) specifically amplifying cDNA molecules that are capable of encoding at least a portion of a polypeptide according to paragraph 7.
28. A method for monitoring the progression of breast cancer in a patient, comprising:
   (a) detecting an amount, within a biological sample, of at least one RNA molecule at a first point in time, the RNA molecule encoding a polypeptide encoded by a nucleotide sequence selected from the group consisting of SEQ ID NO: 78-86, 144, 145, 153, 167, 177, 193, 199, 205, 208, 215, 217, 220, 241, 242, 246, 248, 249, 252, 256, 267, 270, 274, 277, 279, 282, 283, 285-287, 289, 290 and sequences that hybridize thereto under stringent conditions;
   (b) repeating step (a) at a subsequent point in time; and
   (c) comparing the amounts of RNA molecules detected in steps (a) and (b), and therefrom monitoring the progression of breast cancer in the patient.
29. A pharmaceutical composition, comprising a polypeptide according to paragraph 7 and a physiologically acceptable carrier.
30. A pharmaceutical composition for inhibiting the development of breast cancer, comprising a polypeptide and a physiologically acceptable carrier, the polypeptide being encoded by a nucleotide sequence selected from the group consisting of SEQ ID NO: 78-86, 144, 145, 153, 167, 177, 193, 199, 205, 208, 215, 217, 220, 241, 242, 246, 248, 249, 252, 256, 267, 270, 274, 277, 279, 282, 283, 285-287, 289, 290 and sequences that hybridize thereto under stringent conditions.
31. A vaccine, comprising a polypeptide according to paragraph 7 and an immune response enhancer.
32. A vaccine, comprising a DNA molecule according to any one of paragraphs 1-3.
33. A vaccine, comprising a recombinant expression vector comprising a DNA molecule according to any one of paragraphs 1-3.
34. A vaccine for inhibiting the development of breast cancer, comprising a polypeptide and an immune response enhancer, the polypeptide being encoded by a nucleotide sequence selected from the group consisting of SEQ ID NO: 78-86, 144, 145, 153, 167, 177, 193, 199, 205, 208, 215, 217, 220, 241, 242, 246, 248, 249, 252, 256, 267, 270, 274, 277, 279, 282, 283, 285-287, 289, 290 and sequences that hybridize thereto under stringent conditions.
35. A pharmaceutical composition according to either of paragraphs 29 or 30, for use in the manufacture of a medicament for inhibiting the development of breast cancer in a patient, comprising administering to a patient.
36. A vaccine according to any one of paragraphs 31-34, for use in the manufacture of a medicament for inhibiting the development of breast cancer in a patient.
37. A diagnostic kit comprising:
   (a) one or more monoclonal antibodies according to paragraph 9; and
   (b) a detection reagent.
38. A diagnostic kit comprising:
   (a) one or more monoclonal antibodies that bind to a polypeptide encoded by a nucleotide sequence selected from the group consisting of sequences provided in SEQ ID 78-86, 144, 145, 153, 167, 177, 193, 199, 205, 208, 215, 217, 220, 241, 242 and 246, 248, 249, 252, 256, 267, 270, 274, 277, 279, 282, 283, 285-287, 289, 290; and
   (b) a detection reagent.
39. The kit of any one of paragraphs 37 or 38 wherein the monoclonal antibody(s) are immobilized on a solid support.
40. A diagnostic kit comprising two polymerase chain reaction primers, at least one of the primers being specific for an RNA molecule according to paragraph 4.
41. The kit of paragraph 40, wherein at least one of the polymerase chain reaction primers comprises at least about 10 contiguous nucleotides of an RNA molecule according to paragraph 4.
42. A diagnostic kit comprising two polymerase chain reaction primers, at least one of the primers being specific for an RNA molecule encoding a polypeptide encoded by a nucleotide sequence selected from the group consisting of SEQ ID NOS:78-86, 144, 145, 153, 167, 177, 193, 199, 205, 208, 215, 217, 220, 241, 242 246, 248, 249, 252, 256, 267, 270, 274, 277, 279, 282, 283, 285-287, 289 and 290.
43. The kit of paragraph 42, wherein at least one of the polymerase chain reaction primers comprises at least about 10 contiguous nucleotides of an RNA molecule encoding a polypeptide encoded by a nucleotide sequence selected from the group consisting of SEQ ID NOS:78-86, 144, 145, 153, 167, 177, 193, 199, 205, 208, 215, 217, 220, 241, 242 246, 248, 249, 252, 256, 267, 270, 274, 277, 279, 282, 283, 285-287, 289 and 290.
44. A diagnostic kit comprising at least one oligonucleotide probe, the oligonucleotide probe containing at least about 15 contiguous nucleotides of a DNA molecule according to paragraph 4.
45. A diagnostic kit comprising at least one oligonucleotide probe, the oligonucleotide probe comprising at least about 15 contiguous nucleotides of a DNA sequence selected from the group consisting of SEQ ID NOS:78-86, 144, 145, 153, 167, 177, 193, 199, 205, 208, 215, 217, 220, 241, 242 246, 248, 249, 252, 256, 267, 270, 274, 277, 279, 282, 283, 285-287, 289 and 290.
46. A diagnostic kit comprising at least one oligonucleotide probe specific for a DNA molecule according to paragraph 4.
47. The kit of paragraph 46, wherein the oligonucleotide probe comprises at least about 15 contiguous nucleotides of a DNA molecule according to paragraph 4.
48. A diagnostic kit comprising at least one oligonucleotide probe specific for a DNA sequence selected from the group consisting of SEQ ID NOS:78-86,
49. The kit of paragraph 48, wherein the oligonucleotide probe comprises at least about 15 contiguous nucleotides of a DNA sequence selected from the group consisting of SEQ ID NOS:78-86, 144, 145, 153, 167, 177, 193, 199, 205, 208, 215, 217, 220, 241, 242 246, 248, 249, 252, 256, 267, 270, 274, 277, 279, 282, 283, 285-287, 289 and 290.

The following Examples are offered by way of illustration and not by way of limitation.

### EXAMPLES

### EXAMPLE 1

PREPARATION OF BREAST TUMOR-SPECIFIC cDNAs USING

### DIFFERENTIAL DISPLAY RT-PCR

This Example illustrates the preparation of cDNA molecules encoding breast tumor-specific polypeptides using a differential display screen.

### A. Preparation of B18Ag1 cDNA and Characterization of mRNA Expression

Tissue samples were prepared from breast tumor and normal tissue of a patient with breast cancer that was confirmed by pathology after removal from the patient. Normal RNA and tumor RNA was extracted from the samples and mRNA was isolated and converted into cDNA using a (dT)₁₂AG (SEQ ID NO.:130) anchored 3' primer. Differential display PCR was then executed using a randomly chosen primer (CTTCAACCTC) (SEQ ID NO.:103). Amplification conditions were standard buffer containing 1.5 mM MgCl₂, 20 pmol of primer, 500 pmol dNTP, and 1 unit of *Taq* DNA polymerase (Perkin-Elmer, Branchburg, NJ). Forty cycles of amplification were performed using 94°C denaturation for 30 seconds, 42°C annealing for 1 minute, and 72°C extension for 30 seconds. An RNA fingerprint containing 76 amplified products was obtained. Although the RNA fingerprint of breast tumor tissue was over 98% identical to that of the normal breast tissue, a band was repeatedly observed to be specific to the RNA fingerprint pattern of the tumor. This band was cut out of a silver stained gel, subcloned into the T-vector (Novagen, Madison, WI) and sequenced.

The sequence of the cDNA, referred to as B18Ag1, is provided in SEQ ID NO:1. A database search of GENBANK and EMBL revealed that the B18Ag1 fragment initially cloned is 77% identical to the endogenous human retroviral element S71, which is a truncated retroviral element homologous to the Simian Sarcoma Virus (SSV). S71 contains an incomplete *gag* gene, a portion of the *pol* gene and an LTR-like structure at the 3' terminus (*see* Werner et al., Virology 174:225-238 (1990)). B18Agl is also 64% identical to SSV in the region corresponding to the P30 (gag) locus. B18Ag1 contains three separate and incomplete reading frames covering a region which shares considerable homology to a wide variety of gag proteins of retroviruses which infect mammals. In addition, the homology to S71 is not just within the *gag* gene, but spans several kb of sequence including an LTR.

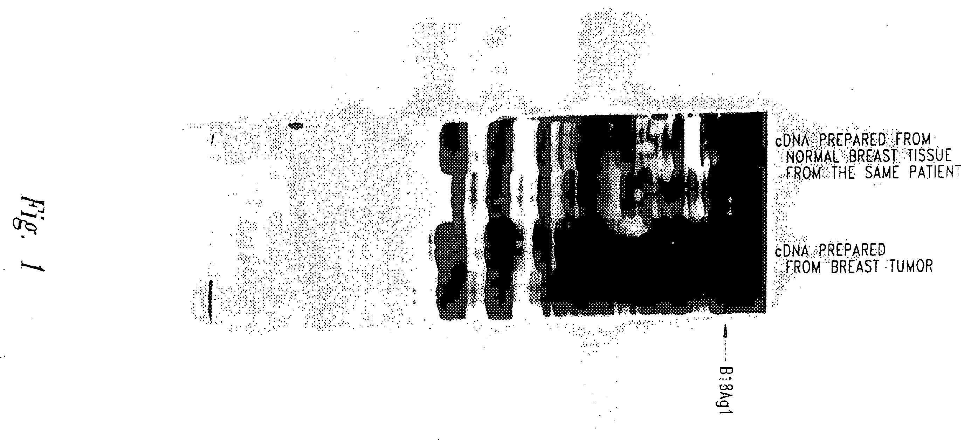

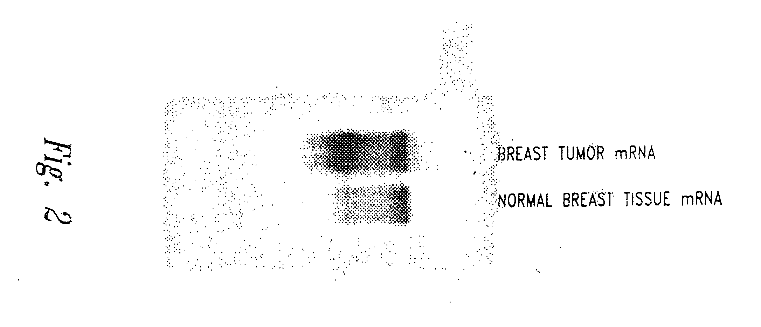

B18Ag1-specific PCR primers were synthesized using computer analysis guidelines. RT-PCR amplification (94°C, 30 seconds; 60°C → 42°C, 30 seconds; 72°C, 30 seconds for 40 cycles) confirmed that B18Ag1 represents an actual mRNA sequence present at relatively high levels in the patient's breast tumor tissue. The primers used in amplification were B18Ag1-1 (CTG CCT GAG CCA CAA ATG) (SEQ ID NO.:128) and B18Ag1-4 (CCG GAG GAG GAA GCT AGA GGA ATA) (SEQ ID NO.:129) at a 3.5 mM magnesium concentration and a pH of 8.5, and B18Ag1-2 (ATG GCT ATT TTC GGG GCC TGA CA) (SEQ ID NO.:126) and B18Ag1-3 (CCG GTA TCT CCT CGT GGG TAT T) (SEQ ID NO.:127) at 2 mM magnesium at pH 9.5. The same experiments showed exceedingly low to nonexistent levels of expression in this patient's normal breast tissue *(see* Figure 1). RT-PCR experiments were then used to show that B18Ag1 mRNA is present in nine other breast tumor samples (from Brazilian and American patients) but absent in, or at exceedingly low levels in, the normal breast tissue corresponding to each cancer patient. RT-PCR analysis has also shown that the B18Ag1 transcript is not present in various normal tissues (including lymph node, myocardium and liver) and present at relatively low levels in PBMC and lung tissue. The presence of B18Ag1 mRNA in breast tumor samples, and its absence from normal breast tissue, has been confirmed by Northern blot analysis, as shown in Figure 2.

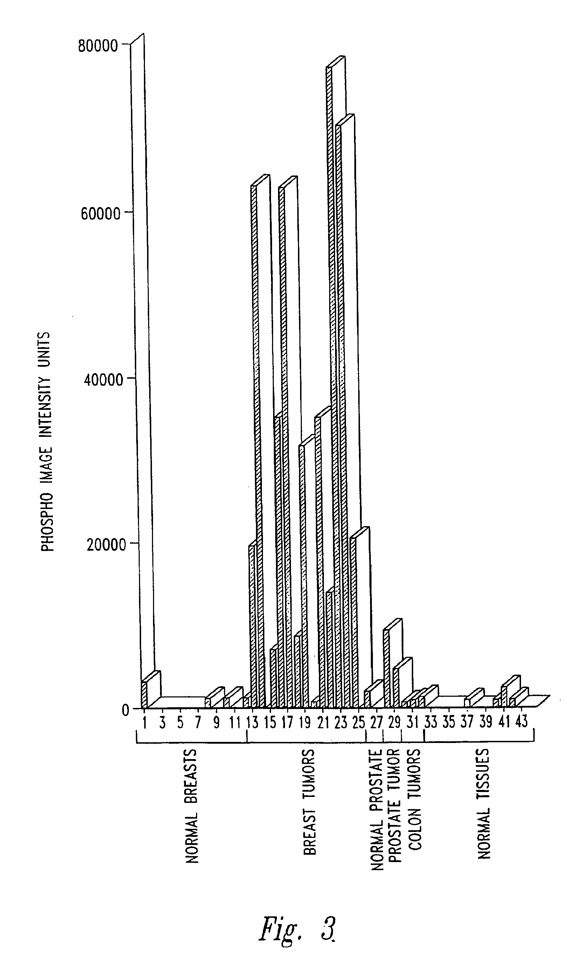

The differential expression of B18Ag1 in breast tumor tissue was also confirmed by RNase protection assays. Figure 3 shows the level of B18Ag1 mRNA in various tissue types as determined in four different RNase protection assays. Lanes 1-12 represent various normal breast tissue samples, lanes 13-25 represent various breast tumor samples; lanes 26-27 represent normal prostate samples; lanes 28-29 represent prostate tumor samples; lanes 30-32 represent colon tumor samples; lane 33 represents normal aorta; lane 34 represents normal small intestine; lane 35 represents normal skin, lane 36 represents normal lymph node; lane 37 represents normal ovary; lane 38 represents normal liver; lane 39 represents normal skeletal muscle; lane 40 represents a first normal stomach sample, lane 41 represents a second normal stomach sample; lane 42 represents a normal lung; lane 43 represents normal kidney; and lane 44 represents normal pancreas. Interexperimental comparison was facilitated by including a positive control RNA of known β-actin message abundance in each assay and normalizing the results of the different assays with respect to this positive control.

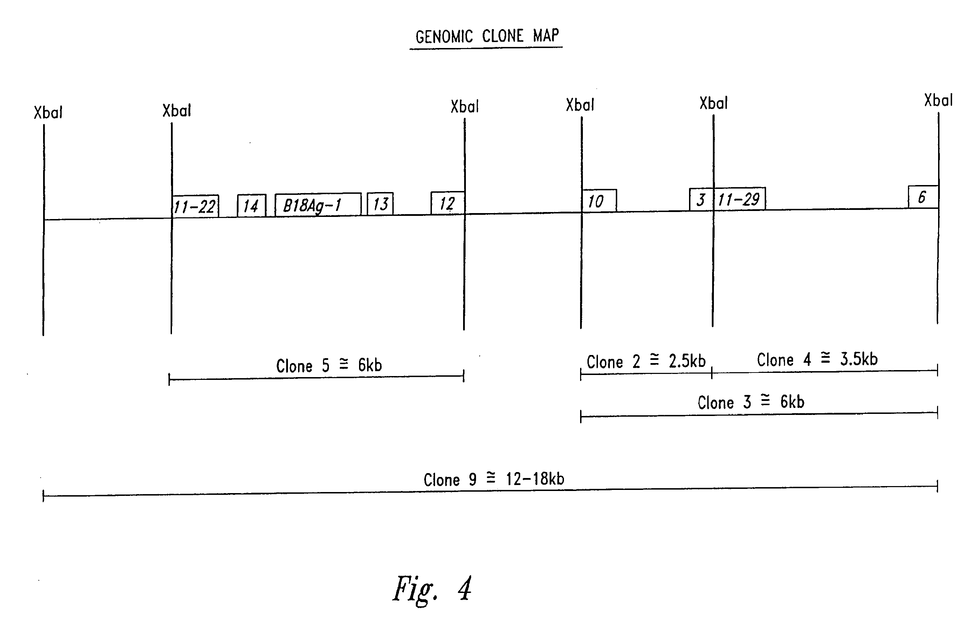

RT-PCR and Southern Blot analysis has shown the B18Ag1 locus to be present in human genomic DNA as a single copy endogenous retroviral element. A genomic clone of approximately 12-18 kb was isolated using the initial B18Ag1 sequence as a probe. Four additional subclones were also isolated by XbaI digestion. Additional retroviral sequences obtained from the ends of the XbaI digests of these clones (located as shown in Figure 4) are shown as SEQ ID NO:3 - SEQ ID NO:10, where SEQ ID NO:3 shows the location of the sequence labeled 10 in Figure 4, SEQ ID NO:4 shows the location of the sequence labeled 11-29, SEQ ID NO:5 shows the location of the sequence labeled 3, SEQ ID NO:6 shows the location of the sequence labeled 6, SEQ ID NO:7 shows the location of the sequence labeled 12, SEQ ID NO:8 shows the location of the sequence labeled 13, SEQ ID NO:9 shows the location of the sequence labeled 14 and SEQ ID NO: 10 shows the location of the sequence labeled 11-22.

Subsequent studies demonstrated that the 12-18 kb genomic clone contains a retroviral element of about 7.75 kb, as shown in Figures 5A and 5B. The sequence of this retroviral element is shown in SEQ ID NO: 141. The numbered line at the top of Figure 5A represents the sense strand sequence of the retroviral genomic clone. The box below this line shows the position of selected restriction sites. The arrows depict the different overlapping clones used to sequence the retroviral element. The direction of the arrow shows whether the single-pass subclone sequence corresponded to the sense or anti-sense strand. Figure 5B is a schematic diagram of the retroviral element containing B18Ag1 depicting the organization of viral genes within the element. The open boxes correspond to predicted reading frames, starting with a methionine, found throughout the element. Each of the six likely reading frames is shown, as indicated to the left of the boxes, with frames 1-3 corresponding to those found on the sense strand.

Using the cDNA of SEQ ID NO:1 as a probe, a longer cDNA was obtained (SEQ ID NO:227) which contains minor nucleotide differences (less than 1%) compared to the genomic sequence shown in SEQ ID NO:141.

### B. Preparation of cDNA Molecules Encoding Other Breast Tumor-Specific Polypeptides

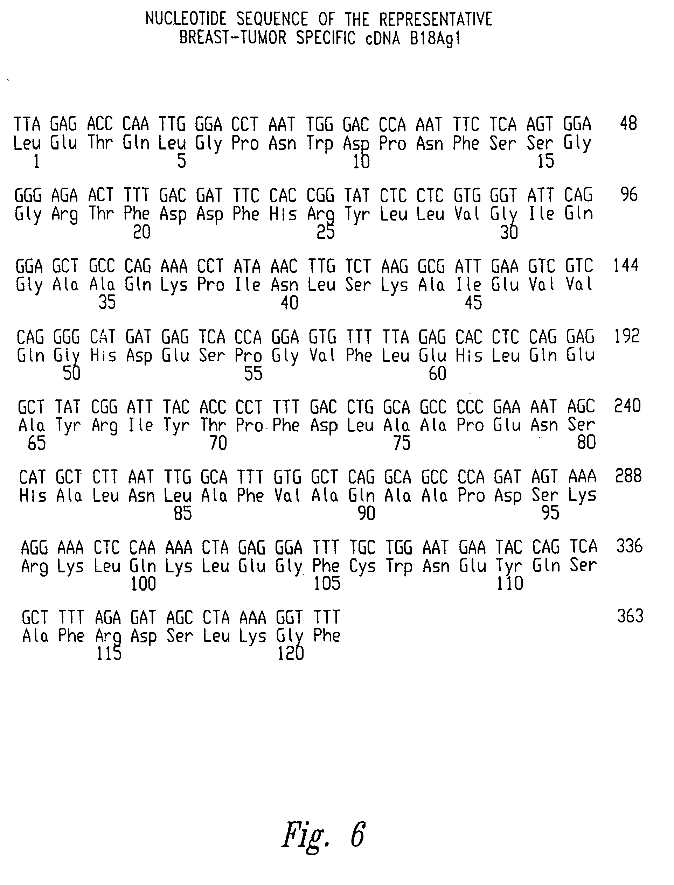

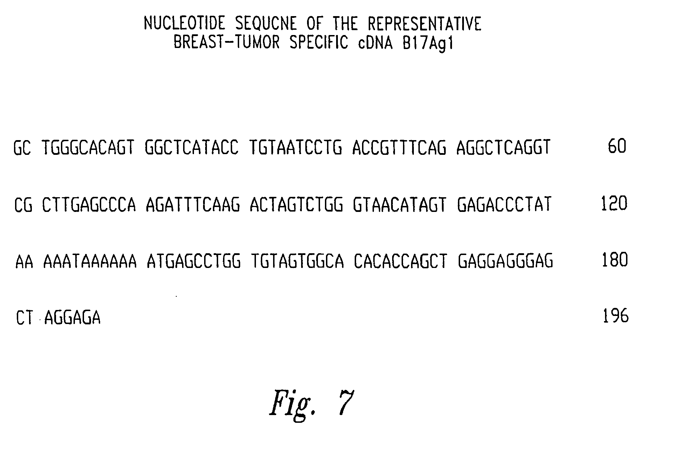

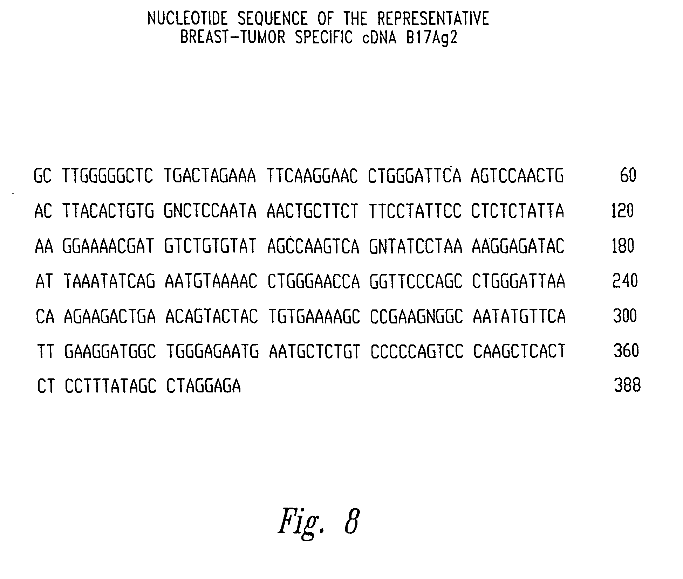

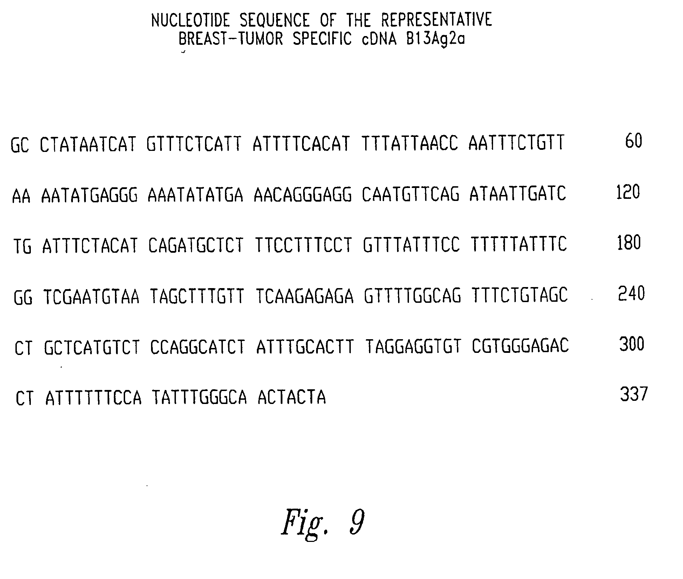

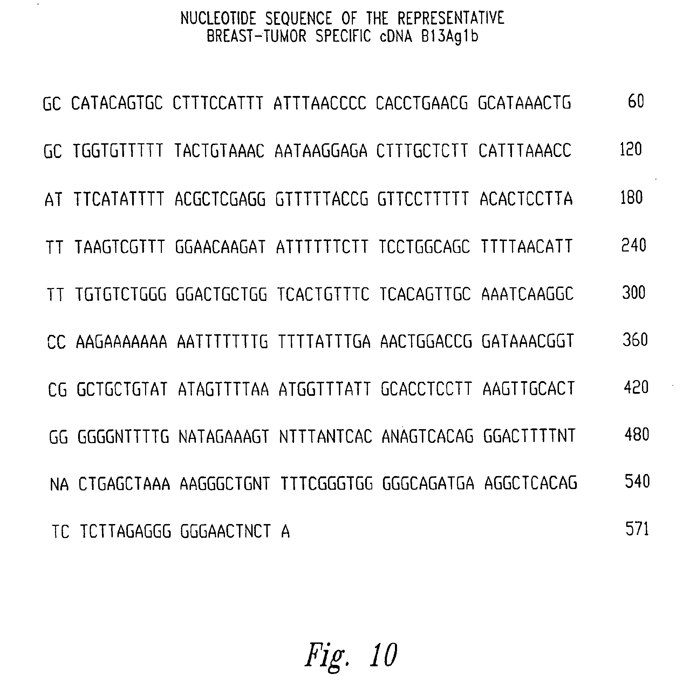

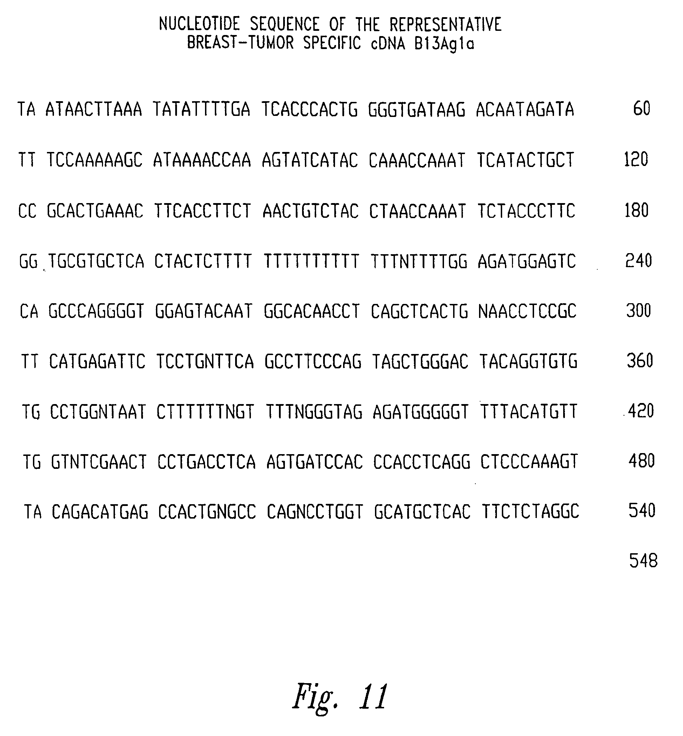

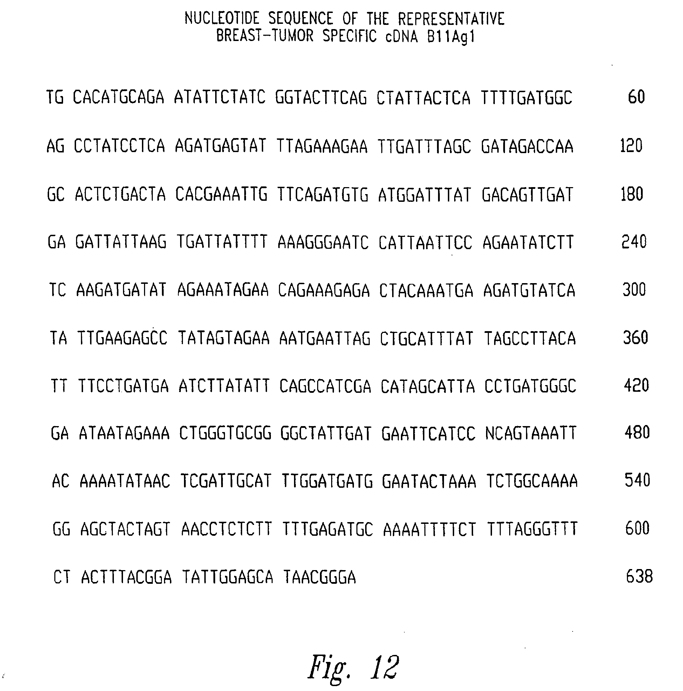

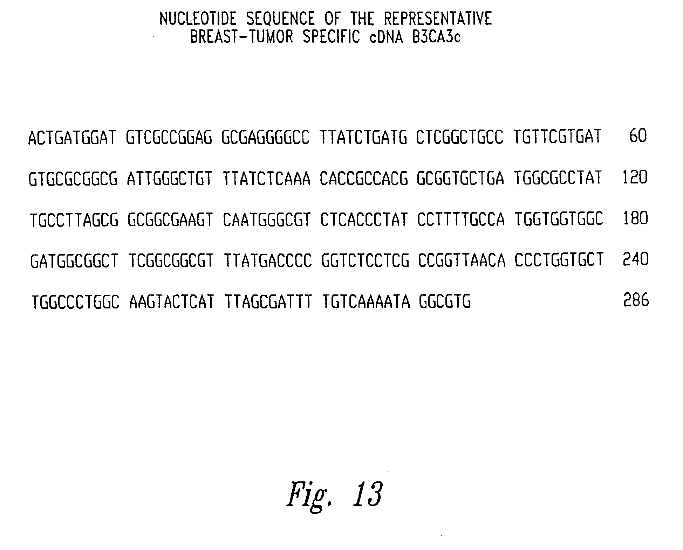

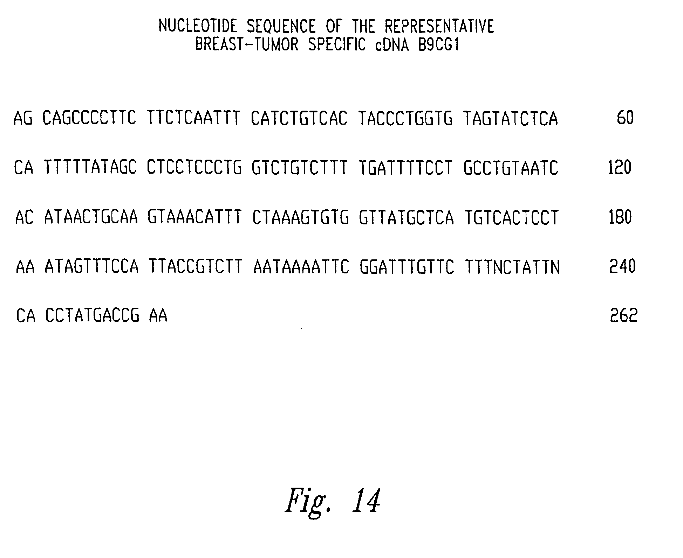

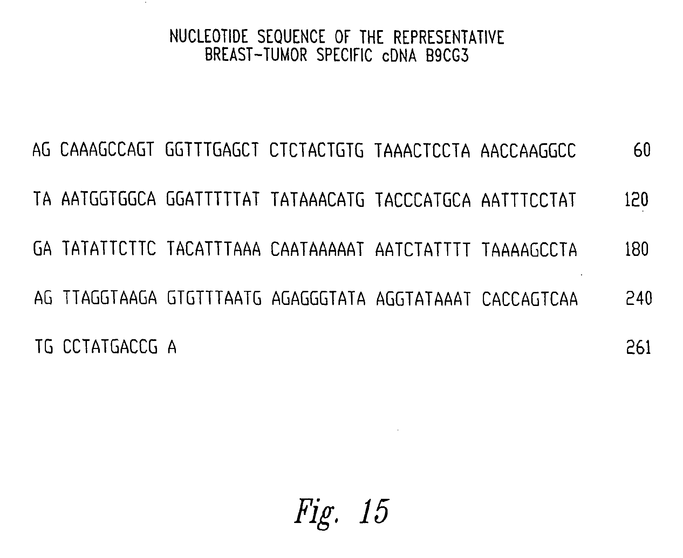

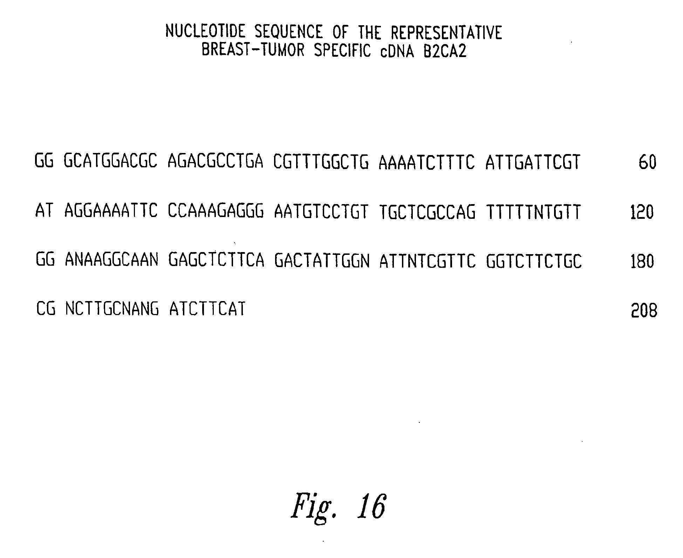

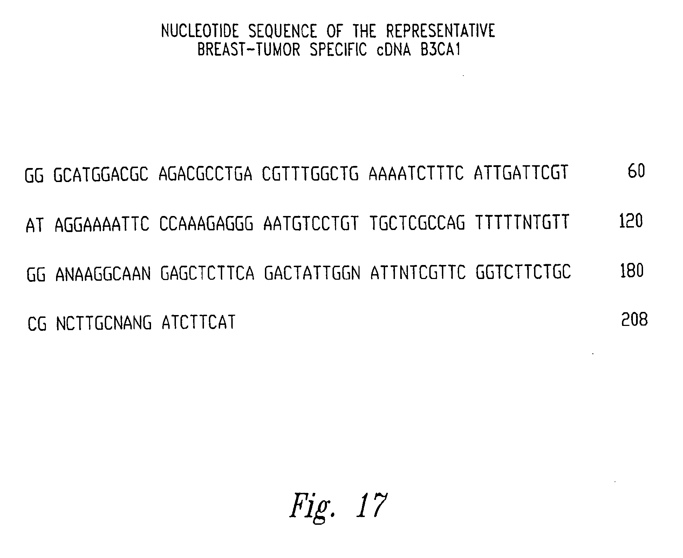

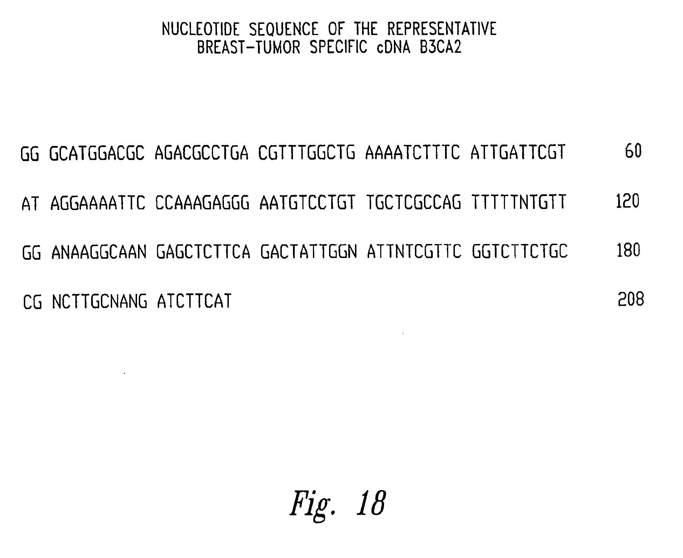

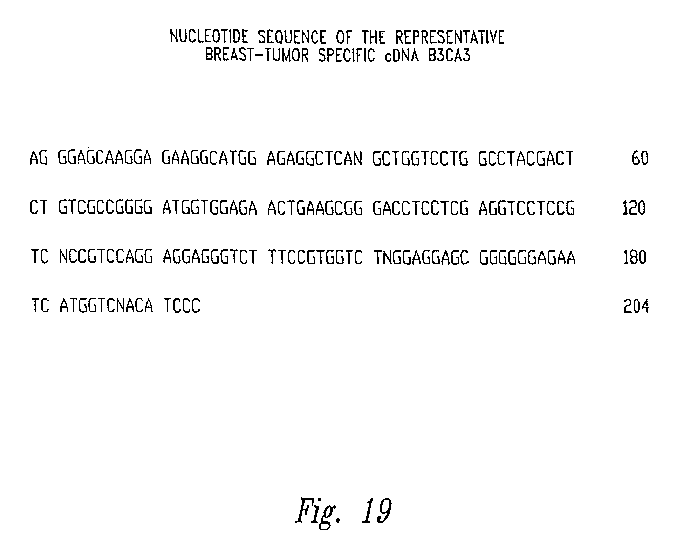

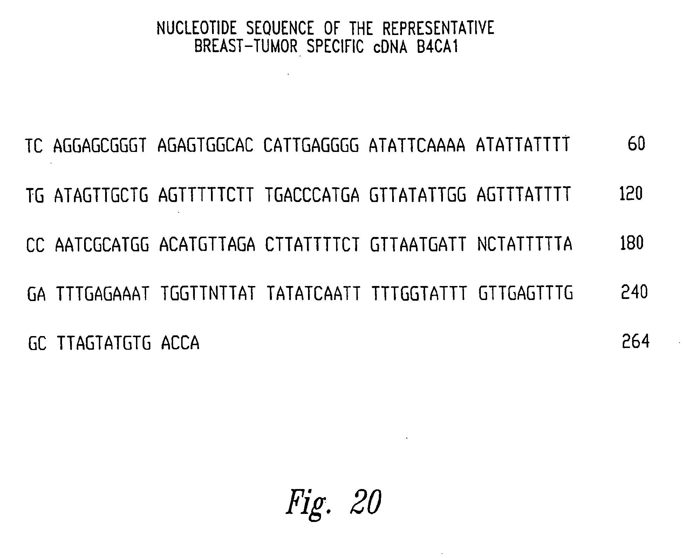

Normal RNA and tumor RNA was prepared and mRNA was isolated and converted into cDNA using a (dT)₁₂AG anchored 3' primer, as described above. Differential display PCR was then executed using the randomly chosen primers SEQ ID NO.: 87-125. Amplification conditions were as noted above, and bands observed to be specific to the RNA fingerprint pattern of the tumor were cut out of a silver stained gel, subcloned into either the T-vector (Novagen, Madison, WI) or the pCRII vector (Invitrogen, San Diego, CA) and sequenced. The sequences are provided in SEQ ID NO:11 - SEQ ID NO:86. Of the 79 sequences isolated, 67 were found to be novel (SEQ ID NO.:11-26 and 28-77) (*see also* Figures 6-20).

An extended DNA sequence (SEQ ID NO: 290) for the antigen B15Ag1 (originally identified partial sequence provided in SEQ ID NO: 27) was obtained in further studies. Comparison of the sequence of SEQ ID NO: 290 with those in the gene bank as described above, revealed homology to the known human β-A activin gene.

Subsequent studies identified an additional 146 sequences (SEQ ID NOS:142-289), of which 115 appeared to be novel (SEQ ID NOS:142, 143, 146-152, 154-166, 168-176, 178-192, 194-198, 200-204, 206, 207, 209-214, 216, 218, 219, 221-240, 243-245, 247, 250, 251, 253, 255, 257-266, 268, 269, 271-273, 275, 276, 278, 280, 281, 284, 288 and 291). To the best of the inventors' knowledge none of the previously identified sequences have heretofore been shown to be expressed at a greater level in human breast tumor tissue than in normal breast tissue.

In further studies, six different splice forms of the antigen B11Ag1 were isolated, with each of the various splice forms containing slightly different versions of the B11Ag1 coding frame. Splice junction sequences define individual exons which, in various patterns and arrangements, make up the various splice forms. Primers were designed to examine the expression pattern of each of the exons using RT-PCR as described below. Each exon was found to show the same expression pattern as the original B11Ag1 clone, with expression being breast tumor, prostate and testis-specific. The determined cDNA sequences for the isolated protein coding exons are provided in SEQ ID NO: 292-297, respectively.

### EXAMPLE 2

### PREPARATION OF B18AG1 DNA FROM HUMAN GENOMIC DNA

This Example illustrates the preparation of B18Ag1 DNA by amplification from human genomic DNA.

B18Ag1 DNA may be prepared from 250 ng human genomic DNA using 20 pmol of B18Ag1 specific primers, 500 pmol dNTPS and 1 unit of *Taq* DNA polymerase (Perkin Elmer, Branchburg, NJ) using the following amplification parameters: 94°C for 30 seconds denaturing, 30 seconds 60°C to 42°C touchdown annealing in 2°C increments every two cycles and 72°C extension for 30 seconds. The last increment (a 42°C annealing temperature) should cycle 25 times. Primers were selected using computer analysis. Primers synthesized were B18Ag1-1, B18Ag1-2, B18Ag1-3, and B18Ag1-4. Primer pairs that may be used are 1+3, 1+4, 2+3, and 2+4.

Following gel electrophoresis, the band corresponding to B18Ag1 DNA may be excised and cloned into a suitable vector.

### EXAMPLE 3

### PREPARATION OF B 18AG 1 DNA FROM BREAST TUMOR CDNA

This Example illustrates the preparation of B18Ag1 DNA by amplification from human breast tumor cDNA.

First strand cDNA is synthesized from RNA prepared from human breast tumor tissue in a reaction mixture containing 500 ng poly A+ RNA, 200 pmol of the primer (T)₁₂AG (i.e., TTT TTT TTT TTT AG) (SEQ ID NO: 130), 1X first strand reverse transcriptase buffer, 6.7 mM DTT, 500 mmol dNTPs, and 1 unit AMV or MMLV reverse transcriptase (from any supplier, such as Gibco-BRL (Grand Island, NY)) in a final volume of 30 µl. After first strand synthesis, the cDNA is diluted approximately 25 fold and 1 µl is used for amplification as described in Example 2. While some primer pairs can result in a heterogeneous population of transcripts, the primers B18Ag1-2 (5'ATG GCT ATT TTC GGG GGC TGA CA) (SEQ ID NO: 126) and B18Ag1-3 (5'CCG GTA TCT CCT CGT GGG TAT T) (SEQ ID NO: 127) yield a single 151 bp amplification product.

### EXAMPLE 4

### IDENTIFICATION OF B-CELL AND T-CELL EPITOPES OF B18AG1

This Example illustrates the identification of B18Ag1 epitopes.

The B18Ag1 sequence can be screened using a variety of computer algorithms. To determine B-cell epitopes, the sequence can be screened for hydrophobicity and hydrophilicity values using the method of Hopp, Prog. Clin. Biol. Res. 172B:367-77 (1985) or, alternatively, Cease et al., J. Exp. Med. 164:1779-84 (1986) or Spouge et al., J. Immunol. 138:204-12 (1987). Additional Class II MHC (antibody or B-cell) epitopes can be predicted using programs such as AMPHI (*e*.*g*., Margalit et al., J. Immunol. 138:2213 (1987)) or the methods of Rothbard and Taylor (e.g., EMBO J. 7:93 (1988)).

Once peptides (15-20 amino acids long) are identified using these techniques, individual peptides can be synthesized using automated peptide synthesis equipment (available from manufacturers such as Perkin Elmer/Applied Biosystems Division, Foster City, CA) and techniques such as Merrifield synthesis. Following synthesis, the peptides can used to screen sera harvested from either normal or breast cancer patients to determine whether patients with breast cancer possess antibodies reactive with the peptides. Presence of such antibodies in breast cancer patient would confirm the immunogenicity of the specific B-cell epitope in question. The peptides can also be tested for their ability to generate a serologic or humoral immune in animals (mice, rats, rabbits, chimps etc.) following immunization *in vivo.* Generation of a peptide-specific antiserum following such immunization further confirms the immunogenicity of the specific B-cell epitope in question.

To identify T-cell epitopes, the B18Ag1 sequence can be screened using different computer algorithms which are useful in identifying 8-10 amino acid motifs within the B18Ag1 sequence which are capable of binding to HLA Class I MHC molecules. (*see, e.g.,* Rammensee et al., Immunogenetics 41:178-228 (1995)). Following synthesis such peptides can be tested for their ability to bind to class I MHC using standard binding assays (*e*.*g*., Sette et al., J. Immunol. 153:5586-92 (1994)) and more importantly can be tested for their ability to generate antigen reactive cytotoxic T-cells following *in vitro* stimulation of patient or normal peripheral mononuclear cells using, for example, the methods of Bakker et al., Cancer Res. 55:5330-34 (1995); Visseren et al., J. Immunol. 154:3991-98 (1995); Kawakami et al., J. Immunol. 154:3961-68 (1995); and Kast et al., J. Immunol. 152:3904-12 (1994). Successful *in vitro* generation of T-cells capable of killing autologous (bearing the same Class I MHC molecules) tumor cells following *in vitro* peptide stimulation further confirms the immunogenicity of the B18Ag1 antigen. Furthermore, such peptides may be used to generate murine peptide and B18Ag1 reactive cytotoxic T-cells following *in vivo* immunization in mice rendered transgenic for expression of a particular human MHC Class I haplotype (Vitiello et al., J. Exp. Med. 173:1007-15 (1991).

A representative list of predicted B18Ag1 B-cell and T-cell epitopes, broken down according to predicted HLA Class I MHC binding antigen, is shown below:
Predicted Th Motifs (B-cell epitopes) (SEQ ID NOS.: 131-133)
   SSGGRTFDDFHRYLLVGI
   QGAAQKPINLSKXIEVVQGHDE
   SPGVFLEHLQEAYRIYTPFDLSA
Predicted HLA A2.1 Motifs (T-cell epitopes) (SEQ ID NOS.: 134-140)
   YLLVGIQGA
   GAAQKPINL
   NLSKXIEVV
   EVVQGHDES
   HLQEAYRIY
   NLAFVAQAA
   FVAQAAPDS

### EXAMPLE 5

### CHARACTERIZATION OF BREAST TUMOR GENES DISCOVERED BY DIFFERENTIAL DISPLAY PCR

The specificity and sensitivity of the breast tumor genes discovered by differential display PCR were determined using RT-PCR. This procedure enabled the rapid evaluation of breast tumor gene mRNA expression semiquantitatively without using large amounts of RNA. Using gene specific primers, mRNA expression levels in a variety of tissues were examined, including 8 breast tumors, 5 normal breasts, 2 prostate tumors, 2 colon tumors, 1 lung tumor, and 14 other normal adult human tissues, including normal prostate, colon, kidney, liver, lung, ovary, pancreas, skeletal muscle, skin, stomach and testes.

To ensure the semiquantitative nature of the RT-PCR, β-actin was used as internal control for each of the tissues examined. Serial dilutions of the first strand cDNAs were prepared and RT-PCR assays performed using β-actin specific primers. A dilution was then selected that enabled the linear range amplification of β-actin template, and which was sensitive enough to reflect the difference in the initial copy number. Using this condition, the β-actin levels were determined for each reverse transcription reaction from each tissue. DNA contamination was minimized by DNase treatment and by assuring a negative result when using first strand cDNA that was prepared without adding reverse transcriptase.

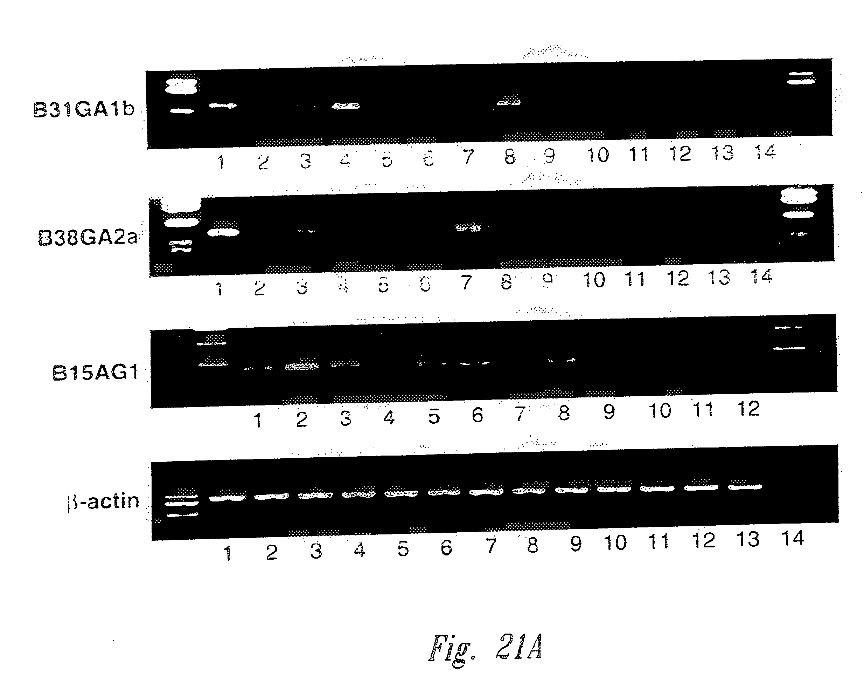

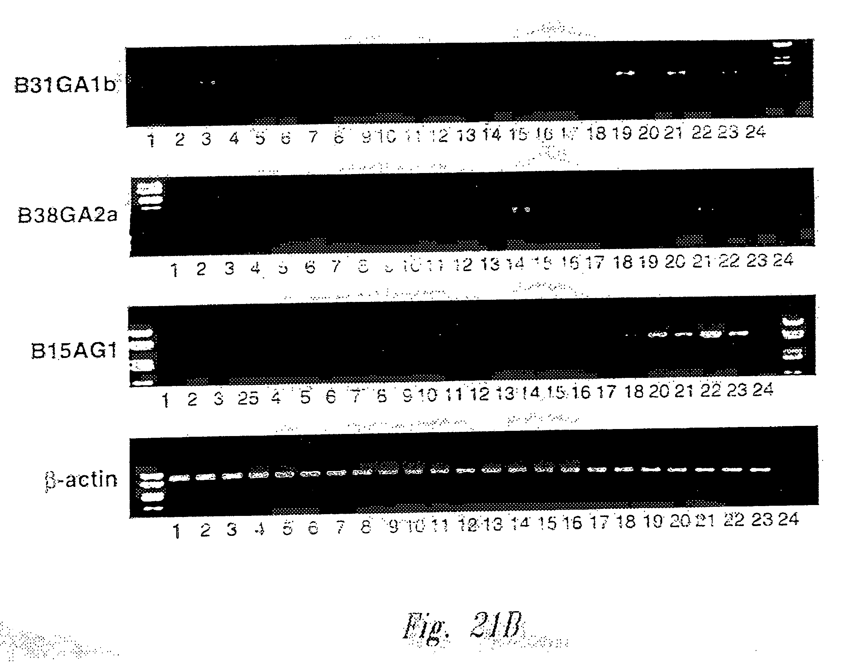

Using gene specific primers, the mRNA expression levels were determined in a variety of tissues. To date, 38 genes have been successfully examined by RT-PCR, five of which exhibit good specificity and sensitivity for breast tumors (B15AG-1, B31GAlb, B38GA2a, B11A1a and B18AG1a). Figures 21A and 21B depict the results for three of these genes: B15AG-1 (SEQ ID NO:27), B31GA1b (SEQ ID NO:148) and B38GA2a (SEQ ID NO. 157). Table I summarizes the expression level of all the genes tested in normal breast tissue and breast tumors, and also in other tissues.

**TABLE I**

| Percentage of Breast Cancer Antigens that are Expressed in Various Tissues | | |
|---|---|---|
| Breast Tissues | Over-expressed in Breast Tumors | 84% |
| | Equally Expressed in Normals and Tumor | 16% |
| Other Tissues | Over-expressed in Breast Tumors but not in any Normal Tissues | 9% |
| | Over-expressed in Breast Tumors but Expressed in Some Normal Tissues | 30% |
| | Over-expressed in Breast Tumors but Equally Expressed in All Other Tissues | 61 % |

From the foregoing, it will be appreciated that, although specific embodiments of the invention have been described herein for the purpose of illustration, various modifications may be made without deviating from the spirit and scope of the invention.

## Claims

1. An isolated DNA molecule, comprising:
(a) a nucleotide sequence of SEQ ID NO: 295;
(b) a variant of said nucleotide sequence that contains one or more nucleotide substitutions, deletions, insertions and/or modifications at no more than 20% of the nucleotide positions, such that the antigenic and/or immunogenic properties of the polypeptide encoded by the nucleotide sequence are retained; or
(c) a nucleotide sequence encoding an epitope of a polypeptide encoded by SEQ ID NO: 295.

2. An isolated DNA molecule encoding an epitope of a polypeptide, wherein said polypeptide is encoded by a nucleotide sequence that:
(a) hybridizes to the complement of a sequence of SEQ ID NO: 295, under stringent conditions; and
(b) is at least 80% identical to a sequence of SEQ ID NO: 295.

3. An isolated DNA or RNA molecule comprising a nucleotide sequence complementary to a DNA molecule according to claim 1 or 2.

4. A recombinant expression vector comprising a DNA molecule according to claim 1 or 2.

5. A host cell transformed or transfected with an expression vector according to claim 4.

6. A polypeptide comprising an amino acid sequence encoded by a DNA molecule according to claim 1 or 2.

7. A polypeptide according to claim 6 wherein said polypeptide comprises an epitope of an amino acid sequence encoded by SEQ ID NO: 295.

8. A monoclonal antibody that binds to a polypeptide according to claim 6.

9. A method for determining the presence of breast cancer in a patient comprising detecting, within a biological sample, at least one polypeptide according to claim 6, and therefrom determining the presence of breast cancer in the patient.

10. The method of claim 9 wherein the biological sample is a portion of a breast tumor.

11. The method of claim 9 wherein the step of detecting comprises contacting the biological sample with a monoclonal antibody according to claim 8.

12. A method for determining the presence of breast cancer in a patient comprising detecting, within a biological sample, an RNA molecule encoding at least one polypeptide according to claim 6, and therefrom determining the presence of breast cancer in the patient.

13. The method of claim 12 wherein the biological sample is a portion of a breast tumor.

14. The method of claim 12 wherein the step of detecting comprises:
(a) preparing cDNA from RNA molecules within the biological sample; and
(b) specifically amplifying cDNA molecules that are capable of encoding at least a portion of a polypeptide according to claim 6, and therefrom determining the presence of breast cancer in the patient.

15. A method for monitoring the progression of breast cancer in a patient, comprising:
(a) detecting an amount, in a biological sample, of at least one polypeptide according to claim 6 at a first point in time;
(b) repeating step (a) at a subsequent point in time; and
(c) comparing the amounts of polypeptide detected in steps (a) and (b), and therefrom monitoring the progression of breast cancer in the patient.

16. The method of claim 15 wherein the biological sample is a portion of a breast tumor.

17. The method of claim 15 wherein the step of detecting comprises contacting a portion of the biological sample with a monoclonal antibody according to claim 8.

18. The method of claim 15 wherein said polypeptide comprises an epitope of an amino acid sequence encoded by a nucleotide sequence of SEQ ID NO: 295.

19. A method for monitoring the progression of breast cancer in a patient, comprising:
(a) detecting an amount, within a biological sample, of at least one RNA molecule encoding a polypeptide according to claim 6 at a first point in time;
(b) repeating step (a) at a subsequent point in time; and
(c) comparing the amounts of RNA molecules detected in steps (a) and (b), and therefrom monitoring the progression of breast cancer in the patient.

20. The method of claim 19 wherein the step of detecting comprises:
(a) preparing cDNA from RNA molecules within the biological sample; and
(b) specifically amplifying cDNA molecules that are capable of encoding at least a portion of a polypeptide according to claim 6.

21. A pharmaceutical composition, comprising a polypeptide according to claim 6 and a physiologically acceptable carrier.

22. A vaccine, comprising a polypeptide according to claim 6 and an immune response enhancer.

23. A vaccine, comprising a DNA molecule according to claim 1 or 2.

24. A vaccine, comprising a recombinant expression vector comprising a DNA molecule according to claim 1 or 2.

25. A pharmaceutical composition according to claim 21, for use in the manufacture of a medicament for inhibiting the development of breast cancer in a patient.

26. A vaccine according to any one of claims 22-24, for use in the manufacture of a medicament for inhibiting the development of breast cancer in a patient.

27. A diagnostic kit comprising:
(a) one or more monoclonal antibodies according to claim 8; and
(b) a detection reagent.

28. The kit of claim 27 wherein the monoclonal antibody(s) are immobilized on a solid support.

29. A diagnostic kit comprising two polymerase chain reaction primers, at least one of the primers being specific for an RNA molecule according to claim 3.

30. The kit of claim 29, wherein at least one of the polymerase chain reaction primers comprises at least about 10 contiguous nucleotides of an RNA molecule according to claim 3.

31. A diagnostic kit comprising at least one oligonucleotide probe, the oligonucleotide probe containing at least about 15 contiguous nucleotides of a DNA molecule according to claim 3.

32. A diagnostic kit comprising at least one oligonucleotide probe specific for a DNA molecule according to claim 3.

33. The kit of claim 32, wherein the oligonucleotide probe comprises at least about 15 contiguous nucleotides of a DNA molecule according to claim 3.
